(19) Europäisches Patentamt
European Patent Office
Office européen des brevets

(11) EP 1 777 301 B1

(12) EUROPEAN PATENT SPECIFICATION

(45) Date of publication and mention of the grant of the patent: **19.12.2012 Bulletin 2012/51**

(51) Int Cl.: *C12Q 1/68* (2006.01)

(21) Application number: **06255436.5**

(22) Date of filing: **23.10.2006**

# (54) Analysis of microRNA

Analyse von microRNA

Analyse de microRNA

(84) Designated Contracting States:
**DE FR GB**

(30) Priority: **21.10.2005 US 256229**

(43) Date of publication of application:
**25.04.2007 Bulletin 2007/17**

(73) Proprietor: **Agilent Technologies, Inc.**
**Santa Clara CA 95051 (US)**

(72) Inventors:
- **Wang, Hui**
  **Loveland,**
  **Colorado 80537-0599 (US)**
- **Curry, Bo U**
  **Loveland,**
  **Colorado 80537-0599 (US)**

(74) Representative: **Grünecker, Kinkeldey, Stockmair & Schwanhäusser et al**
**Leopoldstrasse 4**
**80802 München (DE)**

(56) References cited:
- **THOMSON J.M. ET AL: "A custom microarray platform for analysis of microRNA gene expression" NATURE METHODS, vol. 1, no. 1, October 2004 (2004-10), pages 47-53, XP002417751**
- **BASKERVILLE S. AND BARTEL D.P.: "Microarray profiling of microRNAs reveals frequent coexpression with neighboring miRNAs and host genes" RNA, vol. 11, no. 3, March 2005 (2005-03), pages 241-247, XP002417752**
- **RICCELLI P V ET AL: "Hybridization of single-stranded DNA targets to immobilized complementary DNA probes: comparison of hairpin versus linear capture probes" NUCLEIC ACIDS RESEARCH, OXFORD UNIVERSITY PRESS, SURREY, GB, vol. 29, no. 4, 15 February 2001 (2001-02-15), pages 996-1004, XP002282478 ISSN: 0305-1048**
- **GOFF L A ET AL: "Rational probe optimization and enhanced detection strategy for microRNAs using microarrays" RNA BIOLOGY, LANDES BIOSCIENCE, GEORGETOWN, TX, US, vol. 2, no. 3, July 2005 (2005-07), pages 93-100, XP002400291 ISSN: 1547-6286**
- **LOY A. ET AL: "16S rRNA gene-based oligonucleotide microarray for environmental monitoring of the betaproteobacterial order 'Rhodocyclales'" APPLIED AND ENVIRONMENTAL MICROBIOLOGY, vol. 71, no. 3, March 2005 (2005-03), pages 1373-1386, XP002417753**
- **STOLC VIKTOR ET AL: "Identification of transcribed sequences in Arabidopsis thaliana by using high-resolution genome tiling arrays", PROCEEDINGS OF THE NATIONAL ACADEMY OF SCIENCES OF THE UNITED STATES (PNAS), NATIONAL ACADEMY OF SCIENCE, US, vol. 102, no. 12, 1 March 2005 (2005-03-01), pages 4453-4458, XP002422565, ISSN: 0027-8424, DOI: DOI:10.1073/PNAS.0408203102**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The invention relates generally to methods of biochemical analysis. More specifically, the invention relates to probe sets comprising at least five probes, arrays comprising such probe sets, and method of analysing a sample for microRNAs using such arrays.

Since the discovery of the biological activity of short interfering RNAs (siRNAs) over a decade ago, so called "small RNAs" (i.e., short non-coding regulatory RNAs that have a defined sequence) have become a subject of intense interest in the research community. See Novina et al., Nature 430: 161-164 (2004). Exemplary short RNAs include siRNAs, microRNAs (miRNAs), tiny non-coding RNAs (tncRNAs) and small modulatory RNAs (smRNAs), as well as many others.

Although the exact biological functions of most small RNAs remain a mystery, it is clear that they are abundant in plants and animals, with up to tens of thousands of copies per cell. For example, to date, over 78 Drosophila microRNA species and 300 human microRNA species have been identified. The levels of the individual species of small RNA, in particular microRNA species, appears to vary according to the developmental stage and type of tissue being examined. It is thought that the levels of particular small RNAs may be correlated with particular phenotypes, as well as with the levels of particular mRNAs and proteins. Further, viral microRNAs have been identified, and their presence has been linked to viral latency (see Pfeffer et al., Science, 304: 734-736 (2004)).

The sequences of several hundred miRNAs from a variety of different species, including humans, may be found at the microRNA registry (Griffiths-Jones, Nucl. Acids Res. 2004 32:D109-D111), as found at the world-wide website of the Sanger Institute (Cambridge, UK) (www.sanger.ac.uk/cgi-bin/Rfam/mimalbrowse.pl). The sequences of all of the microRNAs deposited at the microRNA registry, including more than 300 microRNA sequences from humans are disclosed in Lagos-Quintana et al, Science 294:853-858(2001); Grad et al, Mol Cell 11:1253-1263(2003); Mourelatos et al, Genes Dev 16:720-728(2002); Lagos-Quintana et al, Curr Biol 12:735-739(2002); Lagos-Quintana et al, RNA 9: 175-179(2003); Dostie et al, RNA 9:180-186(2003); Lim et al, Science 299:1540(2003); Houbaviy et al, Dev Cell 5: 351-358(2003); Michael et al, Mol Cancer Res 1:882-891(2003); Kim et al, Proc Natl Acad Sci U S A 101:360-365(2004); Suh et al, Dev Biol 270:488-498(2004); Kasashima et al, Biochem Biophys Res Commun 322:403-410(2004); and Xie et al, Nature 434:338-345(2005). MicroRNAs (miRNAs) are a class of single stranded RNAs of approximately 19-25 nt (nucleotides) in length.

Thus, analysis of of miRNA may be of great importance, for example as a research or diagnostic tool. Analytic methods employing polynucleotide arrays have been used for investigating small RNAs, e.g. miRNAs have become a subject of investigation with microarray analysis. See, e.g., Liu et al., Proc. Nat'l Acad. Sci. USA, 101: 9740-9744 (2004); Thomson et al., Nature Methods, 1:1-7 (2004); and Babak et al., RNA, 10: 1813-1819 (2004). A considerable amount of effort is currently being put into developing array platforms to facilitate the analysis of small RNAs, particularly microRNAs. Polynucleotide arrays (such as DNA or RNA arrays) typically include regions of usually different sequence polynucleotides ("capture agents") arranged in a predetermined configuration on a support. The arrays are "addressable" in that these regions (sometimes referenced as "array features") have different predetermined locations ("addresses") on the support of array. The polynucleotide arrays typically are fabricated on planar supports either by depositing previously obtained polynucleotides onto the support in a site specific fashion or by site specific in situ synthesis of the polynucleotides upon the support. After depositing the polynucleotide capture agents onto the support, the support is typically processed (e.g., washed and blocked for example) and stored prior to use.

In use, an array is contacted with a sample or labeled sample containing analytes (typically, but not necessarily, other polynucleotides) under conditions that promote specific binding of the analytes in the sample to one or more of the capture agents present on the array. Thus, the arrays, when exposed to a sample, will undergo a binding reaction with the sample and exhibit an observed binding pattern. This binding pattern can be detected upon interrogating the array. For example all target polynucleotides (for example, DNA) in the sample can be labeled with a suitable label (such as a fluorescent compound), and the label then can be accurately observed (such as by observing the fluorescence pattern) on the array after exposure of the array to the sample. Assuming that the different sequence polynucleotides were correctly deposited in accordance with the predetermined configuration, then the observed binding pattern will be indicative of the presence and/or concentration of one or more components of the sample. Techniques for scanning arrays are described, for example, in U.S. Pat. 5,763,870 and U.S. Pat. 5,945,679. Still other techniques useful for observing an array are described in U.S. Pat 5,721,435.

Thomson et al. (2004) Nature Methods 1, 1-7 disclose a probe set including 124 probes exactly complementary to 124 microRNA sequences.

Baskerville & Bartel (2005) RNA 11, 241-247 disclose probes including a six-carbon linker and a primary amine. The probes were designed to have a predicted $T_m$ of around 55°C. In order to achieve the predicted $T_m$, a probe was either truncated or extended.

Riccelli et al. (2001) Nucleic Acids Research 29, 996-1004 disclose probes including a hairpin that are able to capture relatively more target than linear probes. Overall rates of hybridisation were higher for hairpin probes than for linear probes.

Goff et al. (2005) RNA Biology 2, 93-100 disclose the hybridisation of several different probe sequences against a target miRNA in order to select probes having similar $T_m$ values. Some of the probes in the probe set differ from other probes by lacking at least one base.

There is a continuing need for new methods of analysing microRNA. The presently described invention addresses this need, and others.

The invention thus relates to novel probe sets, arrays, and methods for analysing microRNA in a sample. In certain embodiments, subject probe sets include a plurality of probes, each probe including a target-complementary sequence independently selected from the group consisting of SEQ ID NOS:1-1240.

According to a first aspect of the present invention, there is provided an array as specified in claim 1.

According to a second aspect of the present invention, there is provided a method of analysing microRNAs as specified in claim 16.

The invention finds use in a wide variety of diagnostic and research applications. Additional uses and novel features of this invention shall be set forth in part in the descriptions and examples that follow and in part will become apparent to those skilled in the art upon examination of the following specifications or may be learned by the practice of the invention. Practice of the invention may be realized and attained by means of the instruments, combinations, compositions and methods set forth in the specification and particularly pointed out in the appended claims.

Preferred embodiments of the invention will now be described by way of example only and with reference to the drawings in which

Fig. 1 schematically illustrates a probe of the invention;

Figs. 2A-2C schematically illustrate exemplary probes of the invention;

Fig. 3 schematically illustrate an embodiment of the invention; and

Figs. 4A-4C schematically illustrate exemplary methods of the invention.

To facilitate understanding, identical reference numerals have been used, where practical, to designate corresponding elements that are common to the Figures. Figure components are not drawn to scale.

Before the invention is described in detail, it is to be understood that unless otherwise indicated this invention is not limited to particular materials, reagents, reaction materials, manufacturing processes, or the like, as such may vary. It is also to be understood that the terminology used herein is for purposes of describing particular embodiments only, and is not intended to be limiting. It is also possible in the present invention that steps may be executed in different order where this is logically possible. However, the order described below is preferred.

It must be noted that, as used in the specification and the appended claims, the singular forms "a," "an" and "the" include plural referents unless the context clearly dictates otherwise. Thus, for example, reference to "an insoluble support" includes a plurality of insoluble supports. Similarly, reference to "a microRNA" includes a plurality of different identity (sequence) microRNA species.

Furthermore, where a range of values is provided, it is understood that every intervening value, between the upper and lower limit of that range and any other stated or intervening value in that stated range is encompassed within the invention. Also, it is contemplated that any optional feature of the inventive variations described may be set forth and claimed independently, or in combination with any one or more of the features described herein. It is further noted that the claims may be drafted to exclude any optional element. As such, this statement is intended to serve as antecedent basis for use of such exclusive terminology as "solely," "only," and the like in connection with the recitation of claim elements, or use of a "negative" limitation. In this specification and in the claims that follow, reference will be made to a number of terms that shall be defined to have the following meanings unless a contrary intention is apparent.

"Optional" or "optionally" means that the subsequently described circumstance may or may not occur, so that the description includes instances where the circumstance occurs and instances where it does not. For example, if a step of a process is optional, it means that the step may or may not be performed, and, thus, the description includes embodiments wherein the step is performed and embodiments wherein the step is not performed (i.e. it is omitted). If an element of an apparatus or composition is optional, the element may or may not be present, and, thus, the description includes embodiments wherein the element is present and embodiments wherein the element is not present (i.e. it is omitted).

The terms "determining", "measuring", "evaluating", "assessing" and "assaying" are used interchangeably herein to refer to any form of measurement, and include determining if an element is present or not. These terms include both quantitative and/or qualitative determinations. Assessing may be relative or absolute. "Assessing the presence of" includes determining the amount of something present, as well as determining whether it is present or absent.

The term "using" has its conventional meaning, and, as such, means employing, e.g., putting into service, a method or composition to attain an end. For example, if a program is used to create a file, a program is executed to make a file, the file usually being the output of the program. In another example, if a computer file is used, it is usually accessed, read, and the information stored in the file employed to attain an end. Similarly if a unique identifier, e.g., a barcode is used, the unique identifier is usually read to identify, for example, an object or file associated with the unique identifier.

"Moiety" and "group" are used to refer to a portion of a molecule, typically having a particular functional or structural feature, e.g. a linking group (a portion of a molecule connecting two other portions of the molecule), or an ethyl moiety (a portion of a molecule with a structure closely related to ethane). A moiety is generally bound to one or more other moieties to provide a molecular entity. As a simple example, a hydroxyl moiety bound to an ethyl moiety provides an ethanol molecule. At various points herein, the text may refer to a moiety by the name of the most closely related structure (e.g. an oligonucleotide moiety may be referenced as an oligonucleotide, a mononucleotide moiety may be referenced as a mononucleotide). However, despite this seeming informality of terminology, the appropriate meaning will be clear to those of ordinary skill in the art given the context, e.g. if the referenced term has a portion of its structure replaced with another group, then the referenced term is usually understood to be the moiety. For example, a mononucleotide moiety is a single nucleotide which has a portion of its structure (e.g. a hydrogen atom, hydroxyl group, or other group) replaced by a different moiety (e.g. a linking group, an observable label moiety, or other group). Similarly, an oligonucleotide moiety is an oligonucleotide which has a portion of its structure (e.g. a hydrogen atom, hydroxyl group, or other group) replaced by a different moiety (e.g. a linking group, an observable label moiety, or other group).

The term "nucleic acid" and "polynucleotide" are used interchangeably herein to describe a polymer of any length composed of nucleotides, e.g., deoxyribonucleotides or ribonucleotides, or compounds produced synthetically (e.g., PNA as described in U.S. Patent No. 5,948,902 and the references cited therein) which can hybridize with naturally occurring nucleic acids in a sequence specific manner analogous to that of two naturally occurring nucleic acids, e.g., can participate in Watson-Crick base pairing interactions. An "oligonucleotide" is a molecule containing from 2 to about 100 nucleotide subunits. As used herein in the context of a polynucleotide sequence, the term "bases" (or "base") is synonymous with "nucleotides" (or "nucleotide"), i.e. the monomer subunit of a polynucleotide. The terms "nucleoside" and "nucleotide" are intended to include those moieties that contain not only the known purine and pyrimidine bases, but also other heterocyclic bases that have been modified. Such modifications include methylated purines or pyrimidines, acylated purines or pyrimidines, alkylated riboses or other heterocycles. In addition, the terms "nucleoside" and "nucleotide" include those moieties that contain not only conventional ribose and deoxyribose sugars, but other sugars as well. Modified nucleosides or nucleotides also include modifications on the sugar moiety, e.g., wherein one or more of the hydroxyl groups are replaced with halogen atoms or aliphatic groups, or are functionalized as ethers, amines, or the like. "Analogues" refer to molecules having structural features that are recognized in the literature as being mimetics, derivatives, having analogous structures, or other like terms, and include, for example, polynucleotides incorporating non-natural (not usually occurring in nature) nucleotides, unnatural nucleotide mimetics such as 2'-modified nucleosides, peptide nucleic acids, oligomeric nucleoside phosphonates, and any polynucleotide that has added substituent groups, such as protecting groups or linking moieties.

"Sequence" may refer to a particular sequence of bases and/or may refer to a polynucleotide having the particular sequence of bases and/or may refer to a sub-sequence, i.e. a sequence that is part of a longer sequence. Thus a sequence may be information or may refer to a molecular entity or a portion of a molecular entity, as indicated by the context of the usage.

"Bound" may be used herein to indicate direct or indirect attachment. In the context of chemical structures, "bound" (or "bonded") may refer to the existence of a chemical bond directly joining two moieties or indirectly joining two moieties (e.g. via a linking group or any other intervening portion of the molecule). The chemical bond may be a covalent bond, an ionic bond, a coordination complex, hydrogen bonding, van der Waals interactions, or hydrophobic stacking, or may exhibit characteristics of multiple types of chemical bonds. In certain instances, "bound" includes embodiments where the attachment is direct and also embodiments where the attachment is indirect.

"Isolated" or "purified" generally refers to isolation of a substance (compound, polynucleotide, protein, polypeptide, polypeptide, chromosome, etc.) such that the substance comprises a substantial portion of the sample in which it resides (excluding solvents), i.e. greater than the substance is typically found in its natural or un-isolated state. Typically, a substantial portion of the sample comprises, e.g., greater than 1%, greater than 2%, greater than 5%, greater than 10%, greater than 20%, greater than 50%, or more, usually up to about 90%-100% of the sample (excluding solvents). For example, a sample of isolated RNA will typically comprise at least about 1% total RNA, where percent is calculated in this context as mass (e.g. in micrograms) of total RNA in the sample divided by mass (e.g. in micrograms) of the sum of (total RNA + other constituents in the sample (excluding solvent)). Techniques for purifying polynucleotides and polypeptides of interest are well known in the art and include, for example, gel electrophresis, ion-exchange chromatography, affinity chromatography, flow sorting, and sedimentation according to density. Generally, a substance is purified when it exists in a sample in an amount, relative to other components of the sample, that is not found naturally. In typical embodiments, the sample includes isolated RNA, such as isolated microRNA, prior to use in the present methods.

The term "sample" as used herein relates to a material or mixture of materials, typically, although not necessarily, in fluid form, containing one or more components of interest. The term "mixture", as used herein, refers to a combination of elements, that are interspersed and not in any particular order. A mixture is heterogeneous and not spatially separable into its different constituents. Examples of mixtures of elements include a number of different elements that are dissolved in the same aqueous solution, or a number of different elements attached to a solid support at random or in no particular order in which the different elements are not specially distinct. In other words, a mixture is not addressable. To be specific, an array of surface-bound oligonucleotides, as is commonly known in the art and described below, is not a mixture of surface-bound oligonucleotides because the species of surface-bound oligonucleotides are spatially distinct and the array is addressable.

The term "analyte" is used herein to refer to a known or unknown component of a sample. In certain embodiments of the invention, an analyte may specifically bind to a capture agent on a support surface if the analyte and the capture agent are members of a specific binding pair. In general, analytes are typically RNA or other polynucleotides. Typically, an "analyte" is referenced as a species in a mobile phase (e.g., fluid), to be detected by a "capture agent" which, in some embodiments, is bound to a support, or in other embodiments, is in solution. However, either of the "analyte" or "capture agent" may be the one which is to be evaluated by the other (thus, either one could be an unknown mixture of components of a sample, e.g., polynucleotides, to be evaluated by binding with the other). A "target" references an analyte.

The term "capture agent" refers to an agent that binds an analyte through an interaction that is sufficient to permit the agent to bind and concentrate the analyte from a homogeneous mixture of different analytes. The binding interaction may be mediated by an affinity region of the capture agent. Representative capture agents include polypeptides and polynucleotides, for example antibodies, peptides, or fragments of double stranded or single-stranded DNA or RNA may employed. Capture agents usually are characterized as being capable of exhibiting "specific binding" for one or more analytes.

The term "specific binding" refers to the ability of a capture agent to preferentially bind to a particular analyte that is present in a homogeneous mixture of different analytes. In certain embodiments, a specific binding interaction will discriminate between desirable and undesirable analytes in a sample, in some embodiments more than about 10 to 100-fold or more (e.g., more than about 1000- or 10,000-fold). In certain embodiments, the binding constant of a capture agent and analyte is greater than $10^{-6}$ $M^{-1}$, greater than $10^{7}$ $M^{-1}$, greater than $10^{8}$ $M^{-1}$, greater than $10^{9}$ $M^{-1}$, greater than $10^{10}$ $M^{-1}$, usually up to about $10^{12}$ $M^{-1}$, or even up to about $10^{15}$ $M^{-1}$.

In typical embodiments herein, a "probe" is a capture agent that is directed to a microRNA. An miRNA that a probe is directed to is referenced herein as a "target miRNA". Each probe of the probe set has a respective target miRNA. A probe set consists of its member probes. A probe/target duplex is a structure formed by hybridizing a probe to its target.

The term "pre-determined" refers to an element whose identity is known prior to its use. For example, a "pre-determined analyte" is an analyte whose identity is known prior to any binding to a capture agent. An element may be known by name, sequence, molecular weight, its function, or any other attribute or identifier. In some embodiments, the term "analyte of interest", i.e., a known analyte that is of interest, is used synonymously with the term "pre-determined analyte".

The term "stringent assay conditions" as used herein refers to conditions that are compatible to produce binding pairs of nucleic acids, e.g., probes and targets, of sufficient complementarity to provide for the desired level of specificity in the assay while being incompatible to the formation of binding pairs between binding members of insufficient complementarity to provide for the desired specificity. The term stringent assay conditions refers to the combination of hybridization and wash conditions.

A "stringent hybridization" and "stringent hybridization wash conditions" in the context of nucleic acid hybridization (e.g., as in array, Southern or Northern hybridizations) are sequence dependent, and are different under different experimental conditions. Stringent hybridization conditions that can be used to identify nucleic acids within the scope of the invention can include, e.g., hybridization in a buffer comprising 50% formamide, 5X SSC, and 1% SDS at 42°C, or hybridization in a buffer comprising 5X SSC and 1% SDS at 65°C, both with a wash of 0.1X SSC and 0.1% SDS at 37°C. Exemplary stringent hybridization conditions can also include a hybridization in a buffer of 40% formamide, 1 M NaCl, and 1% SDS at 37°C, and a wash in 1X SSC at 45°C. Alternatively, hybridization to filter-bound DNA in 0.5 M $NaHPO_4$, 7% sodium dodecyl sulfate (SDS), 1 mM EDTA at 65°C, and washing in 0.1X SSC/0.1% SDS at 68°C can be employed. Yet additional stringent hybridization conditions include hybridization at 60°C or higher and 3X SSC (450 mM sodium chloride/45 mM sodium citrate) or incubation at 42°C in a solution containing 30% formamide, 1M NaCl, 0.5% sodium sarcosine, 50 mM MES, pH 6.5. Hybridization buffers suitable for use in the methods described herein are well known in the art and may contain salt, buffer, detergent, chelating agents and other components at pre-determined concentrations.

In certain embodiments, the stringency of the wash conditions may affect the degree to which nucleic acids are specifically hybridized to complementary capture agents. Wash conditions used to identify nucleic acids may include, e.g.: a salt concentration of about 0.02 molar at pH 7 and a temperature of at least about 50°C or about 55°C to about 60°C; or, a salt concentration of about 0.15 M NaCl at 72°C for about 15 minutes; or, a salt concentration of about 0.2X SSC at a temperature of at least about 50°C or about 55°C to about 60°C for about 1 to about 20 minutes; or, multiple washes with a solution with a salt concentration of about 0.1 X SSC containing 0.1% SDS at 20 to 50°C for 1 to 15 minutes; or, equivalent conditions. Stringent conditions for washing can also be, e.g., 0.2X SSC/0.1% SDS at 42°C. In instances wherein the nucleic acid molecules are deoxyoligonucleotides (i.e., oligonucleotides), stringent conditions can include washing in 6X SSC/0.05% sodium pyrophosphate at 37°C (for 14-base oligos), 48°C (for 17-base oligos), 55°C (for 20-base oligos), and 60°C (for 23-base oligos). See, e.g., Ausubel, et al., Short Protocols in Molecular Biology, 3rd ed., Wiley & Sons (1995); Sambrook et al., Molecular Cloning: A Laboratory Manual, Third Edition, Cold Spring Harbor, N.Y. (2001); or Tijssen, Hybridization with Nucleic Acid Probes, Parts I and II (Elsevier, Amsterdam 1993), for detailed descriptions of equivalent hybridization and wash conditions and for reagents and buffers, e.g., SSC buffers and equivalent reagents and conditions.

Those of ordinary skill will readily recognize that alternative but comparable hybridization and wash conditions can be utilized to provide conditions of similar stringency. A specific example of stringent assay conditions is rotating hybridization at a temperature of about 55° C to about 70° C in a salt based hybridization buffer with a total monovalent cation concentration of 1.5M (e.g., as described in U.S. Patent Application No. 09/655,482 filed on September 5, 2000) followed by washes of 0.5X SSC and 0.1X SSC at room temperature and 37°C.

Stringent hybridization conditions may also include a "prehybridization" of aqueous phase nucleic acids with complexity-reducing nucleic acids to suppress repetitive sequences. For example, certain stringent hybridization conditions include, prior to any hybridization to surface-bound polynucleotides, hybridization with Cot-1 DNA or with random sequence synthetic oligonucleotides (e.g. 25-mers), or the like.

Stringent assay conditions are hybridization conditions that are at least as stringent as the above representative conditions, where a given set of conditions are considered to be at least as stringent if substantially no additional binding complexes that lack sufficient complementarity to provide for the desired specificity are produced in the given set of conditions as compared to the above specific conditions, where by "substantially no more" is meant less than about 5-fold more, typically less than about 3-fold more. Other stringent hybridization conditions are known in the art and may also be employed, as appropriate.

The term "array" and the equivalent term "microarray" each reference an ordered array of capture agents for binding to aqueous analytes and the like. An "array" includes any two-dimensional or substantially two-dimensional (as well as a three-dimensional) arrangement of spatially addressable regions (i.e., "features") containing capture agents, particularly polynucleotides, and the like. Any given support may carry one, two, four or more arrays disposed on a surface of a support. Depending upon the use, any or all of the arrays may be the same or different from one another and each may contain multiple spots or features. A typical array may contain one or more, including more than two, more than ten, more than one hundred, more than one thousand, more ten thousand features, or even more than one hundred thousand features, in an area of less than 100 $cm^2$, 20 $cm^2$ or even less than 10 $cm^2$, e.g., less than about 5$cm^2$, including less than about 1 $cm^2$, less than about 1 $mm^2$, e.g., 100 $\mu m^2$, or even smaller. For example, features may have widths (that is, diameter, for a round spot) in the range from a 10 $\mu$m to 1.0 cm. In other embodiments each feature may have a width in the range of 1.0 $\mu$m to 1.0 mm, usually 5.0 $\mu$m to 500 $\mu$m, and more usually 10 $\mu$m to 200 $\mu$m. Non-round features may have area ranges equivalent to that of circular features with the foregoing width (diameter) ranges. At least some, or all, of the features are of the same or different compositions (for example, when any repeats of each feature composition are excluded the remaining features may account for at least 5%, 10%, 20%, 50%, 95%, 99% or 100% of the total number of features). Inter-feature areas will typically (but not essentially) be present which do not carry any nucleic acids (or other biopolymer or chemical moiety of a type of which the features are composed). Such inter-feature areas typically will be present where the arrays are formed by processes involving drop deposition of reagents but may not be present when, for example, photolithographic array fabrication processes are used. It will be appreciated though, that the inter-feature areas, when present, could be of various sizes and configurations.

Arrays can be fabricated by depositing (e.g., by contact- or jet-based methods) either precursor units (such as nucleotide or amino acid monomers) or pre-synthesized capture agent. An array is "addressable" when it has multiple regions of different moieties (e.g., different capture agents) such that a region (i.e., a "feature" or "spot" of the array) at a particular predetermined location (i.e., an "address") on the array will detect a particular target. An "array layout" refers to one or more characteristics of the features, such as feature positioning on the support, one or more feature dimensions, and an indication of a moiety at a given location. "Interrogating" the array refers to obtaining information from the array, especially information about analytes binding to the array. "Hybridization assay" references a process of contacting an array with a mobile phase containing analyte. An "array support" refers to an article that supports an addressable collection of capture agents.

"Linker" references an oligonucleotide moiety that is part of a probe, wherein the linker is bound to the target-complementary sequence. In embodiments in which the probe is bound to a surface of an array support, the target-complementary sequence is bound to the array support via the linker sequence. The linker sequence may be any sequence that does not substantially interfere with hybridization of targets to probes, e.g. the sequence of the probe should be selected to not be complementary to any analytes expected to be assayed. An example used herein is a $(T)_{10}$ linker (ten contiguous Ts), wherein one end of the $(T)_{10}$ linker is bound to the target-complementary sequence, and the probe is bound to the array sequence via the other end of the $(T)_{10}$ linker.

In certain embodiments, a probe includes a Tm enhancement domain that increases the stability of the probe/target duplex. Such "Tm enhancement domains" are described in US 2007/0003940, filed by Wang on July 1, 2005, and entitled "Nucleic Acid Probes for Analysis of Small RNAs and Other Polynucleotides". The Tm enhancement domain may contain a nucleotide clamp and/or a hairpin structure, for example. Briefly, a nucleotide clamp contains a contiguous sequence of up to about 5 nucleotides (i.e., 1, 2, 3, 4 or 5 nucleotides). The identity of the nucleotides employed in the nucleotide clamp may be the same as each other or different to each other. In particular embodiments, the addition of the nucleotide clamp increases the stability of the probe/target duplex, as compared to a probe/target duplex formed in the absence of the clamp. Briefly, a hairpin structure has a loop of at least 3 or 4 nucleotides and a double-stranded stem in which complementary nucleotides bind to each other in an anti-parallel manner. In a duplex formed between a probe containing a hairpin structure and a target, the hairpin region promotes a phenomenon termed stacking (which phenomenon may also be called coaxial stacking) which allows the target to bind more tightly, i.e., more stably, to the probe, as compared to a probe/target duplex formed in the absence of the hairpin structure.

"Complementary" references a property of specific binding between polynucleotides based on the sequences of the polynucleotides. As used herein, a first polynucleotide and a second polynucleotide are complementary if they bind to each other in a hybridization assay under stringent conditions, e.g. if they produce a given or detectable level of signal in a hybridization assay. Portions of polynucleotides are complementary to each other if they follow conventional base-pairing rules, e.g. A pairs with T (or U) and G pairs with C, although small regions (e.g. less than about 5 bases) of mismatch, insertion, or deleted sequence may be present. In this regard, "strictly complementary" is a term used to characterize a first polynucleotide and a second polynucleotide, such as a target and a probe directed to the target, and means that every base in a sequence (or sub-sequence) of contiguous bases in the first polynucleotide has a corresponding complementary base in a corresponding sequence (or sub-sequence) of contiguous bases in the second polynucleotide. "Strictly complementary" means that there are no insertions, deletions, or substitutions in either of the first and second polynucleotides with respect to the other polynucleotide (over the complementary region). Put another way, every base of the complementary region may be paired with its complementary base, i.e. following normal base-pairing rules. The region that is complementary (the complementary region) between a first polynucleotide and a second polynucleotide (e.g. a target analyte and a capture agent) is typically at least about 10 bases long, more typically at least about 12 bases long, still more typically at least about 15 bases long, or at least about 17 bases long. In various typical embodiments, the region that is complementary between a first polynucleotide and a second polynucleotide (e.g. target and a capture agent) may be up to about 30 bases long, or longer, or up to about 27 bases long, up to about 25 bases long, or up to about 23 bases long.

A "target-complementary sequence" references a polynucleotide sequence that is complementary to a target. In the context of a probe which comprises a target-complementary sequence, wherein the probe is directed to a target miRNA, the target-complementary sequence generally is a contiguous nucleotide sequence that is complementary to the nucleotide sequence of the target miRNA and is of a length that is sufficient to provide specific binding between the probe and the target miRNA. In particular embodiments, the target-complementary sequence is complementary to at least 10 bases, at least 12 bases, at least 15 bases, at least 17 bases of the target miRNA, and may be complementary to up to the full length of the target miRNA.

As used herein, "fully-complementary" is a term used to characterize a probe; a "fully-complementary" probe comprises a target-complementary sequence such that the target-complementary sequence is strictly complementary to the full sequence of the target miRNA to which the probe is directed. Therefore, in a probe that is fully-complementary to its target miRNA, every base of the complete target miRNA sequence has a corresponding complementary base in the target-complementary sequence, such that the target-complementary sequence may be aligned base-for-base along the full sequence of a target miRNA to highlight the complementarity. And, under conditions sufficient to allow for hybridization to occur, a fully-complementary probe may hybridize to its target miRNA such that the entire target sequence can be hybridized to the target-complementary sequence, base to base, following normal hybridization rules (e.g. A base pairs with T (or U), and G base pairs with C). The probe that is fully-complementary to its target miRNA may have additional sequences, e.g. at the 5'- and/or 3'-end of the target-complementary sequence, i.e. the fully complementary sequence may be a portion of a longer sequence.

As used herein, "not fully-complementary" is a term used to characterize a probe; a probe that is "not fully-complementary" lacks a sequence of contiguous bases that is strictly complementary to the full sequence of the target miRNA to which the probe is directed. It should be noted that, under conditions sufficient to allow for hybridization to occur, a not fully-complementary probe may hybridize to its target miRNA such that the target-complementary sequence of the probe can be hybridized to a portion of the target miRNA sequence, base to base, following normal hybridization rules (e.g. A base pairs with T (or U), and G base pairs with C). In such a case, the target-complementary sequence that is complementary to the portion of the target miRNA sequence is typically at least one, more typically at least two, still more typically at least 3, at least 4, at least 5 bases shorter than the full sequence of the target miRNA, and may be up to about 10 bases, or possibly up to about 15 bases, shorter than the full sequence of the target miRNA.

If a polynucleotide, e.g. a probe, is "directed to" a target, the polynucleotide has a sequence that is complementary to a sequence in that target and will specifically bind (i.e. hybridize) to that target under hybridization conditions. The hybridization conditions typically are selected to produce binding pairs of nucleic acids, e.g., probes and targets, of sufficient complementarity to provide for the desired level of specificity in the assay while being incompatible to the formation of binding pairs between binding members of insufficient complementarity to provide for the desired specificity. Such hybridization conditions are typically known in the art. Examples of such appropriate hybridization conditions are also disclosed herein for hybridization of a sample to a microarray. The target will typically be an miRNA for embodiments discussed herein.

Accordingly, the invention provides novel probe sets, arrays, and methods for analyzing microRNA in a sample. In certain embodiments, subject probe sets include a plurality of probes, each probe including a target-complementary sequence independently selected from the group consisting of SEQ ID NOS:1-1240. In some embodiments, an array comprising a subject probe set is provided. In particular embodiments of a method for analyzing microRNA in a sample, the sample is contacted with an array comprising a probe set that includes at least five probes. Each of the at least five probes includes a target-complementary sequence independently selected from the group consisting of SEQ ID NOS: 1- 1240. The array is then interrogated to obtain information about miRNAs in the sample.

In further describing the present invention, subject probes and probe sets for detecting target miRNAs will be described first, followed by arrays that include such probes. This is followed by a description of how the subject probes may be used to assess polynucleotides (e.g. microRNAs) in a sample. Finally, representative kits for use in practicing the subject methods will be discussed.

PROBES

As mentioned above and with reference to Fig. 1, the invention provides a probe 102 for detecting a microRNA. The probe typically includes a first region 104 (i.e., a "target-complementary sequence") directed to a target miRNA. In particular embodiments the probe further includes an optional Tm enhancement domain 106. In certain embodiments, the probe 102 further includes a linker 110, and the probe is attached to a solid support 108. As will be described in greater detail below, the solid support may be part of an array, and the array may contain a plurality of different probes for detecting a plurality of target miRNAs. The configuration shown in the figure is typical, with the Tm enhancement domain 106 being attached immediately adjacent to the target-complementary sequence 104, which is attached immediately adjacent to the linker 110, which is attached to solid support 108.

As mentioned above, the probes of the invention (subject probes) may be used to detect microRNAs (miRNAs). The methods and compositions described herein may be used to detect any microRNA for which a sequence has been deposited at the microRNA registry, as well as others. (See Griffiths-Jones, Nucl. Acids Res. 2004 32:D109-D111; as well as the world-wide website of the Sanger Institute, *supra*.) The microRNA registry includes the sequences which are given SEQ ID NOS: 1268-1581 herein. As will be described in greater detail below, the target miRNA to be detected generally has a 3' end or 5' end (depending on which end of the probe for detecting that target miRNA is attached to the solid support) of known sequence.

A subject probe may be in the range of about 10 to about 100 bases in length. In certain embodiments, however, a subject probe may be about 18 to about 70 bases, about 19 to about 60 bases, or about 20 to about 50 bases in length. Target-complementary sequence 104 generally contains a contiguous nucleotide sequence that is complementary to the nucleotide sequence of a corresponding miRNA and is of a length that is sufficient to provide specific binding between the probe and the corresponding miRNA. Since miRNAs are generally in the range of about 19 to about 25 nucleotides (nt) in length, target-complementary sequence 104 is generally at least about 10 nt, at least about 12 nt, or at least about 15 nt in length. In certain embodiments target-complementary sequence 104 may be as long as about 18 nt, as long as about 20 nt, as long as about 22 nt, or as long as about 25 nt in length, or longer. The probe 102, if it is attached to a solid support 108, may be attached via its 3' end or its 5' end. If the probe 102 is attached to a solid support 108 via its 3' end, the nucleotide at the 5' end of the target-complementary sequence 104 of the probe 102 generally base pairs with the 3' terminal nucleotide of a target miRNA to be detected. Conversely, if the probe 102 is attached to a solid support via its 5' end, the nucleotide at the 3' end of the target-complementary sequence 104 of the probe 102 generally base pairs with the 5' terminal nucleotide of a target miRNA to be detected. A subject probe need not be complementary to the entire length of a corresponding target miRNA to provide for assessing the miRNA in a sample, and a target miRNA to be detected need not be complementary to the entire length of a subject probe.

The target-complementary sequence 104 therefore is directed to, i.e., hybridizes to and may be used to detect, a particular target miRNA. In many embodiments, the target-complementary sequence 104 is specific for a particular target miRNA, in that it can detect a particular target miRNA, even in the presence of other RNAs, e.g., other miRNAs, further e.g. total cell RNA or isolated "small RNA" (isolated RNA smaller than about 500 bases in length, e.g. smaller than about 300 bases in length). In other words, a subject probe contains a target-complementary sequence 104 that is complementary to a particular target miRNA.

Table 1 includes a listing of sequences for some possible target-complementary sequences to be used in probes in accordance with the present invention. The table includes the SEQ ID NO: for each sequence, the base listing of the sequence, the name of the target miRNA, as well as the calculated melting temperature of a duplex between the probe and the target miRNA that the probe is directed to Note that each probe included a single base nucleotide clamp (a G) at the 5' end of the target-complementary sequences, which was accounted for in calculating the calculated melting temperature.

Table 1

| SEQ ID NO: | Sequence | Target miRNA | Tm (calc) |
|---|---|---|---|
| 1 | GCACTGATTTCGAATGGT | dme-miR-285 | 58.8 |
| 2 | GCTCCTCAAAGCTGG | dme-miR-2b | 55.4 |
| 3 | TGAGACACACTTTGCCC | dme-miR-3 | 58.8 |
| 4 | CTCACAGTATAATCCTGTGATT | dme-miR-308 | 59.4 |
| 5 | TCAGCTATGCCGACATCT | dme-miR-31a | 59.1 |
| 6 | AAAAAGAACAGCCACTGTG | dme-miR-6 | 58.7 |
| 7 | AACTATACAACCTACTACC | hsa-let-7a | 51.7 |
| 8 | AACTATACAACCTACTACCT | hsa-let-7a | 53.7 |
| 9 | AACCACACAACCTACTA | hsa-let-7b | 52.8 |
| 10 | AACCACACAACCTACTAC | hsa-let-7b | 55.4 |
| 11 | AACCATACAACCTACTAC | hsa-let-7c | 51.6 |
| 12 | AACCATACAACCTACTACC | hsa-let-7c | 55.5 |
| 13 | AACCATACAACCTACTACCT | hsa-let-7c | 57.3 |
| 14 | ACTATGCAACCTACTACCT | hsa-let-7d | 55.8 |
| 15 | ACTATACAACCTCCTACCTC | hsa-let-7e | 57.6 |
| 16 | ACTATACAACCTCCTACCTCA | hsa-let-7e | 59.9 |
| 17 | AACTATACAATCTACTACCT | hsa-let-7f | 50.8 |
| 18 | AACTATACAATCTACTACCTC | hsa-let-7f | 53.3 |
| 19 | AACTATACAATCTACTACCTCA | hsa-let-7f | 55.5 |
| 20 | ACTGTACAAACTACTACCT | hsa-let-7g | 53.6 |
| 21 | ACTGTACAAACTACTACCTC | hsa-let-7g | 56 |
| 22 | ACTGTACAAACTACTACCTCA | hsa-let-7g | 58.2 |
| 23 | ACAGCACAAACTACTACC | hsa-let-7i | 55.3 |
| 24 | ACAGCACAAACTACTACCT | hsa-let-7i | 57.2 |
| 25 | ACAGCACAAACTACTACCTC | hsa-let-7i | 59.4 |
| 26 | TACATACTTCTTTACATTCC | hsa-miR-1 | 52.1 |
| 27 | TACATACTTCTTTACATTCCA | hsa-miR-1 | 54.4 |
| 28 | CACAAGTTCGGATCTAC | hsa-miR-100 | 55.2 |
| 29 | CACAAGTTCGGATCTACG | hsa-miR-100 | 59.2 |
| 30 | CTTCAGTTATCACAGTACTGTA | hsa-miR-101 | 59.1 |
| 31 | TCATAGCCCTGTACAAT | hsa-miR-103 | 51.9 |
| 32 | TCATAGCCCTGTACAATG | hsa-miR-103 | 54.7 |
| 33 | ACAGGAGTCTGAGCA | hsa-miR-105 | 52.5 |
| 34 | ACAGGAGTCTGAGCAT | hsa-miR-105 | 53.6 |
| 35 | ACAGGAGTCTGAGCATT | hsa-miR-105 | 55.4 |
| 36 | ACAGGAGTCTGAGCATTT | hsa-miR-105 | 56.9 |
| 37 | ACAGGAGTCTGAGCATTTG | hsa-miR-105 | 59.3 |
| 38 | GCTACCTGCACTGTAAG | hsa-miR-106a | 56.3 |
| 39 | ATCTGCACTGTCAGCA | hsa-miR-106b | 54.9 |
| 40 | TGATAGCCCTGTACAATGC | hsa-miR-107 | 58.8 |
| 41 | AATGCCCCTAAAAATCC | hsa-miR-108 | 53.2 |
| 42 | AATGCCCCTAAAAATCCT | hsa-miR-108 | 55.3 |

(continued)

| SEQ ID NO: | Sequence | Target miRNA | Tm (calc) |
|---|---|---|---|
| 43 | AATGCCCCTAAAAATCCTT | hsa-miR-108 | 56.8 |
| 44 | AATGCCCCTAAAAATCCTTA | hsa-miR-108 | 57.2 |
| 45 | AATGCCCCTAAAAATCCTTAT | hsa-miR-108 | 57.8 |
| 46 | CACAAATTCGGATCTACAG | hsa-miR-10a | 57.4 |
| 47 | CACAAATTCGGATCTACAGG | hsa-miR-10a | 60.8 |
| 48 | CACAAATTCGGATCTACAGGG | hsa-miR-10a | 63.9 |
| 49 | ACAAATTCGGTTCTACAGG | hsa-miR-10b | 57.7 |
| 50 | ACAAACACCATTGTCA | hsa-miR-122a | 51.3 |
| 51 | ACAAACACCATTGTCAC | hsa-miR-122a | 54.1 |
| 52 | ACAAACACCATTGTCACA | hsa-miR-122a | 56.6 |
| 53 | ACAAACACCATTGTCACAC | hsa-miR-122a | 58.8 |
| 54 | TGGCATTCACCGC | hsa-miR-124a | 51.5 |
| 55 | TGGCATTCACCGCG | hsa-miR-124a | 56.6 |
| 56 | TGGCATTCACCGCGT | hsa-miR-124a | 59.3 |
| 57 | CACAGGTTAAAGGGTCTC | hsa-miR-125a | 58.9 |
| 58 | CACAGGTTAAAGGGTCTCA | hsa-miR-125a | 61.2 |
| 59 | CACAGGTTAAAGGGTCTCAG | hsa-miR-125a | 62.8 |
| 60 | TCACAAGTTAGGGTCTC | hsa-miR-125b | 54.2 |
| 61 | TCACAAGTTAGGGTCTCA | hsa-miR-125b | 56.8 |
| 62 | GCATTATTACTCACGGTAC | hsa-miR-126 | 56.3 |
| 63 | GCATTATTACTCACGGTACG | hsa-miR-126 | 60 |
| 64 | CGCGTACCAAAAGTAATAATG | hsa-miR-126* | 59.7 |
| 65 | AGCCAAGCTCAGACGG | hsa-miR-127 | 59.6 |
| 66 | AAAAGAGACCGGTTC | hsa-miR-128a | 50.8 |
| 67 | AAAAGAGACCGGTTCA | hsa-miR-128a | 53.8 |
| 68 | AAAAGAGACCGGTTCAC | hsa-miR-128a | 56.5 |
| 69 | AAAAGAGACCGGTTCACT | hsa-miR-128a | 58.3 |
| 70 | GAAAGAGACCGGTTCAC | hsa-miR-128b | 58.8 |
| 71 | GAAAGAGACCGGTTCACT | hsa-miR-128b | 60.7 |
| 72 | GAAAGAGACCGGTTCACTG | hsa-miR-128b | 62.9 |
| 73 | GCAAGCCCAGACC | hsa-miR-129 | 53.3 |
| 74 | GCAAGCCCAGACCG | hsa-miR-129 | 58.6 |
| 75 | ATGCCCTTTTAACATTGC | hsa-miR-130a | 55.1 |
| 76 | ATGCCCTTTTAACATTGCA | hsa-miR-130a | 57.5 |
| 77 | ATGCCCTTTCATCATTG | hsa-miR-130b | 52.9 |
| 78 | CGACCATGGCTGTAG | hsa-miR-132 | 55.2 |
| 79 | CGACCATGGCTGTAGA | hsa-miR-132 | 57.9 |
| 80 | ACAGCTGGTTGAAGGG | hsa-miR-133a | 57 |
| 81 | ACAGCTGGTTGAAGGGG | hsa-miR-133a | 61 |
| 82 | ACAGCTGGTTGAAGGGGA | hsa-miR-133a | 63.3 |
| 83 | TAGCTGGTTGAAGGGGA | hsa-miR-133b | 58.9 |
| 84 | TAGCTGGTTGAAGGGGAC | hsa-miR-133b | 61.2 |
| 85 | TAGCTGGTTGAAGGGGACC | hsa-miR-133b | 64.8 |
| 86 | CCCTCTGGTCAACC | hsa-miR-134 | 54.3 |
| 87 | CCCTCTGGTCAACCA | hsa-miR-134 | 57.4 |
| 88 | TCACATAGGAATAAAAAGCCA | hsa-miR-135a | 58 |
| 89 | TCACATAGGAATAAAAAGCCAT | hsa-miR-135a | 58.5 |
| 90 | TCACATAGGAATAAAAAGCCATA | hsa-miR-135a | 58.8 |
| 91 | CACATAGGAATGAAAAGC | hsa-miR-135b | 54.3 |
| 92 | CACATAGGAATGAAAAGCC | hsa-miR-135b | 57.8 |

(continued)

| SEQ ID NO: | Sequence | Target miRNA | Tm (calc) |
|---|---|---|---|
| 93 | TCCATCATCAAAACAAATGGA | hsa-miR-136 | 59.3 |
| 94 | CTACGCGTATTCTTAAG | hsa-miR-137 | 51.8 |
| 95 | CTACGCGTATTCTTAAGC | hsa-miR-137 | 56 |
| 96 | CTACGCGTATTCTTAAGCA | hsa-miR-137 | 58.2 |
| 97 | CTACGCGTATTCTTAAGCAA | hsa-miR-137 | 59.4 |
| 98 | CTACGCGTATTCTTAAGCAAT | hsa-miR-137 | 59.9 |
| 99 | GATTCACAACACCAGC | hsa-miR-138 | 54.9 |
| 100 | GATTCACAACACCAGCT | hsa-miR-138 | 57 |
| 101 | AGACACGTGCACTGTAG | hsa-miR-139 | 57.1 |
| 102 | AGACACGTGCACTGTAGA | hsa-miR-139 | 59.5 |
| 103 | CTACCATAGGGTAAAACCA | hsa-miR-140 | 57.5 |
| 104 | CTACCATAGGGTAAAACCAC | hsa-miR-140 | 59.7 |
| 105 | CCATCTTTACCAGACA | hsa-miR-141 | 52 |
| 106 | CCATCTTTACCAGACAG | hsa-miR-141 | 54.3 |
| 107 | CCATCTTTACCAGACAGT | hsa-miR-141 | 56.9 |
| 108 | CCATCTTTACCAGACAGTG | hsa-miR-141 | 59.2 |
| 109 | TCCATAAAGTAGGAAACACTAC | hsa-miR-142-3p | 58.6 |
| 110 | GTAGTGCTTTCTACTTTAT | hsa-miR-142-5p | 51.8 |
| 111 | GTAGTGCTTTCTACTTTATG | hsa-miR-142-5p | 54.3 |
| 112 | TGAGCTACAGTGCTTCATC | hsa-miR-143 | 58.7 |
| 113 | CTAGTACATCATCTATACTGTA | hsa-miR-144 | 54.5 |
| 114 | AAGGGATTCCTGGGAAAA | hsa-miR-145 | 58.6 |
| 115 | AAGGGATTCCTGGGAAAAC | hsa-miR-145 | 60.9 |
| 116 | AAGGGATTCCTGGGAAAACT | hsa-miR-145 | 62.5 |
| 117 | AACCCATGGAATTCAG | hsa-miR-146a | 51.2 |
| 118 | AACCCATGGAATTCAGT | hsa-miR-146a | 54.2 |
| 119 | AACCCATGGAATTCAGTT | hsa-miR-146a | 55.8 |
| 120 | AACCCATGGAATTCAGTTC | hsa-miR-146a | 58.2 |
| 121 | AACCCATGGAATTCAGTTCT | hsa-miR-146a | 59.9 |
| 122 | AGCCTATGGAATTCAGT | hsa-miR-146b | 52.3 |
| 123 | AGCCTATGGAATTCAGTT | hsa-miR-146b | 54.1 |
| 124 | AGCCTATGGAATTCAGTTC | hsa-miR-146b | 56.5 |
| 125 | AGCCTATGGAATTCAGTTCT | hsa-miR-146b | 58.3 |
| 126 | GCAGAAGCATTTCCAC | hsa-miR-147 | 55.1 |
| 127 | GCAGAAGCATTTCCACA | hsa-miR-147 | 57.8 |
| 128 | ACAAAGTTCTGTAGTGCAC | hsa-miR-148a | 57.4 |
| 129 | ACAAAGTTCTGTAGTGCACT | hsa-miR-148a | 59.1 |
| 130 | ACAAAGTTCTGTGATGCAC | hsa-miR-148b | 58.2 |
| 131 | ACAAAGTTCTGTGATGCACT | hsa-miR-148b | 59.8 |
| 132 | GGAGTGAAGACACG | hsa-miR-149 | 51 |
| 133 | GGAGTGAAGACACGG | hsa-miR-149 | 55.7 |
| 134 | GGAGTGAAGACACGGA | hsa-miR-149 | 58.5 |
| 135 | CACTGGTACAAGGG | hsa-miR-150 | 50.3 |
| 136 | CACTGGTACAAGGGT | hsa-miR-150 | 53.6 |
| 137 | CACTGGTACAAGGGTT | hsa-miR-150 | 55.5 |
| 138 | CACTGGTACAAGGGTTG | hsa-miR-150 | 58.1 |
| 139 | CCTCAAGGAGCTTCAG | hsa-miR-151 | 56.2 |
| 140 | CCTCAAGGAGCTTCAGT | hsa-miR-151 | 58.8 |
| 141 | CCCAAGTTCTGTCATGC | hsa-miR-152 | 58.8 |
| 142 | TCACTTTTGTGACTATGC | hsa-miR-153 | 54 |

(continued)

| SEQ ID NO: | Sequence | Target miRNA | Tm (calc) |
|---|---|---|---|
| 143 | TCACTTTTGTGACTATGCA | hsa-miR-153 | 56.4 |
| 144 | TCACTTTTGTGACTATGCAA | hsa-miR-153 | 57.7 |
| 145 | CGAAGGCAACACG | hsa-miR-154 | 52.3 |
| 146 | CGAAGGCAACACGG | hsa-miR-154 | 56.8 |
| 147 | CGAAGGCAACACGGA | hsa-miR-154 | 59.5 |
| 148 | AATAGGTCAACCGTGT | hsa-miR-154* | 52.5 |
| 149 | AATAGGTCAACCGTGTA | hsa-miR-154* | 53.2 |
| 150 | AATAGGTCAACCGTGTAT | hsa-miR-154* | 54.1 |
| 151 | AATAGGTCAACCGTGTATG | hsa-miR-154* | 56.6 |
| 152 | AATAGGTCAACCGTGTATGA | hsa-miR-154* | 58.8 |
| 153 | AATAGGTCAACCGTGTATGAT | hsa-miR-154* | 59.3 |
| 154 | CCCCTATCACGATTAG | hsa-miR-155 | 52.1 |
| 155 | CCCCTATCACGATTAGC | hsa-miR-155 | 56.8 |
| 156 | CCCCTATCACGATTAGCA | hsa-miR-155 | 59.3 |
| 157 | CCCCTATCACGATTAGCAT | hsa-miR-155 | 59.9 |
| 158 | CACAAACCATTATGTGCTG | hsa-miR-15a | 58.2 |
| 159 | CACAAACCATTATGTGCTGC | hsa-miR-15a | 61.7 |
| 160 | CACAAACCATTATGTGCTGCT | hsa-miR-15a | 63.1 |
| 161 | TGTAAACCATGATGTGC | hsa-miR-15b | 53.1 |
| 162 | TGTAAACCATGATGTGCT | hsa-miR-15b | 55.1 |
| 163 | TGTAAACCATGATGTGCTG | hsa-miR-15b | 57.5 |
| 164 | CGCCAATATTTACGTGC | hsa-miR-16 | 56.8 |
| 165 | CGCCAATATTTACGTGCT | hsa-miR-16 | 58.5 |
| 166 | ACAAGTGCCTTCACTGC | hsa-miR-17-3p | 58.6 |
| 167 | ACTACCTGCACTGTAAGC | hsa-miR-17-5p | 57.5 |
| 168 | ACTACCTGCACTGTAAGCA | hsa-miR-17-5p | 59.8 |
| 169 | ACTCACCGACAGCG | hsa-miR-181a | 54.8 |
| 170 | ACTCACCGACAGCGT | hsa-miR-181a | 57.7 |
| 171 | ACTCACCGACAGCGTT | hsa-miR-181a | 59.2 |
| 172 | CCCACCGACAGCAA | hsa-miR-181b | 57.7 |
| 173 | CCCACCGACAGCAAT | hsa-miR-181b | 58.4 |
| 174 | ACTCACCGACAGGTTGA | hsa-miR-181c | 59.6 |
| 175 | AACCCACCGACAAC | hsa-miR-181d | 52.9 |
| 176 | AACCCACCGACAACA | hsa-miR-181d | 55.9 |
| 177 | AACCCACCGACAACAA | hsa-miR-181d | 57.6 |
| 178 | AACCCACCGACAACAAT | hsa-miR-181d | 58.3 |
| 179 | TGTGAGTTCTACCATTG | hsa-miR-182 | 51.7 |
| 180 | TGTGAGTTCTACCATTGC | hsa-miR-182 | 56.1 |
| 181 | TAGTTGGCAAGTCTAGAACCA | hsa-miR-182* | n/d |
| 182 | TAGTTGGCAAGTCTAGA | hsa-miR-182* | 52 |
| 183 | TAGTTGGCAAGTCTAGAA | hsa-miR-182* | 53.8 |
| 184 | TAGTTGGCAAGTCTAGAAC | hsa-miR-182* | 56.2 |
| 185 | TAGTTGGCAAGTCTAGAACC | hsa-miR-182* | 59.7 |
| 186 | CAGTGAATTCTACCAGT | hsa-miR-183 | 52.9 |
| 187 | CAGTGAATTCTACCAGTG | hsa-miR-183 | 55.6 |
| 188 | CAGTGAATTCTACCAGTGC | hsa-miR-183 | 59.6 |
| 189 | ACCCTTATCAGTTCTCCG | hsa-miR-184 | 57.9 |
| 190 | ACCCTTATCAGTTCTCCGT | hsa-miR-184 | 60.2 |
| 191 | ACCCTTATCAGTTCTCCGTC | hsa-miR-184 | 62.3 |
| 192 | GAACTGCCTTTCTCT | hsa-miR-185 | 50.6 |

(continued)

| SEQ ID NO: | Sequence | Target miRNA | Tm (calc) |
|---|---|---|---|
| 193 | AAGCCCAAAAGGAGAATTC | hsa-miR-186 | 58.5 |
| 194 | AAGCCCAAAAGGAGAATTCT | hsa-miR-186 | 60.2 |
| 195 | AAGCCCAAAAGGAGAATTCTT | hsa-miR-186 | 61.3 |
| 196 | CGGCTGCAACAC | hsa-miR-187 | 50.3 |
| 197 | CGGCTGCAACACA | hsa-miR-187 | 53.7 |
| 198 | CGGCTGCAACACAA | hsa-miR-187 | 55.6 |
| 199 | CGGCTGCAACACAAG | hsa-miR-187 | 57.7 |
| 200 | ACCCTCCACCATGC | hsa-miR-188 | 54.1 |
| 201 | ACCCTCCACCATGCA | hsa-miR-188 | 57.2 |
| 202 | ACTGATATCAGCTCAGTAGG | hsa-miR-189 | 57.5 |
| 203 | TATCTGCACTAGATGCA | hsa-miR-18a | 51.6 |
| 204 | TATCTGCACTAGATGCAC | hsa-miR-18a | 54.3 |
| 205 | TATCTGCACTAGATGCACC | hsa-miR-18a | 57.9 |
| 206 | TATCTGCACTAGATGCACCT | hsa-miR-18a | 59.7 |
| 207 | TAACTGCACTAGATGCA | hsa-miR-18b | 52.5 |
| 208 | TAACTGCACTAGATGCAC | hsa-miR-18b | 55.1 |
| 209 | TAACTGCACTAGATGCACC | hsa-miR-18b | 58.7 |
| 210 | ACCTAATATATCAAACATATCA | hsa-miR-190 | 51.8 |
| 211 | AGCTGCTTTTGGGA | hsa-miR-191 | 50.3 |
| 212 | AGCTGCTTTTGGGAT | hsa-miR-191 | 51.5 |
| 213 | AGCTGCTTTTGGGATT | hsa-miR-191 | 53.4 |
| 214 | GGGGACGAAATCC | hsa-miR-191* | 50.1 |
| 215 | GGGGACGAAATCCA | hsa-miR-191* | 53.7 |
| 216 | GGGGACGAAATCCAA | hsa-miR-191* | 55.7 |
| 217 | GGGGACGAAATCCAAG | hsa-miR-191* | 57.9 |
| 218 | GGCTGTCAATTCATAG | hsa-miR-192 | 50 |
| 219 | GGCTGTCAATTCATAGG | hsa-miR-192 | 54.4 |
| 220 | GGCTGTCAATTCATAGGT | hsa-miR-192 | 57 |
| 221 | GGCTGTCAATTCATAGGTC | hsa-miR-192 | 59.3 |
| 222 | CTGGGACTTTGTAGG | hsa-miR-193a | 51.7 |
| 223 | CTGGGACTTTGTAGGC | hsa-miR-193a | 56.7 |
| 224 | AAAGCGGGACTTTGA | hsa-miR-193b | 52.9 |
| 225 | AAAGCGGGACTTTGAG | hsa-miR-193b | 55.1 |
| 226 | AAAGCGGGACTTTGAGG | hsa-miR-193b | 59.1 |
| 227 | TCCACATGGAGTTGC | hsa-miR-194 | 53.1 |
| 228 | TCCACATGGAGTTGCT | hsa-miR-194 | 55.3 |
| 229 | GCCAATATTTCTGTGCTG | hsa-miR-195 | 56.5 |
| 230 | CCAACAACATGAAACT | hsa-miR-196a | 51.6 |
| 231 | CCAACAACATGAAACTA | hsa-miR-196a | 52.3 |
| 232 | CCAACAACATGAAACTAC | hsa-miR-196a | 54.9 |
| 233 | CCAACAACATGAAACTACC | hsa-miR-196a | 58.4 |
| 234 | CCAACAACAGGAAAC | hsa-miR-196b | 51.9 |
| 235 | CCAACAACAGGAAACT | hsa-miR-196b | 54.1 |
| 236 | CCAACAACAGGAAACTA | hsa-miR-196b | 54.7 |
| 237 | CCAACAACAGGAAACTAC | hsa-miR-196b | 57.2 |
| 238 | GCTGGGTGGAGAA | hsa-miR-197 | 50.7 |
| 239 | GCTGGGTGGAGAAG | hsa-miR-197 | 53.5 |
| 240 | GCTGGGTGGAGAAGG | hsa-miR-197 | 58.4 |
| 241 | CCTATCTCCCCTCTG | hsa-miR-198 | 52.5 |
| 242 | CCTATCTCCCCTCTGG | hsa-miR-198 | 57.2 |

(continued)

| SEQ ID NO: | Sequence | Target miRNA | Tm (calc) |
|---|---|---|---|
| 243 | CCTATCTCCCCTCTGGA | hsa-miR-198 | 60 |
| 244 | GAACAGGTAGTCTGAACAC | hsa-miR-199a | 59.2 |
| 245 | AACCAATGTGCAGACTAC | hsa-miR-199a* | 56.1 |
| 246 | AACCAATGTGCAGACTACT | hsa-miR-199a* | 58 |
| 247 | GAACAGATAGTCTAAACACTG | hsa-miR-199b | 57.5 |
| 248 | TCAGTTTTGCATAGATTTGC | hsa-miR-19a | 56.8 |
| 249 | TCAGTTTTGCATAGATTTGCA | hsa-miR-19a | 58.9 |
| 250 | TCAGTTTTGCATGGATTTG | hsa-miR-19b | 56.5 |
| 251 | ACATCGTTACCAGACAG | hsa-miR-200a | 54.4 |
| 252 | ACATCGTTACCAGACAGT | hsa-miR-200a | 56.9 |
| 253 | TCCAGCACTGTCCG | hsa-miR-200a* | 54.4 |
| 254 | TCCAGCACTGTCCGG | hsa-miR-200a* | 58.9 |
| 255 | GTCATCATTACCAGGC | hsa-miR-200b | 53.2 |
| 256 | GTCATCATTACCAGGCA | hsa-miR-200b | 56 |
| 257 | GTCATCATTACCAGGCAG | hsa-miR-200b | 58 |
| 258 | CCATCATTACCCGG | hsa-miR-200c | 51 |
| 259 | CCATCATTACCCGGC | hsa-miR-200c | 56.3 |
| 260 | CCATCATTACCCGGCA | hsa-miR-200c | 59 |
| 261 | TTTTCCCATGCCCT | hsa-miR-202 | 50.5 |
| 262 | TTTTCCCATGCCCTA | hsa-miR-202 | 51.4 |
| 263 | TTTTCCCATGCCCTAT | hsa-miR-202 | 52.5 |
| 264 | TTTTCCCATGCCCTATA | hsa-miR-202 | 53.2 |
| 265 | TTTTCCCATGCCCTATAC | hsa-miR-202 | 55.9 |
| 266 | TTTTCCCATGCCCTATACC | hsa-miR-202 | 59.5 |
| 267 | AAAGAAGTATATGCATAGGA | hsa-miR-202* | 52.4 |
| 268 | AAAGAAGTATATGCATAGGAA | hsa-miR-202* | 53.9 |
| 269 | AAAGAAGTATATGCATAGGAAA | hsa-miR-202* | 55.3 |
| 270 | CTAGTGGTCCTAAACA | hsa-miR-203 | 51.1 |
| 271 | CTAGTGGTCCTAAACAT | hsa-miR-203 | 52.1 |
| 272 | CTAGTGGTCCTAAACATT | hsa-miR-203 | 53.9 |
| 273 | CTAGTGGTCCTAAACATTT | hsa-miR-203 | 55.5 |
| 274 | CTAGTGGTCCTAAACATTTC | hsa-miR-203 | 57.7 |
| 275 | CTAGTGGTCCTAAACATTTCA | hsa-miR-203 | 59.8 |
| 276 | AGGCATAGGATGACAAAGG | hsa-miR-204 | 58.8 |
| 277 | CAGACTCCGGTGG | hsa-miR-205 | 52.6 |
| 278 | CAGACTCCGGTGGA | hsa-miR-205 | 55.9 |
| 279 | CAGACTCCGGTGGAA | hsa-miR-205 | 57.7 |
| 280 | CAGACTCCGGTGGAAT | hsa-miR-205 | 58.5 |
| 281 | CCACACACTTCCTT | hsa-miR-206 | 50.1 |
| 282 | CCACACACTTCCTTA | hsa-miR-206 | 51 |
| 283 | CCACACACTTCCTTAC | hsa-miR-206 | 54 |
| 284 | CCACACACTTCCTTACA | hsa-miR-206 | 56.7 |
| 285 | CCACACACTTCCTTACAT | hsa-miR-206 | 57.5 |
| 286 | CCACACACTTCCTTACATT | hsa-miR-206 | 58.8 |
| 287 | ACAAGCTTTTTGCTCG | hsa-miR-208 | 53.4 |
| 288 | ACAAGCTTTTTGCTCGTC | hsa-miR-208 | 58.2 |
| 289 | ACAAGCTTTTTGCTCGTCT | hsa-miR-208 | 59.9 |
| 290 | CTACCTGCACTATAAGC | hsa-miR-20a | 52.9 |
| 291 | CTACCTGCACTATAAGCA | hsa-miR-20a | 55.6 |
| 292 | CTACCTGCACTATAAGCAC | hsa-miR-20a | 57.9 |

(continued)

| SEQ ID NO: | Sequence | Target miRNA | Tm (calc) |
|---|---|---|---|
| 293 | CTACCTGCACTATAAGCACT | hsa-miR-20a | 59.6 |
| 294 | CTACCTGCACTATGAG | hsa-miR-20b | 51.2 |
| 295 | CTACCTGCACTATGAGC | hsa-miR-20b | 56 |
| 296 | CTACCTGCACTATGAGCA | hsa-miR-20b | 58.5 |
| 297 | TCAACATCAGTCTGATAAG | hsa-miR-21 | 53.4 |
| 298 | TCAACATCAGTCTGATAAGC | hsa-miR-21 | 57.3 |
| 299 | TCAACATCAGTCTGATAAGCT | hsa-miR-21 | 59 |
| 300 | TCAGCCGCTGTCAC | hsa-miR-210 | 55 |
| 301 | AGGCGAAGGATGACAAAG | hsa-miR-211 | 59.4 |
| 302 | GGCCGTGACTGG | hsa-miR-212 | 51.5 |
| 303 | GGCCGTGACTGGA | hsa-miR-212 | 55.1 |
| 304 | GGCCGTGACTGGAG | hsa-miR-212 | 57.6 |
| 305 | GGTACAATCAACGGT | hsa-miR-213 | 51.6 |
| 306 | GGTACAATCAACGGTC | hsa-miR-213 | 54.6 |
| 307 | GGTACAATCAACGGTCG | hsa-miR-213 | 58.9 |
| 308 | CTGCCTGTCTGTGC | hsa-miR-214 | 54.6 |
| 309 | GTCTGTCAATTCATAGG | hsa-miR-215 | 51 |
| 310 | GTCTGTCAATTCATAGGT | hsa-miR-215 | 53.9 |
| 311 | GTCTGTCAATTCATAGGTC | hsa-miR-215 | 56.3 |
| 312 | GTCTGTCAATTCATAGGTCA | hsa-miR-215 | 58.7 |
| 313 | GTCTGTCAATTCATAGGTCAT | hsa-miR-215 | 59.2 |
| 314 | CACAGTTGCCAGC | hsa-miR-216 | 50.9 |
| 315 | CACAGTTGCCAGCT | hsa-miR-216 | 53.5 |
| 316 | CACAGTTGCCAGCTG | hsa-miR-216 | 56.4 |
| 317 | CACAGTTGCCAGCTGA | hsa-miR-216 | 59.1 |
| 318 | ATCCAATCAGTTCCTG | hsa-miR-217 | 50.1 |
| 319 | ATCCAATCAGTTCCTGA | hsa-miR-217 | 53.1 |
| 320 | ATCCAATCAGTTCCTGAT | hsa-miR-217 | 54 |
| 321 | ATCCAATCAGTTCCTGATG | hsa-miR-217 | 56.6 |
| 322 | ACATGGTTAGATCAAGCAC | hsa-miR-218 | 56.8 |
| 323 | ACATGGTTAGATCAAGCACA | hsa-miR-218 | 59.1 |
| 324 | AGAATTGCGTTTGGA | hsa-miR-219 | 50.3 |
| 325 | AGAATTGCGTTTGGAC | hsa-miR-219 | 53.2 |
| 326 | AGAATTGCGTTTGGACA | hsa-miR-219 | 55.8 |
| 327 | AGAATTGCGTTTGGACAA | hsa-miR-219 | 57.3 |
| 328 | AGAATTGCGTTTGGACAAT | hsa-miR-219 | 57.9 |
| 329 | AGAATTGCGTTTGGACAATC | hsa-miR-219 | 60 |
| 330 | ACAGTTCTTCAACTGGC | hsa-miR-22 | 55.8 |
| 331 | ACAGTTCTTCAACTGGCA | hsa-miR-22 | 58.3 |
| 332 | AAAGTGTCAGATACGG | hsa-miR-220 | 50.5 |
| 333 | AAAGTGTCAGATACGGT | hsa-miR-220 | 53.4 |
| 334 | AAAGTGTCAGATACGGTG | hsa-miR-220 | 56 |
| 335 | AAAGTGTCAGATACGGTGT | hsa-miR-220 | 58.3 |
| 336 | GAAACCCAGCAGACAATG | hsa-miR-221 | 59.8 |
| 337 | GAGACCCAGTAGCCAG | hsa-miR-222 | 57.8 |
| 338 | GGGGTATTTGACAAACTGA | hsa-miR-223 | 59.2 |
| 339 | GGGGTATTTGACAAACTGAC | hsa-miR-223 | 61.4 |
| 340 | GGGGTATTTGACAAACTGACA | hsa-miR-223 | 63.4 |
| 341 | TAAACGGAACCACTAGTGA | hsa-miR-224 | 58.4 |
| 342 | TAAACGGAACCACTAGTGAC | hsa-miR-224 | 60.5 |

(continued)

| SEQ ID NO: | Sequence | Target miRNA | Tm (calc) |
|---|---|---|---|
| 343 | TAAACGGAACCACTAGTGACT | hsa-miR-224 | 62 |
| 344 | GGAAATCCCTGGCAATG | hsa-miR-23a | 58.9 |
| 345 | GGTAATCCCTGGCAATG | hsa-miR-23b | 57.8 |
| 346 | CTGTTCCTGCTGAACTG | hsa-miR-24 | 58.1 |
| 347 | TCAGACCGAGACAAGTG | hsa-miR-25 | 57.6 |
| 348 | GCCTATCCTGGATTAC | hsa-miR-26a | 51.6 |
| 349 | GCCTATCCTGGATTACT | hsa-miR-26a | 53.9 |
| 350 | GCCTATCCTGGATTACTT | hsa-miR-26a | 55.6 |
| 351 | AACCTATCCTGAATTACT | hsa-miR-26b | 50.2 |
| 352 | AACCTATCCTGAATTACTT | hsa-miR-26b | 52 |
| 353 | AACCTATCCTGAATTACTTG | hsa-miR-26b | 54.5 |
| 354 | AACCTATCCTGAATTACTTGA | hsa-miR-26b | 56.7 |
| 355 | AACCTATCCTGAATTACTTGAA | hsa-miR-26b | 58 |
| 356 | GCGGAACTTAGCCAC | hsa-miR-27a | 56.3 |
| 357 | GCGGAACTTAGCCACT | hsa-miR-27a | 58.4 |
| 358 | GCAGAACTTAGCCACTG | hsa-miR-27b | 57.4 |
| 359 | GCAGAACTTAGCCACTGT | hsa-miR-27b | 59.8 |
| 360 | CTCAATAGACTGTGAGC | hsa-miR-28 | 53.8 |
| 361 | CTCAATAGACTGTGAGCT | hsa-miR-28 | 55.8 |
| 362 | CTCAATAGACTGTGAGCTC | hsa-miR-28 | 58.2 |
| 363 | ACAGGATTGAGGGGG | hsa-miR-296 | 54.7 |
| 364 | AAGCGGTTTACCATC | hsa-miR-299-3p | 50.4 |
| 365 | AAGCGGTTTACCATCC | hsa-miR-299-3p | 54.8 |
| 366 | AAGCGGTTTACCATCCC | hsa-miR-299-3p | 58.7 |
| 367 | ATGTATGTGGGACGG | hsa-miR-299-5p | 51.7 |
| 368 | ATGTATGTGGGACGGT | hsa-miR-299-5p | 54.8 |
| 369 | ATGTATGTGGGACGGTA | hsa-miR-299-5p | 55.4 |
| 370 | ATGTATGTGGGACGGTAA | hsa-miR-299-5p | 56.9 |
| 371 | ATGTATGTGGGACGGTAAA | hsa-miR-299-5p | 58.3 |
| 372 | AACCGATTTCAGATGGTG | hsa-miR-29a | 57.1 |
| 373 | AACACTGATTTCAAATGGTG | hsa-miR-29b | 57.2 |
| 374 | AACACTGATTTCAAATGGTGC | hsa-miR-29b | 60:6 |
| 375 | AACACTGATTTCAAATGGTGCT | hsa-miR-29b | 62.1 |
| 376 | ACCGATTTCAAATGGTGC | hsa-miR-29c | 58.3 |
| 377 | ACCGATTTCAAATGGTGCT | hsa-miR-29c | 60 |
| 378 | ACCGATTTCAAATGGTGCTA | hsa-miR-29c | 60.3 |
| 379 | GCTTTGACAATACTATTGC | hsa-miR-301 | 54.8 |
| 380 | GCTTTGACAATACTATTGCA | hsa-miR-301 | 57.1 |
| 381 | TCACCAAAACATGGAAGC | hsa-miR-302a | 58 |
| 382 | TCACCAAAACATGGAAGCA | hsa-miR-302a | 60.2 |
| 383 | TCACCAAAACATGGAAGCAC | hsa-miR-302a | 62.2 |
| 384 | AAAGCAAGTACATCCAC | hsa-miR-302a* | 52.6 |
| 385 | AAAGCAAGTACATCCACG | hsa-miR-302a* | 56.8 |
| 386 | AAAGCAAGTACATCCACGT | hsa-miR-302a* | 59 |
| 387 | CTACTAAAACATGGAAGCAC | hsa-miR-302b | 58.1 |
| 388 | CTACTAAAACATGGAAGCACT | hsa-miR-302b | 59.7 |
| 389 | AGAAAGCACTTCCATG | hsa-miR-302b* | 51.1 |
| 390 | AGAAAGCACTTCCATGT | hsa-miR-302b* | 54 |
| 391 | AGAAAGCACTTCCATGTT | hsa-miR-302b* | 55.6 |
| 392 | AGAAAGCACTTCCATGTTA | hsa-miR-302b* | 56.1 |

(continued)

| SEQ ID NO: | Sequence | Target miRNA | Tm (calc) |
|---|---|---|---|
| 393 | AGAAAGCACTTCCATGTTAA | hsa-miR-302b* | 57.4 |
| 394 | AGAAAGCACTTCCATGTTAAA | hsa-miR-302b* | 58.7 |
| 395 | CCACTGAAACATGGA | hsa-miR-302c | 52 |
| 396 | CCACTGAAACATGGAA | hsa-miR-302c | 53.9 |
| 397 | CCACTGAAACATGGAAG | hsa-miR-302c | 56 |
| 398 | CAGCAGGTACCCCC | hsa-miR-302c* | 57 |
| 399 | ACACTCAAACATGGAAGCA | hsa-miR-302d | 59.4 |
| 400 | GCTGCAAACATCCGA | hsa-miR-30a-3p | 55.8 |
| 401 | GCTGCAAACATCCGAC | hsa-miR-30a-3p | 58.5 |
| 402 | CTTCCAGTCGAGGAT | hsa-miR-30a-5p | 53.4 |
| 403 | CTTCCAGTCGAGGATG | hsa-miR-30a-5p | 56.4 |
| 404 | CTTCCAGTCGAGGATGT | hsa-miR-30a-5p | 59 |
| 405 | AGCTGAGTGTAGGATG | hsa-miR-30b | 51.3 |
| 406 | AGCTGAGTGTAGGATGT | hsa-miR-30b | 54.3 |
| 407 | AGCTGAGTGTAGGATGTT | hsa-miR-30b | 55.9 |
| 408 | AGCTGAGTGTAGGATGTTT | hsa-miR-30b | 57.4 |
| 409 | AGCTGAGTGTAGGATGTTTA | hsa-miR-30b | 57.8 |
| 410 | GCTGAGAGTGTAGGATG | hsa-miR-30c | 55.7 |
| 411 | GCTGAGAGTGTAGGATGT | hsa-miR-30c | 58.3 |
| 412 | CTTCCAGTCGGGGA | hsa-miR-30d | 56.3 |
| 413 | CTTCCAGTCGGGGAT | hsa-miR-30d | 57.2 |
| 414 | GCTGTAAACATCCGAC | hsa-miR-30e-3p | 54.5 |
| 415 | GCTGTAAACATCCGACT | hsa-miR-30e-3p | 56.5 |
| 416 | GCTGTAAACATCCGACTG | hsa-miR-30e-3p | 59 |
| 417 | TCCAGTCAAGGATGTTTACA | hsa-miR-30e-5p | 59.4 |
| 418 | CAGCTATGCCAGCA | hsa-miR-31 | 52.2 |
| 419 | CAGCTATGCCAGCAT | hsa-miR-31 | 53.3 |
| 420 | CAGCTATGCCAGCATC | hsa-miR-31 | 56.1 |
| 421 | CAGCTATGCCAGCATCT | hsa-miR-31 | 58.1 |
| 422 | CAGCTATGCCAGCATCTT | hsa-miR-31 | 59.5 |
| 423 | GCAACTTAGTAATGTGCAA | hsa-miR-32 | 56.5 |
| 424 | GCAACTTAGTAATGTGCAAT | hsa-miR-32 | 57.1 |
| 425 | GCAACTTAGTAATGTGCAATA | hsa-miR-32 | 57.5 |
| 426 | TTCGCCCTCTCAAC | hsa-miR-320 | 52 |
| 427 | TTCGCCCTCTCAACC | hsa-miR-320 | 56.5 |
| 428 | TTCGCCCTCTCAACCC | hsa-miR-320 | 60.7 |
| 429 | AGAGGTCGACCGT | hsa-miR-323 | 50.7 |
| 430 | AGAGGTCGACCGTG | hsa-miR-323 | 54.1 |
| 431 | AGAGGTCGACCGTGT | hsa-miR-323 | 57.1 |
| 432 | AGAGGTCGACCGTGTA | hsa-miR-323 | 57.6 |
| 433 | AGAGGTCGACCGTGTAA | hsa-miR-323 | 59.1 |
| 434 | AGAGGTCGACCGTGTAAT | hsa-miR-323 | 59.7 |
| 435 | CCAGCAGCACCTGG | hsa-miR-324-3p | 57.8 |
| 436 | CCAGCAGCACCTGGG | hsa-miR-324-3p | 62.2 |
| 437 | CCAGCAGCACCTGGGG | hsa-miR-324-3p | 66.2 |
| 438 | ACACCAATGCCCTAGG | hsa-miR-324-5p | 56 |
| 439 | ACACTTACTGGACACC | hsa-miR-325 | 53.1 |
| 440 | ACACTTACTGGACACCT | hsa-miR-325 | 55.3 |
| 441 | ACACTTACTGGACACCTA | hsa-miR-325 | 55.8 |
| 442 | ACACTTACTGGACACCTAC | hsa-miR-325 | 58.2 |

(continued)

| SEQ ID NO: | Sequence | Target miRNA | Tm (calc) |
|---|---|---|---|
| 443 | ACACTTACTGGACACCTACT | hsa-miR-325 | 59.9 |
| 444 | CTGGAGGAAGGGC | hsa-miR-326 | 52.3 |
| 445 | CTGGAGGAAGGGCC | hsa-miR-326 | 57.4 |
| 446 | ACGGAAGGGCAGAGAG | hsa-miR-328 | 59.2 |
| 447 | ACGGAAGGGCAGAGAGG | hsa-miR-328 | 63.2 |
| 448 | ACGGAAGGGCAGAGAGGG | hsa-miR-328 | 66.8 |
| 449 | AAAGAGGTTAACCAGG | hsa-miR-329 | 50.5 |
| 450 | AAAGAGGTTAACCAGGT | hsa-miR-329 | 53.5 |
| 451 | AAAGAGGTTAACCAGGTG | hsa-miR-329 | 56.2 |
| 452 | AAAGAGGTTAACCAGGTGT | hsa-miR-329 | 58.5 |
| 453 | CAATGCAACTACAATGCAC | hsa-miR-33 | 58.5 |
| 454 | TCTCTGCAGGCCG | hsa-miR-330 | 52.6 |
| 455 | TCTCTGCAGGCCGT | hsa-miR-330 | 55.8 |
| 456 | TCTCTGCAGGCCGTG | hsa-miR-330 | 58.8 |
| 457 | TTCTAGGATAGGCCCAGG | hsa-miR-331 | 59.3 |
| 458 | ACATTTTTCGTTATTGCTC | hsa-miR-335 | 54.1 |
| 459 | ACATTTTTCGTTATTGCTCT | hsa-miR-335 | 55.8 |
| 460 | ACATTTTTCGTTATTGCTCTT | hsa-miR-335 | 57.1 |
| 461 | ACATTTTTCGTTATTGCTCTTG | hsa-miR-335 | 59 |
| 462 | AAAGGCATCATATAGGAG | hsa-miR-337 | 51.2 |
| 463 | AAAGGCATCATATAGGAGC | hsa-miR-337 | 55.6 |
| 464 | AAAGGCATCATATAGGAGCT | hsa-miR-337 | 57.4 |
| 465 | AAAGGCATCATATAGGAGCTG | hsa-miR-337 | 59.6 |
| 466 | TCAACAAAATCACTGATGCTG | hsa-miR-338 | 60 |
| 467 | TCAACAAAATCACTGATGCTGG | hsa-miR-338 | 62.9 |
| 468 | TCAACAAAATCACTGATGCTGGA | hsa-miR-338 | 64.6 |
| 469 | TGAGCTCCTGGAGGAC | hsa-miR-339 | 58.5 |
| 470 | GGCTATAAAGTAACTGAGAC | hsa-miR-340 | 56.2 |
| 471 | GGCTATAAAGTAACTGAGACG | hsa-miR-340 | 59.8 |
| 472 | GACGGGTGCGATTTC | hsa-miR-342 | 57.6 |
| 473 | GACGGGTGCGATTTCT | hsa-miR-342 | 59.7 |
| 474 | GCCCTGGACTAGGAG | hsa-miR-345 | 56.4 |
| 475 | GCCCTGGACTAGGAGT | hsa-miR-345 | 59.3 |
| 476 | AGAGGCAGGCATGC | hsa-miR-346 | 53.9 |
| 477 | AGAGGCAGGCATGCG | hsa-miR-346 | 58.8 |
| 478 | AACAACCAGCTAAGACACT | hsa-miR-34a | 58.1 |
| 479 | AACAACCAGCTAAGACACTG | hsa-miR-34a | 60.3 |
| 480 | AACAACCAGCTAAGACACTGC | hsa-miR-34a | 63.7 |
| 481 | CAATCAGCTAATGACAC | hsa-miR-34b | 52.3 |
| 482 | CAATCAGCTAATGACACT | hsa-miR-34b | 54.4 |
| 483 | CAATCAGCTAATGACACTG | hsa-miR-34b | 56.8 |
| 484 | GCAATCAGCTAACTACAC | hsa-miR-34c | 55.4 |
| 485 | GCAATCAGCTAACTACACT | hsa-miR-34c | 57.2 |
| 486 | GTACCCCTGGAGATT | hsa-miR-361 | 52.2 |
| 487 | CTCACACCTAGGTTC | hsa-miR-362 | 51.3 |
| 488 | CTCACACCTAGGTTCC | hsa-miR-362 | 55.8 |
| 489 | CTCACACCTAGGTTCCA | hsa-miR-362 | 58.5 |
| 490 | CTCACACCTAGGTTCCAA | hsa-miR-362 | 59.9 |
| 491 | TTACAGATGGATACCGT | hsa-miR-363 | 52.4 |
| 492 | TTACAGATGGATACCGTG | hsa-miR-363 | 55.1 |

(continued)

| SEQ ID NO: | Sequence | Target miRNA | Tm (calc) |
|---|---|---|---|
| 493 | TTACAGATGGATACCGTGC | hsa-miR-363 | 59.1 |
| 494 | ATAAGGATTTTTAGGGGC | hsa-miR-365 | 53.2 |
| 495 | ATAAGGATTTTTAGGGGCA | hsa-miR-365 | 55.8 |
| 496 | ATAAGGATTTTTAGGGGCAT | hsa-miR-365 | 56.5 |
| 497 | ATAAGGATTTTTAGGGGCATT | hsa-miR-365 | 57.8 |
| 498 | ATAAGGATTTTTAGGGGCATTA | hsa-miR-365 | 58.2 |
| 499 | TCACCATTGCTAAAGTGC | hsa-miR-367 | 56.9 |
| 500 | TCACCATTGCTAAAGTGCA | hsa-miR-367 | 59.2 |
| 501 | AAACGTGGAATTTCCTCTATG | hsa-miR-368 | 59.5 |
| 502 | AAAGATCAACCATGTATTA | hsa-miR-369-3p | 50.3 |
| 503 | AAAGATCAACCATGTATTAT | hsa-miR-369-3p | 51.2 |
| 504 | AAAGATCAACCATGTATTATT | hsa-miR-369-3p | 52.8 |
| 505 | GCGAATATAACACGGT | hsa-miR-369-5p | 52.9 |
| 506 | GCGAATATAACACGGTC | hsa-miR-369-5p | 55.5 |
| 507 | GCGAATATAACACGGTCG | hsa-miR-369-5p | 59.4 |
| 508 | CCAGGTTCCACCCC | hsa-miR-370 | 58.4 |
| 509 | ACACTCAAAAGATGGC | hsa-miR-371 | 51.8 |
| 510 | ACACTCAAAAGATGGCG | hsa-miR-371 | 56.2 |
| 511 | ACACTCAAAAGATGGCGG | hsa-miR-371 | 59.9 |
| 512 | ACGCTCAAATGTCGC | hsa-miR-372 | 54.7 |
| 513 | ACGCTCAAATGTCGCA | hsa-miR-372 | 57.4 |
| 514 | ACGCTCAAATGTCGCAG | hsa-miR-372 | 59.2 |
| 515 | ACACCCCAAAATCGAA | hsa-miR-373 | 54.6 |
| 516 | ACACCCCAAAATCGAAG | hsa-miR-373 | 56.7 |
| 517 | GGAAAGCGCCCCC | hsa-miR-373* | 58.2 |
| 518 | CACTTATCAGGTTGTATTATAA | hsa-miR-374 | 55 |
| 519 | TCACGCGAGCCG | hsa-miR-375 | 53.3 |
| 520 | TCACGCGAGCCGA | hsa-miR-375 | 56.5 |
| 521 | TCACGCGAGCCGAA | hsa-miR-375 | 58.2 |
| 522 | ACGTGGATTTTCCTCTATG | hsa-miR-376a | 56.9 |
| 523 | ACGTGGATTTTCCTCTATGA | hsa-miR-376a | 59.1 |
| 524 | ACGTGGATTTTCCTCTATGAT | hsa-miR-376a | 59.7 |
| 525 | AACATGGATTTTCCTCT | hsa-miR-376b | 51.1 |
| 526 | AACATGGATTTTCCTCTA | hsa-miR-376b | 51.8 |
| 527 | AACATGGATTTTCCTCTAT | hsa-miR-376b | 52.8 |
| 528 | AACATGGATTTTCCTCTATG | hsa-miR-376b | 55.2 |
| 529 | AACATGGATTTTCCTCTATGA | hsa-miR-376b | 57.5 |
| 530 | AACATGGATTTTCCTCTATGAT | hsa-miR-376b | 58 |
| 531 | ACAAAAGTTGCCTTTGTG | hsa-miR-377 | 56 |
| 532 | ACAAAAGTTGCCTTTGTGT | hsa-miR-377 | 58.3 |
| 533 | ACACAGGACCTGGAGTC | hsa-miR-378 | 59.8 |
| 534 | TACGTTCCATAGTCTACC | hsa-miR-379 | 54.2 |
| 535 | TACGTTCCATAGTCTACCA | hsa-miR-379 | 56.7 |
| 536 | AAGATGTGGACCATATTA | hsa-miR-380-3p | 50.6 |
| 537 | AAGATGTGGACCATATTAC | hsa-miR-380-3p | 53.3 |
| 538 | AAGATGTGGACCATATTACA | hsa-miR-380-3p | 55.8 |
| 539 | AAGATGTGGACCATATTACAT | hsa-miR-380-3p | 56.4 |
| 540 | AAGATGTGGACCATATTACATA | hsa-miR-380-3p | 56.9 |
| 541 | GCGCATGTTCTATGG | hsa-miR-380-5p | 52.7 |
| 542 | GCGCATGTTCTATGGT | hsa-miR-380-5p | 55.6 |

(continued)

| SEQ ID NO: | Sequence | Target miRNA | Tm (calc) |
|---|---|---|---|
| 543 | GCGCATGTTCTATGGTC | hsa-miR-380-5p | 58.1 |
| 544 | ACAGAGAGCTTGCC | hsa-miR-381 | 50.3 |
| 545 | ACAGAGAGCTTGCCC | hsa-miR-381 | 55 |
| 546 | ACAGAGAGCTTGCCCT | hsa-miR-381 | 57.3 |
| 547 | ACAGAGAGCTTGCCCTT | hsa-miR-381 | 58.8 |
| 548 | CGAATCCACCACG | hsa-miR-382 | 51.3 |
| 549 | CGAATCCACCACGA | hsa-miR-382 | 54.5 |
| 550 | CGAATCCACCACGAA | hsa-miR-382 | 56.2 |
| 551 | CGAATCCACCACGAAC | hsa-miR-382 | 58.8 |
| 552 | AGCCACAATCACCTTCTG | hsa-miR-383 | 58.9 |
| 553 | AGCCACAATCACCTTCTGA | hsa-miR-383 | 61.1 |
| 554 | AGCCACAATCACCTTCTGAT | hsa-miR-383 | 61.6 |
| 555 | TATGAACAATTTCTAGGAA | hsa-miR-384 | 50.5 |
| 556 | TATGAACAATTTCTAGGAAT | hsa-miR-384 | 51.4 |
| 557 | AGGGGTTCACCGA | hsa-miR-409-3p | 51.3 |
| 558 | AGGGGTTCACCGAG | hsa-miR-409-3p | 54.1 |
| 559 | AGGGGTTCACCGAGC | hsa-miR-409-3p | 59.2 |
| 560 | TGCAAAGTTGCTCG | hsa-miR-409-5p | 50.3 |
| 561 | TGCAAAGTTGCTCGG | hsa-miR-409-5p | 54.7 |
| 562 | TGCAAAGTTGCTCGGG | hsa-miR-409-5p | 58.7 |
| 563 | AACAGGCCATCTGTG | hsa-miR-410 | 52.4 |
| 564 | AACAGGCCATCTGTGT | hsa-miR-410 | 55.3 |
| 565 | AACAGGCCATCTGTGTT | hsa-miR-410 | 57 |
| 566 | AACAGGCCATCTGTGTTA | hsa-miR-410 | 57.4 |
| 567 | AACAGGCCATCTGTGTTAT | hsa-miR-410 | 58.1 |
| 568 | AACAGGCCATCTGTGTTATA | hsa-miR-410 | 58.5 |
| 569 | AACAGGCCATCTGTGTTATAT | hsa-miR-410 | 59 |
| 570 | ACGGCTAGTGGACC | hsa-miR-412 | 53.4 |
| 571 | ACGGCTAGTGGACCA | hsa-miR-412 | 56.6 |
| 572 | ACGGCTAGTGGACCAG | hsa-miR-412 | 58.7 |
| 573 | GGCCTTCTGACCCTAAG | hsa-miR-422a | 59.7 |
| 574 | GGCCTTCTGACCCTAAGT | hsa-miR-422a | 62.1 |
| 575 | GGCCTTCTGACCCTAAGTC | hsa-miR-422a | 64.3 |
| 576 | GGCCTTCTGACTCCA | hsa-miR-422b | 56.7 |
| 577 | GGCCTTCTGACTCCAA | hsa-miR-422b | 58.4 |
| 578 | CTGAGGGGCCTC | hsa-miR-423 | 49.8 |
| 579 | CTGAGGGGCCTCA | hsa-miR-423 | 53.7 |
| 580 | CTGAGGGGCCTCAG | hsa-miR-423 | 56.4 |
| 581 | TTCAAAACATGAATTGCTGCT | hsa-miR-424 | 59.7 |
| 582 | TTCAAAACATGAATTGCTGCTG | hsa-miR-424 | 61.6 |
| 583 | GGCGGACACGAC | hsa-miR-425 | 52.9 |
| 584 | GGCGGACACGACA | hsa-miR-425 | 56.3 |
| 585 | GGCGGACACGACAT | hsa-miR-425 | 57.2 |
| 586 | GGCGGACACGACATT | hsa-miR-425 | 58.8 |
| 587 | ACGGTTTTACCAGAC | hsa-miR-429 | 50.4 |
| 588 | ACGGTTTTACCAGACA | hsa-miR-429 | 53.4 |
| 589 | ACGGTTTTACCAGACAG | hsa-miR-429 | 55.5 |
| 590 | ACGGTTTTACCAGACAGT | hsa-miR-429 | 58 |
| 591 | ACGGTTTTACCAGACAGTA | hsa-miR-429 | 58.4 |
| 592 | ACGGTTTTACCAGACAGTAT | hsa-miR-429 | 58.9 |

(continued)

| SEQ ID NO: | Sequence | Target miRNA | Tm (calc) |
|---|---|---|---|
| 593 | TGCATGACGGCCT | hsa-miR-431 | 51.9 |
| 594 | TGCATGACGGCCTG | hsa-miR-431 | 55.2 |
| 595 | TGCATGACGGCCTGC | hsa-miR-431 | 60 |
| 596 | CCACCCAATGACC | hsa-miR-432 | 50.1 |
| 597 | CCACCCAATGACCT | hsa-miR-432 | 52.9 |
| 598 | CCACCCAATGACCTA | hsa-miR-432 | 53.7 |
| 599 | CCACCCAATGACCTAC | hsa-miR-432 | 56.6 |
| 600 | CCACCCAATGACCTACT | hsa-miR-432 | 58.7 |
| 601 | AGACATGGAGGAGCC | hsa-miR-432* | 54.1 |
| 602 | AGACATGGAGGAGCCA | hsa-miR-432* | 57.2 |
| 603 | AGACATGGAGGAGCCAT | hsa-miR-432* | 57.9 |
| 604 | ACACCGAGGAGCC | hsa-miR-433 | 52.6 |
| 605 | ACACCGAGGAGCCC | hsa-miR-433 | 57.5 |
| 606 | ATGGGACATCCTACAT | hsa-miR-448 | 50.1 |
| 607 | ATGGGACATCCTACATA | hsa-miR-448 | 51 |
| 608 | ATGGGACATCCTACATAT | hsa-miR-448 | 52 |
| 609 | ATGGGACATCCTACATATG | hsa-miR-448 | 54.7 |
| 610 | ATGGGACATCCTACATATGC | hsa-miR-448 | 58.8 |
| 611 | ACCAGCTAACAATACAC | hsa-miR-449 | 51.5 |
| 612 | ACCAGCTAACAATACACT | hsa-miR-449 | 53.6 |
| 613 | ACCAGCTAACAATACACTG | hsa-miR-449 | 56.1 |
| 614 | ACCAGCTAACAATACACTGC | hsa-miR-449 | 59.9 |
| 615 | TATTAGGAACACATCGC | hsa-miR-450 | 52 |
| 616 | TATTAGGAACACATCGCA | hsa-miR-450 | 54.6 |
| 617 | TATTAGGAACACATCGCAA | hsa-miR-450 | 56.1 |
| 618 | TATTAGGAACACATCGCAAA | hsa-miR-450 | 57.4 |
| 619 | TATTAGGAACACATCGCAAAA | hsa-miR-450 | 58.6 |
| 620 | TATTAGGAACACATCGCAAAAA | hsa-miR-450 | 59.7 |
| 621 | AAACTCAGTAATGGTAAC | hsa-miR-451 | 50.5 |
| 622 | AAACTCAGTAATGGTAACG | hsa-miR-451 | 54.6 |
| 623 | AAACTCAGTAATGGTAACGG | hsa-miR-451 | 58.1 |
| 624 | GTCTCAGTTTCCTCTG | hsa-miR-452 | 52.8 |
| 625 | GTCTCAGTTTCCTCTGC | hsa-miR-452 | 57.5 |
| 626 | CTTCTTTGCAGATGAG | hsa-miR-452* | 51.1 |
| 627 | CTTCTTTGCAGATGAGA | hsa-miR-452* | 53.9 |
| 628 | CTTCTTTGCAGATGAGAC | hsa-miR-452* | 56.4 |
| 629 | CTTCTTTGCAGATGAGACT | hsa-miR-452* | 58.2 |
| 630 | CGAACTCACCACG | hsa-miR-453 | 51.2 |
| 631 | CGAACTCACCACGG | hsa-miR-453 | 55.9 |
| 632 | CGAACTCACCACGGA | hsa-miR-453 | 58.6 |
| 633 | AGAGGAGAGCCGTG | hsa-miR-485-3p | 52.8 |
| 634 | AGAGGAGAGCCGTGT | hsa-miR-485-3p | 56 |
| 635 | AGAGGAGAGCCGTGTA | hsa-miR-485-3p | 56.5 |
| 636 | AGAGGAGAGCCGTGTAT | hsa-miR-485-3p | 57.3 |
| 637 | AGAGGAGAGCCGTGTATG | hsa-miR-485-3p | 59.8 |
| 638 | GAATTCATCACGGCC | hsa-miR-485-5p | 53.9 |
| 639 | GAATTCATCACGGCCA | hsa-miR-485-5p | 56.7 |
| 640 | GAATTCATCACGGCCAG | hsa-miR-485-5p | 58.7 |
| 641 | TTGAGAGTGCCATTATC | hsa-miR-488 | 51.7 |
| 642 | TTGAGAGTGCCATTATCT | hsa-miR-488 | 53.8 |

(continued)

| SEQ ID NO: | Sequence | Target miRNA | Tm (calc) |
|---|---|---|---|
| 643 | TTGAGAGTGCCATTATCTG | hsa-miR-488 | 56.3 |
| 644 | TTGAGAGTGCCATTATCTGG | hsa-miR-488 | 59.8 |
| 645 | GCTGCCGTATATGTG | hsa-miR-489 | 51.6 |
| 646 | GCTGCCGTATATGTGA | hsa-miR-489 | 54.5 |
| 647 | GCTGCCGTATATGTGAT | hsa-miR-489 | 55.3 |
| 648 | GCTGCCGTATATGTGATG | hsa-miR-489 | 57.9 |
| 649 | CAGCATGGAGTCCT | hsa-miR-490 | 51.6 |
| 650 | CAGCATGGAGTCCTC | hsa-miR-490 | 54.8 |
| 651 | CAGCATGGAGTCCTCC | hsa-miR-490 | 59.2 |
| 652 | TCCTCATGGAAGGGT | hsa-miR-491 | 53.1 |
| 653 | TCCTCATGGAAGGGTT | hsa-miR-491 | 55 |
| 654 | TCCTCATGGAAGGGTTC | hsa-miR-491 | 57.8 |
| 655 | AAGAATCTTGTCCCGC | hsa-miR-492 | 54.8 |
| 656 | AAGAATCTTGTCCCGCA | hsa-miR-492 | 57.5 |
| 657 | AAGAATCTTGTCCCGCAG | hsa-miR-492 | 59.4 |
| 658 | AATGAAAGCCTACCATG | hsa-miR-493 | 52 |
| 659 | AATGAAAGCCTACCATGT | hsa-miR-493 | 54.7 |
| 660 | AATGAAAGCCTACCATGTA | hsa-miR-493 | 55.2 |
| 661 | AATGAAAGCCTACCATGTAC | hsa-miR-493 | 57.5 |
| 662 | AATGAAAGCCTACCATGTACA | hsa-miR-493 | 59.7 |
| 663 | AAGAGGTTTCCCGT | hsa-miR-494 | 50.1 |
| 664 | AAGAGGTTTCCCGTG | hsa-miR-494 | 53.4 |
| 665 | AAGAGGTTTCCCGTGT | hsa-miR-494 | 56.3 |
| 666 | AAGAGGTTTCCCGTGTA | hsa-miR-494 | 56.8 |
| 667 | AAGAGGTTTCCCGTGTAT | hsa-miR-494 | 57.5 |
| 668 | AAGAGGTTTCCCGTGTATG | hsa-miR-494 | 59.9 |
| 669 | AAAGAAGTGCACCATG | hsa-miR-495 | 51.9 |
| 670 | AAAGAAGTGCACCATGT | hsa-miR-495 | 54.6 |
| 671 | AAAGAAGTGCACCATGTT | hsa-miR-495 | 56.2 |
| 672 | AAAGAAGTGCACCATGTTT | hsa-miR-495 | 57.6 |
| 673 | AAAGAAGTGCACCATGTTTG | hsa-miR-495 | 59.7 |
| 674 | GAGATTGGCCATGTA | hsa-miR-496 | 50 |
| 675 | GAGATTGGCCATGTAA | hsa-miR-496 | 52.1 |
| 676 | GAGATTGGCCATGTAAT | hsa-miR-496 | 53.1 |
| 677 | ACAAACCACAGTGTG | hsa-miR-497 | 50.6 |
| 678 | ACAAACCACAGTGTGC | hsa-miR-497 | 55.5 |
| 679 | ACAAACCACAGTGTGCT | hsa-miR-497 | 57.5 |
| 680 | ACAAACCACAGTGTGCTG | hsa-miR-497 | 59.9 |
| 681 | GAAAAACGCCCCC | hsa-miR-498 | 52.5 |
| 682 | GAAAAACGCCCCCT | hsa-miR-498 | 55 |
| 683 | GAAAAACGCCCCCTG | hsa-miR-498 | 58 |
| 684 | TTAAACATCACTGCAAG | hsa-miR-499 | 50.4 |
| 685 | TTAAACATCACTGCAAGT | hsa-miR-499 | 53.1 |
| 686 | TTAAACATCACTGCAAGTC | hsa-miR-499 | 55.5 |
| 687 | TTAAACATCACTGCAAGTCT | hsa-miR-499 | 57.2 |
| 688 | TTAAACATCACTGCAAGTCTT | hsa-miR-499 | 58.4 |
| 689 | TTAAACATCACTGCAAGTCTTA | hsa-miR-499 | 58.7 |
| 690 | TTAAACATCACTGCAAGTCTTAA | hsa-miR-499 | 59.8 |
| 691 | CAGAATCCTTGCCC | hsa-miR-500 | 51.6 |
| 692 | CAGAATCCTTGCCCA | hsa-miR-500 | 54.8 |

(continued)

| SEQ ID NO: | Sequence | Target miRNA | Tm (calc) |
|---|---|---|---|
| 693 | CAGAATCCTTGCCCAG | hsa-miR-500 | 57 |
| 694 | TCTCACCCAGGGAC | hsa-miR-501 | 53.2 |
| 695 | TCTCACCCAGGGACA | hsa-miR-501 | 56.5 |
| 696 | TCTCACCCAGGGACAA | hsa-miR-501 | 58.2 |
| 697 | TCTCACCCAGGGACAAA | hsa-miR-501 | 59.7 |
| 698 | TAGCACCCAGATAGC | hsa-miR-502 | 50.1 |
| 699 | TAGCACCCAGATAGCA | hsa-miR-502 | 53.3 |
| 700 | TAGCACCCAGATAGCAA | hsa-miR-502 | 55.1 |
| 701 | TAGCACCCAGATAGCAAG | hsa-miR-502 | 57.1 |
| 702 | CTGCAGAACTGTTCC | hsa-miR-503 | 53.2 |
| 703 | CTGCAGAACTGTTCCC | hsa-miR-503 | 57.4 |
| 704 | ATAGAGTGCAGACCAG | hsa-miR-504 | 51.3 |
| 705 | ATAGAGTGCAGACCAGG | hsa-miR-504 | 55.7 |
| 706 | ATAGAGTGCAGACCAGGG | hsa-miR-504 | 59.7 |
| 707 | GAGGAAACCAGCAA | hsa-miR-505 | 50.3 |
| 708 | GAGGAAACCAGCAAG | hsa-miR-505 | 52.9 |
| 709 | GAGGAAACCAGCAAGT | hsa-miR-505 | 55.9 |
| 710 | GAGGAAACCAGCAAGTG | hsa-miR-505 | 58.5 |
| 711 | TCTACTCAGAAGGGTG | hsa-miR-506 | 51.6 |
| 712 | TCTACTCAGAAGGGTGC | hsa-miR-506 | 56.5 |
| 713 | TTCACTCCAAAAGGTG | hsa-miR-507 | 52.1 |
| 714 | TTCACTCCAAAAGGTGC | hsa-miR-507 | 56.7 |
| 715 | TTCACTCCAAAAGGTGCA | hsa-miR-507 | 59.2 |
| 716 | TCTACTCCAAAAGGCT | hsa-miR-508 | 51.5 |
| 717 | TCTACTCCAAAAGGCTA | hsa-miR-508 | 52.3 |
| 718 | TCTACTCCAAAAGGCTAC | hsa-miR-508 | 55 |
| 719 | TCTACTCCAAAAGGCTACA | hsa-miR-508 | 57.5 |
| 720 | TCTACTCCAAAAGGCTACAA | hsa-miR-508 | 58.8 |
| 721 | TCTACTCCAAAAGGCTACAAT | hsa-miR-508 | 59.3 |
| 722 | TCTACCCACAGACGT | hsa-miR-509 | 53 |
| 723 | TCTACCCACAGACGTA | hsa-miR-509 | 53.7 |
| 724 | TCTACCCACAGACGTAC | hsa-miR-509 | 56.5 |
| 725 | TGTGATTGCCACTCT | hsa-miR-510 | 50.9 |
| 726 | TGTGATTGCCACTCTC | hsa-miR-510 | 53.9 |
| 727 | TGTGATTGCCACTCTCC | hsa-miR-510 | 58 |
| 728 | TGTGATTGCCACTCTCCT | hsa-miR-510 | 59.8 |
| 729 | TGACTGCAGAGCAAA | hsa-miR-511 | 51.8 |
| 730 | TGACTGCAGAGCAAAA | hsa-miR-511 | 53.7 |
| 731 | TGACTGCAGAGCAAAAG | hsa-miR-511 | 55.8 |
| 732 | TGACTGCAGAGCAAAAGA | hsa-miR-511 | 58.1 |
| 733 | GACCTCAGCTATGAC | hsa-miR-512-3p | 50.2 |
| 734 | GACCTCAGCTATGACA | hsa-miR-512-3p | 53.4 |
| 735 | GACCTCAGCTATGACAG | hsa-miR-512-3p | 55.7 |
| 736 | GAAAGTGCCCTCAA | hsa-miR-512-5p | 50.3 |
| 737 | GAAAGTGCCCTCAAG | hsa-miR-512-5p | 52.9 |
| 738 | GAAAGTGCCCTCAAGG | hsa-miR-512-5p | 57.3 |
| 739 | ATAAATGACACCTCCCT | hsa-miR-513 | 52.4 |
| 740 | ATAAATGACACCTCCCTG | hsa-miR-513 | 55.2 |
| 741 | ATAAATGACACCTCCCTGT | hsa-miR-513 | 57.6 |
| 742 | ATAAATGACACCTCCCTGTG | hsa-miR-513 | 59.9 |

(continued)

| SEQ ID NO: | Sequence | Target miRNA | Tm (calc) |
|---|---|---|---|
| 743 | CTACTCACAGAAGTGT | hsa-miR-514 | 51 |
| 744 | CTACTCACAGAAGTGTC | hsa-miR-514 | 53.8 |
| 745 | CTACTCACAGAAGTGTCA | hsa-miR-514 | 56.4 |
| 746 | CTACTCACAGAAGTGTCAA | hsa-miR-514 | 57.8 |
| 747 | CTACTCACAGAAGTGTCAAT | hsa-miR-514 | 58.4 |
| 748 | ACGCTCCAAAAGAAG | hsa-miR-515-3p | 50.8 |
| 749 | ACGCTCCAAAAGAAGG | hsa-miR-515-3p | 55.1 |
| 750 | ACGCTCCAAAAGAAGGC | hsa-miR-515-3p | 59.4 |
| 751 | CAGAAAGTGCTTTCTT | hsa-miR-515-5p | 51 |
| 752 | CAGAAAGTGCTTTCTTT | hsa-miR-515-5p | 52.8 |
| 753 | CAGAAAGTGCTTTCTTTT | hsa-miR-515-5p | 54.4 |
| 754 | CAGAAAGTGCTTTCTTTTG | hsa-miR-515-5p | 56.8 |
| 755 | ACCCTCTGAAAGGAA | hsa-miR-516-3p | 50.6 |
| 756 | ACCCTCTGAAAGGAAG | hsa-miR-516-3p | 53.1 |
| 757 | ACCCTCTGAAAGGAAGC | hsa-miR-516-3p | 57.8 |
| 758 | AAAGTGCTTCTTACCTC | hsa-miR-516-5p | 52.1 |
| 759 | AAAGTGCTTCTTACCTCC | hsa-miR-516-5p | 56 |
| 760 | AAAGTGCTTCTTACCTCCA | hsa-miR-516-5p | 58.4 |
| 761 | AGACAGTGCTTCCAT | hsa-miR-517* | 50.1 |
| 762 | AGACAGTGCTTCCATC | hsa-miR-517* | 53.2 |
| 763 | AGACAGTGCTTCCATCT | hsa-miR-517* | 55.4 |
| 764 | AGACAGTGCTTCCATCTA | hsa-miR-517* | 55.9 |
| 765 | AGACAGTGCTTCCATCTAG | hsa-miR-517* | 57.8 |
| 766 | AACACTCTAAAGGGATG | hsa-miR-517a | 51.2 |
| 767 | AACACTCTAAAGGGATGC | hsa-miR-517a | 55.8 |
| 768 | AACACTCTAAAGGGATGCA | hsa-miR-517a | 58.2 |
| 769 | ACACTCTAAAAGGATGC | hsa-miR-517c | 51.9 |
| 770 | ACACTCTAAAAGGATGCA | hsa-miR-517c | 54.6 |
| 771 | ACACTCTAAAAGGATGCAC | hsa-miR-517c | 57 |
| 772 | TCCAGCAAAGGGAA | hsa-miR-518a | 50.3 |
| 773 | TCCAGCAAAGGGAAG | hsa-miR-518a | 52.9 |
| 774 | TCCAGCAAAGGGAAGC | hsa-miR-518a | 57.8 |
| 775 | AAAGGGCTTCCCTTT | hsa-miR-518a-2* | 52.1 |
| 776 | AAAGGGCTTCCCTTTG | hsa-miR-518a-2* | 55.1 |
| 777 | AAAGGGCTTCCCTTTGC | hsa-miR-518a-2* | 59.6 |
| 778 | ACCTCTAAAGGGGAG | hsa-miR-518b | 50 |
| 779 | ACCTCTAAAGGGGAGC | hsa-miR-518b | 55.4 |
| 780 | ACCTCTAAAGGGGAGCG | hsa-miR-518b | 59.9 |
| 781 | CACTCTAAAGAGAAGC | hsa-miR-518c | 50.1 |
| 782 | CACTCTAAAGAGAAGCG | hsa-miR-518c | 54.7 |
| 783 | CACTCTAAAGAGAAGCGC | hsa-miR-518c | 58.8 |
| 784 | CAGAAAGTGCTTCCC | hsa-miR-518c* | 53.5 |
| 785 | CAGAAAGTGCTTCCCT | hsa-miR-518c* | 55.8 |
| 786 | CAGAAAGTGCTTCCCTC | hsa-miR-518c* | 58.3 |
| 787 | GCTCCAAAGGGAAG | hsa-miR-518d | 51 |
| 788 | GCTCCAAAGGGAAGC | hsa-miR-518d | 56.5 |
| 789 | ACACTCTGAAGGGAA | hsa-miR-518e | 50.3 |
| 790 | ACACTCTGAAGGGAAG | hsa-miR-518e | 52.8 |
| 791 | ACACTCTGAAGGGAAGC | hsa-miR-518e | 57.5 |
| 792 | TCCTCTAAAGAGAAGCG | hsa-miR-518f | 54.1 |

(continued)

| SEQ ID NO: | Sequence | Target miRNA | Tm (calc) |
|---|---|---|---|
| 793 | TCCTCTAAAGAGAAGCGC | hsa-miR-518f | 58.4 |
| 794 | AGAGAAAGTGCTTCCC | hsa-miR-518f* | 53.7 |
| 795 | AGAGAAAGTGCTTCCCT | hsa-miR-518f* | 55.9 |
| 796 | AGAGAAAGTGCTTCCCTC | hsa-miR-518f* | 58.4 |
| 797 | GTAACACTCTAAAAGGA | hsa-miR-519a | 50 |
| 798 | GTAACACTCTAAAAGGAT | hsa-miR-519a | 51.1 |
| 799 | GTAACACTCTAAAAGGATG | hsa-miR-519a | 53.7 |
| 800 | GTAACACTCTAAAAGGATGC | hsa-miR-519a | 57.7 |
| 801 | GTAACACTCTAAAAGGATGCA | hsa-miR-519a | 59.8 |
| 802 | AAACCTCTAAAAGGATGC | hsa-miR-519b | 53.9 |
| 803 | AAACCTCTAAAAGGATGCA | hsa-miR-519b | 56.4 |
| 804 | AAACCTCTAAAAGGATGCAC | hsa-miR-519b | 58.6 |
| 805 | ATCCTCTAAAAAGATGCA | hsa-miR-519c | 51.8 |
| 806 | ATCCTCTAAAAAGATGCAC | hsa-miR-519c | 54.4 |
| 807 | ATCCTCTAAAAAGATGCACT | hsa-miR-519c | 56.2 |
| 808 | ATCCTCTAAAAAGATGCACTT | hsa-miR-519c | 57.5 |
| 809 | ATCCTCTAAAAAGATGCACTTT | hsa-miR-519c | 58.7 |
| 810 | ACACTCTAAAGGGAGG | hsa-miR-519d | 51.9 |
| 811 | ACACTCTAAAGGGAGGC | hsa-miR-519d | 56.8 |
| 812 | ACACTCTAAAGGGAGGCA | hsa-miR-519d | 59.3 |
| 813 | ACACTCTAAAAGGAGGC | hsa-miR-519e | 54.4 |
| 814 | ACACTCTAAAAGGAGGCA | hsa-miR-519e | 57 |
| 815 | ACACTCTAAAAGGAGGCAC | hsa-miR-519e | 59.3 |
| 816 | GAAAGTGCTCCCTTT | hsa-miR-519e* | 51.8 |
| 817 | GAAAGTGCTCCCTTTT | hsa-miR-519e* | 53.7 |
| 818 | GAAAGTGCTCCCTTTTG | hsa-miR-519e* | 56.5 |
| 819 | ACAGTCCAAAGGGAA | hsa-miR-520a | 51.4 |
| 820 | ACAGTCCAAAGGGAAG | hsa-miR-520a | 53.8 |
| 821 | ACAGTCCAAAGGGAAGC | hsa-miR-520a | 58.5 |
| 822 | AGAAAGTACTTCCCTCT | hsa-miR-520a4* | 51.7 |
| 823 | AGAAAGTACTTCCCTCTG | hsa-miR-520a* | 54.6 |
| 824 | AGAAAGTACTTCCCTCTGG | hsa-miR-520a* | 58.4 |
| 825 | CCCTCTAAAAGGAAGC | hsa-miR-520b | 53.8 |
| 826 | CCCTCTAAAAGGAAGCA | hsa-miR-520b | 56.6 |
| 827 | CCCTCTAAAAGGAAGCAC | hsa-miR-520b | 59 |
| 828 | AACCCTCTAAAAGGAAG | hsa-miR-520c | 51.7 |
| 829 | AACCCTCTAAAAGGAAGC | hsa-miR-520c | 56.3 |
| 830 | AACCCTCTAAAAGGAAGCA | hsa-miR-520c | 58.7 |
| 831 | AACCCACCAAAGAGA | hsa-miR-520d | 51.4 |
| 832 | AACCCACCAAAGAGAA | hsa-miR-520d | 53.4 |
| 833 | AACCCACCAAAGAGAAG | hsa-miR-520d | 55.6 |
| 834 | AACCCACCAAAGAGAAGC | hsa-miR-520d | 59.9 |
| 835 | CAGAAAGGGCTTCC | hsa-miR-520d* | 51.8 |
| 836 | CAGAAAGGGCTTCCC | hsa-miR-520d* | 56.5 |
| 837 | CAGAAAGGGCTTCCCT | hsa-miR-520d* | 58.6 |
| 838 | CCCTCAAAAAGGAAG | hsa-miR-520e | 50.6 |
| 839 | CCCTCAAAAAGGAAGC | hsa-miR-520e | 55.6 |
| 840 | CCCTCAAAAAGGAAGCA | hsa-miR-520e | 58.3 |
| 841 | ACACTCTAAAGGGAAGC | hsa-miR-520g | 54.4 |
| 842 | ACACTCTAAAGGGAAGCA | hsa-miR-520g | 57 |

(continued)

| SEQ ID NO: | Sequence | Target miRNA | Tm (calc) |
|---|---|---|---|
| 843 | ACACTCTAAAGGGAAGCAC | hsa-miR-520g | 59.3 |
| 844 | ACTCTAAAGGGAAGCA | hsa-miR-520h | 51.5 |
| 845 | ACTCTAAAGGGAAGCAC | hsa-miR-520h | 54.4 |
| 846 | ACTCTAAAGGGAAGCACT | hsa-miR-520h | 56.4 |
| 847 | ACTCTAAAGGGAAGCACTT | hsa-miR-520h | 57.9 |
| 848 | ACTCTAAAGGGAAGCACTTT | hsa-miR-520h | 59.1 |
| 849 | ACACTCTAAAGGGAAGT | hsa-miR-521 | 52.5 |
| 850 | ACACTCTAAAGGGAAGTG | hsa-miR-521 | 55.2 |
| 851 | ACACTCTAAAGGGAAGTGC | hsa-miR-521 | 59.3 |
| 852 | AACACTCTAAAGGGAAC | hsa-miR-522 | 52.1 |
| 853 | AACACTCTAAAGGGAACC | hsa-miR-522 | 56.1 |
| 854 | AACACTCTAAAGGGAACCA | hsa-miR-522 | 58.5 |
| 855 | AACACTCTAAAGGGAACCAT | hsa-miR-522 | 59.1 |
| 856 | CCCTCTATAGGGAAGC | hsa-miR-523 | 54.3 |
| 857 | CCCTCTATAGGGAAGCG | hsa-miR-523 | 58.9 |
| 858 | ACTCCAAAGGGAAGC | hsa-miR-524 | 52.8 |
| 859 | ACTCCAAAGGGAAGCG | hsa-miR-524 | 57.6 |
| 860 | GAGAAAGTGCTTCCC | hsa-miR-524* | 52.8 |
| 861 | GAGAAAGTGCTTCCCT | hsa-miR-524* | 55.2 |
| 862 | GAGAAAGTGCTTCCCTT | hsa-miR-524* | 56.8 |
| 863 | GAGAAAGTGCTTCCCTTT | hsa-miR-524* | 58.3 |
| 864 | AGAAAGTGCATCCCT | hsa-miR-525 | 50.4 |
| 865 | AGAAAGTGCATCCCTC | hsa-miR-525 | 53.5 |
| 866 | AGAAAGTGCATCCCTCT | hsa-miR-525 | 55.7 |
| 867 | AGAAAGTGCATCCCTCTG | hsa-miR-525 | 58.3 |
| 868 | GCTCTAAAGGGAAGC | hsa-miR-525* | 51.9 |
| 869 | GCTCTAAAGGGAAGCG | hsa-miR-525* | 56.7 |
| 870 | AGAAAGTGCTTCCCT | hsa-miR-526a | 50.6 |
| 871 | AGAAAGTGCTTCCCTC | hsa-miR-526a | 53.7 |
| 872 | AGAAAGTGCTTCCCTCT | hsa-miR-526a | 55.9 |
| 873 | AGAAAGTGCTTCCCTCTA | hsa-miR-526a | 56.4 |
| 874 | AGAAAGTGCTTCCCTCTAG | hsa-miR-526a | 58.3 |
| 875 | AACAGAAAGTGCTTCC | hsa-miR-526b | 52 |
| 876 | AACAGAAAGTGCTTCCC | hsa-miR-526b | 56.1 |
| 877 | AACAGAAAGTGCTTCCCT | hsa-miR-526b | 58 |
| 878 | GCCTCTAAAAGGAAGC | hsa-miR-526b* | 53.8 |
| 879 | GCCTCTAAAAGGAAGCA | hsa-miR-526b* | 56.6 |
| 880 | GCCTCTAAAAGGAAGCAC | hsa-miR-526b* | 59 |
| 881 | AACAGAAAGCGCTTC | hsa-miR-526c | 51.5 |
| 882 | AACAGAAAGCGCTTCC | hsa-miR-526c | 55.6 |
| 883 | AACAGAAAGCGCTTCCC | hsa-miR-526c | 59.4 |
| 884 | AGAAAGGGCTTCCC | hsa-miR-527 | 51 |
| 885 | AGAAAGGGCTTCCCT | hsa-miR-527 | 53.6 |
| 886 | AGAAAGGGCTTCCCTT | hsa-miR-527 | 55.5 |
| 887 | AGAAAGGGCTTCCCTTT | hsa-miR-527 | 57.1 |
| 888 | AGAAAGGGCTTCCCTTTG | hsa-miR-527 | 59.7 |
| 889 | CAACAAAATCACTAGTCTTCC | hsa-miR-7 | 59.5 |
| 890 | TCATACAGCTAGATAACCA | hsa-miR-9 | 53.4 |
| 891 | TCATACAGCTAGATAACCAA | hsa-miR-9 | 54.9 |
| 892 | TCATACAGCTAGATAACCAAA | hsa-miR-9 | 56.3 |

(continued)

| SEQ ID NO: | Sequence | Target miRNA | Tm (calc) |
|---|---|---|---|
| 893 | TCATACAGCTAGATAACCAAAG | hsa-miR-9 | 57.9 |
| 894 | TCATACAGCTAGATAACCAAAGA | hsa-miR-9 | 59.8 |
| 895 | ACTTTCGGTTATCTAGC | hsa-miR-9* | 51.5 |
| 896 | ACTTTCGGTTATCTAGCT | hsa-miR-9* | 53.6 |
| 897 | ACTTTCGGTTATCTAGCTT | hsa-miR-9* | 55.1 |
| 898 | ACTTTCGGTTATCTAGCTTT | hsa-miR-9* | 56.5 |
| 899 | ACTTTCGGTTATCTAGCTTTA | hsa-miR-9* | 56.9 |
| 900 | CAGGCCGGGACAAG | hsa-miR-92 | 58.6 |
| 901 | CAGGCCGGGACAAGT | hsa-miR-92 | 61.3 |
| 902 | CAGGCCGGGACAAGTG | hsa-miR-92 | 63.8 |
| 903 | CTACCTGCACGAACAG | hsa-miR-93 | 56.7 |
| 904 | TGCTCAATAAATACCCG | hsa-miR-95 | 52.3 |
| 905 | TGCTCAATAAATACCCGT | hsa-miR-95 | 54.9 |
| 906 | TGCTCAATAAATACCCGTT | hsa-miR-95 | 56.4 |
| 907 | TGCTCAATAAATACCCGTTG | hsa-miR-95 | 58.6 |
| 908 | GCAAAAATGTGCTAGTGC | hsa-miR-96 | 57.7 |
| 909 | GCAAAAATGTGCTAGTGCC | hsa-miR-96 | 61.1 |
| 910 | GCAAAAATGTGCTAGTGCCA | hsa-miR-96 | 63.1 |
| 911 | AACAATACAACTTACTACCTC | hsa-miR-98 | 55.4 |
| 912 | CACAAGATCGGATCTACG | hsa-miR-99a | 58.4 |
| 913 | CGCAAGGTCGGTTC | hsa-miR-99b | 56.7 |
| 914 | CGCAAGGTCGGTTCT | hsa-miR-99b | 58.8 |
| 915 | CGCAAGGTCGGTTCTA | hsa-miR-99b | 59.2 |
| 916 | AACTATACAACCTACTACCTC | hsa-let-7a | 56 |
| 917 | AACTATACAACCTACTACCTCA | hsa-let-7a | 58.2 |
| 918 | AACCACACAACCTACT | hsa-let-7b | 52.1 |
| 919 | AACCACACAACCTACTACC | hsa-let-7b | 59.1 |
| 920 | AACCATACAACCTACTACCTC | hsa-let-7c | 59.5 |
| 921 | ACTATGCAACCTACTACC | hsa-let-7d | 53.8 |
| 922 | ACTATGCAACCTACTACCTC | hsa-let-7d | 58.1 |
| 923 | ACTATGCAACCTACTACCTCT | hsa-let-7d | 59.7 |
| 924 | ACTATACAACCTCCTACC | hsa-let-7e | 53.2 |
| 925 | ACTATACAACCTCCTACCT | hsa-let-7e | 55.2 |
| 926 | CACAAACCATTATGTGC | hsa-miR-15a | 53.9 |
| 927 | CACAAACCATTATGTGCT | hsa-miR-15a | 55.9 |
| 928 | CGCCAATATTTACGTG | hsa-miR-16 | 52.5 |
| 929 | ACAAGTGCCTTCACT | hsa-miR-17-3p | 51.2 |
| 930 | ACAAGTGCCTTCACTG | hsa-miR-17-3p | 54.2 |
| 931 | ACTACCTGCACTGTAA | hsa-miR-17-5p | 50.8 |
| 932 | ACTACCTGCACTGTAAG | hsa-miR-17-5p | 53.1 |
| 933 | TCAGTTTTGCATAGATTT | hsa-miR-19a | 50.4 |
| 934 | TCAGTTTTGCATAGATTTG | hsa-miR-19a | 53 |
| 935 | TCAGTTTTGCATGGAT | hsa-miR-19b | 50.6 |
| 936 | TCAGTTTTGCATGGATT | hsa-miR-19b | 52.5 |
| 937 | TCAGTTTTGCATGGATTT | hsa-miR-19b | 54.1 |
| 938 | TCAACATCAGTCTGATAA | hsa-miR-21 | 51.3 |
| 939 | TCAACATCAGTCTGATAAGCTA | hsa-miR-21 | 59.3 |
| 940 | ACAGTTCTTCAACTGG | hsa-miR-22 | 51.1 |
| 941 | GGAAATCCCTGGCA | hsa-miR-23a | 53.2 |
| 942 | GGAAATCCCTGGCAA | hsa-miR-23a | 55.2 |

(continued)

| SEQ ID NO: | Sequence | Target miRNA | Tm (calc) |
|---|---|---|---|
| 943 | GGAAATCCCTGGCAAT | hsa-miR-23a | 56.1 |
| 944 | ACTGATATCAGCTCAGT | hsa-miR-189 | 51 |
| 945 | ACTGATATCAGCTCAGTA | hsa-miR-189 | 51.8 |
| 946 | ACTGATATCAGCTCAGTAG | hsa-miR-189 | 53.8 |
| 947 | CTGTTCCTGCTGAA | hsa-miR-24 | 50 |
| 948 | CTGTTCCTGCTGAAC | hsa-miR-24 | 53.2 |
| 949 | CTGTTCCTGCTGAACT | hsa-miR-24 | 55.4 |
| 950 | TCAGACCGAGACAAG | hsa-miR-25 | 52 |
| 951 | TCAGACCGAGACAAGT | hsa-miR-25 | 54.9 |
| 952 | GCCTATCCTGGATTACTTG | hsa-miR-26a | 58.2 |
| 953 | GCGGAACTTAGCCA | hsa-miR-27a | 53.3 |
| 954 | AACCGATTTCAGATGG | hsa-miR-29a | 51.7 |
| 955 | AACCGATTTCAGATGGT | hsa-miR-29a | 54.5 |
| 956 | GCTGCAAACATCCG | hsa-miR-30a-3p | 52.9 |
| 957 | CTTCCAGTCGAGGA | hsa-miR-30a-5p | 52.3 |
| 958 | GCAACTTAGTAATGTGC | hsa-miR-32 | 52.4 |
| 959 | GCAACTTAGTAATGTGCA | hsa-miR-32 | 55 |
| 960 | CAATGCAACTACAATGC | hsa-miR-33 | 53.9 |
| 961 | CAATGCAACTACAATGCA | hsa-miR-33 | 56.3 |
| 962 | CTACCTGCACGAAC | hsa-miR-93 | 51.5 |
| 963 | CTACCTGCACGAACA | hsa-miR-93 | 54.6 |
| 964 | GCAAAAATGTGCTAGT | hsa-miR-96 | 50.7 |
| 965 | GCAAAAATGTGCTAGTG | hsa-miR-96 | 53.5 |
| 966 | AACAATACAACTTACTACC | hsa-miR-98 | 51.3 |
| 967 | AACAATACAACTTACTACCT | hsa-miR-98 | 53.2 |
| 968 | AACAATACAACTTACTACCTCA | hsa-miR-98 | 57.6 |
| 969 | CACAAGATCGGATCT | hsa-miR-99a | 50.7 |
| 970 | CACAAGATCGGATCTA | hsa-miR-99a | 51.5 |
| 971 | CACAAGATCGGATCTAC | hsa-miR-99a | 54.3 |
| 972 | CACAAGTTCGGATCT | hsa-miR-100 | 51.7 |
| 973 | CACAAGTTCGGATCTA | hsa-miR-100 | 52.4 |
| 974 | CTTCAGTTATCACAGTAC | hsa-miR-101 | 52.3 |
| 975 | CTTCAGTTATCACAGTACT | hsa-miR-101 | 54.3 |
| 976 | CTTCAGTTATCACAGTACTG | hsa-miR-101 | 56.7 |
| 977 | CTTCAGTTATCACAGTACTGT | hsa-miR-101 | 58.8 |
| 978 | AACACTGATTTCAAATGG | hsa-miR-29b | 52.4 |
| 979 | AACACTGATTTCAAATGGT | hsa-miR-29b | 54.9 |
| 980 | TCATAGCCCTGTACAA | hsa-miR-103 | 50.9 |
| 981 | TCATAGCCCTGTACAATGC | hsa-miR-103 | 58.8 |
| 982 | GCTACCTGCACTGT | hsa-miR-106a | 51.5 |
| 983 | GCTACCTGCACTGTA | hsa-miR-106a | 52.3 |
| 984 | GCTACCTGCACTGTAA | hsa-miR-106a | 54.2 |
| 985 | TGATAGCCCTGTACAA | hsa-miR-107 | 50.9 |
| 986 | TGATAGCCCTGTACAAT | hsa-miR-107 | 51.9 |
| 987 | TGATAGCCCTGTACAATG | hsa-miR-107 | 54.7 |
| 988 | GAACAGGTAGTCTGAA | hsa-miR-199a | 51.2 |
| 989 | GAACAGGTAGTCTGAAC | hsa-miR-199a | 54.2 |
| 990 | GAACAGGTAGTCTGAACA | hsa-miR-199a | 56.8 |
| 991 | AACCAATGTGCAGAC | hsa-miR-199a* | 50.6 |
| 992 | AACCAATGTGCAGACT | hsa-miR-199a* | 52.9 |

(continued)

| SEQ ID NO: | Sequence | Target miRNA | Tm (calc) |
|---|---|---|---|
| 993 | AACCAATGTGCAGACTA | hsa-miR-199a* | 53.6 |
| 994 | ACAAGCTTTTTGCTCGT | hsa-miR-208 | 55.9 |
| 995 | ACAAAGTTCTGTAGTGC | hsa-miR-148a | 52.5 |
| 996 | ACAAAGTTCTGTAGTGCA | hsa-miR-148a | 55.1 |
| 997 | GCTGAGAGTGTAGGA | hsa-miR-30c | 51.6 |
| 998 | GCTGAGAGTGTAGGAT | hsa-miR-30c | 52.7 |
| 999 | GCTGAGAGTGTAGGATGTT | hsa-miR-30c | 59.6 |
| 1000 | CTTCCAGTCGGGGATG | hsa-miR-30d | 60 |
| 1001 | AGACACGTGCACTG | hsa-miR-139 | 51.4 |
| 1002 | AGACACGTGCACTGT | hsa-miR-139 | 54.5 |
| 1003 | AGACACGTGCACTGTA | hsa-miR-139 | 55.1 |
| 1004 | GCAGAAGCATTTCCA | hsa-miR-147 | 52.2 |
| 1005 | CAACAAAATCACTAGTC | hsa-miR-7 | 50.4 |
| 1006 | CAACAAAATCACTAGTCT | hsa-miR-7 | 52.5 |
| 1007 | CAACAAAATCACTAGTCTT | hsa-miR-7 | 54 |
| 1008 | CAACAAAATCACTAGTCTTC | hsa-miR-7 | 56.3 |
| 1009 | CACAAATTCGGATCTA | hsa-miR-10a | 50.2 |
| 1010 | CACAAATTCGGATCTAC | hsa-miR-10a | 53 |
| 1011 | CACAAATTCGGATCTACA | hsa-miR-10a | 55.6 |
| 1012 | ACAAATTCGGTTCTACA | hsa-miR-10b | 52 |
| 1013 | ACAAATTCGGTTCTACAG | hsa-miR-10b | 54.1 |
| 1014 | AACAACCAGCTAAGAC | hsa-miR-34a | 50.9 |
| 1015 | AACAACCAGCTAAGACA | hsa-miR-34a | 53.8 |
| 1016 | AACAACCAGCTAAGACAC | hsa-miR-34a | 56.3 |
| 1017 | ACTCACCGACAGGT | hsa-miR-181c | 52.1 |
| 1018 | ACTCACCGACAGGTT | hsa-miR-181c | 54.2 |
| 1019 | ACTCACCGACAGGTTG | hsa-miR-181c | 57.1 |
| 1020 | TGTGAGTTCTACCATTGCC | hsa-miR-182 | 59.7 |
| 1021 | GAACAGATAGTCTAAACA | hsa-miR-199b | 50 8 |
| 1022 | GAACAGATAGTCTAAACAC | hsa-miR-199b | 53.4 |
| 1023 | GAACAGATAGTCTAAACACT | hsa-miR-199b | 55.3 |
| 1024 | AGGCATAGGATGACAA | hsa-miR-204 | 51 |
| 1025 | AGGCATAGGATGACAAA | hsa-miR-204 | 53 |
| 1026 | AGGCATAGGATGACAAAG | hsa-miR-204 | 55.1 |
| 1027 | TCAGCCGCTGTCACA | hsa-miR-210 | 57.9 |
| 1028 | AGGCGAAGGATGAC | hsa-miR-211 | 50.9 |
| 1029 | AGGCGAAGGATGACA | hsa-miR-211 | 54.1 |
| 1030 | AGGCGAAGGATGACAA | hsa-miR-211 | 55.9 |
| 1031 | AGGCGAAGGATGACAAA | hsa-miR-211 | 57.5 |
| 1032 | CTGCCTGTCTGTGCC | hsa-miR-214 | 59 |
| 1033 | ACATGGTTAGATCAAGC | hsa-miR-218 | 51.7 |
| 1034 | ACATGGTTAGATCAAGCA | hsa-miR-218 | 54.4 |
| 1035 | GAAACCCAGCAGAC | hsa-miR-221 | 51.5 |
| 1036 | GAAACCCAGCAGACA | hsa-miR-221 | 54.8 |
| 1037 | GAAACCCAGCAGACAA | hsa-miR-221 | 56.5 |
| 1038 | GAAACCCAGCAGACAAT | hsa-miR-221 | 57.3 |
| 1039 | GAGACCCAGTAGCC | hsa-miR-222 | 52.1 |
| 1040 | GAGACCCAGTAGCCA | hsa-miR-222 | 55.5 |
| 1041 | GGGGTATTTGACAAAC | hsa-miR-223 | 52.1 |
| 1042 | GGGGTATTTGACAAACT | hsa-miR-223 | 54.3 |

(continued)

| SEQ ID NO: | Sequence | Target miRNA | Tm (calc) |
|---|---|---|---|
| 1043 | GGGGTATTTGACAAACTG | hsa-miR-223 | 56.9 |
| 1044 | TAAACGGAACCACTAG | hsa-miR-224 | 50.6 |
| 1045 | TAAACGGAACCACTAGT | hsa-miR-224 | 53.5 |
| 1046 | TAAACGGAACCACTAGTG | hsa-miR-224 | 56.1 |
| 1047 | ACTGTACAAACTACTACC | hsa-let-7g | 51.6 |
| 1048 | ACAGCACAAACTACTAC | hsa-let-7i | 51.5 |
| 1049 | GGTAATCCCTGGCA | hsa-miR-23b | 51.9 |
| 1050 | GGTAATCCCTGGCAA | hsa-miR-23b | 54 |
| 1051 | GGTAATCCCTGGCAAT | hsa-miR-23b | 55 |
| 1052 | GCAGAACTTAGCCAC | hsa-miR-27b | 52.4 |
| 1053 | GCAGAACTTAGCCACT | hsa-miR-27b | 54.7 |
| 1054 | AGCTGAGTGTAGGATGTTTAC | hsa-miR-30b | 59.9 |
| 1055 | TCACAAGTTAGGGTCT | hsa-miR-125b | 51.3 |
| 1056 | TCACAAGTTAGGGTCTCAG | hsa-miR-125b | 58.7 |
| 1057 | ATGCCCTTTTAACATTG | hsa-miR-130a | 50.7 |
| 1058 | ATGCCCTTTTAACATTGCAC | hsa-miR-130a | 59.6 |
| 1059 | CGACCATGGCTGTA | hsa-miR-132 | 52.8 |
| 1060 | ACAGCTGGTTGAAGG | hsa-miR-133a | 52.6 |
| 1061 | TCACATAGGAATAAAAAGC | hsa-miR-135a | 52.2 |
| 1062 | TCACATAGGAATAAAAAGCC | hsa-miR-135a | 55.8 |
| 1063 | CTACCATAGGGTAAAAC | hsa-miR-140 | 51 |
| 1064 | CTACCATAGGGTAAAACC | hsa-miR-140 | 55 |
| 1065 | TCCATAAAGTAGGAAACA | hsa-miR-142-3p | 51.7 |
| 1066 | TCCATAAAGTAGGAAACAC | hsa-miR-142-3p | 54.3 |
| 1067 | TCCATAAAGTAGGAAACACT | hsa-miR-142-3p | 56.1 |
| 1068 | TCCATAAAGTAGGAAACACTA | hsa-miR-142-3p | 56.6 |
| 1069 | GTAGTGCTTTCTACTTT | hsa-miR-142-5p | 50.1 |
| 1070 | GTAGTGCTTTCTACTTTA | hsa-miR-142-5p | 50.9 |
| 1071 | TGAGCTACAGTGCTTC | hsa-miR-143 | 52.9 |
| 1072 | TGAGCTACAGTGCTTCA | hsa-miR-143 | 55.7 |
| 1073 | TGAGCTACAGTGCTTCAT | hsa-miR-143 | 56.4 |
| 1074 | CTAGTACATCATCTATACTG | hsa-miR-144 | 51.5 |
| 1075 | CTAGTACATCATCTATACTGT | hsa-miR-144 | 53.9 |
| 1076 | AAGGGATTCCTGGGA | hsa-miR-145 | 53.4 |
| 1077 | AAGGGATTCCTGGGAA | hsa-miR-145 | 55.3 |
| 1078 | AAGGGATTCCTGGGAAA | hsa-miR-145 | 57.1 |
| 1079 | CCCAAGTTCTGTCA | hsa-miR-152 | 50 |
| 1080 | CCCAAGTTCTGTCAT | hsa-miR-152 | 51.2 |
| 1081 | CCCAAGTTCTGTCATG | hsa-miR-152 | 54.3 |
| 1082 | AGCTGCTTTTGGGATTC | hsa-miR-191 | 56.1 |
| 1083 | AGCTGCTTTTGGGATTCC | hsa-miR-191 | 59.9 |
| 1084 | TCATACAGCTAGATAACC | hsa-miR-9 | 50.7 |
| 1085 | CACAGGTTAAAGGGT | hsa-miR-125a | 51.4 |
| 1086 | CACAGGTTAAAGGGTC | hsa-miR-125a | 54.3 |
| 1087 | CACAGGTTAAAGGGTCT | hsa-miR-125a | 56.4 |
| 1088 | GCATTATTACTCACGG | hsa-miR-126 | 50.4 |
| 1089 | GCATTATTACTCACGGT | hsa-miR-126 | 53.3 |
| 1090 | GCATTATTACTCACGGTA | hsa-miR-126 | 53.9 |
| 1091 | CGCGTACCAAAAGT | hsa-miR-126* | 52 |
| 1092 | CGCGTACCAAAAGTA | hsa-miR-126* | 52.8 |

(continued)

| SEQ ID NO: | Sequence | Target miRNA | Tm (calc) |
|---|---|---|---|
| 1093 | CGCGTACCAAAAGTAA | hsa-miR-126* | 54.5 |
| 1094 | CGCGTACCAAAAGTAAT | hsa-miR-126* | 55.3 |
| 1095 | CGCGTACCAAAAGTAATA | hsa-miR-126* | 55.8 |
| 1096 | CGCGTACCAAAAGTAATAA | hsa-miR-126* | 57.1 |
| 1097 | CGCGTACCAAAAGTAATAAT | hsa-miR-126* | 57.7 |
| 1098 | AGCCAAGCTCAGAC | hsa-miR-127 | 50.3 |
| 1099 | AGCCAAGCTCAGACG | hsa-miR-127 | 55.4 |
| 1100 | CCCTCTGGTCAACCAG | hsa-miR-134 | 59.6 |
| 1101 | TCCATCATCAAAACAAA | hsa-miR-136 | 50.3 |
| 1102 | TCCATCATCAAAACAAAT | hsa-miR-136 | 51.2 |
| 1103 | TCCATCATCAAAACAAATG | hsa-miR-136 | 53.8 |
| 1104 | TCCATCATCAAAACAAATGG | hsa-miR-136 | 57.3 |
| 1105 | ACCCTTATCAGTTCTCC | hsa-miR-184 | 536 |
| 1106 | GAACTGCCTTTCTCTC | hsa-miR-185 | 53.7 |
| 1107 | GAACTGCCTTTCTCTCC | hsa-miR-185 | 57.8 |
| 1108 | AAGCCCAAAAGGAGA | hsa-miR-186 | 51.7 |
| 1109 | AAGCCCAAAAGGAGAA | hsa-miR-186 | 53.7 |
| 1110 | AAGCCCAAAAGGAGAAT | hsa-miR-186 | 54.6 |
| 1111 | AAGCCCAAAAGGAGAATT | hsa-miR-186 | 56.2 |
| 1112 | ACCCTCCACCATGCAA | hsa-miR-188 | 58.9 |
| 1113 | TCCACATGGAGTTGCTG | hsa-miR-194 | 58 |
| 1114 | GCCAATATTTCTGTGC | hsa-miR-195 | 51.7 |
| 1115 | GCCAATATTTCTGTGCT | hsa-miR-195 | 53.9 |
| 1116 | GAAAGAGACCGGTT | hsa-miR-128b | 50.1 |
| 1117 | GAAAGAGACCGGTTC | hsa-miR-128b | 53.3 |
| 1118 | GAAAGAGACCGGTTCA | hsa-miR-128b | 56.2 |
| 1119 | ATCTGCACTGTCAGC | hsa-miR-106b | 51.9 |
| 1120 | ATCTGCACTGTCAGCAC | hsa-miR-106b | 57.5 |
| 1121 | ATCTGCACTGTCAGCACT | hsa-miR-106b | 59.4 |
| 1122 | ACCGATTTCAAATGGT | hsa-miR-29c | 51.4 |
| 1123 | ACCGATTTCAAATGGTG | hsa-miR-29c | 54.2 |
| 1124 | ACATCGTTACCAGACA | hsa-miR-200a | 52.2 |
| 1125 | ACATCGTTACCAGACAGTG | hsa-miR-200a | 59.3 |
| 1126 | TCACCAAAACATGGAA | hsa-miR-302a | 51.6 |
| 1127 | TCACCAAAACATGGAAG | hsa-miR-302a | 53.7 |
| 1128 | GCAATCAGCTAACTACA | hsa-miR-34c | 52.7 |
| 1129 | GCAATCAGCTAACTACACTG | hsa-miR-34c | 59.4 |
| 1130 | GCTTTGACAATACTATTG | hsa-miR-301 | 50.6 |
| 1131 | GCTTTGACAATACTATTGCAC | hsa-miR-301 | 59.2 |
| 1132 | ACAGGATTGAGGGGGG | hsa-miR-296 | 59.3 |
| 1133 | ATGCCCTTTCATCATTGC | hsa-miR-130b | 57.2 |
| 1134 | ATGCCCTTTCATCATTGCA | hsa-miR-130b | 59.6 |
| 1135 | GCTGTAAACATCCGA | hsa-miR-30e-3p | 51.5 |
| 1136 | TCCAGTCAAGGATGT | hsa-miR-30e-5p | 50.1 |
| 1137 | TCCAGTCAAGGATGTT | hsa-miR-30e-5p | 52.2 |
| 1138 | TCCAGTCAAGGATGTTT | hsa-miR-30e-5p | 54.1 |
| 1139 | TCCAGTCAAGGATGTTTA | hsa-miR-30e-5p | 54.7 |
| 1140 | TCCAGTCAAGGATGTTTAC | hsa-miR-30e-5p | 57.1 |
| 1141 | GTACCCCTGGAGATTC | hsa-miR-361 | 55.3 |
| 1142 | GTACCCCTGGAGATTCT | hsa-miR-361 | 57.5 |

(continued)

| SEQ ID NO: | Sequence | Target miRNA | Tm (calc) |
|---|---|---|---|
| 1143 | CTACTAAAACATGGAAGC | hsa-miR-302b | 53.5 |
| 1144 | CTACTAAAACATGGAAGCA | hsa-miR-302b | 55.9 |
| 1145 | CAGCAGGTACCCCCA | hsa-miR-302c* | 60 |
| 1146 | ACACTCAAACATGGAA | hsa-miR-302d | 50.5 |
| 1147 | ACACTCAAACATGGAAG | hsa-miR-302d | 52.8 |
| 1148 | ACACTCAAACATGGAAGC | hsa-miR-302d | 57.1 |
| 1149 | TCACCATTGCTAAAGTG | hsa-miR-367 | 52.6 |
| 1150 | AAACGTGGAATTTCCT | hsa-miR-368 | 51.6 |
| 1151 | AAACGTGGAATTTCCTC | hsa-miR-368 | 54.3 |
| 1152 | AAACGTGGAATTTCCTCT | hsa-miR-368 | 56.2 |
| 1153 | AAACGTGGAATTTCCTCTA | hsa-miR-368 | 56.7 |
| 1154 | AAACGTGGAATTTCCTCTAT | hsa-miR-368 | 57.4 |
| 1155 | ACACCCCAAAATCGA | hsa-miR-373 | 52.7 |
| 1156 | GGAAAGCGCCCCCA | hsa-miR-373* | 61.3 |
| 1157 | CACTTATCAGGTTGTATT | hsa-miR-374 | 51.5 |
| 1158 | CACTTATCAGGTTGTATTA | hsa-miR-374 | 52.2 |
| 1159 | CACTTATCAGGTTGTATTAT | hsa-miR-374 | 53 |
| 1160 | CACTTATCAGGTTGTATTATA | hsa-miR-374 | 53.6 |
| 1161 | ACGTGGATTTTCCTC | hsa-miR-376a | 506 |
| 1162 | ACGTGGATTTTCCTCT | hsa-miR-376a | 52.9 |
| 1163 | ACGTGGATTTTCCTCTA | hsa-miR-376a | 53.6 |
| 1164 | ACGTGGATTTTCCTCTAT | hsa-miR-376a | 54.5 |
| 1165 | ACAAAAGTTGCCTTTG | hsa-miR-377 | 50.8 |
| 1166 | ACAAAAGTTGCCTTTGT | hsa-miR-377 | 53.5 |
| 1167 | ACACAGGACCTGGA | hsa-miR-378 | 51.6 |
| 1168 | ACACAGGACCTGGAG | hsa-miR-378 | 54.2 |
| 1169 | ACACAGGACCTGGAGT | hsa-miR-378 | 57.2 |
| 1170 | GGCCTTCTGACTCC | hsa-miR-422b | 53.5 |
| 1171 | TACGTTCCATAGTCTAC | hsa-miR-379 | 50.2 |
| 1172 | AGCCACAATCACCTT | hsa-miR-383 | 51.2 |
| 1173 | AGCCACAATCACCTTC | hsa-miR-383 | 54.2 |
| 1174 | AGCCACAATCACCTTCT | hsa-miR-383 | 56.3 |
| 1175 | GGCTATAAAGTAACTGAG | hsa-miR-340 | 51.2 |
| 1176 | GGCTATAAAGTAACTGAGA | hsa-miR-340 | 53.8 |
| 1177 | ACGGAAGGGCAGAG | hsa-miR-328 | 54 |
| 1178 | ACGGAAGGGCAGAGA | hsa-miR-328 | 57 |
| 1179 | GACGGGTGCGATTT | hsa-miR-342 | 54.8 |
| 1180 | CCTCAAGGAGCTTC | hsa-miR-151 | 50.6 |
| 1181 | CCTCAAGGAGCTTCA | hsa-miR-151 | 53.9 |
| 1182 | ACAAAGTTCTGTGATGC | hsa-miR-148b | 53.3 |
| 1183 | ACAAAGTTCTGTGATGCA | hsa-miR-148b | 55.9 |
| 1184 | TTCTAGGATAGGCCCA | hsa-miR-331 | 52.9 |
| 1185 | TTCTAGGATAGGCCCAG | hsa-miR-331 | 55.2 |
| 1186 | ACACCAATGCCCTAG | hsa-miR-324-5p | 51.5 |
| 1187 | TCAACAAAATCACTGATG | hsa-miR-338 | 52.1 |
| 1188 | TCAACAAAATCACTGATGC | hsa-miR-338 | 56.2 |
| 1189 | TCAACAAAATCACTGATGCT | hsa-miR-338 | 57.9 |
| 1190 | TGAGCTCCTGGAGG | hsa-miR-339 | 52.3 |
| 1191 | TGAGCTCCTGGAGGA | hsa-miR-339 | 55.6 |
| 1192 | ACATTTTTCGTTATTGCT | hsa-miR-335 | 51.7 |

(continued)

| SEQ ID NO: | Sequence | Target miRNA | Tm (calc) |
|---|---|---|---|
| 1193 | TAGCTGGTTGAAGGG | hsa-miR-133b | 51.7 |
| 1194 | TAGCTGGTTGAAGGGG | hsa-miR-133b | 56.2 |
| 1195 | GCCCTGGACTAGGA | hsa-miR-345 | 53.8 |
| 1196 | GGCCTTCTGACCCT | hsa-miR-422a | 55.2 |
| 1197 | GGCCTTCTGACCCTA | hsa-miR-422a | 55.8 |
| 1198 | GGCCTTCTGACCCTAA | hsa-miR-422a | 57.6 |
| 1199 | CTGAGGGGCCTCAGA | hsa-miR-423 | 59.4 |
| 1200 | TTCAAAACATGAATTGC | hsa-miR-424 | 50 |
| 1201 | TTCAAAACATGAATTGCT | hsa-miR-424 | 52.1 |
| 1202 | TTCAAAACATGAATTGCTG | hsa-miR-424 | 54.5 |
| 1203 | TTCAAAACATGAATTGCTGC | hsa-miR-424 | 58.2 |
| 1204 | TGAGACACACTTTGC | dme-miR-3 | 50.5 |
| 1205 | TGAGACACACTTTGCC | dme-miR-3 | 54.8 |
| 1206 | ACTATACAACCTACTACCT | dme-let-7 | 52 |
| 1207 | ACTATACAACCTACTACCTC | dme-let-7 | 54.5 |
| 1208 | ACTATACAACCTACTACCTCA | dme-let-7 | 56.9 |
| 1209 | TAGGAGAGAGAAAAAGAC | dme-miR-14 | 52.3 |
| 1210 | TAGGAGAGAGAAAAAGACT | dme-miR-14 | 54.3 |
| 1211 | TAGGAGAGAGAAAAAGACTG | dme-miR-14 | 56.7 |
| 1212 | TAGGAGAGAGAAAAAGACTGA | dme-miR-14 | 58.9 |
| 1213 | TCAGCTATGCCGAC | dme-miR-31a | 50.5 |
| 1214 | TCAGCTATGCCGACA | dme-miR-31a | 53.7 |
| 1215 | TCAGCTATGCCGACAT | dme-miR-31a | 54.6 |
| 1216 | TCAGCTATGCCGACATC | dme-miR-31a | 57.2 |
| 1217 | GCTCCTCAAAGCTG | dme-miR-2b | 50.7 |
| 1218 | AAAAAGAACAGCCACT | dme-miR-6 | 51 |
| 1219 | AAAAAGAACAGCCACTG | dme-miR-6 | 53.9 |
| 1220 | AAAAAGAACAGCCACTGT | dme-miR-6 | 56.4 |
| 1221 | CGGGGCGAGAGAAT | dme-miR-184* | 56.7 |
| 1222 | CGGGGCGAGAGAATG | dme-miR-184* | 59.5 |
| 1223 | GCACTGATTTCGAATG | dme-miR-285 | 52.3 |
| 1224 | GCACTGATTTCGAATGG | dme-miR-285 | 56.4 |
| 1225 | CTCACAGTATAATCCTGT | dme-miR-308 | 52.8 |
| 1226 | CTCACAGTATAATCCTGTG | dme-miR-308 | 55.4 |
| 1227 | CTCACAGTATAATCCTGTGA | dme-miR-308 | 57.6 |
| 1228 | CTCACAGTATAATCCTGTGAT | dme-miR-308 | 58.3 |
| 1229 | CGCCAGTAAGCGGA | dme-miR-316 | 57 |
| 1230 | CGCCAGTAAGCGGAA | dme-miR-316 | 58.6 |
| 1231 | GCTCATCAAAGCTGG | dme-miR-2a | 52.6 |
| 1232 | GCTCATCAAAGCTGGC | dme-miR-2a | 57.5 |
| 1233 | GCTCATCAAAGCTGGCT | dme-miR-2a | 59.4 |
| 1234 | GCCCATCAAAGCTG | dme-miR-2c | 51.6 |
| 1235 | GCCCATCAAAGCTGG | dme-miR-2c | 56.3 |
| 1236 | CAGCTATTCCGACAT | dme-miR-31b | 50.5 |
| 1237 | CAGCTATTCCGACATC | dme-miR-31b | 53.5 |
| 1238 | CAGCTATTCCGACATCT | dme-miR-31b | 55.6 |
| 1239 | CAGCTATTCCGACATCTT | dme-miR-31b | 57.1 |
| 1240 | CAGCTATTCCGACATCTTG | dme-miR-31b | 59.4 |

In particular embodiments, each probe includes a target-complementary sequence selected from SEQ ID NOs:

1-1240. SEQ ID NOS: 1-1240 include sequences selected based on known miRNA sequences, which are given SEQ ID NOS: 1268-1581 (and are listed in Table 4, *infra*). In certain embodiments, the target-complementary sequence of each probe is independently selected from SEQ ID NOS: 916-1240. In particular embodiments, a probe having a target-complementary sequence selected from SEQ ID NOS: 1241 to 1250 may be employed, particularly in addition to probes having target-complementary sequences selected from SEQ ID NOs: 1-1240, or selected from SEQ ID NOS: 916-1240. In particular embodiments, a probe having a target-complementary sequence selected from SEQ ID NOS: 1241 to 1250 may be employed in addition to probes having target-complementary sequences selected from SEQ ID NOS: 290-296.

In particular embodiments, a subject probe may include a Tm enhancement domain, a linker, or both. Some examples are shown in Table 2, which lists some example target-complementary sequences (SEQ ID NOS: 7-14, listed from 5' to 3') along with the same sequences having a linker and Tm enhancement domain. By inspection of the sequences listed in Table 2, it will be apparent that the sequences of SEQ ID NOS:1252-1259 include the sequences of SEQ ID NOS:7-14, respectively, plus a single base "G" (a nucleotide clamp) at the 5' end and a T(10) linker at the 3' end. The sequences of SEQ ID NOS: 1260-1267 include the sequences of SEQ ID NOS:1252-1259 plus a further hairpin Tm enhancement domain having a sequence of CGCTCGGGTTTTCCCGAGCG (SEQ ID NO:1251). A subject probe includes a target-complementary sequence, plus an optional linker and/or an optional Tm enhancement domain; examples of subject probes having target-complementary sequences selected from SEQ ID NOS:7-14 are given in Table 2.

Table 2

| SEQ ID NO: | Sequence | Target miRNA |
|---|---|---|
| 7 | AACTATACAACCTACTACC | hsa-let-7a |
| 1252 | GAACTATACAACCTACTACCTTTTTTTTTT | hsa-let-7a |
| 1260 | CGCTCGGGTTTTCCCGAGCGGAACTATACA ACCTACTACCTTTTTTTTTT | hsa-let-7a |
| | | |
| 8 | AACTATACAACCTACTACCT | hsa-let-7a |
| 1253 | GAACTATACAACCTACTACCTTTTTTTTTTT | hsa-let-7a |
| 1261 | CGCTCGGGTTTTCCCGAGCGGAACTATACA ACCTACTACCTTTTTTTTTTT | hsa-let-7a |
| | | |
| 9 | AACCACACAACCTACTA | hsa-let-7b |
| 1254 | GAACCACACAACCTACTATTTTTTTTTT | hsa-let-7b |
| 1262 | CGCTCGGGTTTTCCCGAGCGGAACCACACA ACCTACTATTTTTTTTTT | hsa-let-7b |
| | | |
| 10 | AACCACACAACCTACTAC | hsa-let-7b |
| 1255 | GAACCACACAACCTACTACTTTTTTTTTT | hsa-let-7b |
| 1263 | CGCTCGGGTTTTCCCGAGCGGAACCACACA ACCTACTACTTTTTTTTTT | hsa-let-7b |
| | | |
| 11 | AACCATACAACCTACTAC | hsa-let-7c |
| 1256 | GAACCATACAACCTACTACTTTTTTTTTT | hsa-let-7c |
| 1264 | CGCTCGGGTTTTCCCGAGCGGAACCATACA ACCTACTACTTTTTTTTTT | hsa-let-7c |
| | | |
| 12 | AACCATACAACCTACTACC | hsa-let-7c |
| 1257 | GAACCATACAACCTACTACCTTTTTTTTTT | hsa-let-7c |

(continued)

| SEQ ID NO: | Sequence | Target miRNA |
|---|---|---|
| 1265 | CGCTCGGGTTTTCCCGAGCGGAACCATACA ACCTACTACCTTTTTTTTTT | hsa-let-7c |
| | | |
| 13 | AACCATACAACCTACTACCT | hsa-let-7c |
| 1258 | GAACCATACAACCTACTACCTTTTTTTTTTT | hsa-let-7c |
| 1266 | CGCTCGGGTTTTCCCGAGCGGAACCATACA ACCTACTACCTTTTTTTTTTT | hsa-let-7c |
| | | |
| 14 | ACTATGCAACCTACTACCT | hsa-let-7d |
| 1259 | GACTATGCAACCTACTACCTTTTTTTTTTT | hsa-let-7d |
| 1267 | CGCTCGGGTTTTCCCGAGCGGACTATGCAA CCTACTACCTTTTTTTTTTT | hsa-let-7d |

Tm ENHANCEMENT DOMAIN:

As noted above, a subject probe generally contains a target-complementary sequence 104 that base-pairs with a target miRNA to form a probe/target duplex. In particular embodiments the probe includes a Tm enhancement domain 106 that increases stability of the probe/target duplex. Tm enhancement domain 106 may increase probe/target duplex stability via a number of mechanisms, including, for example, by providing a nucleotide clamp 106a (as illustrated in Fig. 2A) to which an extended polynucleotide, e.g., extended miRNA, may bind, or by providing a hairpin structure 106b (as illustrated in Fig. 2B) that increases stability via coaxial stacking. In certain embodiments and as illustrated in Fig. ' 2C, Tm enhancement domain 106 may contain both a nucleotide clamp 106a and a hairpin structure 106b. The sequence of the Tm enhancement domain 106 is generally unrelated to the sequence of the target-complementary sequence 104. Further description of such Tm enhancement domains is provided in copending US 2007/0003940.

As mentioned above and as illustrated in Fig. 2A, Tm enhancement domain 106 is immediately adjacent to target-complementary sequence 104 and may contain a nucleotide clamp 106a, where.a nucleotide clamp 106a contains a contiguous sequence of up to about 5 nucleotides (i.e., 1, 2, 3, 4 or 5 nucleotides). The identity of the nucleotides employed in the nucleotide clamp may be the same as each other or different from each other. As illustrated in Fig. 2A, a nucleotide clamp contains $N_{1-5}$, wherein "N" is any nucleotide, particularly a G or a C, possibly an A, T, or U, or a modified base. In certain embodiments, illustrated in Fig. 4A, a subject probe 102 containing a nucleotide clamp 106a is employed in a method in which the miRNA 120 to be detected by the probe 102 is extended 122, i.e. has additional nucleotide(s) added to the miRNA (in certain embodiments during labeling of the miRNA) to produce an extended miRNA 124. The extended miRNA includes an extended portion 126 having contiguous sequence of up to about 5 nucleotides (i.e., 1, 2, 3, 4 or 5 nucleotides) (illustrated in Fig. 4A as $N^*_{1-5}$), wherein extended portion 126 typically is complementary to the nucleotide clamp 106a (i.e. $N^*_{1-5}$ illustrates a contiguous sequence of up to about 5 nucleotides that is complementary to $N_{1-5}$). In use, the miRNA, e.g. the extended miRNA, is contacted 125 with the probe under conditions sufficient to provide for specific binding of the probe to the miRNA to form a probe/target duplex. In the probe/target duplex formed between a probe 102 containing a nucleotide clamp 106a and an extended miRNA 124, the extended portion 126 of the extended miRNA 124 base-pairs with the nucleotide clamp 106a of the probe 102 and the non-extended miRNA sequence 128 base pairs with target-complementary sequence 104. In particular embodiments of particular probes, nucleotide clamp 106a (having nucleotides designated $N_{1-5}$) may be shorter than the extended portion 126 or the miRNA; e.g. the probe may be designed in this way to reduce the base pairing of the extended miRNA 124 to the nucleotide clamp 106a, for example to obtain a set of probes that are characterized as having similar melting temperatures (Tm).

Also as mentioned above and as illustrated in Fig. 2B, Tm enhancement domain 106 is immediately adjacent to target-complementary sequence 104 and may contain a hairpin structure 106b, where a hairpin structure 106b has a loop 112 of at least 3 or 4 nucleotides (up to about 8 or 10 nucleotides) and a double-stranded stem 114 (of about 6 to about 20 base pairs) in which complementary nucleotides bind to each other in an anti-parallel manner. The hairpin structure 106b may contain from approximately 5 to about 45 nucleotides, e.g., about 8 to about 30 nucleotides. As shown in Fig. 4B and Fig. 4C, when miRNA 124, 123 is bound to a probe 102 containing a hairpin region 106b, a terminal nucleotide of the miRNA generally occupies a position that is immediately adjacent to a terminal nucleotide of the probe 102. In effect, in this embodiment, the probe/target duplex produced by binding of a miRNA 124, 123 to a probe 102 resembles a long hairpin structure containing a nick in the stem of the hairpin. The description of the embodiments of Fig. 4B and Fig 4C is essentially the same as that given for the embodiment of Fig. 4A, with the following considerations. The embodiment illustrated in Fig. 4B shows a method in which the miRNA 120 to be detected by the probe 102 is extended 122 (in certain embodiments during labeling of the miRNA) to produce an extended miRNA 124, which is then hybridized to the probe 102. The embodiment illustrated in Fig. 4C shows a method in which the miRNA 120 to be detected by the probe 102 is not extended 122, and the unextended miRNA is then hybridized to the probe 102. The hairpin structure may assist in increasing probe specificity by preferentially binding to miRNAs (having known length and sequence), rather than pre-miRNAs or pri-miRNAs (i.e., precursor RNAs that are cleaved to produce miRNAs) in a sample. The presence of hairpin structure, in certain embodiments, allows a probe to discriminate between a miRNA and a precursor of that miRNA that is present in same sample. Also, the extra stabilization provided by helical stacking is present only when the target miRNA is the proper length.

LINKER:

As noted above, a probe may include a linker that is bound to the target-complementary sequence. In embodiments in which the probe is bound to a surface of an array support, the target-complementary sequence is bound to the array support via the linker. The linker sequence may be any sequence that does not substantially interfere with hybridization of targets to probes, e.g. the sequence of the probe should be selected to not be complementary to any analytes expected to be assayed. An example used herein is a $(T)_{10}$ linker (ten contiguous Ts), wherein one end of the $(T)_{10}$ linker is bound to the target-complementary sequence, and the probe is bound to the array surface via the other end of the $(T)_{10}$ linker. Thus, the sequence and length of the linker can be varied (such as 0-20 nucleotides).

PROBE SET:

In particular embodiments, a probe set comprising subject probes is provided. In particular embodiments, a probe set includes at least five subject probes, wherein each of said at least five subject probes has a target-complementary sequence independently selected from SEQ ID NOs: 1-1240. In some embodiments, a probe set includes at least 10 subject probes, at least 20 subject probes, at least 50 subject probes, at least 100 subject probes, at least 200 subject probes, or more subject probes, such as up to 1000 subject probes, up to 2000 subject probes, or even more subject probes, and each of the subject probes has a target-complementary sequence independently selected from SEQ ID NOs: 1-1240. In certain embodiments, the target-complementary sequence of each probe is independently selected from the group consisting of SEQ ID NOS: 916-1240. Each probe of the probe set may include a linker and/or Tm enhancement domain, as described above.

In particular embodiments, the probe set further includes at least one probe having a target-complementary sequence selected from SEQ ID NOS: 1241 to 1250. SEQ ID NOS: 1241 to 1250 are directed to a few miRNAs, such as human miR-20a and miR-20b, in which sequence homologous miRNAs differ by one 5' end nucleotide and one nucleotide in the middle of the miRNA sequences. In such cases, additional Tm matching probes are generated by successively removing 3' nucleotides of the miRNA in the probe-target base pairing (i.e., by removing base pairing sequence from the 5' end of the target-complementary sequence of the probe). In use, the results of hybridization experiments with these probes will be compared to the results with 5' modified probes (probes with target complementary sequences selected from SEQ ID NOS:290-296). Table 3 compares the sequences of SEQ ID NOS: 1241 to 1250 (having successive nucleotides deleted from the 3' end) with the sequences of SEQ ID NOS:290-296 (having successive nucleotides deleted from the 5' end).

Table 3

| SEQ ID NO: | Sequence | Target miRNA | Tm (calc) |
|---|---|---|---|
| 1241 | CTGCACTATAAGCACTTTA | hsa-miR-20a | 50.7 |
| 1242 | CCTGCACTATAAGCACTTTA | hsa-miR-20a | 54.5 |
| 1243 | ACCTGCACTATAAGCACTTTA | hsa-miR-20a | 56.8 |
| 1244 | TACCTGCACTATAAGCACTTTA | hsa-miR-20a | 57.2 |
| 1245 | CTACCTGCACTATAAGCACTTTA | hsa-miR-20a | 58.8 |
| 290 | CTACCTGCACTATAAGC | hsa-miR-20a | 52.9 |
| 291 | CTACCTGCACTATAAGCA | hsa-miR-20a | 55.6 |

(continued)

| SEQ ID NO: | Sequence | Target miRNA | Tm (calc) |
|---|---|---|---|
| 292 | CTACCTGCACTATAAGCAC | hsa-miR-20a | 57.9 |
| 293 | CTACCTGCACTATAAGCACT | hsa-miR-20a | 59.6 |
| 1377 | UAAAGUGCUUAUAGUGCAGGUAG | (hsa-miR-20a) | |
| | | | |
| 1246 | CTGCACTATGAGCACTTTG | hsa-miR-20b | 55.6 |
| 1247 | CCTGCACTATGAGCACTTTG | hsa-miR-20b | 59.2 |
| 1248 | ACCTGCACTATGAGCACTTTG | hsa-miR-20b | 61.3 |
| 1249 | TACCTGCACTATGAGCACTTTG | hsa-miR-20b | 61.5 |
| 1250 | CTACCTGCACTATGAGCACTTTG | hsa-miR-20b | 62.9 |
| 294 | CTACCTGCACTATGAG | hsa-miR-20b | 51.2 |
| 295 | CTACCTGCACTATGAGC | hsa-miR-20b | 56.0 |
| 296 | CTACCTGCACTATGAGCA | hsa-miR-20b | 58.5 |
| 1378 | CAAAGUGCUCAUAGUGCAGGUAG | (hsa-miR-20b) | |

Note that the sequences of human miRNAs miR-20a and miR-20b are given in SEQ ID NOs:1377 and 1378. Thus, in addition to any other probes in a probe set of the present invention, in certain embodiments the probe set may further include at least one probe with a target complementary sequence selected from SEQ ID NOS:290-296 and at least one corresponding probe with a target complementary sequence selected from SEQ ID NOS: 1241 to 1250. In this regard, the corresponding probe is directed to the same target miRNA that the probe with a target complementary sequence selected from SEQ ID NOS:290-296 is directed to.

NESTED SET:

Referring now to Table 1, it can be seen that the sequences given in SEQ ID NOS:1-1250 include nested sets of sequences, in which a first (longer) sequence shares the same sequence of a second (shorter) sequence, but includes one or more additional bases. An example is SEQ ID NOS:33-37, in which the longest sequence (SEQ ID NO:37) comprises the same sequence of the shorter sequences plus 1, 2, 3, or 4 bases (relative to SEQ IDS 36, 35, 34, 33, respectively):

33 ACAGGAGTCTGAGCA
34 ACAGGAGTCTGAGCAT
35 ACAGGAGTCTGAGCATT
36 ACAGGAGTCTGAGCATTT
37 ACAGGAGTCTGAGCATTTG

As used herein, "nested set" references two or more sequences bearing such a relationship to each other. The "nested set" may include two sequences wherein the longer of the two sequences comprises that shorter of the two sequences plus 1, 2, 3, 4, or 5, or more bases. In certain embodiments, the nested set may include three or more sequences.

In particular embodiments, a subject probe set may include two, three, or more probes that are characterized as having target-complementary sequences that form a nested set. In other words, more than one member of a given nested set selected from the sequences of SEQ ID NOS:1-1250 may be included in a subject probe set. In such embodiments, the target-complementary sequence of one probe of the probe set will be shorter or longer by about I to about 5 bases, compared to the target-complementary sequence of another probe of the probe set, but otherwise the two target-complementary sequences share the same "common" sequence. That is, one probe of the probe set will have target-complementary sequence that is shorter or longer by about, e.g. 1, 2, 3, 4, or 5 bases (but will otherwise share a common sequence), compared to the target-complementary sequence of another probe of the probe set, wherein both probes are directed to the same target miRNA. In some such embodiments, the base (or bases) that are omitted are typically those from the 3'-end of the target-complementary sequence (see, e.g. selected sequences in SEQ ID NOS:

1-1240). In certain embodiments, the base (or bases) that are omitted are typically those from the 5'-end of the target-complementary sequence (see, e.g. selected sequences in SEQ ID NOS: 1241-1250). In particular embodiments, the target-complementary sequence of a first probe of the probe set differs from the target-complementary sequence of a second probe of the probe set by lacking at least one base (e.g. lacking at least two bases, lacking at least three bases, lacking up to about five bases) relative to the target-complementary sequence of the second probe, wherein the first probe and second probe are directed to the same miRNA.

SIMILAR Tm:

In particular embodiments, the probes of the probe set are selected such that the probe/target duplexes formed will have similar thermal stabilities. The melting temperature ('Tm') of the probe/target duplexes should be high enough to eliminate or reduce any nonspecific binding (e.g. preventing non-complementary sequences from forming double-stranded structures). In such embodiments, the melting temperatures of at least 80% of the probe/target duplexes will be within about 15° C of each other, typically within about 12° C of each other, about 10° C of each other, or about 5° C of each other. In certain embodiments, the difference between the maximum and minimum melting temperatures is less than about 20°C, less than about 15°C, less than about 10°C, or less than about 5°C. In some embodiments, probe sequences may be selected based on experimental determinations of the melting temperatures or calculations of the theoretical melting temperatures. In certain embodiments, putative probe sequences may first be selected based on calculations of their theoretical melting temperatures and then be confirmed experimentally. Methods for determining the melting temperature of nucleic acid duplexes are known in the art. See for example, Sambrook and Russell (2001) Molecular Cloning: A Laboratory Handbook, 10.38-10.41 and 10.47.

A value for melting temperature can be determined mathematically using equations and algorithms known in the art. For duplex oligonucleotides shorter than 25 bp, "The Wallace Rule" can be used in which:

$$\text{Tm (in °C)} = 2(A+T) + 4(C+G), \text{ where}$$

(A+T) - the sum of the A and T residues in the oligonucleotide,
(C+G) - the sum of G and C residues in the oligonucleotide

(see Wallace et al., Nucleic Acids Res. (1979) 6: 3543-3557). Computer programs for estimating Tm are also available (see, e.g., Le Novere, Bioinformatics (2001) 17(12): 1226-1227). VisualOmp (DNA Software, Inc., Ann Arbor, MI) is an example of commercially available software for calculating nucleic acid duplex melting temperature.

As illustrated by the left hand graph of Fig. 3, the Tms of the set of probe sequences that are complementary to the polynucleotides (e.g., miRNAs) of a population of polynucleotides are distributed across a Tm spread (a Tm spread being difference in temperature between the highest and lowest Tm of the set). As illustrated in this graph, the Tms may have an approximate normal distribution and form an approximate bell-shaped curve when plotted as shown. As illustrated in the middle graph of Fig. 3, in designing probes for the polynucleotides, the length of the complementary sequences having a higher Tm is decreased (thereby decreasing the Tm of those sequences) and stability sequences are added to the complementary sequences having a lower Tm (thereby increasing the Tm of those sequences). As illustrated in the right-hand graph of Fig. 3, once the Tms of the population of complementary sequences have been adjusted by reducing the length of the sequences or by adding Tm enhancement domains, the spread of the Tms of the population is significantly reduced. Such a reduction in Tm spread is highly desirable in microarray analysis.

ARRAYS

In certain embodiments of the invention a subject probe is a "surface-bound probe", where such a probe is bound, usually covalently but in certain embodiments non-covalently, to a surface of a solid substrate, e.g., a sheet, bead, or other structure. In certain embodiments, a surface-bound probe may be immobilized on a surface of a planar support, e.g., as part of an array.

A subject array may contain a plurality of features (e.g., 2 or more, about 5 or more, about 10 or more, about 15 or more, about 20 or more, about 30 or more, about 50 or more, about 100 or more, about 200 or more, about 500 or more, about 1000 or more, usually up to about 10,000 or about 20,000 or more features, etc.), each feature containing a capture agent capable of binding a target. In embodiments in accordance with the present invention, the array includes features wherein the capture agents of at least some of the features are probes directed to miRNAs as described above. In certain embodiments, the array may also include features wherein the capture agent of each feature is not directed to a miRNA, e.g. the target of the capture agent is some other target, e.g. mRNA, other small RNA, or other polynucleotide, or the capture agent is a 'control'. In particular embodiments the array has at least five different subject probes attached to the array support. In some embodiments, at least 10, at least 20, at least 40, at least 100, or at least 200 different subject probes are attached to the array support. In certain embodiments, at least 5%, at least 10%, at least 20%, or at least 40% of the features of an array contain a subject probe. As few as one and as many as all of the subject probes of a subject array may contain a Tm enhancement domain. In certain embodiments, at least 5%, at least 10% or at least 20% of the subject . ;: : probes of an array contain a Tm enhancement domain.

The nucleic acids in a feature are a mixture of nucleic acids having different sequences, e.g. two or more different probes are co-located at the same feature.

A subject array typically may have at least five different subject probes, i.e. the array includes a probe set having at least five different subject probes. However, in certain embodiments, a subject array may include a probe set having at least 10, at least 20, at least 50, at least 100, or at least 200 subject probes that are directed to (i.e., may be used to detect) a corresponding number of miRNAs. In particular embodiments, the subject arrays may include probes for detecting at least a portion or all of the identified miRNAs of a particular organism, e.g. human.

In general, methods for the preparation of nucleic acid arrays, particularly oligonucleotide arrays, are well known in the art (see, e.g., Harrington et al,. Curr Opin Microbiol. (2000) 3:285-91, and Lipshutz et al., Nat Genet. (1999) 21: 20-4) and need not be described in any great detail. The subject arrays can be fabricated using any means available, including drop deposition from pulse jets or from fluid-filled tips, etc., or using photolithographic means. Either polynucleotide precursor units (such as nucleotide monomers), in the case of *in situ* fabrication, or previously synthesized polynucleotides can be deposited. Such methods are described in detail in, for example U.S. patents 6,242,266, 6,232,072, 6,180,351; 6,171,797, 6,323,043, etc.

In certain embodiments, an array of the invention may contain probes that all have a similar Tm. The spread of Tms of such arrays may be less than about 10°C, less than about 5°C, or less than about 2°C, for example. The spread of Tms of an array may be theoretically determined, or, in certain embodiments, experimentally determined.

METHODS FOR ASSESSING miRNA IN A SAMPLE

The subject invention provides a method of analysing a sample for miRNA, e.g. assessing for the presence or amount of a miRNA. In general, the method includes the following steps: a) contacting the sample with a array comprising a subject probe set (such as described above) under conditions sufficient for specific binding to occur; and b) interrogating the array to obtain information about miRNAs in the sample. Interrogating the array typically involves detecting the presence of any detectable label associated with the probes of the probe set on the array, thereby evaluating the amount of the respective target miRNAs in the sample.

In embodiments in which a probe containing a nucleotide clamp is employed, miRNAs in a sample containing the miRNAs may be extended to add nucleotides that are complementary to the nucleotide clamp of the nucleic acid probe. The addition of the nucleotides to the miRNAs may be done before, simultaneously with, or after labeling. In representative embodiments, a mononucleotide, di-nucleotide, tri-nucleotide, tetra-nucleotide or penta-nucleotide moiety is added to either the 3' or the 5' ends of the miRNAs (e.g. sample comprising isolated miRNAs) using an enzyme, e.g., an RNA or DNA ligase or terminal transferase. A variety of RNA and DNA ligases may be purchased from a variety of vendors (e.g., Pharmacia, Piscataway, NJ, New England Biolabs, Berverly MA, and Roche Diagnostics, Indianapolis, IN) and employed according to the instructions supplied therewith. In an embodiment of particular interest, the nucleotide (s) added to the miRNA are covalently linked to a label, e.g., a fluorophore, such that the miRNA is labeled by the addition of the nucleotide label moiety. Labeled mononucleotides, di-nucleotides, tri-nucleotides, tetra-nucleotides, penta-nucleotides or higher order labeled polynucleotides are termed "nucleotide label moieties" herein. Further description of such Tm enhancement domains is provided in copending US 2006/0172317 filed on Jan. 31, 2005 by Wang entitled "RNA Labeling Method."

For example, and as illustrated in Fig. 4A, nucleotides ($N^*_{1-5}$) complementary to the nucleotide clamp of the probe are ligated to a terminus of a miRNA to produce an extended miRNA. The extended miRNA hybridizes to a nucleic acid probe containing a nucleotide clamp ($N_{1-5}$). The added nucleotides of the extended miRNA base pair with the clamp of the probe whereas the remainder of the extended miRNA base pair with the target-complementary sequence of the probe. As illustrated in Fig. 4B, nucleotides ($N^*_{1-5}$) complementary to the nucleotide clamp of the probe are ligated to a terminus of a miRNA to produce an extended miRNA. The extended miRNA hybridizes to a probe containing a nucleotide clamp ($N_{1-5}$) and a hairpin. The added nucleotides of the extended miRNA base pair with the clamp of the probe whereas the remainder of the extended miRNA base pairs with the target-complementary sequence of the probe. The coaxial stacking and the nucleotide clamping increase the stability of the probe/target duplex. Finally and with reference to Fig. 4C, an miRNA hybridizes to a probe containing a hairpin structure. The miRNA base pairs with the target-complementary sequence of the probe and coaxial stacking increases the stability of the probe/target duplex.

In certain embodiments a subject array is employed to assess a sample of microRNAs that is prepared from a cell. Methods for preparing samples of miRNAs from cells are well known in the art (see, e.g., Lagos-Quintana et al, Science 294:853-858(2001); Grad et al, Mol Cell 11:1253-1263 (2003); Mourelatos et al, Genes Dev 16:720-728(2002); Lagos-Quintana et al, Curr Biol 12:735-739(2002); Lagos-Quintana et al, RNA 9:175-179(2003) and other references cited above).

The sample is usually labeled to make a population of labeled miRNAs. In general, a sample may be labeled using methods that are well known in the art (e.g., using DNA ligase, terminal transferase, or by labeling the RNA backbone, etc.; see, e.g., Ausubel, et al., Short Protocols in Molecular Biology, 3rd ed., Wiley & Sons 1995 and Sambrook et al., Molecular Cloning: A Laboratory Manual, Third Edition, 2001 Cold Spring Harbor, N.Y.), and, accordingly, such methods do not need to be described here in great detail. In particular embodiments, the sample is usually labeled with fluorescent label, which labels will be described in greater detail below.

Fluorescent dyes of particular interest include: xanthene dyes, e.g. fluorescein and rhodamine dyes, such as fluorescein isothiocyanate (FITC), 6 carboxyfluorescein (commonly known by the abbreviations FAM and F), 6 carboxy-2',4',7',4,7-hexachlorofluorescein (HEX), 6 carboxy 4', 5' dichloro 2', 7' dimethoxyfluorescein (JOE or J), N,N,N',N' tetramethyl 6 carboxyrhodamine (TAMRA or T), 6 carboxy X rhodamine (ROX or R), 5 carboxyrhodamine 6G (R6G5 or G5), 6 carboxyrhodamine 6G (R6G6 or G6), and rhodamine 110; cyanine dyes, e.g. Cy3, Cy5 and Cy7 dyes; Alexa dyes, e.g. Alexa-fluor-555; coumarins, e.g. umbelliferone; benzimide dyes, e.g. Hoechst 33258; phenanthridine dyes, e.g. Texas Red; ethidium dyes; acridine dyes; carbazole dyes; phenoxazine dyes; porphyrin dyes; polymethine dyes, e.g. cyanine dyes such as Cy3, Cy5, etc; BODIPY dyes and quinoline dyes. Specific fluorophores of interest that are commonly used in subject applications include: Pyrene, Coumarin, Diethylaminocoumarin, FAM, Fluorescein Chlorotriazinyl, Fluorescein, R110, Eosin, JOE, R6G, Tetramethylrhodamine, TAMRA, Lissamine, ROX, Napthofluorescein, Texas Red, Napthofluorescein, Cy3, and Cy5, etc.

In some embodiments, after labeling the labeled sample is contacted with a subject probe (e.g. a member of a subject probe set, e.g. of an array comprising the probe set) under stringent hybridization conditions, and any binding of labeled miRNA to a probe is detected by detecting the label associated with the probe.

In certain embodiments, binding of labeled miRNAs in the labeled sample is assessed with respect to binding of at least one control labeled sample. In one example, a suitable control labeled sample may be made from a control cell population, as will be described in greater detail below.

In certain embodiments, a sample and a control sample may be prepared and labeled, and relative binding of the labeled miRNAs in the samples to a subject probe may be assessed. Since the subject probe may be a surface-bound probe that is present at a feature of an array, in many embodiments, the samples are labeled and contacted with at least one array containing a subject probe, under stringent hybridization conditions.

In practicing the subject methods, the samples may be labeled to provide at least two different populations of labeled miRNAs that are to be compared. The populations of miRNAs may be labeled with the same label or different labels, depending on the actual assay protocol employed. For example, where each population is to be contacted with different but identical arrays, each population of miRNAs may be labeled with the same label. Alternatively, where both populations are to be simultaneously contacted with a single array of surface-bound probes, i.e., co-hybridized to the same array of immobilized probes, the two different populations are generally distinguishably labeled with respect to each other.

The samples are sometimes labeled using "distinguishable" labels in that the labels that can be independently detected and measured, even when the labels are mixed. In other words, the amounts of label present (e.g., the amount of fluorescence) for each of the labels are separately determinable, even when the labels are co-located (e.g., in the same tube or in the same duplex molecule or in the same feature of an array). Suitable distinguishable fluorescent label pairs useful in the subject methods include Cy-3 and Cy-5 (Amersham Inc., Piscataway, NJ), Quasar 570 and Quasar 670 (Biosearch Technology, Novato CA), Alexafluor555 and Alexafluor647 (Molecular Probes, Eugene, OR), BODIPY V-1002 and BODIPY V1005 (Molecular Probes, Eugene, OR), POPO-3 and TOTO-3 (Molecular Probes, Eugene, OR), fluorescein and Texas red (Dupont, Bostan MA) and POPRO3 and TOPRO3 (Molecular Probes, Eugene, OR). Further suitable distinguishable detectable labels may be described in Kricka et al. (Ann. Clin. Biochem. 39:114-29, 2002).

Accordingly, in certain embodiments, at least a first population of miRNAs and a second population of miRNAs are produced from two different miRNA-containing samples, e.g., two populations of cells. As indicated above, depending on the particular assay protocol (e.g., whether both populations are to be hybridized simultaneously to a single array or whether each population is to be hybridized to two different but substantially identical, if not identical, arrays) the populations may be labeled with the same or different labels. As such, a feature of certain embodiments is that the different populations of miRNAs are labeled with the same label such that they are not distinguishably labeled. In yet other embodiments, a feature of the different populations of labeled nucleic acids is that the first and second labels are distinguishable from each other.

Generally, the subject methods comprise the following major steps: (1) provision of an array containing surface-bound subject probes; (2) hybridization of a population of labeled miRNAs to the surface-bound probes under conditions sufficient to provide for specific binding, e.g. typically under stringent hybridization conditions; (3) post-hybridization washes to remove nucleic acids not bound in the hybridization; and (4) detection of the hybridized miRNAs. The reagents used in each of these steps and their conditions for use may vary depending on the particular application.

As indicated above, hybridization is carried out under suitable hybridization conditions, which may vary in stringency as desired, typical conditions are sufficient to produce probe/target complexes on an array surface between complementary binding members, i.e., between surface-bound subject probes and complementary labeled miRNAs in a sample. In certain embodiments, stringent hybridization conditions may be employed. Representative stringent hybridization conditions that may be employed in these embodiments are provided above.

Thus, after nucleic acid purification of labeled miRNAs from unincorporated label, the populations of labeled miRNAs are usually contacted with an array of surface-bound probes, as discussed above, under conditions such that nucleic acid hybridization to the surface-bound probes can occur, e.g., in a buffer containing 50% formamide, 5X SSC and 1% SDS at 42°C, or in a buffer containing 5X SSC and 1% SDS at 65°C, both with a wash of 0.2X SSC and 0.1% SDS at 65°C, for example.

The above hybridization step may include agitation of the surface-bound probes and the sample of labeled miRNAs, where the agitation may be accomplished using any convenient protocol, e.g., shaking, rotating, spinning, and the like.

Standard hybridization techniques (e.g. under conditions sufficient to provide for specific binding of target miRNAs in the sample to the probes on the array) are used to hybridize a sample to a nucleic acid array. Suitable methods are described in many references (e.g., Kallioniemi et al., Science 258:818-821 (1992) and WO 93/18186). Several guides to general techniques are available, e.g., Tijssen, Hybridization with Nucleic Acid Probes, Parts I and II (Elsevier, Amsterdam 1993). For descriptions of techniques suitable for in situ hybridizations, see Gall et al. Meth. Enzymol., 21:470-480 (1981); and Angerer et al. in Genetic Engineering: Principles and Methods (Setlow and Hollaender, Eds.) Vol 7, pgs 43-65 (Plenum Press, New York 1985). See also United States Patent Nos: 6,335,167; 6,197,501; 5,830,645; and 5,665,549. Hybridizing the sample to the array is typically performed under stringent hybridization conditions, as described herein and as known in the art. Selection of appropriate conditions, including temperature, salt concentration, polynucleotide concentration, time(duration) of hybridization, stringency of washing conditions, and the like will depend on experimental design, including source of sample, identity of capture agents, degree of complementarity expected, etc., and may be determined as a matter of routine experimentation for those of ordinary skill in the art.

Following hybridization, the array-surface bound polynucleotides are typically washed to remove unbound nucleic acids. Washing may be performed using any convenient washing protocol, where the washing conditions are typically stringent, as described above.

Following hybridization and washing, as described above, the hybridization of the target miRNAs to the probes is then detected using standard techniques of reading the array, i.e. the array is interrogated. Reading the resultant hybridized array may be accomplished by illuminating the array and reading the location and intensity of resulting fluorescence at each feature of the array to detect any binding complexes (e.g. probe/target duplexes) on the surface of the array. For example, a scanner may be used for this purpose that is similar to the AGILENT MICROARRAY SCANNER available from Agilent Technologies, Palo Alto, CA. Other suitable devices and methods are described in U.S. Pat. No. 6,756,202 and U.S. Pat. No. 6,406,849. However, arrays may be read by any other method or apparatus than the foregoing, with other reading methods including other optical techniques (for example, detecting chemiluminescent or electroluminescent labels) or electrical, techniques (where each feature is provided with an electrode to detect hybridization at that feature in a manner disclosed in U.S. Pat. 6,221,583 and elsewhere). In the case of indirect labelling, subsequent treatment of the array with the appropriate reagents may be employed to enable reading of the array. Some methods of detection, such as surface plasmon resonance, do not require any labeling of nucleic acids, and are suitable for some embodiments.

Results from the reading or evaluating may be raw results (such as fluorescence intensity readings for each feature in one or more color channels) or may be processed results (such as those obtained by subtracting a background measurement, or by rejecting a reading for a feature which is below a predetermined threshold, normalizing the results, and/or forming conclusions based on the pattern read from the array (such as whether or not a particular target sequence may have been present in the sample, or whether or not a pattern indicates a particular condition of an organism from which the sample came).

By "normalization" is meant that data corresponding to the two populations of polynucleotides are globally normalized to each other, and/or normalized to data obtained from controls (e.g., internal controls produce data that are predicted to equal in value in all of the data groups). Normalization generally involves multiplying each numerical value for one data group by a value that allows the direct comparison of those amounts to amounts in a second data group. Several normalization strategies have been described (Quackenbush et al, Nat Genet. 32 Suppl:496-501, 2002, Bilban et al Curr Issues Mol Biol. 4:57-64, 2002, Finkelstein et al, Plant Mol Biol.48(1-2):119-31, 2002, and Hegde et al, Biotechniques. 29:548-554, 2000). Specific examples of normalization suitable for use in the subject methods include linear normalization methods, non-linear normalization methods, e.g., using lowest local regression to paired data as a function of signal intensity, signal-dependent non-linear normalization, qspline normalization and spatial normalization, as described in Workman et al., (Genome Biol. 2002 3, 1-16). In certain embodiments, the numerical value associated with a feature signal is converted into a log number, either before or after normalization occurs. Data may be normalized to data obtained using a support-bound polynucleotide probe for a polynucleotide of known concentration, for example.

In certain embodiments, results from interrogating the array are used to assess the level of binding of the population of miRNAs from the sample to subject probes on the array. The term "level of binding" means any assessment of binding (e.g. a quantitative or qualitative, relative or absolute assessment), usually done, as is known in the art, by detecting signal (i.e., pixel brightness) from a label associated with the sample miRNA, e.g. the sample is labeled. The level of binding of labeled miRNA to probe is typically obtained by measuring the surface density of the bound label (or of a signal resulting from the label). Accordingly, since the arrays used in the subject assays may contain probes for a plurality of different miRNAs, the presence of a plurality of different miRNAs in a sample may be assessed. The subject methods are therefore suitable for simultaneous assessment of a plurality of miRNAs in a sample.

In certain embodiments, a surface-bound probe may be assessed by evaluating its binding to two populations of nucleic acids that are distinguishably labeled. In these embodiments, for a single surface-bound probe of interest, the results obtained from hybridization with a first population of labeled nucleic acids may be compared to results obtained from hybridization with the second population of nucleic acids, usually after normalization of the data. The results may be expressed using any convenient means, e.g., as a number or numerical ratio, etc.

In typical embodiments of methods in accordance with the present invention, an isolated RNA sample may be labeled, e.g. with Cy5 or Cy3, and hybridized onto an array as follows: The labeled RNA is desalted (e.g. with BioRad MICRO BIO-SPIN™ 6 columns, as directed by BioRad instructions) to remove excess observable label remaining from the labeling reaction. The desalted sample of RNA is added to solution containing water and carrier (25-mer DNA with random sequence). The resulting solution is heated at about 100° C for approximately 1 minute per 10 microliters of solution, and then immediately cooled on ice. The cooled solution is then added to hybridization buffer and mixed carefully. The final solution is then contacted with the array, e.g. in a SUREHYB hybridization chamber (Agilent Part Number:G2534A), and placed on rotisserie of hybridization oven overnight. The hybridization temperature is typically in the range from about 50° C to about 60° C, or in the range from about 55° C to about 60° C,, although temperatures outside this range (e.g. in the range from about 30° C to about 65° C, or in the range from about 45° C to about 65° C) may be used depending on the other experimental parameters, e.g. hybridization buffer composition and wash conditions. After the hybridization is complete, the array is washed thoroughly and dried with nitrogen as needed. The array is scanned (e.g. with an Agilent Scanner, Agilent Product Number: G2565BA). The data is then evaluated (e.g. using Agilent Feature Extraction Software, Agilent Product Number: G2567AA) for hybridization efficiency and specificity. Data may be further analyzed, e.g. using Spotfire software and Microsoft Excel.

Also provided by the subject invention are kits for practicing the subject methods, as described above. The subject kits include at least a probe set, as described above. For example, a kit may include an array support having the probe set attached to the surface of the array support. In certain embodiments the subject kits may also include reagents for isolating RNA from a source to provide an isolated sample of RNA. In some embodiments the subject kits optionally also include one or more constituents selected from reagents for labeling RNA, reagents for contacting the sample of RNA with the probe set (e.g., enzymes for use with the subject methods such as described above, control samples, reagents for performing an array hybridization, combinations thereof, etc.) The various components of the kit may be present in separate containers or certain compatible components may be precombined into a single container, as desired.

In addition to above-mentioned components, the subject kits may further include instructions for using the components of the kit to practice the subject methods, i.e., to instructions for sample analysis. The instructions for practicing the subject methods are generally recorded on a suitable recording medium. For example, the instructions may be printed on a suitable material, such as paper or plastic, etc. As such, the instructions may be present in the kits as a package insert, in the labeling of the container of the kit or components thereof (i.e., associated with the packaging or subpackaging) etc. In other embodiments, the instructions are present as an electronic storage data file present on a suitable computer readable storage medium, e.g., CD-ROM, diskette, etc. In yet other embodiments, the actual instructions are not present in the kit, but means for obtaining the instructions from a remote source, e.g., via the internet, are provided. An example of this embodiment is a kit that includes a web address where the instructions can be viewed and/or from which the instructions can be downloaded. As with the instructions, this means for obtaining the instructions is recorded on a suitable material.

The practice of the present invention will employ, unless otherwise indicated, conventional techniques of synthetic organic chemistry, biochemistry, molecular biology, and the like, which are within the skill of the art. Such techniques are explained fully in the literature. Unless otherwise defined herein, all technical and scientific terms used herein have the same meaning as commonly understood by one of ordinary skill in the art to which the invention belongs. The description herein is set forth so as to provide those of ordinary skill in the art with a complete disclosure and description of how to perform the methods and use the compositions disclosed and claimed herein. Efforts have been made to ensure accuracy with respect to numbers (e.g., amounts, temperature, etc.) but some errors and deviations should be accounted for. Unless indicated otherwise, parts are parts by weight, temperature is in °C and pressure is at or near atmospheric. Standard temperature and pressure are defined as 20° C and 1 atmosphere. Some known sequences of miRNA are listed in Table 4.

Table 4

| SEQ ID NO: | Sequence | Name |
|---|---|---|
| 1268 | UGAGGUAGUAGGUUGUAUAGUU | hsa-let-7a |
| 1269 | UGAGGUAGUAGGUUGUGUGGUU | hsa-let-7b |
| 1270 | UGAGGUAGUAGGUUGUAUGGUU | hsa-let-7c |
| 1271 | AGAGGUAGUAGGUUGCAUAGU | hsa-let-7d |
| 1272 | UGAGGUAGGAGGUUGUAUAGU | hsa-let-7e |
| 1273 | UGAGGUAGUAGAUUGUAUAGUU | hsa-let-7f |
| 1274 | UGAGGUAGUAGUUUGUACAGU | hsa-let-7g |
| 1275 | UGAGGUAGUAGUUUGUGCUGU | hsa-let-7i |
| 1276 | UGGAAUGUAAAGAAGUAUGUA | hsa-miR-1 |
| 1277 | AACCCGUAGAUCCGAACUUGUG | hsa-miR-100 |
| 1278 | UACAGUACUGUGAUAACUGAAG | hsa-miR-101 |
| 1279 | AGCAGCAUUGUACAGGGCUAUGA | hsa-miR-103 |
| 1280 | UCAAAUGCUCAGACUCCUGU | hsa-miR-105 |
| 1281 | AAAAGUGCUUACAGUGCAGGUAGC | hsa-miR-106a |
| 1282 | UAAAGUGCUGACAGUGCAGAU | hsa-miR-106b |
| 1283 | AGCAGCAUUGUACAGGGCUAUCA | hsa-miR-107 |
| 1284 | AUAAGGAUUUUUAGGGGCAUU | hsa-miR-108 |
| 1285 | UACCCUGUAGAUCCGAAUUUGUG | hsa-miR-10a |
| 1286 | UACCCUGUAGAACCGAAUUUGU | hsa-miR-10b |
| 1287 | UGGAGUGUGACAAUGGUGUUUGU | hsa-miR-122a |
| 1288 | UUAAGGCACGCGGUGAAUGCCA | hsa-miR-124a |
| 1289 | UCCCUGAGACCCUUUAACCUGUG | hsa-miR-125a |
| 1290 | UCCCUGAGACCCUAACUUGUGA | hsa-miR-125b |
| 1291 | UCGUACCGUGAGUAAUAAUGC | hsa-miR-126 |
| 1292 | CAUUAUUACUUUUGGUACGCG | hsa-miR-126* |
| 1293 | UCGGAUCCGUCUGAGCUUGGCU | hsa-miR-127 |
| 1294 | UCACAGUGAACCGGUCUCUUUU | hsa-miR-128a |
| 1295 | UCACAGUGAACCGGUCUCUUUC | hsa-miR-128b |
| 1296 | CUUUUUGCGGUCUGGGCUUGC | hsa-miR-129 |
| 1297 | CAGUGCAAUGUUAAAAGGGCAU | hsa-miR-130a |
| 1298 | CAGUGCAAUGAUGAAAGGGCAU | hsa-miR-130b |
| 1299 | UAACAGUCUACAGCCAUGGUCG | hsa-miR-132 |
| 1300 | UUGGUCCCCUUCAACCAGCUGU | hsa-miR-133a |
| 1301 | UUGGUCCCCUUCAACCAGCUA | hsa-miR-133b |
| 1302 | UGUGACUGGUUGACCAGAGGG | hsa-miR-134 |
| 1303 | UAUGGCUUUUUAUUCCUAUGUGA | hsa-miR-135a |
| 1304 | UAUGGCUUUUCAUUCCUAUGUG | hsa-miR-135b |
| 1305 | ACUCCAUUUGUUUUGAUGAUGGA | hsa-miR-136 |
| 1306 | UAUUGCUUAAGAAUACGCGUAG | hsa-miR-137 |
| 1307 | AGCUGGUGUUGUGAAUC | hsa-miR-138 |
| 1308 | UCUACAGUGCACGUGUCU | hsa-miR-139 |
| 1309 | AGUGGUUUUACCCUAUGGUAG | hsa-miR-140 |
| 1310 | UAACACUGUCUGGUAAAGAUGG | hsa-miR-141 |
| 1311 | UGUAGUGUUUCCUACUUUAUGGA | hsa-miR-142-3p |
| 1312 | CAUAAAGUAGAAAGCACUAC | hsa-miR-142-5p |
| 1313 | UGAGAUGAAGCACUGUAGCUCA | hsa-miR-143 |
| 1314 | UACAGUAUAGAUGAUGUACUAG | hsa-miR-144 |

(continued)

| SEQ ID NO: | Sequence | Name |
|---|---|---|
| 1315 | GUCCAGUUUUCCCAGGAAUCCCUU | hsa-miR-145 |
| 1316 | UGAGAACUGAAUUCCAUGGGUU | hsa-miR-146a |
| 1317 | UGAGAACUGAAUUCCAUAGGCU | hsa-miR-146b |
| 1318 | GUGUGUGGAAAUGCUUCUGC | hsa-miR-147 |
| 1319 | UCAGUGCACUACAGAACUUUGU | hsa-miR-148a |
| 1320 | UCAGUGCAUCACAGAACUUUGU | hsa-miR-148b |
| 1321 | UCUGGCUCCGUGUCUUCACUCC | hsa-miR-149 |
| 1322 | UCUCCCAACCCUUGUACCAGUG | hsa-miR-150 |
| 1323 | ACUAGACUGAAGCUCCUUGAGG | hsa-miR-151 |
| 1324 | UCAGUGCAUGACAGAACUUGGG | hsa-miR-152 |
| 1325 | UUGCAUAGUCACAAAAGUGA | hsa-miR-153 |
| 1326 | UAGGUUAUCCGUGUUGCCUUCG | hsa-miR-154 |
| 1327 | AAUCAUACACGGUUGACCUAUU | hsa-miR-154* |
| 1328 | UUAAUGCUAAUCGUGAUAGGGG | hsa-miR-155 |
| 1329 | UAGCAGCACAUAAUGGUUUGUG | hsa-miR-15a |
| 1330 | UAGCAGCACAUCAUGGUUUACA | hsa-miR-15b |
| 1331 | UAGCAGCACGUAAAUAUUGGCG | hsa-miR-16 |
| 1332 | ACUGCAGUGAAGGCACUUGU | hsa-miR-17-3p |
| 1333 | CAAAGUGCUUACAGUGCAGGUAGU | hsa-miR-17-5p |
| 1334 | AACAUUCAACGCUGUCGGUGAGU | hsa-miR-181a |
| 1335 | AACAUUCAUUGCUGUCGGUGGG | hsa-miR-181b |
| 1336 | AACAUUCAACCUGUCGGUGAGU | hsa-miR-181c |
| 1337 | AACAUUCAUUGUUGUCGGUGGGUU | hsa-miR-181d |
| 1338 | UUUGGCAAUGGUAGAACUCACA | hsa-miR-182 |
| 1339 | UGGUUCUAGACUUGCCAACUA | hsa-miR-182* |
| 1340 | UAUGGCACUGGUAGAAUUCACUG | hsa-miR-183 |
| 1341 | UGGACGGAGAACUGAUAAGGGU | hsa-miR-184 |
| 1342 | UGGAGAGAAAGGCAGUUC | hsa-miR-185 |
| 1343 | CAAAGAAUUCUCCUUUUGGGCUU | hsa-miR-186 |
| 1344 | UCGUGUCUUGUGUUGCAGCCG | hsa-miR-187 |
| 1345 | CAUCCCUUGCAUGGUGGAGGGU | hsa-miR-188 |
| 1346 | GUGCCUACUGAGCUGAUAUCAGU | hsa-miR-189 |
| 1347 | UAAGGUGCAUCUAGUGCAGAUA | hsa-miR-18a |
| 1348 | UAAGGUGCAUCUAGUGCAGUUA | hsa-miR-18b |
| 1349 | UGAUAUGUUUGAUAUAUUAGGU | hsa-miR-190 |
| 1350 | CAACGGAAUCCCAAAAGCAGCU | hsa-miR-191 |
| 1351 | GCUGCGCUUGGAUUUCGUCCCC | hsa-miR-191* |
| 1352 | CUGACCUAUGAAUUGACAGCC | hsa-miR-192 |
| 1353 | AACUGGCCUACAAAGUCCCAG | hsa-miR-193a |
| 1354 | AACUGGCCCUCAAAGUCCCGCUUU | hsa-miR-193b |
| 1355 | UGUAACAGCAACUCCAUGUGGA | hsa-miR-194 |
| 1356 | UAGCAGCACAGAAAUAUUGGC | hsa-miR-195 |
| 1357 | UAGGUAGUUUCAUGUUGUUGG | hsa-miR-196a |
| 1358 | UAGGUAGUUUCCUGUUGUUGG | hsa-miR-196b |
| 1359 | UUCACCACCUUCUCCACCCAGC | hsa-miR-197 |
| 1360 | GGUCCAGAGGGGAGAUAGG | hsa-miR-198 |
| 1361 | UACAGUAGUCUGCACAUUGGUU | hsa-miR-199a* |
| 1362 | CCCAGUGUUCAGACUACCUGUUC | hsa-miR-199a |
| 1363 | CCCAGUGUUUAGACUAUCUGUUC | hsa-miR-199b |
| 1364 | UGUGCAAAUCUAUGCAAAACUGA | hsa-miR-19a |

(continued)

| SEQ ID NO: | Sequence | Name |
|---|---|---|
| 1365 | UGUGCAAAUCCAUGCAAAACUGA | hsa-miR-19b |
| 1366 | CAUCUUACCGGACAGUGCUGGA | hsa-miR-200a* |
| 1367 | UAACACUGUCUGGUAACGAUGU | hsa-miR-200a |
| 1368 | UAAUACUGCCUGGUAAUGAUGAC | hsa-miR-200b |
| 1369 | UAAUACUGCCGGGUAAUGAUGG | hsa-miR-200c |
| 1370 | AGAGGUAUAGGGCAUGGGAAAA | hsa-miR-202 |
| 1371 | UUUCCUAUGCAUAUACUUCUUU | hsa-miR-202* |
| 1372 | GUGAAAUGUUUAGGACCACUAG | hsa-miR-203 |
| 1373 | UUCCCUUUGUCAUCCUAUGCCU | hsa-miR-204 |
| 1374 | UCCUUCAUUCCACCGGAGUCUG | hsa-miR-205 |
| 1375 | UGGAAUGUAAGGAAGUGUGUGG | hsa-miR-206 |
| 1376 | AUAAGACGAGCAAAAAGCUUGU | hsa-miR-208 |
| 1377 | UAAAGUGCUUAUAGUGCAGGUAG | hsa-miR-20a |
| 1378 | CAAAGUGCUCAUAGUGCAGGUAG | hsa-miR-20b |
| 1379 | UAGCUUAUCAGACUGAUGUUGA | hsa-miR-21 |
| 1380 | CUGUGCGUGUGACAGCGGCUGA | hsa-miR-210 |
| 1381 | UUCCCUUUGUCAUCCUUCGCCU | hsa-miR-211 |
| 1382 | UAACAGUCUCCAGUCACGGCC | hsa-miR-212 |
| 1383 | ACCAUCGACCGUUGAUUGUACC | hsa-miR-213 |
| 1384 | ACAGCAGGCACAGACAGGCAG | hsa-miR-214 |
| 1385 | AUGACCUAUGAAUUGACAGAC | hsa-miR-215 |
| 1386 | UAAUCUCAGCUGGCAACUGUG | hsa-miR-216 |
| 1387 | UACUGCAUCAGGAACUGAUUGGAU | hsa-miR-217 |
| 1388 | UUGUGCUUGAUCUAACCAUGU | hsa-miR-218 |
| 1389 | UGAUUGUCCAAACGCAAUUCU | hsa-miR-219 |
| 1390 | AAGCUGCCAGUUGAAGAACUGU | hsa-miR-22 |
| 1391 | CCACACCGUAUCUGACACUUU | hsa-miR-220 |
| 1392 | AGCUACAUUGUCUGCUGGGUUUC | hsa-miR-221 |
| 1393 | AGCUACAUCUGGCUACUGGGUCUC | hsa-miR-222 |
| 1394 | UGUCAGUUUGUCAAAUACCCC | hsa-miR-223 |
| 1395 | CAAGUCACUAGUGGUUCCGUUUA | hsa-miR-224 |
| 1396 | AUCACAUUGCCAGGGAUUUCC | hsa-miR-23a |
| 1397 | AUCACAUUGCCAGGGAUUACC | hsa-miR-23b |
| 1398 | UGGCUCAGUUCAGCAGGAACAG | hsa-miR-24 |
| 1399 | CAUUGCACUUGUCUCGGUCUGA | hsa-miR-25 |
| 1400 | UUCAAGUAAUCCAGGAUAGGC | hsa-miR-26a |
| 1401 | UUCAAGUAAUUCAGGAUAGGUU | hsa-miR-26b |
| 1402 | UUCACAGUGGCUAAGUUCCGC | hsa-miR-27a |
| 1403 | UUCACAGUGGCUAAGUUCUGC | hsa-miR-27b |
| 1404 | AAGGAGCUCACAGUCUAUUGAG | hsa-miR-28 |
| 1405 | AGGGCCCCCCCUCAAUCCUGU | hsa-miR-296 |
| 1406 | UAUGUGGGAUGGUAAACCGCUU | hsa-miR-299-3p |
| 1407 | UGGUUUACCGUCCCACAUACAU | hsa-miR-299-5p |
| 1408 | UAGCACCAUCUGAAAUCGGUU | hsa-miR-29a |
| 1409 | UAGCACCAUUUGAAAUCAGUGUU | hsa-miR-29b |
| 1410 | UAGCACCAUUUGAAAUCGGU | hsa-miR-29c |
| 1411 | CAGUGCAAUAGUAUUGUCAAAGC | hsa-miR-301 |
| 1412 | UAAACGUGGAUGUACUUGCUUU | hsa-miR-302a* |
| 1413 | ACUUUAACAUGGAAGUGCUUUCU | hsa-miR-302b* |
| 1414 | UUUAACAUGGGGGUACCUGCUG | hsa-miR-302c* |

(continued)

| SEQ ID NO: | Sequence | Name |
|---|---|---|
| 1415 | UAAGUGCUUCCAUGUUUUGGUGA | hsa-miR-302a |
| 1416 | UAAGUGCUUCCAUGUUUUAGUAG | hsa-miR-302b |
| 1417 | UAAGUGCUUCCAUGUUUCAGUGG | hsa-miR-302c |
| 1418 | UAAGUGCUUCCAUGUUUGAGUGU | hsa-miR-302d |
| 1419 | CUUUCAGUCGGAUGUUUGCAGC | hsa-miR-30a-3p |
| 1420 | UGUAAACAUCCUCGACUGGAAG | hsa-miR-30a-5p |
| 1421 | UGUAAACAUCCUACACUCAGCU | hsa-miR-30b |
| 1422 | UGUAAACAUCCUACACUCUCAGC | hsa-miR-30c |
| 1423 | UGUAAACAUCCCCGACUGGAAG | hsa-miR-30d |
| 1424 | CUUUCAGUCGGAUGUUUACAGC | hsa-miR-30e-3p |
| 1425 | UGUAAACAUCCUUGACUGGA | hsa-miR-30e-5p |
| 1426 | GGCAAGAUGCUGGCAUAGCUG | hsa-miR-31 |
| 1427 | UAUUGCACAUUACUAAGUUGC | hsa-miR-32 |
| 1428 | AAAAGCUGGGUUGAGAGGGCGAA | hsa-miR-320 |
| 1429 | GCACAUUACACGGUCGACCUCU | hsa-miR-323 |
| 1430 | CCACUGCCCCAGGUGCUGCUGG | hsa-miR-324-3p |
| 1431 | CGCAUCCCCUAGGGCAUUGGUGU | hsa-miR-324-5p |
| 1432 | CCUAGUAGGUGUCCAGUAAGUGU | hsa-miR-325 |
| 1433 | CCUCUGGGCCCUUCCUCCAG | hsa-miR-326 |
| 1434 | CUGGCCCUCUCUGCCCUUCCGU | hsa-miR-328 |
| 1435 | AACACACCUGGUUAACCUCUUU | hsa-miR-329 |
| 1436 | GUGCAUUGUAGUUGCAUUG | hsa-miR-33 |
| 1437 | GCAAAGCACACGGCCUGCAGAGA | hsa-miR-330 |
| 1438 | GCCCCUGGGCCUAUCCUAGAA | hsa-miR-331 |
| 1439 | UCAAGAGCAAUAACGAAAAAUGU | hsa-miR-335 |
| 1440 | UCCAGCUCCUAUAUGAUGCCUUU | hsa-miR-337 |
| 1441 | UCCAGCAUCAGUGAUUUUGUUGA | hsa-miR-338 |
| 1442 | UCCCUGUCCUCCAGGAGCUCA | hsa-miR-339 |
| 1443 | UCCGUCUCAGUUACUUUAUAGCC | hsa-miR-340 |
| 1444 | UCUCACACAGAAAUCGCACCCGUC | hsa-miR-342 |
| 1445 | UGCUGACUCCUAGUCCAGGGC | hsa-miR-345 |
| 1446 | UGUCUGCCCGCAUGCCUGCCUCU | hsa-miR-346 |
| 1447 | UGGCAGUGUCUUAGCUGGUUGUU | hsa-miR-34a |
| 1448 | UAGGCAGUGUCAUUAGCUGAUUG | hsa-miR-34b |
| 1449 | AGGCAGUGUAGUUAGCUGAUUGC | hsa-miR-34c |
| 1450 | UUAUCAGAAUCUCCAGGGGUAC | hsa-miR-361 |
| 1451 | AAUCCUUGGAACCUAGGUGUGAG | hsa-miR-362 |
| 1452 | AUUGCACGGUAUCCAUCUGUAA | hsa-miR-363 |
| 1453 | UAAUGCCCCUAAAAAUCCUUAU | hsa-miR-365 |
| 1454 | AAUUGCACUUUAGCAAUGGUGA | hsa-miR-367 |
| 1455 | ACAUAGAGGAAAUUCCACGUUU | hsa-miR-368 |
| 1456 | AAUAAUACAUGGUUGAUCUUU | hsa-miR-369-3p |
| 1457 | AGAUCGACCGUGUUAUAUUCGC | hsa-miR-369-5p |
| 1458 | GCCUGCUGGGGUGGAACCUGG | hsa-miR-370 |
| 1459 | GUGCCGCCAUCUUUUGAGUGU | hsa-miR-371 |
| 1460 | AAAGUGCUGCGACAUUUGAGCGU | hsa-miR-372 |
| 1461 | GAAGUGCUUCGAUUUUGGGGUGU | hsa-miR-373 |
| 1462 | ACUCAAAAUGGGGGCGCUUUCC | hsa-miR-373* |
| 1463 | UUAUAAUACAACCUGAUAAGUG | hsa-miR-374 |
| 1464 | UUUGUUCGUUCGGCUCGCGUGA | hsa-miR-375 |

(continued)

| SEQ ID NO: | Sequence | Name |
|---|---|---|
| 1465 | AUCAUAGAGGAAAAUCCACGU | hsa-miR-376a |
| 1466 | AUCAUAGAGGAAAAUCCAUGUU | hsa-miR-376b |
| 1467 | AUCACACAAAGGCAACUUUUGU | hsa-miR-377 |
| 1468 | CUCCUGACUCCAGGUCCUGUGU | hsa-miR-378 |
| 1469 | UGGUAGACUAUGGAACGUA | hsa-miR-379 |
| 1470 | UAUGUAAUAUGGUCCACAUCUU | hsa-miR-380-3p |
| 1471 | UGGUUGACCAUAGAACAUGCGC | hsa-miR-380-5p |
| 1472 | UAUACAAGGGCAAGCUCUCUGU | hsa-miR-381 |
| 1473 | GAAGUUGUUCGUGGUGGAUUCG | hsa-miR-382 |
| 1474 | AGAUCAGAAGGUGAUUGUGGCU | hsa-miR-383 |
| 1475 | AUUCCUAGAAAUUGUUCAUA | hsa-miR-384 |
| 1476 | CGAAUGUUGCUCGGUGAACCCCU | hsa-miR-409-3p |
| 1477 | AGGUUACCCGAGCAACUUUGCA | hsa-miR-409-5p |
| 1478 | AAUAUAACACAGAUGGCCUGUU | hsa-miR-410 |
| 1479 | ACUUCACCUGGUCCACUAGCCGU | hsa-miR-412 |
| 1480 | CUGGACUUAGGGUCAGAAGGCC | hsa-miR-422a |
| 1481 | CUGGACUUGGAGUCAGAAGGCC | hsa-miR-422b |
| 1482 | AGCUCGGUCUGAGGCCCCUCAG | hsa-miR-423 |
| 1483 | CAGCAGCAAUUCAUGUUUUGAA | hsa-miR-424 |
| 1484 | AUCGGGAAUGUCGUGUCCGCC | hsa-miR-425 |
| 1485 | UAAUACUGUCUGGUAAAACCGU | hsa-miR-429 |
| 1486 | UGUCUUGCAGGCCGUCAUGCA | hsa-miR-431 |
| 1487 | UCUUGGAGUAGGUCAUUGGGUGG | hsa-miR-432 |
| 1488 | CUGGAUGGCUCCUCCAUGUCU | hsa-miR-432* |
| 1489 | AUCAUGAUGGGCUCCUCGGUGU | hsa-miR-433 |
| 1490 | UUGCAUAUGUAGGAUGUCCCAU | hsa-miR-448 |
| 1491 | UGGCAGUGUAUUGUUAGCUGGU | hsa-miR-449 |
| 1492 | UUUUUGCGAUGUGUUCCUAAUA | hsa-miR-450 |
| 1493 | AAACCGUUACCAUUACUGAGUUU | hsa-miR-451 |
| 1494 | UGUUUGCAGAGGAAACUGAGAC | hsa-miR-452 |
| 1495 | UCAGUCUCAUCUGCAAAGAAG | hsa-miR-452* |
| 1496 | GAGGUUGUCCGUGGUGAGUUCG | hsa-miR-453 |
| 1497 | GUCAUACACGGCUCUCCUCU | hsa-miR-485-3p |
| 1498 | AGAGGCUGGCCGUGAUGAAUUC | hsa-miR-485-5p |
| 1499 | CCCAGAUAAUGGCACUCUCAA | hsa-miR-488 |
| 1500 | AGUGACAUCACAUAUACGGCAGC | hsa-miR-489 |
| 1501 | CAACCUGGAGGACUCCAUGCUG | hsa-miR-490 |
| 1502 | AGUGGGGAACCCUUCCAUGAGGA | hsa-miR-491 |
| 1503 | AGGACCUGCGGGACAAGAUUCUU | hsa-miR-492 |
| 1504 | UUGUACAUGGUAGGCUUUCAUU | hsa-miR-493 |
| 1505 | UGAAACAUACACGGGAAACCUCUU | hsa-miR-494 |
| 1506 | AAACAAACAUGGUGCACUUCUUU | hsa-miR-495 |
| 1507 | AUUACAUGGCCAAUCUC | hsa-miR-496 |
| 1508 | CAGCAGCACACUGUGGUUUGU | hsa-miR-497 |
| 1509 | UUUCAAGCCAGGGGGCGUUUUUC | hsa-miR-498 |
| 1510 | UUAAGACUUGCAGUGAUGUUUAA | hsa-miR-499 |
| 1511 | AUGCACCUGGGCAAGGAUUCUG | hsa-miR-500 |
| 1512 | AAUCCUUUGUCCCUGGGUGAGA | hsa-miR-501 |
| 1513 | AUCCUUGCUAUCUGGGUGCUA | hsa-miR-502 |
| 1514 | UAGCAGCGGGAACAGUUCUGCAG | hsa-miR-503 |

(continued)

| SEQ ID NO: | Sequence | Name |
|---|---|---|
| 1515 | AGACCCUGGUCUGCACUCUAU | hsa-miR-504 |
| 1516 | GUCAACACUUGCUGGUUUCCUC | hsa-miR-505 |
| 1517 | UAAGGCACCCUUCUGAGUAGA | hsa-miR-506 |
| 1518 | UUUUGCACCUUUUGGAGUGAA | hsa-miR-507 |
| 1519 | UGAUUGUAGCCUUUUGGAGUAGA | hsa-miR-508 |
| 1520 | UGAUUGGUACGUCUGUGGGUAGA | hsa-miR-509 |
| 1521 | UACUCAGGAGAGUGGCAAUCACA | hsa-miR-510 |
| 1522 | GUGUCUUUUGCUCUGCAGUCA | hsa-miR-511 |
| 1523 | AAGUGCUGUCAUAGCUGAGGUC | hsa-miR-512-3p |
| 1524 | CACUCAGCCUUGAGGGCACUUUC | hsa-miR-512-5p |
| 1525 | UUCACAGGGAGGUGUCAUUUAU | hsa-miR-513 |
| 1526 | AUUGACACUUCUGUGAGUAG | hsa-miR-514 |
| 1527 | GAGUGCCUUCUUUUGGAGCGU | hsa-miR-515-3p |
| 1528 | UUCUCCAAAAGAAAGCACUUUCUG | hsa-miR-515-5p |
| 1529 | UGCUUCCUUUCAGAGGGU | hsa-miR-516-3p |
| 1530 | AUCUGGAGGUAAGAAGCACUUU | hsa-miR-516-5p |
| 1531 | CCUCUAGAUGGAAGCACUGUCU | hsa-miR-517* |
| 1532 | AUCGUGCAUCCCUUUAGAGUGUU | hsa-miR-517a |
| 1533 | UCGUGCAUCCCUUUAGAGUGUU | hsa-miR-517b |
| 1534 | AUCGUGCAUCCUUUUAGAGUGU | hsa-miR-517c |
| 1535 | UCUGCAAAGGGAAGCCCUUU | hsa-miR-518a-2* |
| 1536 | UCUCUGGAGGGAAGCACUUUCUG | hsa-miR-518c* |
| 1537 | CUCUAGAGGGAAGCACUUUCUCU | hsa-miR-518f* |
| 1538 | AAAGCGCUUCCCUUUGCUGGA | hsa-miR-518a |
| 1539 | CAAAGCGCUCCCCUUUAGAGGU | hsa-miR-518b |
| 1540 | CAAAGCGCUUCUCUUUAGAGUG | hsa-miR-518c |
| 1541 | CAAAGCGCUUCCCUUUGGAGC | hsa-miR-518d |
| 1542 | AAAGCGCUUCCCUUCAGAGUGU | hsa-miR-518e |
| 1543 | AAAGCGCUUCUCUUUAGAGGA | hsa-miR-518f |
| 1544 | UUCUCCAAAAGGGAGCACUUUC | hsa-miR-519e* |
| 1545 | AAAGUGCAUCCUUUUAGAGUGUUAC | hsa-miR-519a |
| 1546 | AAAGUGCAUCCUUUUAGAGGUUU | hsa-miR-519b |
| 1547 | AAAGUGCAUCUUUUUAGAGGAU | hsa-miR-519c |
| 1548 | CAAAGUGCCUCCCUUUAGAGUGU | hsa-miR-519d |
| 1549 | AAAGUGCCUCCUUUUAGAGUGU | hsa-miR-519e |
| 1550 | CUCCAGAGGGAAGUACUUUCU | hsa-miR-520a* |
| 1551 | UCUACAAAGGGAAGCCCUUUCUG | hsa-miR-520d* |
| 1552 | AAAGUGCUUCCCUUUGGACUGU | hsa-miR-520a |
| 1553 | AAAGUGCUUCCUUUUAGAGGG | hsa-miR-520b |
| 1554 | AAAGUGCUUCCUUUUAGAGGGUU | hsa-miR-520c |
| 1555 | AAAGUGCUUCUCUUUGGUGGGUU | hsa-miR-520d |
| 1556 | AAAGUGCUUCCUUUUUGAGGG | hsa-miR-520e |
| 1557 | AAGUGCUUCCUUUUAGAGGGUU | hsa-miR-520f |
| 1558 | ACAAAGUGCUUCCCUUUAGAGUGU | hsa-miR-520g |
| 1559 | ACAAAGUGCUUCCCUUUAGAGU | hsa-miR-520h |
| 1560 | AACGCACUUCCCUUUAGAGUGU | hsa-miR-521 |
| 1561 | AAAAUGGUUCCCUUUAGAGUGUU | hsa-miR-522 |
| 1562 | AACGCGCUUCCCUAUAGAGGG | hsa-miR-523 |
| 1563 | GAAGGCGCUUCCCUUUGGAGU | hsa-miR-524 |
| 1564 | CUACAAAGGGAAGCACUUUCUC | hsa-miR-524* |

(continued)

| SEQ ID NO: | Sequence | Name |
|---|---|---|
| 1565 | CUCCAGAGGGAUGCACUUUCU | hsa-miR-525 |
| 1566 | GAAGGCGCUUCCCUUUAGAGC | hsa-miR-525* |
| 1567 | AAAGUGCUUCCUUUUAGAGGC | hsa-miR-526b* |
| 1568 | CUCUAGAGGGAAGCACUUUCU | hsa-miR-526a |
| 1569 | CUCUUGAGGGAAGCACUUUCUGUU | hsa-miR-526b |
| 1570 | CUCUAGAGGGAAGCGCUUUCUGUU | hsa-miR-526c |
| 1571 | CUGCAAAGGGAAGCCCUUUCU | hsa-miR-527 |
| 1572 | UGGAAGACUAGUGAUUUUGUUG | hsa-miR-7 |
| 1573 | UCUUUGGUUAUCUAGCUGUAUGA | hsa-miR-9 |
| 1574 | UAAAGCUAGAUAACCGAAAGU | hsa-miR-9* |
| 1575 | UAUUGCACUUGUCCCGGCCUG | hsa-miR-92 |
| 1576 | AAAGUGCUGUUCGUGCAGGUAG | hsa-miR-93 |
| 1577 | UUCAACGGGUAUUUAUUGAGCA | hsa-miR-95 |
| 1578 | UUUGGCACUAGCACAUUUUUGC | hsa-miR-96 |
| 1579 | UGAGGUAGUAAGUUGUAUUGUU | hsa-miR-98 |
| 1580 | AACCCGUAGAUCCGAUCUUGUG | hsa-miR-99a |
| 1581 | CACCCGUAGAACCGACCUUGCG | hsa-miR-99b |

While the foregoing embodiments of the invention have been set forth in considerable detail for the purpose of making a complete disclosure of the invention, it will be apparent to those of skill in the art that numerous changes may be made in such details without departing from the principles of the invention. Accordingly, the invention should be limited only by the following claims.

## Claims

1. An array for analysing small RNAs comprising:

   an array support (108); and
   a set of probes on the array support, wherein the set of probes includes a plurality of probes (102) that bind specifically and are strictly complementary to a single target small RNA via a target-complementary sequence of a length sufficient to provide specific bindng between the probe and the corresponding small RNA, and wherein probes of the set each have a same common sequence and differ from a next probe of the set by lacking at least one base from one of the ends of the target complementary sequence of the probe and wherein each probe of the set is present on the array support as a discrete feature.

2. An array as claimed in claim 1, wherein the target-complementary sequence is complementary to at least 10 bases of the target small RNA.

3. An array as claimed in claim 1or 2, wherein the set of probes includes at least three probes.

4. An array as claimed in claim 1, 2 or 3, wherein the set of probes includes at least five probes.

5. An array as claimed in claim 4, wherein the set of probes comprises the target-' complementary sequences of SEQ ID Nos: 33 to 37.

6. An array as claimed in claim 4, wherein each of the at least five probes has a target-complementary sequence (104) independently selected from the group consisting of SEQ ID NOS: 1 to 1240.

7. An array as claimed in any of claims 1 to 4, wherein the probe set includes at least one probe (102) having a target-complementary sequence (104) independently selected from the group consisting of SEQ ID NOS:1241 to 1250.

8. An array as claimed in any of claims 1 to 4, wherein the set of probes comprises a target-complementary sequence selected from the group consisting of SEQ ID NOS: 7 to 14.

9. An array as claimed in any preceding claim, wherein the probe set comprises at least 20 probes (102).

10. An array as claimed in any preceding claim, including at least 1000 features.

11. An array as claimed in any preceding claim, wherein each of the probes (102) comprises a linker sequence (110), a target-complementary sequence (104), and a domain that increases the stability of the probe/target duplex (106), wherein the target-complementary sequence (104) and the domain that increases the stability of the probe/target duplex (106) for each probe is bound to the array support (108) via the linker sequence (110) of said probe.

**12.** An array as claimed in claim 11, wherein the domain that increases the stability of the probe/target duplex (106) of at least one of the at least five probes comprises a hairpin sequence (106b).

**13.** An array as claimed in any preceding claim, wherein each of the probes (102) in the probe set is characterized as having a Tm in the range from about 50°C to about 60°C when hybridized with its respective target small RNA.

**14.** An array as claimed in any preceding claim, wherein each of the probes (102) in the probe set is characterized as having a Tm in the range from about 55°C to about 60°C when hybridized with its respective target small RNA.

**15.** An array as claimed in any preceding claim, wherein the small RNA is a miRNA.

**16.** A method of analysing a sample for miRNAs (120), the method comprising contacting (125) a sample with an array as claimed in claim 15, and interrogating the array to obtain information about miRNAs in the sample.

**17.** A method as claimed in claim 16, wherein contacting (125) the sample with the array is performed under stringent assay conditions.

**18.** A method as claimed in claim 16 or 17, wherein contacting (125) the sample with the array includes incubating the sample on the array at a temperature in the range from about 50°C to about 60°C.

**19.** A method as claimed in claim 16, 17 or 18, wherein each of the probes (102) in the probe set has a Tm in the range from about 50°C to about 60°C when hybridised with its target miRNA.

**20.** A method as claimed in any of claims 16 to 19, wherein the probes include a nucleotide clamp (106a) comprising a contiguous sequence of up to about five nucleotides, and wherein the method includes extending (122) the miRNA (120).

**Patentansprüche**

**1.** Array zum Analysieren von kleinen RNAs, umfassend:

eine Array-Unterlage (108) und
einen Sondensatz auf der Array-Unterlage, wobei der Sondensatz eine Vielfalt von Sonden (102) einschließt, die mittels einer zielkomplementären Sequenz mit einer Länge, die ausreicht, um spezifisches Binden zwischen der Sonde und der entsprechenden kleinen RNA bereitzustellen, spezifisch an eine einzelne kleine Ziel-RNA binden und zu dieser strikt komplementär sind, und wobei Sonden des Satzes jeweils eine gleiche gemeinsame Sequenz aufweisen und sich von einer nächsten Sonde des Satzes dadurch unterscheiden, dass ihnen mindestens eine Base von einem der Enden der zielkomplementären Sequenz der Sonde fehlt, und wobei jede Sonde des Satzes auf der Array-Unterlage als diskrete Einheit vorliegt.

**2.** Array wie in Anspruch 1 beansprucht, wobei die zielkomplementäre Sequenz zu mindestens 10 Basen der kleinen Ziel-RNA komplementär ist.

**3.** Array wie in Anspruch 1 oder 2 beansprucht, wobei der Sondensatz mindestens drei Sonden einschließt.

**4.** Array wie in Anspruch 1, 2 oder 3 beansprucht, wobei der Sondensatz mindestens fünf Sonden einschließt.

**5.** Array wie in Anspruch 4 beansprucht, wobei der Sondensatz die zielkomplementären Sequenzen der SEQ ID NO: 33 bis 37 umfasst.

**6.** Array wie in Anspruch 4 beansprucht, wobei jede der mindestens fünf Sonden eine zielkomplementäre Sequenz (104) aufweist, unabhängig ausgewählt aus der Gruppe, bestehend aus SEQ ID NO: 1 bis 1240.

**7.** Array wie in einem der Ansprüche 1 bis 4 beansprucht, wobei der Sondensatz mindestens eine Sonde (102) einschließt, die eine zielkomplementäre Sequenz (104) aufweist, unabhängig ausgewählt aus der Gruppe, bestehend aus SEQ ID NO: 1241 bis 1250.

**8.** Array wie in einem der Ansprüche 1 bis 4 beansprucht, wobei der Sondensatz eine zielkomplementäre Sequenz aufweist, ausgewählt aus der Gruppe, bestehend aus SEQ ID NO: 7 bis 14.

**9.** Array wie in einem vorhergehenden Anspruch beansprucht, wobei der Sondensatz mindestens 20 Sonden (102) umfasst.

**10.** Array wie in einem vorhergehenden Anspruch beansprucht, der mindestens 1000 Einheiten einschließt.

**11.** Array wie in einem vorhergehenden Anspruch beansprucht, wobei jede der Sonden (102) eine Linker-Sequenz (110), eine zielkomplementäre Sequenz (104) und eine Domäne, welche die Stabilität des Sonde/Ziel-Duplexes erhöht (106), umfasst, wobei die zielkomplementäre Sequenz (104) und die Domäne, welche die Stabilität des Sonde/Ziel-Duplexes erhöht (106), für jede Sonde mittels der Linker-Sequenz (110) der Sonde an die Array-Unterlage (108) gebunden ist.

**12.** Array wie in Anspruch 11 beansprucht, wobei die Domäne, welche die Stabilität des Sonde/Ziel-Duplexes erhöht (106), von mindestens einer der mindestens fünf Sonden eine Hairpin-Sequenz (106b) umfasst.

**13.** Array wie in einem vorhergehenden Anspruch beansprucht, wobei jede der Sonden (102) in dem Sondensatz **dadurch gekennzeichnet ist, dass** sie eine Tm im Bereich von etwa 50°C bis etwa 60°C aufweist, wenn sie mit ihrer jeweiligen kleinen Ziel-RNA hybridisiert wird.

**14.** Array wie in einem vorhergehenden Anspruch beansprucht, wobei jede der Sonden (102) in dem Sondensatz **dadurch gekennzeichnet ist, dass** sie eine Tm im Bereich von etwa 55°C bis etwa 60°C aufweist, wenn sie mit ihrer jeweiligen kleinen Ziel-RNA hybridisiert wird.

**15.** Array wie in einem vorhergehenden Anspruch beansprucht, wobei es sich bei der kleinen RNA um eine miRNA handelt.

**16.** Verfahren zum Analysieren einer Probe auf miRNAs (120), wobei das Verfahren umfasst
In-Kontakt-Bringen (125) einer Probe mit einem wie in Anspruch 15 beanspruchten Array und Abfragen des Arrays, um Information über miRNAs in der Probe zu erhalten.

**17.** Verfahren wie in Anspruch 16 beansprucht, wobei das In-Kontakt-Bringen (125) der Probe mit dem Array unter stringenten Assaybedingungen durchgeführt wird.

**18.** Verfahren wie in Anspruch 16 oder 17 beansprucht, wobei das In-Kontakt-Bringen (125) der Probe mit dem Array das Inkubieren der Probe auf dem Array bei einer Temperatur im Bereich von etwa 50°C bis etwa 60°C einschließt.

**19.** Verfahren wie in Anspruch 16, 17 oder 18 beansprucht, wobei jede der Sonden (102) in dem Sondensatz eine Tm im Bereich von etwa 50°C bis etwa 60°C aufweist, wenn sie mit ihrer Ziel-miRNA hybridisiert wird.

**20.** Verfahren wie in einem der Ansprüche 16 bis 19 beansprucht, wobei die Sonden eine Nucleotid-Klammer (106a) einschließen, die eine zusammenhängende Sequenz von bis zu etwa fünf Nucleotiden umfasst, und wobei das Verfahren das Verlängern (122) der miRNA (120) einschließt.

## Revendications

**1.** Biopuce pour analyser de petits ARN comprenant :

un support de biopuce (108) ; et
un jeu de sondes sur le support de biopuce, le jeu de sondes comprenant une pluralité de sondes (102) qui se lient spécifiquement à et sont strictement complémentaires d'un seul petit ARN cible via une séquence complémentaire d'une cible ayant une longueur suffisante pour permettre une liaison spécifique entre la sonde et le petit ARN correspondant, dans lequel chacune des sondes du jeu a une même séquence commune et diffère d'une sonde suivante du jeu en étant dépourvue d'au moins une base depuis l'une des extrémités de la séquence complémentaire d'une cible de la sonde, et dans lequel chaque sonde du jeu est présente sur le support de biopuce sous la forme d'une caractéristique discrète.

**2.** Biopuce selon la revendication 1, dans laquelle la séquence complémentaire d'une cible est complémentaire d'au moins 10 bases du petit ARN cible.

**3.** Biopuce selon la revendication 1 ou 2, dans laquelle le jeu de sondes comprend au moins trois sondes.

**4.** Biopuce selon la revendication 1, 2 ou 3, dans laquelle le jeu de sondes comprend au moins cinq sondes.

**5.** Biopuce selon la revendication 4, dans laquelle le jeu de sondes comprend les séquences complémentaires d'une cible des SEQ ID NO : 33 à 37.

**6.** Biopuce selon la revendication 4, dans laquelle chacune des au moins cinq sondes a une séquence complémentaire d'une cible (104) indépendamment choisie dans l'ensemble constitué par les SEQ ID NO : 1 à 1240.

**7.** Biopuce selon l'une quelconque des revendications 1 à 4, dans laquelle le jeu de sondes comprend au moins une sonde (102) ayant une séquence complémentaire d'une cible (104) indépendamment choisie dans l'ensemble constitué par les SEQ ID NO : 1241 à 1250.

**8.** Biopuce selon l'une quelconque des revendications 1 à 4, dans laquelle le jeu de sondes comprend une séquence complémentaire d'une cible choisie dans l'ensemble constitué par les SEQ ID NO : 7 à 14.

**9.** Biopuce selon l'une quelconque des revendications précédentes, dans laquelle le jeu de sondes comprend au moins 20 sondes (102).

**10.** Biopuce selon l'une quelconque des revendications précédentes, comprenant au moins 1000 caractéristiques.

**11.** Biopuce selon l'une quelconque des revendications précédentes, dans laquelle chacune des sondes (102) comprend une séquence de liaison (110), une séquence complémentaire d'une cible (104), et un domaine qui augmente la stabilité du duplex sonde/cible (106), et dans laquelle la séquence complémentaire d'une cible (104) et le domaine qui augmente la stabilité du duplex sonde/cible (106) pour chaque sonde sont liés au support de biopuce (108) via la séquence de liaison (110) de ladite sonde.

**12.** Biopuce selon la revendication 11, dans laquelle le domaine qui augmente la stabilité du duplex sonde/cible (106) d'au moins l'une des au moins cinq sondes comprend une séquence en épingle à cheveux (106b).

**13.** Biopuce selon l'une quelconque des revendications précédentes, dans laquelle chacune des sondes (102) dans le jeu de sondes est **caractérisée en ce qu'**elle a une valeur Tm située dans la plage allant d'environ 50°C à environ 60°C quand elle est hybridée avec son petit ARN cible respectif.

**14.** Biopuce selon l'une quelconque des revendications précédentes, dans laquelle chacune des sondes (102) dans le jeu de sondes est **caractérisée en ce qu'**elle a une valeur Tm située dans la plage allant d'environ 55°C à environ 60°C quand elle est hybridée avec son petit ARN cible respectif.

**15.** Biopuce selon l'une quelconque des revendications précédentes, dans laquelle le petit ARN est un ARNmi.

**16.** Procédé pour analyser les ARNmi (120) dans un échantillon, lequel procédé comprend la mise en contact (125) d'un échantillon avec une biopuce telle que revendiquée dans la revendication 15, et l'interrogation de la biopuce pour obtenir des informations concernant les ARNmi dans l'échantillon.

**17.** Procédé selon la revendication 16, dans lequel la mise en contact (125) de l'échantillon avec la biopuce est effectuée dans des conditions de dosage stringentes.

**18.** Procédé selon la revendication 16 ou 17, dans lequel la mise en contact (125) de l'échantillon avec la biopuce comprend l'incubation de l'échantillon sur la biopuce à une température située dans la plage allant d'environ 50°C à environ 60°C.

**19.** Procédé selon la revendication 16, 17 ou 18, dans lequel chacune des sondes (102) dans le jeu de sondes a une valeur Tm située dans la plage allant d'environ 50°C à environ 60°C quand elle est hybridée avec son ARNmi cible.

**20.** Procédé selon l'une quelconque des revendications 16 à 19, dans lequel les sondes comprennent une pince de nucléotides (106a) comprenant une séquence contiguë de jusqu'à environ cinq nucléotides, lequel procédé comprend l'extension (122) de l'ARNmi (120).

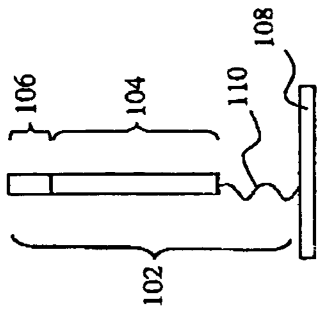


Fig. 1

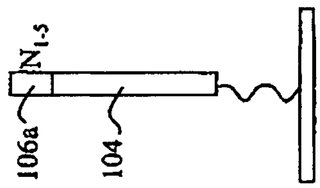


Fig. 2A

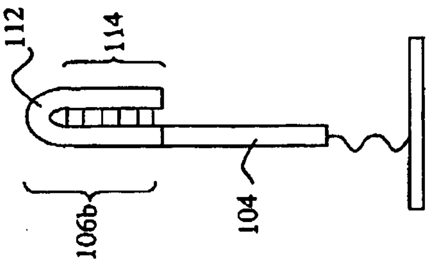


Fig. 2B

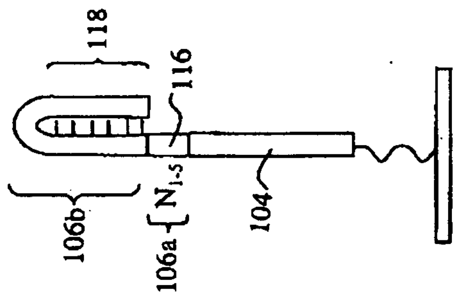


Fig. 2C

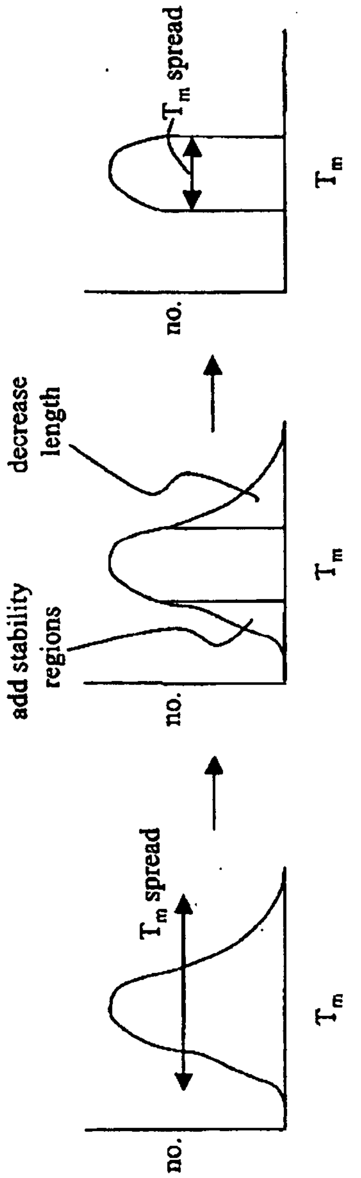
no.
$T_m$ spread
$T_m$
add stability regions
decrease length
no.
$T_m$
no.
$T_m$ spread
$T_m$

Fig. 3

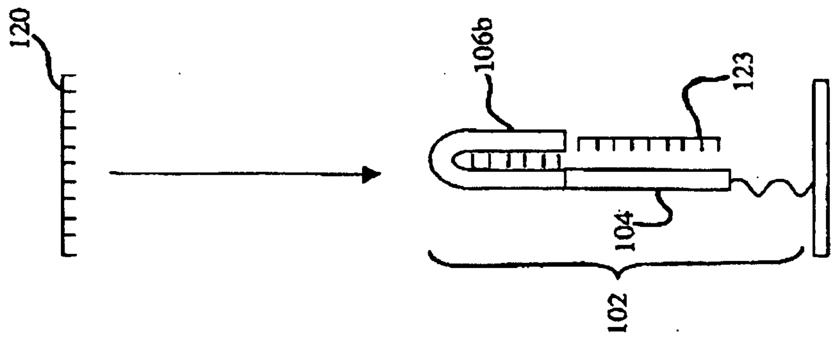


Fig. 4C

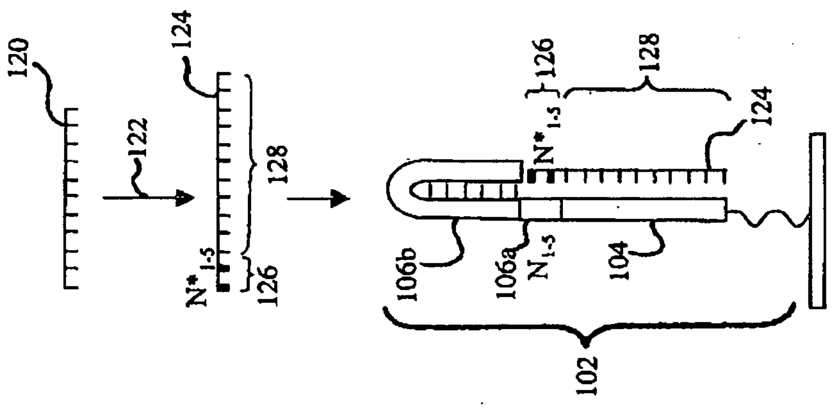


Fig. 4B

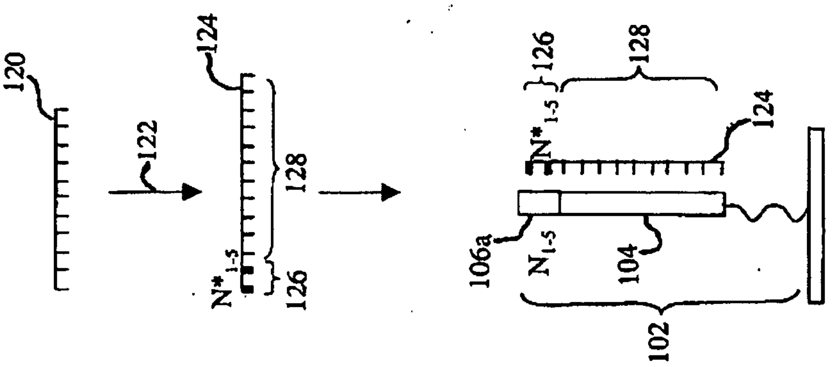


Fig. 4A

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5763870 A
- US 5945679 A
- US 5721435 A
- US 5948902 A
- US 65548200 A
- US 20070003940 A, Wang
- US 6242266 B
- US 6232072 B
- US 6180351 B
- US 6171797 B
- US 6323043 B
- US 20060172317 A, Wang
- WO 9318186 A
- US 6335167 B
- US 6197501 B
- US 5830645 A
- US 5665549 A
- US 6756202 B
- US 6406849 B
- US 6221583 B

### Non-patent literature cited in the description


- **NOVINA et al.** *Nature,* 2004, vol. 430, 161-164
- **PFEFFER et al.** *Science,* 2004, vol. 304, 734-736
- **GRIFFITHS-JONES.** *Nucl. Acids Res.,* 2004, vol. 32, D109-D111
- **LAGOS-QUINTANA et al.** *Science,* 2001, vol. 294, 853-858
- **GRAD et al.** *Mol Cell,* 2003, vol. 11, 1253-1263
- **MOURELATOS et al.** *Genes Dev,* 2002, vol. 16, 720-728
- **LAGOS-QUINTANA et al.** *Curr Biol,* 2002, vol. 12, 735-739
- **LAGOS-QUINTANA et al.** *RNA,* 2003, vol. 9, 175-179
- **DOSTIE et al.** *RNA,* 2003, vol. 9, 180-186
- **LIM et al.** *Science,* 2003, vol. 299, 1540
- **HOUBAVIY et al.** *Dev Cell,* 2003, vol. 5, 351-358
- **MICHAEL et al.** *Mol Cancer Res,* 2003, vol. 1, 882-891
- **KIM et al.** *Proc Natl Acad Sci U S A,* 2004, vol. 101, 360-365
- **SUH et al.** *Dev Biol,* 2004, vol. 270, 488-498
- **KASASHIMA et al.** *Biochem Biophys Res Commun,* 2004, vol. 322, 403-410
- **XIE et al.** *Nature,* 2005, vol. 434, 338-345
- **LIU et al.** *Proc. Nat'l Acad. Sci. USA,* 2004, vol. 101, 9740-9744
- **THOMSON et al.** *Nature Methods,* 2004, vol. 1, 1-7
- **BABAK et al.** *RNA,* 2004, vol. 10, 1813-1819
- **BASKERVILLE ; BARTEL.** *RNA,* 2005, vol. 11, 241-247
- **RICCELLI et al.** *Nucleic Acids Research,* 2001, vol. 29, 996-1004
- **GOFF et al.** *RNA Biology,* 2005, vol. 2, 93-100
- **AUSUBEL et al.** Short Protocols in Molecular Biology. Wiley & Sons, 1995
- **SAMBROOK et al.** Molecular Cloning: A Laboratory Manual. Cold Spring Harbor, 2001
- **TIJSSEN.** Hybridization with Nucleic Acid Probes. Elsevier, 1993
- **SAMBROOK ; RUSSELL.** Molecular Cloning: A Laboratory Handbook. 2001, 10.38-10.4110.47
- **WALLACE et al.** *Nucleic Acids Res.,* 1979, vol. 6, 3543-3557
- **LE NOVERE.** *Bioinformatics,* 2001, vol. 17 (12), 1226-1227
- **HARRINGTON et al.** *Curr Opin Microbiol.,* 2000, vol. 3, 285-91
- **LIPSHUTZ et al.** *Nat Genet,* 1999, vol. 21, 20-4
- **KRICKA et al.** *Ann. Clin. Biochem.,* 2002, vol. 39, 114-29
- **KALLIONIEMI et al.** *Science,* 1992, vol. 258, 818-821
- **TIJSSEN.** Hybridization with Nucleic Acid Probes, Parts I and II. Elsevier, 1993
- **GALL et al.** *Meth. Enzymol.,* 1981, vol. 21, 470-480
- **ANGERER et al.** Genetic Engineering: Principles and Methods. Plenum Press, 1985, vol. 7, 43-65
- **QUACKENBUSH et al.** *Nat Genet.,* 2002, vol. 32, 496-501
- **BILBAN et al.** *Curr Issues Mol Biol.,* 2002, vol. 4, 57-64

- **FINKELSTEIN et al.** *Plant Mol Biol.,* 2002, vol. 48 (1-2), 119-31
- **HEGDE et al.** *Biotechniques,* 2000, vol. 29, 548-554
- **WORKMAN et al.** *Genome Biol.,* 2002, vol. 3, 1-16

SEQUENCE LISTING

<110> Wang, Hui

<120> Analysis of microRNA

<130> ED/GM/N19320

<160> 1581

<170> PatentIn version 3.3

<210> 1
<211> 18
<212> DNA
<213> Drosophila melanogaster

<400> 1
gcactgattt cgaatggt 18

<210> 2
<211> 15
<212> DNA
<213> Drosophila melanogaster

<400> 2
gctcctcaaa gctgg 15

<210> 3
<211> 17
<212> DNA
<213> Drosophila melanogaster

<400> 3
tgagacacac tttgccc 17

<210> 4
<211> 22
<212> DNA
<213> Drosophila melanogaster

<400> 4
ctcacagtat aatcctgtga tt 22

<210> 5
<211> 18
<212> DNA
<213> Drosophila melanogaster

<400> 5
tcagctatgc cgacatct 18

<210> 6
<211> 19
<212> DNA
<213> Drosophila melanogaster

<400> 6
aaaaagaaca gccactgtg 19

<210> 7
<211> 19
<212> DNA
<213> Homo sapiens

<400> 7
aactatacaa cctactacc 19

<210> 8
<211> 20
<212> DNA
<213> Homo sapiens

<400> 8
aactatacaa cctactacct 20

<210> 9
<211> 17
<212> DNA
<213> Homo sapiens

<400> 9
aaccacacaa cctacta 17

<210> 10
<211> 18

<212> DNA
<213> Homo sapiens

<400> 10
aaccacacaa cctactac 18

<210> 11
<211> 18
<212> DNA
<213> Homo sapiens

<400> 11
aaccatacaa cctactac 18

<210> 12
<211> 19
<212> DNA
<213> Homo sapiens

<400> 12
aaccatacaa cctactacc 19

<210> 13
<211> 20
<212> DNA
<213> Homo sapiens

<400> 13
aaccatacaa cctactacct 20

<210> 14
<211> 19
<212> DNA
<213> Homo sapiens

<400> 14
actatgcaac ctactacct 19

<210> 15
<211> 20
<212> DNA
<213> Homo sapiens

<400> 15
actatacaac ctcctacctc 20

<210> 16
<211> 21
<212> DNA
<213> Homo sapiens

<400> 16
actatacaac ctcctacctc a 21

<210> 17
<211> 20
<212> DNA
<213> Homo sapiens

<400> 17
aactatacaa tctactacct 20

<210> 18
<211> 21
<212> DNA
<213> Homo sapiens

<400> 18
aactatacaa tctactacct c 21

<210> 19
<211> 22
<212> DNA
<213> Homo sapiens

<400> 19
aactatacaa tctactacct ca 22

<210> 20
<211> 19
<212> DNA
<213> Homo sapiens

<400> 20
actgtacaaa ctactacct 19

<210> 21
<211> 20
<212> DNA
<213> Homo sapiens

<400> 21
actgtacaaa ctactacctc 20

<210> 22
<211> 21
<212> DNA
<213> Homo sapiens

<400> 22
actgtacaaa ctactacctc a 21

<210> 23
<211> 18
<212> DNA
<213> Homo sapiens

<400> 23
acagcacaaa ctactacc 18

<210> 24
<211> 19
<212> DNA
<213> Homo sapiens

<400> 24
acagcacaaa ctactacct 19

<210> 25
<211> 20
<212> DNA
<213> Homo sapiens

<400> 25
acagcacaaa ctactacctc 20

<210> 26
<211> 20
<212> DNA
<213> Homo sapiens

<400> 26
tacatacttc tttacattcc 20

<210> 27
<211> 21
<212> DNA
<213> Homo sapiens

<400> 27
tacatacttc tttacattcc a 21

<210> 28
<211> 17
<212> DNA
<213> Homo sapiens

<400> 28
cacaagttcg gatctac 17

<210> 29
<211> 18
<212> DNA
<213> Homo sapiens

<400> 29
cacaagttcg gatctacg 18

<210> 30
<211> 22
<212> DNA
<213> Homo sapiens

<400> 30
cttcagttat cacagtactg ta 22

<210> 31
<211> 17
<212> DNA
<213> Homo sapiens

<400> 31
tcatagccct gtacaat 17

<210> 32
<211> 18

<212> DNA
<213> Homo sapiens

<400> 32
tcatagccct gtacaatg 18

<210> 33
<211> 15
<212> DNA
<213> Homo sapiens

<400> 33
acaggagtct gagca 15

<210> 34
<211> 16
<212> DNA
<213> Homo sapiens

<400> 34
acaggagtct gagcat 16

<210> 35
<211> 17
<212> DNA
<213> Homo sapiens

<400> 35
acaggagtct gagcatt 17

<210> 36
<211> 18
<212> DNA
<213> Homo sapiens

<400> 36
acaggagtct gagcattt 18

<210> 37
<211> 19
<212> DNA
<213> Homo sapiens

<400> 37
acaggagtct gagcatttg 19

<210> 38
<211> 17
<212> DNA
<213> Homo sapiens

<400> 38
gctacctgca ctgtaag 17

<210> 39
<211> 16
<212> DNA
<213> Homo sapiens

<400> 39
atctgcactg tcagca 16

<210> 40
<211> 19
<212> DNA
<213> Homo sapiens

<400> 40
tgatagccct gtacaatgc 19

<210> 41
<211> 17
<212> DNA
<213> Homo sapiens

<400> 41
aatgccccta aaaatcc 17

<210> 42
<211> 18
<212> DNA
<213> Homo sapiens

<400> 42
aatgccccta aaaatcct 18

<210> 43
<211> 19
<212> DNA
<213> Homo sapiens

<400> 43
aatgccccta aaaatcctt 19

<210> 44
<211> 20
<212> DNA
<213> Homo sapiens

<400> 44
aatgccccta aaaatcctta 20

<210> 45
<211> 21
<212> DNA
<213> Homo sapiens

<400> 45
aatgccccta aaaatcctta t 21

<210> 46
<211> 19
<212> DNA
<213> Homo sapiens

<400> 46
cacaaattcg gatctacag 19

<210> 47
<211> 20
<212> DNA
<213> Homo sapiens

<400> 47
cacaaattcg gatctacagg 20

<210> 48
<211> 21
<212> DNA
<213> Homo sapiens

<400> 48
cacaaattcg gatctacagg g 21

<210> 49
<211> 19
<212> DNA
<213> Homo sapiens

<400> 49
acaaattcgg ttctacagg 19

<210> 50
<211> 16
<212> DNA
<213> Homo sapiens

<400> 50
acaaacacca ttgtca 16

<210> 51
<211> 17
<212> DNA
<213> Homo sapiens

<400> 51
acaaacacca ttgtcac 17

<210> 52
<211> 18
<212> DNA
<213> Homo sapiens

<400> 52
acaaacacca ttgtcaca 18

<210> 53
<211> 19
<212> DNA
<213> Homo sapiens

<400> 53
acaaacacca ttgtcacac 19

<210> 54
<211> 13

<212> DNA
<213> Homo sapiens

<400> 54
tggcattcac cgc 13

<210> 55
<211> 14
<212> DNA
<213> Homo sapiens

<400> 55
tggcattcac cgcg 14

<210> 56
<211> 15
<212> DNA
<213> Homo sapiens

<400> 56
tggcattcac cgcgt 15

<210> 57
<211> 18
<212> DNA
<213> Homo sapiens

<400> 57
cacaggttaa agggtctc 18

<210> 58
<211> 19
<212> DNA
<213> Homo sapiens

<400> 58
cacaggttaa agggtctca 19

<210> 59
<211> 20
<212> DNA
<213> Homo sapiens

<400> 59
cacaggttaa agggtctcag 20

<210> 60
<211> 17
<212> DNA
<213> Homo sapiens

<400> 60
tcacaagtta gggtctc 17

<210> 61
<211> 18
<212> DNA
<213> Homo sapiens

<400> 61
tcacaagtta gggtctca 18

<210> 62
<211> 19
<212> DNA
<213> Homo sapiens

<400> 62
gcattattac tcacggtac 19

<210> 63
<211> 20
<212> DNA
<213> Homo sapiens

<400> 63
gcattattac tcacggtacg 20

<210> 64
<211> 21
<212> DNA
<213> Homo sapiens

<400> 64
cgcgtaccaa aagtaataat g 21

<210> 65
<211> 16
<212> DNA
<213> Homo sapiens

<400> 65
agccaagctc agacgg 16

<210> 66
<211> 15
<212> DNA
<213> Homo sapiens

<400> 66
aaaagagacc ggttc 15

<210> 67
<211> 16
<212> DNA
<213> Homo sapiens

<400> 67
aaaagagacc ggttca 16

<210> 68
<211> 17
<212> DNA
<213> Homo sapiens

<400> 68
aaaagagacc ggttcac 17

<210> 69
<211> 18
<212> DNA
<213> Homo sapiens

<400> 69
aaaagagacc ggttcact 18

<210> 70
<211> 17
<212> DNA
<213> Homo sapiens

<400> 70
gaaagagacc ggttcac 17

<210> 71
<211> 18
<212> DNA
<213> Homo sapiens

<400> 71
gaaagagacc ggttcact 18

<210> 72
<211> 19
<212> DNA
<213> Homo sapiens

<400> 72
gaaagagacc ggttcactg 19

<210> 73
<211> 13
<212> DNA
<213> Homo sapiens

<400> 73
gcaagcccag acc 13

<210> 74
<211> 14
<212> DNA
<213> Homo sapiens

<400> 74
gcaagcccag accg 14

<210> 75
<211> 18
<212> DNA
<213> Homo sapiens

<400> 75
atgccctttt aacattgc 18

<210> 76
<211> 19

<212> DNA
<213> Homo sapiens

<400> 76
atgccctttt aacattgca 19

<210> 77
<211> 17
<212> DNA
<213> Homo sapiens

<400> 77
atgccctttc atcattg 17

<210> 78
<211> 15
<212> DNA
<213> Homo sapiens

<400> 78
cgaccatggc tgtag 15

<210> 79
<211> 16
<212> DNA
<213> Homo sapiens

<400> 79
cgaccatggc tgtaga 16

<210> 80
<211> 16
<212> DNA
<213> Homo sapiens

<400> 80
acagctggtt gaaggg 16

<210> 81
<211> 17
<212> DNA
<213> Homo sapiens

<400> 81
acagctggtt gaagggg 17

<210> 82
<211> 18
<212> DNA
<213> Homo sapiens

<400> 82
acagctggtt gaagggga 18

<210> 83
<211> 17
<212> DNA
<213> Homo sapiens

<400> 83
tagctggttg aagggga 17

<210> 84
<211> 18
<212> DNA
<213> Homo sapiens

<400> 84
tagctggttg aaggggac 18

<210> 85
<211> 19
<212> DNA
<213> Homo sapiens

<400> 85
tagctggttg aaggggacc 19

<210> 86
<211> 14
<212> DNA
<213> Homo sapiens

<400> 86
ccctctggtc aacc 14

<210> 87
<211> 15
<212> DNA
<213> Homo sapiens

<400> 87
ccctctggtc aacca 15

<210> 88
<211> 21
<212> DNA
<213> Homo sapiens

<400> 88
tcacatagga ataaaaagcc a 21

<210> 89
<211> 22
<212> DNA
<213> Homo sapiens

<400> 89
tcacatagga ataaaaagcc at 22

<210> 90
<211> 23
<212> DNA
<213> Homo sapiens

<400> 90
tcacatagga ataaaaagcc ata 23

<210> 91
<211> 18
<212> DNA
<213> Homo sapiens

<400> 91
cacataggaa tgaaaagc 18

<210> 92
<211> 19
<212> DNA
<213> Homo sapiens

<400> 92
cacataggaa tgaaaagcc 19

<210> 93
<211> 21
<212> DNA
<213> Homo sapiens

<400> 93
tccatcatca aaacaaatgg a 21

<210> 94
<211> 17
<212> DNA
<213> Homo sapiens

<400> 94
ctacgcgtat tcttaag 17

<210> 95
<211> 18
<212> DNA
<213> Homo sapiens

<400> 95
ctacgcgtat tcttaagc 18

<210> 96
<211> 19
<212> DNA
<213> Homo sapiens

<400> 96
ctacgcgtat tcttaagca 19

<210> 97
<211> 20
<212> DNA
<213> Homo sapiens

<400> 97
ctacgcgtat tcttaagcaa 20

<210> 98
<211> 21

<212> DNA
<213> Homo sapiens

<400> 98
ctacgcgtat tcttaagcaa t 21

<210> 99
<211> 16
<212> DNA
<213> Homo sapiens

<400> 99
gattcacaac accagc 16

<210> 100
<211> 17
<212> DNA
<213> Homo sapiens

<400> 100
gattcacaac accagct 17

<210> 101
<211> 17
<212> DNA
<213> Homo sapiens

<400> 101
agacacgtgc actgtag 17

<210> 102
<211> 18
<212> DNA
<213> Homo sapiens

<400> 102
agacacgtgc actgtaga 18

<210> 103
<211> 19
<212> DNA
<213> Homo sapiens

<400> 103
ctaccatagg gtaaaacca 19

<210> 104
<211> 20
<212> DNA
<213> Homo sapiens

<400> 104
ctaccatagg gtaaaaccac 20

<210> 105
<211> 16
<212> DNA
<213> Homo sapiens

<400> 105
ccatctttac cagaca 16

<210> 106
<211> 17
<212> DNA
<213> Homo sapiens

<400> 106
ccatctttac cagacag 17

<210> 107
<211> 18
<212> DNA
<213> Homo sapiens

<400> 107
ccatctttac cagacagt 18

<210> 108
<211> 19
<212> DNA
<213> Homo sapiens

<400> 108
ccatctttac cagacagtg 19

<210> 109
<211> 22
<212> DNA
<213> Homo sapiens

<400> 109
tccataaagt aggaaacact ac 22

<210> 110
<211> 19
<212> DNA
<213> Homo sapiens

<400> 110
gtagtgcttt ctactttat 19

<210> 111
<211> 20
<212> DNA
<213> Homo sapiens

<400> 111
gtagtgcttt ctactttatg 20

<210> 112
<211> 19
<212> DNA
<213> Homo sapiens

<400> 112
tgagctacag tgcttcatc 19

<210> 113
<211> 22
<212> DNA
<213> Homo sapiens

<400> 113
ctagtacatc atctatactg ta 22

<210> 114
<211> 18
<212> DNA
<213> Homo sapiens

<400> 114
aagggattcc tgggaaaa 18

<210> 115
<211> 19
<212> DNA
<213> Homo sapiens

<400> 115
aagggattcc tgggaaaac 19

<210> 116
<211> 20
<212> DNA
<213> Homo sapiens

<400> 116
aagggattcc tgggaaaact 20

<210> 117
<211> 16
<212> DNA
<213> Homo sapiens

<400> 117
aacccatgga attcag 16

<210> 118
<211> 17
<212> DNA
<213> Homo sapiens

<400> 118
aacccatgga attcagt 17

<210> 119
<211> 18
<212> DNA
<213> Homo sapiens

<400> 119
aacccatgga attcagtt 18

<210> 120
<211> 19

<212> DNA
<213> Homo sapiens

<400> 120
aacccatgga attcagttc 19

<210> 121
<211> 20
<212> DNA
<213> Homo sapiens

<400> 121
aacccatgga attcagttct 20

<210> 122
<211> 17
<212> DNA
<213> Homo sapiens

<400> 122
agcctatgga attcagt 17

<210> 123
<211> 18
<212> DNA
<213> Homo sapiens

<400> 123
agcctatgga attcagtt 18

<210> 124
<211> 19
<212> DNA
<213> Homo sapiens

<400> 124
agcctatgga attcagttc 19

<210> 125
<211> 20
<212> DNA
<213> Homo sapiens

<400> 125
agcctatgga attcagttct 20

<210> 126
<211> 16
<212> DNA
<213> Homo sapiens

<400> 126
gcagaagcat ttccac 16

<210> 127
<211> 17
<212> DNA
<213> Homo sapiens

<400> 127
gcagaagcat ttccaca 17

<210> 128
<211> 19
<212> DNA
<213> Homo sapiens

<400> 128
acaaagttct gtagtgcac 19

<210> 129
<211> 20
<212> DNA
<213> Homo sapiens

<400> 129
acaaagttct gtagtgcact 20

<210> 130
<211> 19
<212> DNA
<213> Homo sapiens

<400> 130
acaaagttct gtgatgcac 19

<210> 131
<211> 20
<212> DNA
<213> Homo sapiens

<400> 131
acaaagttct gtgatgcact 20

<210> 132
<211> 14
<212> DNA
<213> Homo sapiens

<400> 132
ggagtgaaga cacg 14

<210> 133
<211> 15
<212> DNA
<213> Homo sapiens

<400> 133
ggagtgaaga cacgg 15

<210> 134
<211> 16
<212> DNA
<213> Homo sapiens

<400> 134
ggagtgaaga cacgga 16

<210> 135
<211> 14
<212> DNA
<213> Homo sapiens

<400> 135
cactggtaca aggg 14

<210> 136
<211> 15
<212> DNA
<213> Homo sapiens

<400> 136
cactggtaca agggt 15

<210> 137
<211> 16
<212> DNA
<213> Homo sapiens

<400> 137
cactggtaca agggtt 16

<210> 138
<211> 17
<212> DNA
<213> Homo sapiens

<400> 138
cactggtaca agggttg 17

<210> 139
<211> 16
<212> DNA
<213> Homo sapiens

<400> 139
cctcaaggag cttcag 16

<210> 140
<211> 17
<212> DNA
<213> Homo sapiens

<400> 140
cctcaaggag cttcagt 17

<210> 141
<211> 17
<212> DNA
<213> Homo sapiens

<400> 141
cccaagttct gtcatgc 17

<210> 142
<211> 18

<212> DNA
<213> Homo sapiens

<400> 142
tcacttttgt gactatgc 18

<210> 143
<211> 19
<212> DNA
<213> Homo sapiens

<400> 143
tcacttttgt gactatgca 19

<210> 144
<211> 20
<212> DNA
<213> Homo sapiens

<400> 144
tcacttttgt gactatgcaa 20

<210> 145
<211> 13
<212> DNA
<213> Homo sapiens

<400> 145
cgaaggcaac acg 13

<210> 146
<211> 14
<212> DNA
<213> Homo sapiens

<400> 146
cgaaggcaac acgg 14

<210> 147
<211> 15
<212> DNA
<213> Homo sapiens

<400> 147
cgaaggcaac acgga 15

<210> 148
<211> 16
<212> DNA
<213> Homo sapiens

<400> 148
aataggtcaa ccgtgt 16

<210> 149
<211> 17
<212> DNA
<213> Homo sapiens

<400> 149
aataggtcaa ccgtgta 17

<210> 150
<211> 18
<212> DNA
<213> Homo sapiens

<400> 150
aataggtcaa ccgtgtat 18

<210> 151
<211> 19
<212> DNA
<213> Homo sapiens

<400> 151
aataggtcaa ccgtgtatg 19

<210> 152
<211> 20
<212> DNA
<213> Homo sapiens

<400> 152
aataggtcaa ccgtgtatga 20

<210> 153
<211> 21
<212> DNA
<213> Homo sapiens

<400> 153
aataggtcaa ccgtgtatga t 21

<210> 154
<211> 16
<212> DNA
<213> Homo sapiens

<400> 154
cccctatcac gattag 16

<210> 155
<211> 17
<212> DNA
<213> Homo sapiens

<400> 155
cccctatcac gattagc 17

<210> 156
<211> 18
<212> DNA
<213> Homo sapiens

<400> 156
cccctatcac gattagca 18

<210> 157
<211> 19
<212> DNA
<213> Homo sapiens

<400> 157
cccctatcac gattagcat 19

<210> 158
<211> 19
<212> DNA
<213> Homo sapiens

<400> 158
cacaaaccat tatgtgctg 19

<210> 159
<211> 20
<212> DNA
<213> Homo sapiens

<400> 159
cacaaaccat tatgtgctgc 20

<210> 160
<211> 21
<212> DNA
<213> Homo sapiens

<400> 160
cacaaaccat tatgtgctgc t 21

<210> 161
<211> 17
<212> DNA
<213> Homo sapiens

<400> 161
tgtaaaccat gatgtgc 17

<210> 162
<211> 18
<212> DNA
<213> Homo sapiens

<400> 162
tgtaaaccat gatgtgct 18

<210> 163
<211> 19
<212> DNA
<213> Homo sapiens

<400> 163
tgtaaaccat gatgtgctg 19

<210> 164
<211> 17

<212> DNA
<213> Homo sapiens

<400> 164
cgccaatatt tacgtgc 17

<210> 165
<211> 18
<212> DNA
<213> Homo sapiens

<400> 165
cgccaatatt tacgtgct 18

<210> 166
<211> 17
<212> DNA
<213> Homo sapiens

<400> 166
acaagtgcct tcactgc 17

<210> 167
<211> 18
<212> DNA
<213> Homo sapiens

<400> 167
actacctgca ctgtaagc 18

<210> 168
<211> 19
<212> DNA
<213> Homo sapiens

<400> 168
actacctgca ctgtaagca 19

<210> 169
<211> 14
<212> DNA
<213> Homo sapiens

<400> 169
actcaccgac agcg 14

<210> 170
<211> 15
<212> DNA
<213> Homo sapiens

<400> 170
actcaccgac agcgt 15

<210> 171
<211> 16
<212> DNA
<213> Homo sapiens

<400> 171
actcaccgac agcgtt 16

<210> 172
<211> 14
<212> DNA
<213> Homo sapiens

<400> 172
cccaccgaca gcaa 14

<210> 173
<211> 15
<212> DNA
<213> Homo sapiens

<400> 173
cccaccgaca gcaat 15

<210> 174
<211> 17
<212> DNA
<213> Homo sapiens

<400> 174
actcaccgac aggttga 17

<210> 175
<211> 14
<212> DNA
<213> Homo sapiens

<400> 175
aacccaccga caac 14

<210> 176
<211> 15
<212> DNA
<213> Homo sapiens

<400> 176
aacccaccga caaca 15

<210> 177
<211> 16
<212> DNA
<213> Homo sapiens

<400> 177
aacccaccga caacaa 16

<210> 178
<211> 17
<212> DNA
<213> Homo sapiens

<400> 178
aacccaccga caacaat 17

<210> 179
<211> 17
<212> DNA
<213> Homo sapiens

<400> 179
tgtgagttct accattg 17

<210> 180
<211> 18
<212> DNA
<213> Homo sapiens

<400> 180
tgtgagttct accattgc 18

<210> 181
<211> 21
<212> DNA
<213> Homo sapiens

<400> 181
tagttggcaa gtctagaacc a 21

<210> 182
<211> 17
<212> DNA
<213> Homo sapiens

<400> 182
tagttggcaa gtctaga 17

<210> 183
<211> 18
<212> DNA
<213> Homo sapiens

<400> 183
tagttggcaa gtctagaa 18

<210> 184
<211> 19
<212> DNA
<213> Homo sapiens

<400> 184
tagttggcaa gtctagaac 19

<210> 185
<211> 20
<212> DNA
<213> Homo sapiens

<400> 185
tagttggcaa gtctagaacc 20

<210> 186
<211> 17

<212> DNA
<213> Homo sapiens

<400> 186
cagtgaattc taccagtv 17

<210> 187
<211> 18
<212> DNA
<213> Homo sapiens

<400> 187
cagtgaattc taccagtg 18

<210> 188
<211> 19
<212> DNA
<213> Homo sapiens

<400> 188
cagtgaattc taccagtgc 19

<210> 189
<211> 18
<212> DNA
<213> Homo sapiens

<400> 189
acccttatca gttctccg 18

<210> 190
<211> 19
<212> DNA
<213> Homo sapiens

<400> 190
acccttatca gttctccgt 19

<210> 191
<211> 20
<212> DNA
<213> Homo sapiens

<400> 191
acccttatca gttctccgtc 20

<210> 192
<211> 15
<212> DNA
<213> Homo sapiens

<400> 192
gaactgcctt tctct 15

<210> 193
<211> 19
<212> DNA
<213> Homo sapiens

<400> 193
aagcccaaaa ggagaattc 19

<210> 194
<211> 20
<212> DNA
<213> Homo sapiens

<400> 194
aagcccaaaa ggagaattct 20

<210> 195
<211> 21
<212> DNA
<213> Homo sapiens

<400> 195
aagcccaaaa ggagaattct t 21

<210> 196
<211> 12
<212> DNA
<213> Homo sapiens

<400> 196
cggctgcaac ac 12

<210> 197
<211> 13
<212> DNA
<213> Homo sapiens

<400> 197
cggctgcaac aca 13

<210> 198
<211> 14
<212> DNA
<213> Homo sapiens

<400> 198
cggctgcaac acaa 14

<210> 199
<211> 15
<212> DNA
<213> Homo sapiens

<400> 199
cggctgcaac acaag 15

<210> 200
<211> 14
<212> DNA
<213> Homo sapiens

<400> 200
accctccacc atgc 14

<210> 201
<211> 15
<212> DNA
<213> Homo sapiens

<400> 201
accctccacc atgca 15

<210> 202
<211> 20
<212> DNA
<213> Homo sapiens

<400> 202
actgatatca gctcagtagg 20

<210> 203
<211> 17
<212> DNA
<213> Homo sapiens

<400> 203
tatctgcact agatgca 17

<210> 204
<211> 18
<212> DNA
<213> Homo sapiens

<400> 204
tatctgcact agatgcac 18

<210> 205
<211> 19
<212> DNA
<213> Homo sapiens

<400> 205
tatctgcact agatgcacc 19

<210> 206
<211> 20
<212> DNA
<213> Homo sapiens

<400> 206
tatctgcact agatgcacct 20

<210> 207
<211> 17
<212> DNA
<213> Homo sapiens

<400> 207
taactgcact agatgca 17

<210> 208
<211> 18

<212> DNA
<213> Homo sapiens

<400> 208
taactgcact agatgcac 18

<210> 209
<211> 19
<212> DNA
<213> Homo sapiens

<400> 209
taactgcact agatgcaccv 19

<210> 210
<211> 22
<212> DNA
<213> Homo sapiens

<400> 210
acctaatata tcaaacatat ca 22

<210> 211
<211> 14
<212> DNA
<213> Homo sapiens

<400> 211
agctgctttt ggga 14

<210> 212
<211> 15
<212> DNA
<213> Homo sapiens

<400> 212
agctgctttt gggat 15

<210> 213
<211> 16
<212> DNA
<213> Homo sapiens

<400> 213
agctgctttt gggatt 16

<210> 214
<211> 13
<212> DNA
<213> Homo sapiens

<400> 214
ggggacgaaa tcc 13

<210> 215
<211> 14
<212> DNA
<213> Homo sapiens

<400> 215
ggggacgaaa tcca 14

<210> 216
<211> 15
<212> DNA
<213> Homo sapiens

<400> 216
ggggacgaaa tccaa 15

<210> 217
<211> 16
<212> DNA
<213> Homo sapiens

<400> 217
ggggacgaaa tccaag 16

<210> 218
<211> 16
<212> DNA
<213> Homo sapiens

<400> 218
ggctgtcaat tcatag 16

<210> 219
<211> 17
<212> DNA
<213> Homo sapiens

<400> 219
ggctgtcaat tcatagg 17

<210> 220
<211> 18
<212> DNA
<213> Homo sapiens

<400> 220
ggctgtcaat tcataggt 18

<210> 221
<211> 19
<212> DNA
<213> Homo sapiens

<400> 221
ggctgtcaat tcataggtc 19

<210> 222
<211> 15
<212> DNA
<213> Homo sapiens

<400> 222
ctgggacttt gtagg 15

<210> 223
<211> 16
<212> DNA
<213> Homo sapiens

<400> 223
ctgggacttt gtaggc 16

<210> 224
<211> 15
<212> DNA
<213> Homo sapiens

<400> 224
aaagcgggac tttga 15

<210> 225
<211> 16
<212> DNA
<213> Homo sapiens

<400> 225
aaagcgggac tttgag 16

<210> 226
<211> 17
<212> DNA
<213> Homo sapiens

<400> 226
aaagcgggac tttgagg 17

<210> 227
<211> 15
<212> DNA
<213> Homo sapiens

<400> 227
tccacatgga gttgc 15

<210> 228
<211> 16
<212> DNA
<213> Homo sapiens

<400> 228
tccacatgga gttgct 16

<210> 229
<211> 18
<212> DNA
<213> Homo sapiens

<400> 229
gccaatattt ctgtgctg 18

<210> 230
<211> 16

<212> DNA
<213> Homo sapiens

<400> 230
ccaacaacat gaaact 16

<210> 231
<211> 17
<212> DNA
<213> Homo sapiens

<400> 231
ccaacaacat gaaacta 17

<210> 232
<211> 18
<212> DNA
<213> Homo sapiens

<400> 232
ccaacaacat gaaactac 18

<210> 233
<211> 19
<212> DNA
<213> Homo sapiens

<400> 233
ccaacaacat gaaactacc 19

<210> 234
<211> 15
<212> DNA
<213> Homo sapiens

<400> 234
ccaacaacag gaaac 15

<210> 235
<211> 16
<212> DNA
<213> Homo sapiens

<400> 235
ccaacaacag gaaact 16

<210> 236
<211> 17
<212> DNA
<213> Homo sapiens

<400> 236
ccaacaacag gaaacta 17

<210> 237
<211> 18
<212> DNA
<213> Homo sapiens

<400> 237
ccaacaacag gaaactac 18

<210> 238
<211> 13
<212> DNA
<213> Homo sapiens

<400> 238
gctgggtgga gaa 13

<210> 239
<211> 14
<212> DNA
<213> Homo sapiens

<400> 239
gctgggtgga gaagv 14

<210> 240
<211> 15
<212> DNA
<213> Homo sapiens

<400> 240
gctgggtgga gaagg 15

<210> 241
<211> 15
<212> DNA
<213> Homo sapiens

<400> 241
cctatctccc ctctg 15

<210> 242
<211> 16
<212> DNA
<213> Homo sapiens

<400> 242
cctatctccc ctctgg 16

<210> 243
<211> 17
<212> DNA
<213> Homo sapiens

<400> 243
cctatctccc ctctgga 17

<210> 244
<211> 19
<212> DNA
<213> Homo sapiens

<400> 244
gaacaggtag tctgaacac 19

<210> 245
<211> 18
<212> DNA
<213> Homo sapiens

<400> 245
aaccaatgtg cagactac 18

<210> 246
<211> 19
<212> DNA
<213> Homo sapiens

<400> 246
aaccaatgtg cagactact 19

<210> 247
<211> 21
<212> DNA
<213> Homo sapiens

<400> 247
gaacagatag tctaaacact g 21

<210> 248
<211> 20
<212> DNA
<213> Homo sapiens

<400> 248
tcagttttgc atagatttgc 20

<210> 249
<211> 21
<212> DNA
<213> Homo sapiens

<400> 249
tcagttttgc atagatttgc a 21

<210> 250
<211> 19
<212> DNA
<213> Homo sapiens

<400> 250
tcagttttgc atggatttg 19

<210> 251
<211> 17
<212> DNA
<213> Homo sapiens

<400> 251
acatcgttac cagacag 17

<210> 252
<211> 18

<212> DNA
<213> Homo sapiens

<400> 252
acatcgttac cagacagt 18

<210> 253
<211> 14
<212> DNA
<213> Homo sapiens

<400> 253
tccagcactg tccg 14

<210> 254
<211> 15
<212> DNA
<213> Homo sapiens

<400> 254
tccagcactg tccgg 15

<210> 255
<211> 16
<212> DNA
<213> Homo sapiens

<400> 255
gtcatcatta ccaggc 16

<210> 256
<211> 17
<212> DNA
<213> Homo sapiens

<400> 256
gtcatcatta ccaggca 17

<210> 257
<211> 18
<212> DNA
<213> Homo sapiens

<400> 257
gtcatcatta ccaggcag 18

<210> 258
<211> 14
<212> DNA
<213> Homo sapiens

<400> 258
ccatcattac ccgg 14

<210> 259
<211> 15
<212> DNA
<213> Homo sapiens

<400> 259
ccatcattac ccggc 15

<210> 260
<211> 16
<212> DNA
<213> Homo sapiens

<400> 260
ccatcattac ccggca 16

<210> 261
<211> 14
<212> DNA
<213> Homo sapiens

<400> 261
ttttcccatg ccct 14

<210> 262
<211> 15
<212> DNA
<213> Homo sapiens

<400> 262
ttttcccatg cccta 15

<210> 263
<211> 16
<212> DNA
<213> Homo sapiens

<400> 263
ttttcccatg ccctat 16

<210> 264
<211> 17
<212> DNA
<213> Homo sapiens

<400> 264
ttttcccatg ccctata 17

<210> 265
<211> 18
<212> DNA
<213> Homo sapiens

<400> 265
ttttcccatg ccctatac 18

<210> 266
<211> 19
<212> DNA
<213> Homo sapiens

<400> 266
ttttcccatg ccctatacc 19

<210> 267
<211> 20
<212> DNA
<213> Homo sapiens

<400> 267
aaagaagtat atgcatagga 20

<210> 268
<211> 21
<212> DNA
<213> Homo sapiens

<400> 268
aaagaagtat atgcatagga a 21

<210> 269
<211> 22
<212> DNA
<213> Homo sapiens

<400> 269
aaagaagtat atgcatagga aa 22

<210> 270
<211> 16
<212> DNA
<213> Homo sapiens

<400> 270
ctagtggtcc taaaca 16

<210> 271
<211> 17
<212> DNA
<213> Homo sapiens

<400> 271
ctagtggtcc taaacat 17

<210> 272
<211> 18
<212> DNA
<213> Homo sapiens

<400> 272
ctagtggtcc taaacatt 18

<210> 273
<211> 19
<212> DNA
<213> Homo sapiens

<400> 273
ctagtggtcc taaacattt 19

<210> 274
<211> 20

<212> DNA
<213> Homo sapiens

<400> 274
ctagtggtcc taaacatttc 20

<210> 275
<211> 21
<212> DNA
<213> Homo sapiens

<400> 275
ctagtggtcc taaacatttc a 21

<210> 276
<211> 19
<212> DNA
<213> Homo sapiens

<400> 276
aggcatagga tgacaaagg 19

<210> 277
<211> 13
<212> DNA
<213> Homo sapiens

<400> 277
cagactccgg tgg 13

<210> 278
<211> 14
<212> DNA
<213> Homo sapiens

<400> 278
cagactccgg tgga 14

<210> 279
<211> 15
<212> DNA
<213> Homo sapiens

<400> 279
cagactccgg tggaa 15

<210> 280
<211> 16
<212> DNA
<213> Homo sapiens

<400> 280
cagactccgg tggaat 16

<210> 281
<211> 14
<212> DNA
<213> Homo sapiens

<400> 281
ccacacactt cctt 14

<210> 282
<211> 15
<212> DNA
<213> Homo sapiens

<400> 282
ccacacactt cctta 15

<210> 283
<211> 16
<212> DNA
<213> Homo sapiens

<400> 283
ccacacactt ccttac 16

<210> 284
<211> 17
<212> DNA
<213> Homo sapiens

<400> 284
ccacacactt ccttaca 17

<210> 285
<211> 18
<212> DNA
<213> Homo sapiens

<400> 285
ccacacactt ccttacat 18

<210> 286
<211> 19
<212> DNA
<213> Homo sapiens

<400> 286
ccacacactt ccttacatt ' 19

<210> 287
<211> 16
<212> DNA
<213> Homo sapiens

<400> 287
acaagctttt tgctcg 16

<210> 288
<211> 18
<212> DNA
<213> Homo sapiens

<400> 288
acaagctttt tgctcgtc 18

<210> 289
<211> 19
<212> DNA
<213> Homo sapiens

<400> 289
acaagctttt tgctcgtct 19

<210> 290
<211> 17
<212> DNA
<213> Homo sapiens

<400> 290
ctacctgcac tataagc 17

<210> 291
<211> 18
<212> DNA
<213> Homo sapiens

<400> 291
ctacctgcac tataagca 18

<210> 292
<211> 19
<212> DNA
<213> Homo sapiens

<400> 292
ctacctgcac tataagcac 19

<210> 293
<211> 20
<212> DNA
<213> Homo sapiens

<400> 293
ctacctgcac tataagcact 20

<210> 294
<211> 16
<212> DNA
<213> Homo sapiens

<400> 294
ctacctgcac tatgag 16

<210> 295
<211> 17
<212> DNA
<213> Homo sapiens

<400> 295
ctacctgcac tatgagc 17

<210> 296
<211> 18

<212> DNA
<213> Homo sapiens

<400> 296
ctacctgcac tatgagca 18

<210> 297
<211> 19
<212> DNA
<213> Homo sapiens

<400> 297
tcaacatcag tctgataag 19

<210> 298
<211> 20
<212> DNA
<213> Homo sapiens

<400> 298
tcaacatcag tctgataagc 20

<210> 299
<211> 21
<212> DNA
<213> Homo sapiens

<400> 299
tcaacatcag tctgataagc t 21

<210> 300
<211> 14
<212> DNA
<213> Homo sapiens

<400> 300
tcagccgctg tcac 14

<210> 301
<211> 18
<212> DNA
<213> Homo sapiens

<400> 301
aggcgaagga tgacaaag 18

<210> 302
<211> 12
<212> DNA
<213> Homo sapiens

<400> 302
ggccgtgact gg 12

<210> 303
<211> 13
<212> DNA
<213> Homo sapiens

<400> 303
ggccgtgact gga 13

<210> 304
<211> 14
<212> DNA
<213> Homo sapiens

<400> 304
ggccgtgact ggag 14

<210> 305
<211> 15
<212> DNA
<213> Homo sapiens

<400> 305
ggtacaatca acggt 15

<210> 306
<211> 16
<212> DNA
<213> Homo sapiens

<400> 306
ggtacaatca acggtc 16

<210> 307
<211> 17
<212> DNA
<213> Homo sapiens

<400> 307
ggtacaatca acggtcg 17

<210> 308
<211> 14
<212> DNA
<213> Homo sapiens

<400> 308
ctgcctgtct gtgc 14

<210> 309
<211> 17
<212> DNA
<213> Homo sapiens

<400> 309
gtctgtcaat tcatagg 17

<210> 310
<211> 18
<212> DNA
<213> Homo sapiens

<400> 310
gtctgtcaat tcataggt 18

<210> 311
<211> 19
<212> DNA
<213> Homo sapiens

<400> 311
gtctgtcaat tcataggtc 19

<210> 312
<211> 20
<212> DNA
<213> Homo sapiens

<400> 312
gtctgtcaat tcataggtca 20

<210> 313
<211> 21
<212> DNA
<213> Homo sapiens

<400> 313
gtctgtcaat tcataggtca t 21

<210> 314
<211> 13
<212> DNA
<213> Homo sapiens

<400> 314
cacagttgcc agc 13

<210> 315
<211> 14
<212> DNA
<213> Homo sapiens

<400> 315
cacagttgcc agct 14

<210> 316
<211> 15
<212> DNA
<213> Homo sapiens

<400> 316
cacagttgcc agctg 15

<210> 317
<211> 16
<212> DNA
<213> Homo sapiens

<400> 317
cacagttgcc agctga 16

<210> 318
<211> 16

<212> DNA
<213> Homo sapiens

<400> 318
atccaatcag ttcctg 16

<210> 319
<211> 17
<212> DNA
<213> Homo sapiens

<400> 319
atccaatcag ttcctga 17

<210> 320
<211> 18
<212> DNA
<213> Homo sapiens

<400> 320
atccaatcag ttcctgat 18

<210> 321
<211> 19
<212> DNA
<213> Homo sapiens

<400> 321
atccaatcag ttcctgatg 19

<210> 322
<211> 19
<212> DNA
<213> Homo sapiens

<400> 322
acatggttag atcaagcac 19

<210> 323
<211> 20
<212> DNA
<213> Homo sapiens

<400> 323
acatggttag atcaagcaca 20

<210> 324
<211> 15
<212> DNA
<213> Homo sapiens

<400> 324
agaattgcgt ttgga 15

<210> 325
<211> 16
<212> DNA
<213> Homo sapiens

<400> 325
agaattgcgt ttggac 16

<210> 326
<211> 17
<212> DNA
<213> Homo sapiens

<400> 326
agaattgcgt ttggaca 17

<210> 327
<211> 18
<212> DNA
<213> Homo sapiens

<400> 327
agaattgcgt ttggacaa 18

<210> 328
<211> 19
<212> DNA
<213> Homo sapiens

<400> 328
agaattgcgt ttggacaat 19

<210> 329
<211> 20
<212> DNA
<213> Homo sapiens

<400> 329
agaattgcgt ttggacaatc 20

<210> 330
<211> 17
<212> DNA
<213> Homo sapiens

<400> 330
acagttcttc aactggc 17

<210> 331
<211> 18
<212> DNA
<213> Homo sapiens

<400> 331
acagttcttc aactggca 18

<210> 332
<211> 16
<212> DNA
<213> Homo sapiens

<400> 332
aaagtgtcag atacgg 16

<210> 333
<211> 17
<212> DNA
<213> Homo sapiens

<400> 333
aaagtgtcag atacggt 17

<210> 334
<211> 18
<212> DNA
<213> Homo sapiens

<400> 334
aaagtgtcag atacggtg 18

<210> 335
<211> 19
<212> DNA
<213> Homo sapiens

<400> 335
aaagtgtcag atacggtgt 19

<210> 336
<211> 18
<212> DNA
<213> Homo sapiens

<400> 336
gaaacccagc agacaatg 18

<210> 337
<211> 16
<212> DNA
<213> Homo sapiens

<400> 337
gagacccagt agccag 16

<210> 338
<211> 19
<212> DNA
<213> Homo sapiens

<400> 338
ggggtatttg acaaactga 19

<210> 339
<211> 20
<212> DNA
<213> Homo sapiens

<400> 339
ggggtatttg acaaactgac 20

<210> 340
<211> 21

<212> DNA
<213> Homo sapiens

<400> 340
ggggtatttg acaaactgac a 21

<210> 341
<211> 19
<212> DNA
<213> Homo sapiens

<400> 341
taaacggaac cactagtga 19

<210> 342
<211> 20
<212> DNA
<213> Homo sapiens

<400> 342
taaacggaac cactagtgac 20

<210> 343
<211> 21
<212> DNA
<213> Homo sapiens

<400> 343
taaacggaac cactagtgac t 21

<210> 344
<211> 17
<212> DNA
<213> Homo sapiens

<400> 344
ggaaatccct ggcaatg 17

<210> 345
<211> 17
<212> DNA
<213> Homo sapiens

<400> 345
ggtaatccct ggcaatg 17

<210> 346
<211> 17
<212> DNA
<213> Homo sapiens

<400> 346
ctgttcctgc tgaactg 17

<210> 347
<211> 17
<212> DNA
<213> Homo sapiens

<400> 347
tcagaccgag acaagtg 17

<210> 348
<211> 16
<212> DNA
<213> Homo sapiens

<400> 348
gcctatcctg gattac 16

<210> 349
<211> 17
<212> DNA
<213> Homo sapiens

<400> 349
gcctatcctg gattact 17

<210> 350
<211> 18
<212> DNA
<213> Homo sapiens

<400> 350
gcctatcctg gattactt 18

<210> 351
<211> 18
<212> DNA
<213> Homo sapiens

<400> 351
aacctatcct gaattact 18

<210> 352
<211> 19
<212> DNA
<213> Homo sapiens

<400> 352
aacctatcct gaattactt 19

<210> 353
<211> 20
<212> DNA
<213> Homo sapiens

<400> 353
aacctatcct gaattacttg 20

<210> 354
<211> 21
<212> DNA
<213> Homo sapiens

<400> 354
aacctatcct gaattacttg a 21

<210> 355
<211> 22
<212> DNA
<213> Homo sapiens

<400> 355
aacctatcct gaattacttg aa 22

<210> 356
<211> 15
<212> DNA
<213> Homo sapiens

<400> 356
gcggaactta gccac 15

<210> 357
<211> 16
<212> DNA
<213> Homo sapiens

<400> 357
gcggaactta gccact 16

<210> 358
<211> 17
<212> DNA
<213> Homo sapiens

<400> 358
gcagaactta gccactg 17

<210> 359
<211> 18
<212> DNA
<213> Homo sapiens

<400> 359
gcagaactta gccactgt 18

<210> 360
<211> 17
<212> DNA
<213> Homo sapiens

<400> 360
ctcaatagac tgtgagc 17

<210> 361
<211> 18
<212> DNA
<213> Homo sapiens

<400> 361
ctcaatagac tgtgagct 18

<210> 362
<211> 19

<212> DNA
<213> Homo sapiens

<400> 362
ctcaatagac tgtgagctc 19

<210> 363
<211> 15
<212> DNA
<213> Homo sapiens

<400> 363
acaggattga ggggg 15

<210> 364
<211> 15
<212> DNA
<213> Homo sapiens

<400> 364
aagcggttta ccatc 15

<210> 365
<211> 16
<212> DNA
<213> Homo sapiens

<400> 365
aagcggttta ccatcc 16

<210> 366
<211> 17
<212> DNA
<213> Homo sapiens

<400> 366
aagcggttta ccatccc 17

<210> 367
<211> 15
<212> DNA
<213> Homo sapiens

<400> 367
atgtatgtgg gacgg 15

<210> 368
<211> 16
<212> DNA
<213> Homo sapiens

<400> 368
atgtatgtgg gacggt 16

<210> 369
<211> 17
<212> DNA
<213> Homo sapiens

<400> 369
atgtatgtgg gacggta 17

<210> 370
<211> 18
<212> DNA
<213> Homo sapiens

<400> 370
atgtatgtgg gacggtaa 18

<210> 371
<211> 19
<212> DNA
<213> Homo sapiens

<400> 371
atgtatgtgg gacggtaaa 19

<210> 372
<211> 18
<212> DNA
<213> Homo sapiens

<400> 372
aaccgatttc agatggtg 18

<210> 373
<211> 20
<212> DNA
<213> Homo sapiens

<400> 373
aacactgatt tcaaatggtg 20

<210> 374
<211> 21
<212> DNA
<213> Homo sapiens

<400> 374
aacactgatt tcaaatggtg c 21

<210> 375
<211> 22
<212> DNA
<213> Homo sapiens

<400> 375
aacactgatt tcaaatggtg ct 22

<210> 376
<211> 18
<212> DNA
<213> Homo sapiens

<400> 376
accgatttca aatggtgc 18

<210> 377
<211> 19
<212> DNA
<213> Homo sapiens

<400> 377
accgatttca aatggtgct 19

<210> 378
<211> 20
<212> DNA
<213> Homo sapiens

<400> 378
accgatttca aatggtgcta 20

<210> 379
<211> 19
<212> DNA
<213> Homo sapiens

<400> 379
gctttgacaa tactattgc 19

<210> 380
<211> 20
<212> DNA
<213> Homo sapiens

<400> 380
gctttgacaa tactattgca 20

<210> 381
<211> 18
<212> DNA
<213> Homo sapiens

<400> 381
tcaccaaaac atggaagc 18

<210> 382
<211> 19
<212> DNA
<213> Homo sapiens

<400> 382
tcaccaaaac atggaagca 19

<210> 383
<211> 20
<212> DNA
<213> Homo sapiens

<400> 383
tcaccaaaac atggaagcac 20

<210> 384
<211> 17

<212> DNA
<213> Homo sapiens

<400> 384
aaagcaagta catccac 17

<210> 385
<211> 18
<212> DNA
<213> Homo sapiens

<400> 385
aaagcaagta catccacg 18

<210> 386
<211> 19
<212> DNA
<213> Homo sapiens

<400> 386
aaagcaagta catccacgt 19

<210> 387
<211> 20
<212> DNA
<213> Homo sapiens

<400> 387
ctactaaaac atggaagcac 20

<210> 388
<211> 21
<212> DNA
<213> Homo sapiens

<400> 388
ctactaaaac atggaagcac t 21

<210> 389
<211> 16
<212> DNA
<213> Homo sapiens

<400> 389
agaaagcact tccatg 16

<210> 390
<211> 17
<212> DNA
<213> Homo sapiens

<400> 390
agaaagcact tccatgt 17

<210> 391
<211> 18
<212> DNA
<213> Homo sapiens

<400> 391
agaaagcact tccatgtt 18

<210> 392
<211> 19
<212> DNA
<213> Homo sapiens

<400> 392
agaaagcact tccatgtta 19

<210> 393
<211> 20
<212> DNA
<213> Homo sapiens

<400> 393
agaaagcact tccatgttaa 20

<210> 394
<211> 21
<212> DNA
<213> Homo sapiens

<400> 394
agaaagcact tccatgttaa a 21

<210> 395
<211> 15
<212> DNA
<213> Homo sapiens

<400> 395
ccactgaaac atgga 15

<210> 396
<211> 16
<212> DNA
<213> Homo sapiens

<400> 396
ccactgaaac atggaa 16

<210> 397
<211> 17
<212> DNA
<213> Homo sapiens

<400> 397
ccactgaaac atggaag 17

<210> 398
<211> 14
<212> DNA
<213> Homo sapiens

<400> 398
cagcaggtac cccc 14

<210> 399
<211> 19
<212> DNA
<213> Homo sapiens

<400> 399
acactcaaac atggaagca 19

<210> 400
<211> 15
<212> DNA
<213> Homo sapiens

<400> 400
gctgcaaaca tccga 15

<210> 401
<211> 16
<212> DNA
<213> Homo sapiens

<400> 401
gctgcaaaca tccgac 16

<210> 402
<211> 15
<212> DNA
<213> Homo sapiens

<400> 402
cttccagtcg aggat 15

<210> 403
<211> 16
<212> DNA
<213> Homo sapiens

<400> 403
cttccagtcg aggatg 16

<210> 404
<211> 17
<212> DNA
<213> Homo sapiens

<400> 404
cttccagtcg aggatgt 17

<210> 405
<211> 16
<212> DNA
<213> Homo sapiens

<400> 405
agctgagtgt aggatg 16

<210> 406
<211> 17

<212> DNA
<213> Homo sapiens

<400> 406
agctgagtgt aggatgt 17

<210> 407
<211> 18
<212> DNA
<213> Homo sapiens

<400> 407
agctgagtgt aggatgtt 18

<210> 408
<211> 19
<212> DNA
<213> Homo sapiens

<400> 408
agctgagtgt aggatgttt 19

<210> 409
<211> 20
<212> DNA
<213> Homo sapiens

<400> 409
agctgagtgt aggatgttta 20

<210> 410
<211> 17
<212> DNA
<213> Homo sapiens

<400> 410
gctgagagtg taggatg 17

<210> 411
<211> 18
<212> DNA
<213> Homo sapiens

<400> 411
gctgagagtg taggatgt 18

<210> 412
<211> 14
<212> DNA
<213> Homo sapiens

<400> 412
cttccagtcg ggga 14

<210> 413
<211> 15
<212> DNA
<213> Homo sapiens

<400> 413
cttccagtcg gggat 15

<210> 414
<211> 16
<212> DNA
<213> Homo sapiens

<400> 414
gctgtaaaca tccgac 16

<210> 415
<211> 17
<212> DNA
<213> Homo sapiens

<400> 415
gctgtaaaca tccgact 17

<210> 416
<211> 18
<212> DNA
<213> Homo sapiens

<400> 416
gctgtaaaca tccgactg 18

<210> 417
<211> 20
<212> DNA
<213> Homo sapiens

<400> 417
tccagtcaag gatgtttaca 20

<210> 418
<211> 14
<212> DNA
<213> Homo sapiens

<400> 418
cagctatgcc agca 14

<210> 419
<211> 15
<212> DNA
<213> Homo sapiens

<400> 419
cagctatgcc agcat 15

<210> 420
<211> 16
<212> DNA
<213> Homo sapiens

<400> 420
cagctatgcc agcatc 16

<210> 421
<211> 17
<212> DNA
<213> Homo sapiens

<400> 421
cagctatgcc agcatct 17

<210> 422
<211> 18
<212> DNA
<213> Homo sapiens

<400> 422
cagctatgcc agcatctt 18

<210> 423
<211> 19
<212> DNA
<213> Homo sapiens

<400> 423
gcaacttagt aatgtgcaa 19

<210> 424
<211> 20
<212> DNA
<213> Homo sapiens

<400> 424
gcaacttagt aatgtgcaat 20

<210> 425
<211> 21
<212> DNA
<213> Homo sapiens

<400> 425
gcaacttagt aatgtgcaat a 21

<210> 426
<211> 14
<212> DNA
<213> Homo sapiens

<400> 426
ttcgccctct caac 14

<210> 427
<211> 15
<212> DNA
<213> Homo sapiens

<400> 427
ttcgccctct caacc 15

<210> 428
<211> 16

<212> DNA
<213> Homo sapiens

<400> 428
ttcgccctct caaccc 16

<210> 429
<211> 13
<212> DNA
<213> Homo sapiens

<400> 429
agaggtcgac cgt 13

<210> 430
<211> 14
<212> DNA
<213> Homo sapiens

<400> 430
agaggtcgac cgtg 14

<210> 431
<211> 15
<212> DNA
<213> Homo sapiens

<400> 431
agaggtcgac cgtgt 15

<210> 432
<211> 16
<212> DNA
<213> Homo sapiens

<400> 432
agaggtcgac cgtgta 16

<210> 433
<211> 17
<212> DNA
<213> Homo sapiens

<400> 433
agaggtcgac cgtgtaa 17

<210> 434
<211> 18
<212> DNA
<213> Homo sapiens

<400> 434
agaggtcgac cgtgtaat 18

<210> 435
<211> 14
<212> DNA
<213> Homo sapiens

<400> 435
ccagcagcac ctgg 14

<210> 436
<211> 15
<212> DNA
<213> Homo sapiens

<400> 436
ccagcagcac ctggg 15

<210> 437
<211> 16
<212> DNA
<213> Homo sapiens

<400> 437
ccagcagcac ctgggg 16

<210> 438
<211> 16
<212> DNA
<213> Homo sapiens

<400> 438
acaccaatgc cctagg 16

<210> 439
<211> 16
<212> DNA
<213> Homo sapiens

<400> 439
acacttactg gacacc 16

<210> 440
<211> 17
<212> DNA
<213> Homo sapiens

<400> 440
acacttactg gacacct 17

<210> 441
<211> 18
<212> DNA
<213> Homo sapiens

<400> 441
acacttactg gacaccta 18

<210> 442
<211> 19
<212> DNA
<213> Homo sapiens

<400> 442
acacttactg gacacctac 19

<210> 443
<211> 20
<212> DNA
<213> Homo sapiens

<400> 443
acacttactg gacacctact 20

<210> 444
<211> 13
<212> DNA
<213> Homo sapiens

<400> 444
ctggaggaag ggc 13

<210> 445
<211> 14
<212> DNA
<213> Homo sapiens

<400> 445
ctggaggaag ggcc 14

<210> 446
<211> 16
<212> DNA
<213> Homo sapiens

<400> 446
acggaagggc agagag 16

<210> 447
<211> 17
<212> DNA
<213> Homo sapiens

<400> 447
acggaagggc agagagg 17

<210> 448
<211> 18
<212> DNA
<213> Homo sapiens

<400> 448
acggaagggc agagaggg 18

<210> 449
<211> 16
<212> DNA
<213> Homo sapiens

<400> 449
aaagaggtta accagg 16

<210> 450
<211> 17

<212> DNA
<213> Homo sapiens

<400> 450
aaagaggtta accaggt 17

<210> 451
<211> 18
<212> DNA
<213> Homo sapiens

<400> 451
aaagaggtta accaggtg 18

<210> 452
<211> 19
<212> DNA
<213> Homo sapiens

<400> 452
aaagaggtta accaggtgt 19

<210> 453
<211> 19
<212> DNA
<213> Homo sapiens

<400> 453
caatgcaact acaatgcac 19

<210> 454
<211> 13
<212> DNA
<213> Homo sapiens

<400> 454
tctctgcagg ccg 13

<210> 455
<211> 14
<212> DNA
<213> Homo sapiens

<400> 455
tctctgcagg ccgt 14

<210> 456
<211> 15
<212> DNA
<213> Homo sapiens

<400> 456
tctctgcagg ccgtg 15

<210> 457
<211> 18
<212> DNA
<213> Homo sapiens

<400> 457
ttctaggata ggcccagg 18

<210> 458
<211> 19
<212> DNA
<213> Homo sapiens

<400> 458
acatttttcg ttattgctc 19

<210> 459
<211> 20
<212> DNA
<213> Homo sapiens

<400> 459
acatttttcg ttattgctct 20

<210> 460
<211> 21
<212> DNA
<213> Homo sapiens

<400> 460
acatttttcg ttattgctct t 21

<210> 461
<211> 22
<212> DNA
<213> Homo sapiens

<400> 461
acatttttcg ttattgctct tg 22

<210> 462
<211> 18
<212> DNA
<213> Homo sapiens

<400> 462
aaaggcatca tataggag 18

<210> 463
<211> 19
<212> DNA
<213> Homo sapiens

<400> 463
aaaggcatca tataggagc 19

<210> 464
<211> 20
<212> DNA
<213> Homo sapiens

<400> 464
aaaggcatca tataggagct 20

<210> 465
<211> 21
<212> DNA
<213> Homo sapiens

<400> 465
aaaggcatca tataggagct g 21

<210> 466
<211> 21
<212> DNA
<213> Homo sapiens

<400> 466
tcaacaaaat cactgatgct g 21

<210> 467
<211> 22
<212> DNA
<213> Homo sapiens

<400> 467
tcaacaaaat cactgatgct gg 22

<210> 468
<211> 23
<212> DNA
<213> Homo sapiens

<400> 468
tcaacaaaat cactgatgct gga 23

<210> 469
<211> 16
<212> DNA
<213> Homo sapiens

<400> 469
tgagctcctg gaggac 16

<210> 470
<211> 20
<212> DNA
<213> Homo sapiens

<400> 470
ggctataaag taactgagac 20

<210> 471
<211> 21
<212> DNA
<213> Homo sapiens

<400> 471
ggctataaag taactgagac g 21

<210> 472
<211> 15

<212> DNA
<213> Homo sapiens

<400> 472
gacgggtgcg atttc 15

<210> 473
<211> 16
<212> DNA
<213> Homo sapiens

<400> 473
gacgggtgcg atttct 16

<210> 474
<211> 15
<212> DNA
<213> Homo sapiens

<400> 474
gccctggact aggag 15

<210> 475
<211> 16
<212> DNA
<213> Homo sapiens

<400> 475
gccctggact aggagt 16

<210> 476
<211> 14
<212> DNA
<213> Homo sapiens

<400> 476
agaggcaggc atgc 14

<210> 477
<211> 15
<212> DNA
<213> Homo sapiens

<400> 477
agaggcaggc atgcg 15

<210> 478
<211> 19
<212> DNA
<213> Homo sapiens

<400> 478
aacaaccagc taagacact 19

<210> 479
<211> 20
<212> DNA
<213> Homo sapiens

<400> 479
aacaaccagc taagacactg 20

<210> 480
<211> 21
<212> DNA
<213> Homo sapiens

<400> 480
aacaaccagc taagacactg c 21

<210> 481
<211> 17
<212> DNA
<213> Homo sapiens

<400> 481
caatcagcta atgacac 17

<210> 482
<211> 18
<212> DNA
<213> Homo sapiens

<400> 482
caatcagcta atgacact 18

<210> 483
<211> 19
<212> DNA
<213> Homo sapiens

<400> 483
caatcagcta atgacactg 19

<210> 484
<211> 18
<212> DNA
<213> Homo sapiens

<400> 484
gcaatcagct aactacac 18

<210> 485
<211> 19
<212> DNA
<213> Homo sapiens

<400> 485
gcaatcagct aactacact 19

<210> 486
<211> 15
<212> DNA
<213> Homo sapiens

<400> 486
gtacccctgg agatt 15

<210> 487
<211> 15
<212> DNA
<213> Homo sapiens

<400> 487
ctcacaccta ggttc 15

<210> 488
<211> 16
<212> DNA
<213> Homo sapiens

<400> 488
ctcacaccta ggttcc 16

<210> 489
<211> 17
<212> DNA
<213> Homo sapiens

<400> 489
ctcacaccta ggttcca 17

<210> 490
<211> 18
<212> DNA
<213> Homo sapiens

<400> 490
ctcacaccta ggttccaa 18

<210> 491
<211> 17
<212> DNA
<213> Homo sapiens

<400> 491
ttacagatgg ataccgt 17

<210> 492
<211> 18
<212> DNA
<213> Homo sapiens

<400> 492
ttacagatgg ataccgtg 18

<210> 493
<211> 19
<212> DNA
<213> Homo sapiens

<400> 493
ttacagatgg ataccgtgc 19

<210> 494
<211> 18

<212> DNA
<213> Homo sapiens

<400> 494
ataaggattt ttaggggc 18

<210> 495
<211> 19
<212> DNA
<213> Homo sapiens

<400> 495
ataaggattt ttaggggca 19

<210> 496
<211> 20
<212> DNA
<213> Homo sapiens

<400> 496
ataaggattt ttaggggcat 20

<210> 497
<211> 21
<212> DNA
<213> Homo sapiens

<400> 497
ataaggattt ttaggggcat t 21

<210> 498
<211> 22
<212> DNA
<213> Homo sapiens

<400> 498
ataaggattt ttaggggcat ta 22

<210> 499
<211> 18
<212> DNA
<213> Homo sapiens

<400> 499
tcaccattgc taaagtgc 18

<210> 500
<211> 19
<212> DNA
<213> Homo sapiens

<400> 500
tcaccattgc taaagtgca 19

<210> 501
<211> 21
<212> DNA
<213> Homo sapiens

<400> 501
aaacgtggaa tttcctctat g 21

<210> 502
<211> 19
<212> DNA
<213> Homo sapiens

<400> 502
aaagatcaac catgtatta 19

<210> 503
<211> 20
<212> DNA
<213> Homo sapiens

<400> 503
aaagatcaac catgtattat 20

<210> 504
<211> 21
<212> DNA
<213> Homo sapiens

<400> 504
aaagatcaac catgtattat t 21

<210> 505
<211> 16
<212> DNA
<213> Homo sapiens

<400> 505
gcgaatataa cacggt 16

<210> 506
<211> 17
<212> DNA
<213> Homo sapiens

<400> 506
gcgaatataa cacggtc 17

<210> 507
<211> 18
<212> DNA
<213> Homo sapiens

<400> 507
gcgaatataa cacggtcg 18

<210> 508
<211> 14
<212> DNA
<213> Homo sapiens

<400> 508
ccaggttcca cccc 14

<210> 509
<211> 16
<212> DNA
<213> Homo sapiens

<400> 509
acactcaaaa gatggc 16

<210> 510
<211> 17
<212> DNA
<213> Homo sapiens

<400> 510
acactcaaaa gatggcg 17

<210> 511
<211> 18
<212> DNA
<213> Homo sapiens

<400> 511
acactcaaaa gatggcgg 18

<210> 512
<211> 15
<212> DNA
<213> Homo sapiens

<400> 512
acgctcaaat gtcgc 15

<210> 513
<211> 16
<212> DNA
<213> Homo sapiens

<400> 513
acgctcaaat gtcgca 16

<210> 514
<211> 17
<212> DNA
<213> Homo sapiens

<400> 514
acgctcaaat gtcgcag 17

<210> 515
<211> 16
<212> DNA
<213> Homo sapiens

<400> 515
acaccccaaa atcgaa 16

<210> 516
<211> 17

<212> DNA
<213> Homo sapiens

<400> 516
acaccccaaa atcgaag 17

<210> 517
<211> 13
<212> DNA
<213> Homo sapiens

<400> 517
ggaaagcgcc ccc 13

<210> 518
<211> 22
<212> DNA
<213> Homo sapiens

<400> 518
cacttatcag gttgtattat aa 22

<210> 519
<211> 12
<212> DNA
<213> Homo sapiens

<400> 519
tcacgcgagc cg 12

<210> 520
<211> 13
<212> DNA
<213> Homo sapiens

<400> 520
tcacgcgagc cga 13

<210> 521
<211> 14
<212> DNA
<213> Homo sapiens

<400> 521
tcacgcgagc cgaa 14

<210> 522
<211> 19
<212> DNA
<213> Homo sapiens

<400> 522
acgtggattt tcctctatg 19

<210> 523
<211> 20
<212> DNA
<213> Homo sapiens

<400> 523
acgtggattt tcctctatga 20

<210> 524
<211> 21
<212> DNA
<213> Homo sapiens

<400> 524
acgtggattt tcctctatga t 21

<210> 525
<211> 17
<212> DNA
<213> Homo sapiens

<400> 525
aacatggatt ttcctct 17

<210> 526
<211> 18
<212> DNA
<213> Homo sapiens

<400> 526
aacatggatt ttcctcta 18

<210> 527
<211> 19
<212> DNA
<213> Homo sapiens

<400> 527
aacatggatt ttcctctat 19

<210> 528
<211> 20
<212> DNA
<213> Homo sapiens

<400> 528
aacatggatt ttcctctatg 20

<210> 529
<211> 21
<212> DNA
<213> Homo sapiens

<400> 529
aacatggatt ttcctctatg a 21

<210> 530
<211> 22
<212> DNA
<213> Homo sapiens

<400> 530
aacatggatt ttcctctatg at 22

<210> 531
<211> 18
<212> DNA
<213> Homo sapiens

<400> 531
acaaaagttg cctttgtg 18

<210> 532
<211> 19
<212> DNA
<213> Homo sapiens

<400> 532
acaaaagttg cctttgtgt 19

<210> 533
<211> 17
<212> DNA
<213> Homo sapiens

<400> 533
acacaggacc tggagtc 17

<210> 534
<211> 18
<212> DNA
<213> Homo sapiens

<400> 534
tacgttccat agtctacc 18

<210> 535
<211> 19
<212> DNA
<213> Homo sapiens

<400> 535
tacgttccat agtctacca 19

<210> 536
<211> 18
<212> DNA
<213> Homo sapiens

<400> 536
aagatgtgga ccatatta 18

<210> 537
<211> 19
<212> DNA
<213> Homo sapiens

<400> 537
aagatgtgga ccatattac 19

<210> 538
<211> 20

<212> DNA
<213> Homo sapiens

<400> 538
aagatgtgga ccatattaca 20

<210> 539
<211> 21
<212> DNA
<213> Homo sapiens

<400> 539
aagatgtgga ccatattaca t 21

<210> 540
<211> 22
<212> DNA
<213> Homo sapiens

<400> 540
aagatgtgga ccatattaca ta 22

<210> 541
<211> 15
<212> DNA
<213> Homo sapiens

<400> 541
gcgcatgttc tatgg 15

<210> 542
<211> 16
<212> DNA
<213> Homo sapiens

<400> 542
gcgcatgttc tatggt 16

<210> 543
<211> 17
<212> DNA
<213> Homo sapiens

<400> 543
gcgcatgttc tatggtc 17

<210> 544
<211> 14
<212> DNA
<213> Homo sapiens

<400> 544
acagagagct tgcc 14

<210> 545
<211> 15
<212> DNA
<213> Homo sapiens

<400> 545
acagagagct tgccc 15

<210> 546
<211> 16
<212> DNA
<213> Homo sapiens

<400> 546
acagagagct tgccct 16

<210> 547
<211> 17
<212> DNA
<213> Homo sapiens

<400> 547
acagagagct tgccctt 17

<210> 548
<211> 13
<212> DNA
<213> Homo sapiens

<400> 548
cgaatccacc acg 13

<210> 549
<211> 14
<212> DNA
<213> Homo sapiens

<400> 549
cgaatccacc acga 14

<210> 550
<211> 15
<212> DNA
<213> Homo sapiens

<400> 550
cgaatccacc acgaa 15

<210> 551
<211> 16
<212> DNA
<213> Homo sapiens

<400> 551
cgaatccacc acgaac 16

<210> 552
<211> 18
<212> DNA
<213> Homo sapiens

<400> 552 18
agccacaatc accttctg 18

<210> 553
<211> 19
<212> DNA
<213> Homo sapiens

<400> 553 19
agccacaatc accttctga 19

<210> 554
<211> 20
<212> DNA
<213> Homo sapiens

<400> 554
agccacaatc accttctgat 20

<210> 555
<211> 19
<212> DNA
<213> Homo sapiens

<400> 555
tatgaacaat ttctaggaa 19

<210> 556
<211> 20
<212> DNA
<213> Homo sapiens

<400> 556
tatgaacaat ttctaggaat 20

<210> 557
<211> 13
<212> DNA
<213> Homo sapiens

<400> 557
aggggttcac cga 13

<210> 558
<211> 14
<212> DNA
<213> Homo sapiens

<400> 558
aggggttcac cgag 14

<210> 559
<211> 15
<212> DNA
<213> Homo sapiens

<400> 559
aggggttcac cgagc 15

<210> 560
<211> 14

<212> DNA
<213> Homo sapiens

<400> 560
tgcaaagttg ctcg 14

<210> 561
<211> 15
<212> DNA
<213> Homo sapiens

<400> 561
tgcaaagttg ctcgg 15

<210> 562
<211> 16
<212> DNA
<213> Homo sapiens

<400> 562
tgcaaagttg ctcggg 16

<210> 563
<211> 15
<212> DNA
<213> Homo sapiens

<400> 563
aacaggccat ctgtg 15

<210> 564
<211> 16
<212> DNA
<213> Homo sapiens

<400> 564
aacaggccat ctgtgt 16

<210> 565
<211> 17
<212> DNA
<213> Homo sapiens

<400> 565
aacaggccat ctgtgtt 17

<210> 566
<211> 18
<212> DNA
<213> Homo sapiens

<400> 566
aacaggccat ctgtgtta 18

<210> 567
<211> 19
<212> DNA
<213> Homo sapiens

<400> 567
aacaggccat ctgtgttat 19

<210> 568
<211> 20
<212> DNA
<213> Homo sapiens

<400> 568
aacaggccat ctgtgttata 20

<210> 569
<211> 21
<212> DNA
<213> Homo sapiens

<400> 569
aacaggccat ctgtgttata t 21

<210> 570
<211> 14
<212> DNA
<213> Homo sapiens

<400> 570
acggctagtg gacc 14

<210> 571
<211> 15
<212> DNA
<213> Homo sapiens

<400> 571
acggctagtg gacca 15

<210> 572
<211> 16
<212> DNA
<213> Homo sapiens

<400> 572
acggctagtg gaccag 16

<210> 573
<211> 17
<212> DNA
<213> Homo sapiens

<400> 573
ggccttctga ccctaag 17

<210> 574
<211> 18
<212> DNA
<213> Homo sapiens

<400> 574
ggccttctga ccctaagt 18

<210> 575
<211> 19
<212> DNA
<213> Homo sapiens

<400> 575
ggccttctga ccctaagtc 19

<210> 576
<211> 15
<212> DNA
<213> Homo sapiens

<400> 576
ggccttctga ctcca 15

<210> 577
<211> 16
<212> DNA
<213> Homo sapiens

<400> 577
ggccttctga ctccaa 16

<210> 578
<211> 12
<212> DNA
<213> Homo sapiens

<400> 578
ctgaggggcc tc 12

<210> 579
<211> 13
<212> DNA
<213> Homo sapiens

<400> 579
ctgaggggcc tca 13

<210> 580
<211> 14
<212> DNA
<213> Homo sapiens

<400> 580
ctgaggggcc tcag 14

<210> 581
<211> 21
<212> DNA
<213> Homo sapiens

<400> 581
ttcaaaacat gaattgctgc t 21

<210> 582
<211> 22

<212> DNA
<213> Homo sapiens

<400> 582
ttcaaaacat gaattgctgc tg 22

<210> 583
<211> 12
<212> DNA
<213> Homo sapiens

<400> 583
ggcggacacg ac 12

<210> 584
<211> 13
<212> DNA
<213> Homo sapiens

<400> 584
ggcggacacg aca 13

<210> 585
<211> 14
<212> DNA
<213> Homo sapiens

<400> 585
ggcggacacg acat 14

<210> 586
<211> 15
<212> DNA
<213> Homo sapiens

<400> 586
ggcggacacg acatt 15

<210> 587
<211> 15
<212> DNA
<213> Homo sapiens

<400> 587
acggttttac cagac 15

<210> 588
<211> 16
<212> DNA
<213> Homo sapiens

<400> 588
acggttttac cagaca 16

<210> 589
<211> 17
<212> DNA
<213> Homo sapiens

<400> 589
acggttttac cagacag 17

<210> 590
<211> 18
<212> DNA
<213> Homo sapiens

<400> 590
acggttttac cagacagt 18

<210> 591
<211> 19
<212> DNA
<213> Homo sapiens

<400> 591
acggttttac cagacagta 19

<210> 592
<211> 20
<212> DNA
<213> Homo sapiens

<400> 592
acggttttac cagacagtat 20

<210> 593
<211> 13
<212> DNA
<213> Homo sapiens

<400> 593
tgcatgacgg cct 13

<210> 594
<211> 14
<212> DNA
<213> Homo sapiens

<400> 594
tgcatgacgg cctg 14

<210> 595
<211> 15
<212> DNA
<213> Homo sapiens

<400> 595
tgcatgacgg cctgc 15

<210> 596
<211> 13
<212> DNA
<213> Homo sapiens

<400> 596
ccacccaatg acc 13

<210> 597
<211> 14
<212> DNA
<213> Homo sapiens

<400> 597
ccacccaatg acct 14

<210> 598
<211> 15
<212> DNA
<213> Homo sapiens

<400> 598
ccacccaatg accta 15

<210> 599.
<211> 16
<212> DNA
<213> Homo sapiens

<400> 599
ccacccaatg acctac 16

<210> 600
<211> 17
<212> DNA
<213> Homo sapiens

<400> 600
ccacccaatg acctact 17

<210> 601
<211> 15
<212> DNA
<213> Homo sapiens

<400> 601
agacatggag gagcc 15

<210> 602
<211> 16
<212> DNA
<213> Homo sapiens

<400> 602
agacatggag gagcca 16

<210> 603
<211> 17
<212> DNA
<213> Homo sapiens

<400> 603
agacatggag gagccat 17

<210> 604
<211> 13

<212> DNA
<213> Homo sapiens

<400> 604
acaccgagga gcc 13

<210> 605
<211> 14
<212> DNA
<213> Homo sapiens

<400> 605
acaccgagga gccc 14

<210> 606
<211> 16
<212> DNA
<213> Homo sapiens

<400> 606
atgggacatc ctacat 16

<210> 607
<211> 17
<212> DNA
<213> Homo sapiens

<400> 607
atgggacatc ctacata 17

<210> 608
<211> 18
<212> DNA
<213> Homo sapiens

<400> 608
atgggacatc ctacatat 18

<210> 609
<211> 19
<212> DNA
<213> Homo sapiens

<400> 609
atgggacatc ctacatatg 19

<210> 610
<211> 20
<212> DNA
<213> Homo sapiens

<400> 610
atgggacatc ctacatatgc 20

<210> 611
<211> 17
<212> DNA
<213> Homo sapiens

<400> 611
accagctaac aatacac 17

<210> 612
<211> 18
<212> DNA
<213> Homo sapiens

<400> 612
accagctaac aatacact 18

<210> 613
<211> 19
<212> DNA
<213> Homo sapiens

<400> 613
accagctaac aatacactg 19

<210> 614
<211> 20
<212> DNA
<213> Homo sapiens

<400> 614
accagctaac aatacactgc 20

<210> 615
<211> 17
<212> DNA
<213> Homo sapiens

<400> 615
tattaggaac acatcgc 17

<210> 616
<211> 18
<212> DNA
<213> Homo sapiens

<400> 616
tattaggaac acatcgca 18

<210> 617
<211> 19
<212> DNA
<213> Homo sapiens

<400> 617
tattaggaac acatcgcaa 19

<210> 618
<211> 20
<212> DNA
<213> Homo sapiens

<400> 618
tattaggaac acatcgcaaa 20

<210> 619
<211> 21
<212> DNA
<213> Homo sapiens

<400> 619
tattaggaac acatcgcaaa a 21

<210> 620
<211> 22
<212> DNA
<213> Homo sapiens

<400> 620
tattaggaac acatcgcaaa aa 22

<210> 621
<211> 18
<212> DNA
<213> Homo sapiens

<400> 621
aaactcagta atggtaac 18

<210> 622
<211> 19
<212> DNA
<213> Homo sapiens

<400> 622
aaactcagta atggtaacg 19

<210> 623
<211> 20
<212> DNA
<213> Homo sapiens

<400> 623
aaactcagta atggtaacgg 20

<210> 624
<211> 16
<212> DNA
<213> Homo sapiens

<400> 624
gtctcagttt cctctg 16

<210> 625
<211> 17
<212> DNA
<213> Homo sapiens

<400> 625
gtctcagttt cctctgc 17

<210> 626
<211> 16

<212> DNA
<213> Homo sapiens

<400> 626
cttctttgca gatgag 16

<210> 627
<211> 17
<212> DNA
<213> Homo sapiens

<400> 627
cttctttgca gatgaga 17

<210> 628
<211> 18
<212> DNA
<213> Homo sapiens

<400> 628
cttctttgca gatgagac 18

<210> 629
<211> 19
<212> DNA
<213> Homo sapiens

<400> 629
cttctttgca gatgagact 19

<210> 630
<211> 13
<212> DNA
<213> Homo sapiens

<400> 630
cgaactcacc acg 13

<210> 631
<211> 14
<212> DNA
<213> Homo sapiens

<400> 631
cgaactcacc acgg 14

<210> 632
<211> 15
<212> DNA
<213> Homo sapiens

<400> 632
cgaactcacc acgga 15

<210> 633
<211> 14
<212> DNA
<213> Homo sapiens

<400> 633
agaggagagc cgtg 14

<210> 634
<211> 15
<212> DNA
<213> Homo sapiens

<400> 634
agaggagagc cgtgt 15

<210> 635
<211> 16
<212> DNA
<213> Homo sapiens

<400> 635
agaggagagc cgtgta 16

<210> 636
<211> 17
<212> DNA
<213> Homo sapiens

<400> 636
agaggagagc cgtgtat 17

<210> 637
<211> 18
<212> DNA
<213> Homo sapiens

<400> 637
agaggagagc cgtgtatg 18

<210> 638
<211> 15
<212> DNA
<213> Homo sapiens

<400> 638
gaattcatca cggcc 15

<210> 639
<211> 16
<212> DNA
<213> Homo sapiens

<400> 639
gaattcatca cggcca 16

<210> 640
<211> 17
<212> DNA
<213> Homo sapiens

<400> 640
gaattcatca cggccag 17

<210> 641
<211> 17
<212> DNA
<213> Homo sapiens

<400> 641
ttgagagtgc cattatc 17

<210> 642
<211> 18
<212> DNA
<213> Homo sapiens

<400> 642
ttgagagtgc cattatct 18

<210> 643
<211> 19
<212> DNA
<213> Homo sapiens

<400> 643
ttgagagtgc cattatctg 19

<210> 644
<211> 20
<212> DNA
<213> Homo sapiens

<400> 644
ttgagagtgc cattatctgg 20

<210> 645
<211> 15
<212> DNA
<213> Homo sapiens

<400> 645
gctgccgtat atgtg 15

<210> 646
<211> 16
<212> DNA
<213> Homo sapiens

<400> 646
gctgccgtat atgtga 16

<210> 647
<211> 17
<212> DNA
<213> Homo sapiens

<400> 647
gctgccgtat atgtgat 17

<210> 648
<211> 18

<212> DNA
<213> Homo sapiens

<400> 648
gctgccgtat atgtgatg 18

<210> 649
<211> 14
<212> DNA
<213> Homo sapiens

<400> 649
cagcatggag tcct 14

<210> 650
<211> 15
<212> DNA
<213> Homo sapiens

<400> 650
cagcatggag tcctc 15

<210> 651
<211> 16
<212> DNA
<213> Homo sapiens

<400> 651
cagcatggag tcctcc 16

<210> 652
<211> 15
<212> DNA
<213> Homo sapiens

<400> 652
tcctcatgga agggt 15

<210> 653
<211> 16
<212> DNA
<213> Homo sapiens

<400> 653
tcctcatgga agggtt 16

<210> 654
<211> 17
<212> DNA
<213> Homo sapiens

<400> 654
tcctcatgga agggttc 17

<210> 655
<211> 16
<212> DNA
<213> Homo sapiens

<400> 655
aagaatcttg tcccgc 16

<210> 656
<211> 17
<212> DNA
<213> Homo sapiens

<400> 656
aagaatcttg tcccgca 17

<210> 657
<211> 18
<212> DNA
<213> Homo sapiens

<400> 657
aagaatcttg tcccgcag 18

<210> 658
<211> 17
<212> DNA
<213> Homo sapiens

<400> 658
aatgaaagcc taccatg 17

<210> 659
<211> 18
<212> DNA
<213> Homo sapiens

<400> 659
aatgaaagcc taccatgt 18

<210> 660
<211> 19
<212> DNA
<213> Homo sapiens

<400> 660
aatgaaagcc taccatgta 19

<210> 661
<211> 20
<212> DNA
<213> Homo sapiens

<400> 661
aatgaaagcc taccatgtac 20

<210> 662
<211> 21
<212> DNA
<213> Homo sapiens

<400> 662
aatgaaagcc taccatgtac a 21

<210> 663
<211> 14
<212> DNA
<213> Homo sapiens

<400> 663
aagaggtttc ccgt 14

<210> 664
<211> 15
<212> DNA
<213> Homo sapiens

<400> 664
aagaggtttc ccgtg 15

<210> 665
<211> 16
<212> DNA
<213> Homo sapiens

<400> 665
aagaggtttc ccgtgt 16

<210> 666
<211> 17
<212> DNA
<213> Homo sapiens

<400> 666
aagaggtttc ccgtgta 17

<210> 667
<211> 18
<212> DNA
<213> Homo sapiens

<400> 667
aagaggtttc ccgtgtat 18

<210> 668
<211> 19
<212> DNA
<213> Homo sapiens

<400> 668
aagaggtttc ccgtgtatg 19

<210> 669
<211> 16
<212> DNA
<213> Homo sapiens

<400> 669
aaagaagtgc accatg 16

<210> 670
<211> 17

<212> DNA
<213> Homo sapiens

<400> 670
aaagaagtgc accatgt 17

<210> 671
<211> 18
<212> DNA
<213> Homo sapiens

<400> 671
aaagaagtgc accatgtt 18

<210> 672
<211> 19
<212> DNA
<213> Homo sapiens

<400> 672
aaagaagtgc accatgttt 19

<210> 673
<211> 20
<212> DNA
<213> Homo sapiens

<400> 673
aaagaagtgc accatgtttg 20

<210> 674
<211> 15
<212> DNA
<213> Homo sapiens

<400> 674
gagattggcc atgta 15

<210> 675
<211> 16
<212> DNA
<213> Homo sapiens

<400> 675
gagattggcc atgtaa 16

<210> 676
<211> 17
<212> DNA
<213> Homo sapiens

<400> 676
gagattggcc atgtaat 17

<210> 677
<211> 15
<212> DNA
<213> Homo sapiens

<400> 677
acaaaccaca gtgtg 15

<210> 678
<211> 16
<212> DNA
<213> Homo sapiens

<400> 678
acaaaccaca gtgtgc 16

<210> 679
<211> 17
<212> DNA
<213> Homo sapiens

<400> 679
acaaaccaca gtgtgct 17

<210> 680
<211> 18
<212> DNA
<213> Homo sapiens

<400> 680
acaaaccaca gtgtgctg 18

<210> 681
<211> 13
<212> DNA
<213> Homo sapiens

<400> 681
gaaaaacgcc ccc 13

<210> 682
<211> 14
<212> DNA
<213> Homo sapiens

<400> 682
gaaaaacgcc ccct 14

<210> 683
<211> 15
<212> DNA
<213> Homo sapiens

<400> 683
gaaaaacgcc ccctg 15

<210> 684
<211> 17
<212> DNA
<213> Homo sapiens

<400> 684
ttaaacatca ctgcaag 17

<210> 685
<211> 18
<212> DNA
<213> Homo sapiens

<400> 685
ttaaacatca ctgcaagt 18

<210> 686
<211> 19
<212> DNA
<213> Homo sapiens

<400> 686
ttaaacatca ctgcaagtc 19

<210> 687
<211> 20
<212> DNA
<213> Homo sapiens

<400> 687
ttaaacatca ctgcaagtct 20

<210> 688
<211> 21
<212> DNA
<213> Homo sapiens

<400> 688
ttaaacatca ctgcaagtct t 21

<210> 689
<211> 22
<212> DNA
<213> Homo sapiens

<400> 689
ttaaacatca ctgcaagtct ta 22

<210> 690
<211> 23
<212> DNA
<213> Homo sapiens

<400> 690 23
ttaaacatca ctgcaagtct taa 23

<210> 691
<211> 14
<212> DNA
<213> Homo sapiens

<400> 691
cagaatcctt gccc 14

<210> 692
<211> 15

<212> DNA
<213> Homo sapiens

<400> 692
cagaatcctt gccca 15

<210> 693
<211> 16
<212> DNA
<213> Homo sapiens

<400> 693
cagaatcctt gcccag 16

<210> 694
<211> 14
<212> DNA
<213> Homo sapiens

<400> 694
tctcacccag ggac 14

<210> 695
<211> 15
<212> DNA
<213> Homo sapiens

<400> 695
tctcacccag ggaca 15

<210> 696
<211> 16
<212> DNA
<213> Homo sapiens

<400> 696
tctcacccag ggacaa 16

<210> 697
<211> 17
<212> DNA
<213> Homo sapiens

<400> 697
tctcacccag ggacaaa 17

<210> 698
<211> 15
<212> DNA
<213> Homo sapiens

<400> 698
tagcacccag atagc 15

<210> 699
<211> 16
<212> DNA
<213> Homo sapiens

<400> 699
tagcacccag atagca 16

<210> 700
<211> 17
<212> DNA
<213> Homo sapiens

<400> 700
tagcacccag atagcaa 17

<210> 701
<211> 18
<212> DNA
<213> Homo sapiens

<400> 701
tagcacccag atagcaag 18

<210> 702
<211> 15
<212> DNA
<213> Homo sapiens

<400> 702
ctgcagaact gttcc 15

<210> 703
<211> 16
<212> DNA
<213> Homo sapiens

<400> 703
ctgcagaact gttccc 16

<210> 704
<211> 16
<212> DNA
<213> Homo sapiens

<400> 704
atagagtgca gaccag 16

<210> 705
<211> 17
<212> DNA
<213> Homo sapiens

<400> 705
atagagtgca gaccagg 17

<210> 706
<211> 18
<212> DNA
<213> Homo sapiens

<400> 706
atagagtgca gaccaggg 18

<210> 707
<211> 14
<212> DNA
<213> Homo sapiens

<400> 707
gaggaaacca gcaa 14

<210> 708
<211> 15
<212> DNA
<213> Homo sapiens

<400> 708
gaggaaacca gcaag 15

<210> 709
<211> 16
<212> DNA
<213> Homo sapiens

<400> 709
gaggaaacca gcaagt 16

<210> 710
<211> 17
<212> DNA
<213> Homo sapiens

<400> 710
gaggaaacca gcaagtg 17

<210> 711
<211> 16
<212> DNA
<213> Homo sapiens

<400> 711
tctactcaga agggtg 16

<210> 712
<211> 17
<212> DNA
<213> Homo sapiens

<400> 712
tctactcaga agggtgc 17

<210> 713
<211> 16
<212> DNA
<213> Homo sapiens

<400> 713
ttcactccaa aaggtg 16

<210> 714
<211> 17

<212> DNA
<213> Homo sapiens

<400> 714
ttcactccaa aaggtgc 17

<210> 715
<211> 18
<212> DNA
<213> Homo sapiens

<400> 715
ttcactccaa aaggtgca 18

<210> 716
<211> 16
<212> DNA
<213> Homo sapiens

<400> 716
tctactccaa aaggct 16

<210> 717
<211> 17
<212> DNA
<213> Homo sapiens

<400> 717
tctactccaa aaggcta 17

<210> 718
<211> 18
<212> DNA
<213> Homo sapiens

<400> 718
tctactccaa aaggctac 18

<210> 719
<211> 19
<212> DNA
<213> Homo sapiens

<400> 719
tctactccaa aaggctaca 19

<210> 720
<211> 20
<212> DNA
<213> Homo sapiens

<400> 720
tctactccaa aaggctacaa 20

<210> 721
<211> 21
<212> DNA
<213> Homo sapiens

<400> 721
tctactccaa aaggctacaa t 21

<210> 722
<211> 15
<212> DNA
<213> Homo sapiens

<400> 722
tctacccaca gacgt 15

<210> 723
<211> 16
<212> DNA
<213> Homo sapiens

<400> 723
tctacccaca gacgta 16

<210> 724
<211> 17
<212> DNA
<213> Homo sapiens

<400> 724
tctacccaca gacgtac 17

<210> 725
<211> 15
<212> DNA
<213> Homo sapiens

<400> 725
tgtgattgcc actct 15

<210> 726
<211> 16
<212> DNA
<213> Homo sapiens

<400> 726
tgtgattgcc actctc 16

<210> 727
<211> 17
<212> DNA
<213> Homo sapiens

<400> 727
tgtgattgcc actctcc 17

<210> 728
<211> 18
<212> DNA
<213> Homo sapiens

<400> 728
tgtgattgcc actctcct 18

<210> 729
<211> 15
<212> DNA
<213> Homo sapiens

<400> 729
tgactgcaga gcaaa 15

<210> 730
<211> 16
<212> DNA
<213> Homo sapiens

<400> 730
tgactgcaga gcaaaa 16

<210> 731
<211> 17
<212> DNA
<213> Homo sapiens

<400> 731
tgactgcaga gcaaaag 17

<210> 732
<211> 18
<212> DNA
<213> Homo sapiens

<400> 732
tgactgcaga gcaaaaga 18

<210> 733
<211> 15
<212> DNA
<213> Homo sapiens

<400> 733
gacctcagct atgac 15

<210> 734
<211> 16
<212> DNA
<213> Homo sapiens

<400> 734
gacctcagct atgaca 16

<210> 735
<211> 17
<212> DNA
<213> Homo sapiens

<400> 735
gacctcagct atgacag 17

<210> 736
<211> 14

<212> DNA
<213> Homo sapiens

<400> 736
gaaagtgccc tcaa 14

<210> 737
<211> 15
<212> DNA
<213> Homo sapiens

<400> 737
gaaagtgccc tcaag 15

<210> 738
<211> 16
<212> DNA
<213> Homo sapiens

<400> 738
gaaagtgccc tcaagg 16

<210> 739
<211> 17
<212> DNA
<213> Homo sapiens

<400> 739
ataaatgaca cctccct 17

<210> 740
<211> 18
<212> DNA
<213> Homo sapiens

<400> 740
ataaatgaca cctccctg 18

<210> 741
<211> 19
<212> DNA
<213> Homo sapiens

<400> 741
ataaatgaca cctccctgt 19

<210> 742
<211> 20
<212> DNA
<213> Homo sapiens

<400> 742
ataaatgaca cctccctgtg 20

<210> 743
<211> 16 .
<212> DNA
<213> Homo sapiens

<400> 743
ctactcacag aagtgt 16

<210> 744
<211> 17
<212> DNA
<213> Homo sapiens

<400> 744
ctactcacag aagtgtc 17

<210> 745
<211> 18
<212> DNA
<213> Homo sapiens

<400> 745
ctactcacag aagtgtca 18

<210> 746
<211> 19
<212> DNA
<213> Homo sapiens

<400> 746
ctactcacag aagtgtcaa 19

<210> 747
<211> 20
<212> DNA
<213> Homo sapiens

<400> 747
ctactcacag aagtgtcaat 20

<210> 748
<211> 15
<212> DNA
<213> Homo sapiens

<400> 748
acgctccaaa agaag 15

<210> 749
<211> 16
<212> DNA
<213> Homo sapiens

<400> 749
acgctccaaa agaagg 16

<210> 750
<211> 17
<212> DNA
<213> Homo sapiens

<400> 750
acgctccaaa agaaggc 17

<210> 751
<211> 16
<212> DNA
<213> Homo sapiens

<400> 751
cagaaagtgc tttctt 16

<210> 752
<211> 17
<212> DNA
<213> Homo sapiens

<400> 752
cagaaagtgc tttcttt 17

<210> 753
<211> 18
<212> DNA
<213> Homo sapiens

<400> 753
cagaaagtgc tttctttt 18

<210> 754
<211> 19
<212> DNA
<213> Homo sapiens

<400> 754
cagaaagtgc tttcttttg 19

<210> 755
<211> 15
<212> DNA
<213> Homo sapiens

<400> 755
accctctgaa aggaa 15

<210> 756
<211> 16
<212> DNA
<213> Homo sapiens

<400> 756
accctctgaa aggaag 16

<210> 757
<211> 17
<212> DNA
<213> Homo sapiens

<400> 757
accctctgaa aggaagc 17

<210> 758
<211> 17

<212> DNA
<213> Homo sapiens

<400> 758
aaagtgcttc ttacctc 17

<210> 759
<211> 18
<212> DNA
<213> Homo sapiens

<400> 759
aaagtgcttc ttacctcc 18

<210> 760
<211> 19
<212> DNA
<213> Homo sapiens

<400> 760
aaagtgcttc ttacctcca 19

<210> 761
<211> 15
<212> DNA
<213> Homo sapiens

<400> 761
agacagtgct tccat 15

<210> 762
<211> 16
<212> DNA
<213> Homo sapiens

<400> 762 16
agacagtgct tccatc 16

<210> 763
<211> 17
<212> DNA
<213> Homo sapiens

<400> 763
agacagtgct tccatct 17

<210> 764
<211> 18
<212> DNA
<213> Homo sapiens

<400> 764
agacagtgct tccatcta 18

<210> 765
<211> 19
<212> DNA
<213> Homo sapiens

<400> 765
agacagtgct tccatctag 19

<210> 766
<211> 17
<212> DNA
<213> Homo sapiens

<400> 766
aacactctaa agggatg 17

<210> 767
<211> 18
<212> DNA
<213> Homo sapiens

<400> 767
aacactctaa agggatgc 18

<210> 768
<211> 19
<212> DNA
<213> Homo sapiens

<400> 768
aacactctaa agggatgca 19

<210> 769
<211> 17
<212> DNA
<213> Homo sapiens

<400> 769
acactctaaa aggatgc 17

<210> 770
<211> 18
<212> DNA
<213> Homo sapiens

<400> 770
acactctaaa aggatgca 18

<210> 771
<211> 19
<212> DNA
<213> Homo sapiens

<400> 771
acactctaaa aggatgcac 19

<210> 772
<211> 14
<212> DNA
<213> Homo sapiens

<400> 772
tccagcaaag ggaa 14

<210> 773
<211> 15
<212> DNA
<213> Homo sapiens

<400> 773
tccagcaaag ggaag 15

<210> 774
<211> 16
<212> DNA
<213> Homo sapiens

<400> 774
tccagcaaag ggaagc 16

<210> 775
<211> 15
<212> DNA
<213> Homo sapiens

<400> 775
aaagggcttc ccttt 15

<210> 776
<211> 16
<212> DNA
<213> Homo sapiens

<400> 776
aaagggcttc cctttg 16

<210> 777
<211> 17
<212> DNA
<213> Homo sapiens

<400> 777
aaagggcttc cctttgc 17

<210> 778
<211> 15
<212> DNA
<213> Homo sapiens

<400> 778
acctctaaag gggag 15

<210> 779
<211> 16
<212> DNA
<213> Homo sapiens

<400> 779
acctctaaag gggagc 16

<210> 780
<211> 17

<212> DNA
<213> Homo sapiens

<400> 780
acctctaaag gggagcg 17

<210> 781
<211> 16
<212> DNA
<213> Homo sapiens

<400> 781
cactctaaag agaagc 16

<210> 782
<211> 17
<212> DNA
<213> Homo sapiens

<400> 782
cactctaaag agaagcg 17

<210> 783
<211> 18
<212> DNA
<213> Homo sapiens

<400> 783
cactctaaag agaagcgc 18

<210> 784
<211> 15
<212> DNA
<213> Homo sapiens

<400> 784
cagaaagtgc ttccc 15

<210> 785
<211> 16
<212> DNA
<213> Homo sapiens

<400> 785
cagaaagtgc ttccct 16

<210> 786
<211> 17
<212> DNA
<213> Homo sapiens

<400> 786
cagaaagtgc ttccctc 17

<210> 787
<211> 14
<212> DNA
<213> Homo sapiens

<400> 787
gctccaaagg gaag 14

<210> 788
<211> 15
<212> DNA
<213> Homo sapiens

<400> 788
gctccaaagg gaagc 15

<210> 789
<211> 15
<212> DNA
<213> Homo sapiens

<400> 789
acactctgaa gggaa 15

<210> 790
<211> 16
<212> DNA
<213> Homo sapiens

<400> 790
acactctgaa gggaag 16

<210> 791
<211> 17
<212> DNA
<213> Homo sapiens

<400> 791
acactctgaa gggaagc 17

<210> 792
<211> 17
<212> DNA
<213> Homo sapiens

<400> 792
tcctctaaag agaagcg 17

<210> 793
<211> 18
<212> DNA
<213> Homo sapiens

<400> 793
tcctctaaag agaagcgc 18

<210> 794
<211> 16
<212> DNA
<213> Homo sapiens

<400> 794
agagaaagtg cttccc 16

<210> 795
<211> 17
<212> DNA
<213> Homo sapiens

<400> 795
agagaaagtg cttccct 17

<210> 796
<211> 18
<212> DNA
<213> Homo sapiens

<400> 796
agagaaagtg cttccctc 18

<210> 797
<211> 17
<212> DNA
<213> Homo sapiens

<400> 797
gtaacactct aaaagga 17

<210> 798
<211> 18
<212> DNA
<213> Homo sapiens

<400> 798
gtaacactct aaaaggat 18

<210> 799
<211> 19
<212> DNA
<213> Homo sapiens

<400> 799
gtaacactct aaaaggatg 19

<210> 800
<211> 20
<212> DNA
<213> Homo sapiens

<400> 800
gtaacactct aaaaggatgc 20

<210> 801
<211> 21
<212> DNA
<213> Homo sapiens

<400> 801
gtaacactct aaaaggatgc a 21

<210> 802
<211> 18

<212> DNA
<213> Homo sapiens

<400> 802
aaacctctaa aaggatgc 18

<210> 803
<211> 19
<212> DNA
<213> Homo sapiens

<400> 803
aaacctctaa aaggatgca 19

<210> 804
<211> 20
<212> DNA
<213> Homo sapiens

<400> 804
aaacctctaa aaggatgcac 20

<210> 805
<211> 18
<212> DNA
<213> Homo sapiens

<400> 805
atcctctaaa aagatgca 18

<210> 806
<211> 19
<212> DNA
<213> Homo sapiens

<400> 806
atcctctaaa aagatgcac 19

<210> 807
<211> 20
<212> DNA
<213> Homo sapiens

<400> 807
atcctctaaa aagatgcact 20

<210> 808
<211> 21
<212> DNA
<213> Homo sapiens

<400> 808
atcctctaaa aagatgcact t 21

<210> 809
<211> 22
<212> DNA
<213> Homo sapiens

<400> 809
atcctctaaa aagatgcact tt 22

<210> 810
<211> 16
<212> DNA
<213> Homo sapiens

<400> 810
acactctaaa gggagg 16

<210> 811
<211> 17
<212> DNA
<213> Homo sapiens

<400> 811
acactctaaa gggaggc 17

<210> 812
<211> 18
<212> DNA
<213> Homo sapiens

<400> 812
acactctaaa gggaggca 18

<210> 813
<211> 17
<212> DNA
<213> Homo sapiens

<400> 813
acactctaaa aggaggc 17

<210> 814
<211> 18
<212> DNA
<213> Homo sapiens

<400> 814
acactctaaa aggaggca 18

<210> 815
<211> 19
<212> DNA
<213> Homo sapiens

<400> 815
acactctaaa aggaggcac 19

<210> 816
<211> 15
<212> DNA
<213> Homo sapiens

<400> 816
gaaagtgctc ccttt 15

<210> 817
<211> 16
<212> DNA
<213> Homo sapiens

<400> 817
gaaagtgctc cctttt 16

<210> 818
<211> 17
<212> DNA
<213> Homo sapiens

<400> 818
gaaagtgctc ccttttg 17

<210> 819
<211> 15
<212> DNA
<213> Homo sapiens

<400> 819
acagtccaaa gggaa 15

<210> 820
<211> 16
<212> DNA
<213> Homo sapiens

<400> 820
acagtccaaa gggaag 16

<210> 821
<211> 17
<212> DNA
<213> Homo sapiens

<400> 821
acagtccaaa gggaagc 17

<210> 822
<211> 17
<212> DNA
<213> Homo sapiens

<400> 822
agaaagtact tccctct 17

<210> 823
<211> 18
<212> DNA
<213> Homo sapiens

<400> 823
agaaagtact tccctctg 18

<210> 824
<211> 19

<212> DNA
<213> Homo sapiens

<400> 824
agaaagtact tccctctgg 19

<210> 825
<211> 16
<212> DNA
<213> Homo sapiens

<400> 825
ccctctaaaa ggaagc 16

<210> 826
<211> 17
<212> DNA
<213> Homo sapiens

<400> 826
ccctctaaaa ggaagca 17

<210> 827
<211> 18
<212> DNA
<213> Homo sapiens

<400> 827
ccctctaaaa ggaagcac 18

<210> 828
<211> 17
<212> DNA
<213> Homo sapiens

<400> 828
aaccctctaa aaggaag 17

<210> 829
<211> 18
<212> DNA
<213> Homo sapiens

<400> 829
aaccctctaa aaggaagc 18

<210> 830
<211> 19
<212> DNA
<213> Homo sapiens

<400> 830
aaccctctaa aaggaagca 19

<210> 831
<211> 15
<212> DNA
<213> Homo sapiens

<400> 831
aacccaccaa agaga 15

<210> 832
<211> 16
<212> DNA
<213> Homo sapiens

<400> 832
aacccaccaa agagaa 16

<210> 833
<211> 17
<212> DNA
<213> Homo sapiens

<400> 833
aacccaccaa agagaag 17

<210> 834
<211> 18
<212> DNA
<213> Homo sapiens

<400> 834
aacccaccaa agagaagc 18

<210> 835
<211> 14
<212> DNA
<213> Homo sapiens

<400> 835
cagaaagggc ttcc 14

<210> 836
<211> 15
<212> DNA
<213> Homo sapiens

<400> 836
cagaaagggc ttccc 15

<210> 837
<211> 16
<212> DNA
<213> Homo sapiens

<400> 837
cagaaagggc ttccct 16

<210> 838
<211> 15
<212> DNA
<213> Homo sapiens

<400> 838
ccctcaaaaa ggaag 15

<210> 839
<211> 16
<212> DNA
<213> Homo sapiens

<400> 839
ccctcaaaaa ggaagc 16

<210> 840
<211> 17
<212> DNA
<213> Homo sapiens

<400> 840
ccctcaaaaa ggaagca 17

<210> 841
<211> 17
<212> DNA
<213> Homo sapiens

<400> 841
acactctaaa gggaagc 17

<210> 842
<211> 18
<212> DNA
<213> Homo sapiens

<400> 842
acactctaaa gggaagca 18

<210> 843
<211> 19
<212> DNA
<213> Homo sapiens

<400> 843
acactctaaa gggaagcac 19

<210> 844
<211> 16
<212> DNA
<213> Homo sapiens

<400> 844
actctaaagg gaagca 16

<210> 845
<211> 17
<212> DNA
<213> Homo sapiens

<400> 845
actctaaagg gaagcac 17

<210> 846
<211> 18

<212> DNA
<213> Homo sapiens

<400> 846
actctaaagg gaagcact 18

<210> 847
<211> 19
<212> DNA
<213> Homo sapiens

<400> 847
actctaaagg gaagcactt 19

<210> 848
<211> 20
<212> DNA
<213> Homo sapiens

<400> 848
actctaaagg gaagcacttt 20

<210> 849
<211> 17
<212> DNA
<213> Homo sapiens

<400> 849
acactctaaa gggaagt 17

<210> 850
<211> 18
<212> DNA
<213> Homo sapiens

<400> 850
acactctaaa gggaagtg 18

<210> 851
<211> 19
<212> DNA
<213> Homo sapiens

<400> 851
acactctaaa gggaagtgc 19

<210> 852
<211> 17
<212> DNA
<213> Homo sapiens

<400> 852
aacactctaa agggaac 17

<210> 853
<211> 18
<212> DNA
<213> Homo sapiens

<400> 853
aacactctaa agggaacc 18

<210> 854
<211> 19
<212> DNA
<213> Homo sapiens

<400> 854
aacactctaa agggaacca 19

<210> 855
<211> 20
<212> DNA
<213> Homo sapiens

<400> 855
aacactctaa agggaaccat 20

<210> 856
<211> 16
<212> DNA
<213> Homo sapiens

<400> 856
ccctctatag ggaagc 16

<210> 857
<211> 17
<212> DNA
<213> Homo sapiens

<400> 857
ccctctatag ggaagcg 17

<210> 858
<211> 15
<212> DNA
<213> Homo sapiens

<400> 858
actccaaagg gaagc 15

<210> 859
<211> 16
<212> DNA
<213> Homo sapiens

<400> 859
actccaaagg gaagcg 16

<210> 860
<211> 15
<212> DNA
<213> Homo sapiens

<400> 860
gagaaagtgc ttccc 15

<210> 861
<211> 16
<212> DNA
<213> Homo sapiens

<400> 861
gagaaagtgc ttccct 16

<210> 862
<211> 17
<212> DNA
<213> Homo sapiens

<400> 862
gagaaagtgc ttccctt 17

<210> 863
<211> 18
<212> DNA
<213> Homo sapiens

<400> 863
gagaaagtgc ttcccttt 18

<210> 864
<211> 15
<212> DNA
<213> Homo sapiens

<400> 864
agaaagtgca tccct 15

<210> 865
<211> 16
<212> DNA
<213> Homo sapiens

<400> 865
agaaagtgca tccctc 16

<210> 866
<211> 17
<212> DNA
<213> Homo sapiens

<400> 866
agaaagtgca tccctct 17

<210> 867
<211> 18
<212> DNA
<213> Homo sapiens

<400> 867
agaaagtgca tccctctg 18

<210> 868
<211> 15

<212> DNA
<213> Homo sapiens

<400> 868
gctctaaagg gaagc 15

<210> 869
<211> 16
<212> DNA
<213> Homo sapiens

<400> 869
gctctaaagg gaagcg 16

<210> 870
<211> 15
<212> DNA
<213> Homo sapiens

<400> 870
agaaagtgct tccct 15

<210> 871
<211> 16
<212> DNA
<213> Homo sapiens

<400> 871
agaaagtgct tccctc 16

<210> 872
<211> 17
<212> DNA
<213> Homo sapiens

<400> 872
agaaagtgct tccctct 17

<210> 873
<211> 18
<212> DNA
<213> Homo sapiens

<400> 873
agaaagtgct tccctcta 18

<210> 874
<211> 19
<212> DNA
<213> Homo sapiens

<400> 874
agaaagtgct tccctctag 19

<210> 875
<211> 16
<212> DNA
<213> Homo sapiens

<400> 875
aacagaaagt gcttcc 16

<210> 876
<211> 17
<212> DNA
<213> Homo sapiens

<400> 876
aacagaaagt gcttccc 17

<210> 877
<211> 18
<212> DNA
<213> Homo sapiens

<400> 877
aacagaaagt gcttccct 18

<210> 878
<211> 16
<212> DNA
<213> Homo sapiens

<400> 878
gcctctaaaa ggaagc 16

<210> 879
<211> 17
<212> DNA
<213> Homo sapiens

<400> 879
gcctctaaaa ggaagca 17

<210> 880
<211> 18
<212> DNA
<213> Homo sapiens

<400> 880
gcctctaaaa ggaagcac 18

<210> 881
<211> 15
<212> DNA
<213> Homo sapiens

<400> 881
aacagaaagc gcttc 15

<210> 882
<211> 16
<212> DNA
<213> Homo sapiens

<400> 882
aacagaaagc gcttcc 16

<210> 883
<211> 17
<212> DNA
<213> Homo sapiens

<400> 883
aacagaaagc gcttccc 17

<210> 884
<211> 14
<212> DNA
<213> Homo sapiens

<400> 884
agaaagggct tccc 14

<210> 885
<211> 15
<212> DNA
<213> Homo sapiens

<400> 885
agaaagggct tccct 15

<210> 886
<211> 16
<212> DNA
<213> Homo sapiens

<400> 886
agaaagggct tccctt 16

<210> 887
<211> 17
<212> DNA
<213> Homo sapiens

<400> 887
agaaagggct tcccttt 17

<210> 888
<211> 18
<212> DNA
<213> Homo sapiens

<400> 888
agaaagggct tccctttg 18

<210> 889
<211> 21
<212> DNA
<213> Homo sapiens

<400> 889
caacaaaatc actagtcttc c 21

<210> 890
<211> 19

<212> DNA
<213> Homo sapiens

<400> 890
tcatacagct agataacca 19

<210> 891
<211> 20
<212> DNA
<213> Homo sapiens

<400> 891
tcatacagct agataaccaa 20

<210> 892
<211> 21
<212> DNA
<213> Homo sapiens

<400> 892
tcatacagct agataaccaa a 21

<210> 893
<211> 22
<212> DNA
<213> Homo sapiens

<400> 893
tcatacagct agataaccaa ag 22

<210> 894
<211> 23
<212> DNA
<213> Homo sapiens

<400> 894
tcatacagct agataaccaa aga 23

<210> 895
<211> 17
<212> DNA
<213> Homo sapiens

<400> 895
actttcggtt atctagc 17

<210> 896
<211> 18
<212> DNA
<213> Homo sapiens

<400> 896
actttcggtt atctagct 18

<210> 897
<211> 19
<212> DNA
<213> Homo sapiens

<400> 897
actttcggtt atctagctt 19

<210> 898
<211> 20
<212> DNA
<213> Homo sapiens

<400> 898
actttcggtt atctagcttt 20

<210> 899
<211> 21
<212> DNA
<213> Homo sapiens

<400> 899
actttcggtt atctagcttt a 21

<210> 900
<211> 14
<212> DNA
<213> Homo sapiens

<400> 900
caggccggga caag 14

<210> 901
<211> 15
<212> DNA
<213> Homo sapiens

<400> 901
caggccggga caagt 15

<210> 902
<211> 16
<212> DNA
<213> Homo sapiens

<400> 902
caggccggga caagtg 16

<210> 903
<211> 16
<212> DNA
<213> Homo sapiens

<400> 903
ctacctgcac gaacag 16

<210> 904
<211> 17
<212> DNA
<213> Homo sapiens

<400> 904
tgctcaataa atacccg 17

<210> 905
<211> 18
<212> DNA
<213> Homo sapiens

<400> 905
tgctcaataa atacccgt 18

<210> 906
<211> 19
<212> DNA
<213> Homo sapiens

<400> 906
tgctcaataa atacccgtt 19

<210> 907
<211> 20
<212> DNA
<213> Homo sapiens

<400> 907
tgctcaataa atacccgttg 20

<210> 908
<211> 18
<212> DNA
<213> Homo sapiens

<400> 908
gcaaaaatgt gctagtgc 18

<210> 909
<211> 19
<212> DNA
<213> Homo sapiens

<400> 909
gcaaaaatgt gctagtgcc 19

<210> 910
<211> 20
<212> DNA
<213> Homo sapiens

<400> 910
gcaaaaatgt gctagtgcca 20

<210> 911
<211> 21
<212> DNA
<213> Homo sapiens

<400> 911
aacaatacaa cttactacct c 21

<210> 912
<211> 18

<212> DNA
<213> Homo sapiens

<400> 912
cacaagatcg gatctacg 18

<210> 913
<211> 14
<212> DNA
<213> Homo sapiens

<400> 913
cgcaaggtcg gttc 14

<210> 914
<211> 15
<212> DNA
<213> Homo sapiens

<400> 914
cgcaaggtcg gttct 15

<210> 915
<211> 16
<212> DNA
<213> Homo sapiens

<400> 915
cgcaaggtcg gttcta 16

<210> 916
<211> 21
<212> DNA
<213> Homo sapiens

<400> 916
aactatacaa cctactacct c 21

<210> 917
<211> 22
<212> DNA
<213> Homo sapiens

<400> 917
aactatacaa cctactacct ca 22

<210> 918
<211> 16
<212> DNA
<213> Homo sapiens

<400> 918
aaccacacaa cctact 16

<210> 919
<211> 19
<212> DNA
<213> Homo sapiens

<400> 919
aaccacacaa cctactacc 19

<210> 920
<211> 21
<212> DNA
<213> Homo sapiens

<400> 920
aaccatacaa cctactacct c 21

<210> 921
<211> 18
<212> DNA
<213> Homo sapiens

<400> 921
actatgcaac ctactacc 18

<210> 922
<211> 20
<212> DNA
<213> Homo sapiens

<400> 922
actatgcaac ctactacctc 20

<210> 923
<211> 21
<212> DNA
<213> Homo sapiens

<400> 923
actatgcaac ctactacctc t 21

<210> 924
<211> 18
<212> DNA
<213> Homo sapiens

<400> 924
actatacaac ctcctacc 18

<210> 925
<211> 19
<212> DNA
<213> Homo sapiens

<400> 925
actatacaac ctcctacct 19

<210> 926
<211> 17
<212> DNA
<213> Homo sapiens

<400> 926
cacaaaccat tatgtgc 17

<210> 927
<211> 18
<212> DNA
<213> Homo sapiens

<400> 927
cacaaaccat tatgtgct 18

<210> 928
<211> 16
<212> DNA
<213> Homo sapiens

<400> 928
cgccaatatt tacgtg 16

<210> 929
<211> 15
<212> DNA
<213> Homo sapiens

<400> 929
acaagtgcct tcact 15

<210> 930
<211> 16
<212> DNA
<213> Homo sapiens

<400> 930
acaagtgcct tcactg 16

<210> 931
<211> 16
<212> DNA
<213> Homo sapiens

<400> 931
actacctgca ctgtaa 16

<210> 932
<211> 17
<212> DNA
<213> Homo sapiens

<400> 932
actacctgca ctgtaag 17

<210> 933
<211> 18
<212> DNA
<213> Homo sapiens

<400> 933
tcagttttgc atagattt 18

<210> 934
<211> 19

<212> DNA
<213> Homo sapiens

<400> 934
tcagttttgc atagatttg 19

<210> 935
<211> 16
<212> DNA
<213> Homo sapiens

<400> 935
tcagttttgc atggat 16

<210> 936
<211> 17
<212> DNA
<213> Homo sapiens

<400> 936
tcagttttgc atggatt 17

<210> 937
<211> 18
<212> DNA
<213> Homo sapiens

<400> 937
tcagttttgc atggattt 18

<210> 938
<211> 18
<212> DNA
<213> Homo sapiens

<400> 938
tcaacatcag tctgataa 18

<210> 939
<211> 22
<212> DNA
<213> Homo sapiens

<400> 939
tcaacatcag tctgataagc ta 22

<210> 940
<211> 16
<212> DNA
<213> Homo sapiens

<400> 940
acagttcttc aactgg 16

<210> 941
<211> 14
<212> DNA
<213> Homo sapiens

<400> 941
ggaaatccct ggca 14

<210> 942
<211> 15
<212> DNA
<213> Homo sapiens

<400> 942
ggaaatccct ggcaa 15

<210> 943
<211> 16
<212> DNA
<213> Homo sapiens

<400> 943
ggaaatccct ggcaat 16

<210> 944
<211> 17
<212> DNA
<213> Homo sapiens

<400> 944
actgatatca gctcagt 17

<210> 945
<211> 18
<212> DNA
<213> Homo sapiens

<400> 945
actgatatca gctcagta 18

<210> 946
<211> 19
<212> DNA
<213> Homo sapiens

<400> 946
actgatatca gctcagtag 19

<210> 947
<211> 14
<212> DNA
<213> Homo sapiens

<400> 947
ctgttcctgc tgaa 14

<210> 948
<211> 15
<212> DNA
<213> Homo sapiens

<400> 948
ctgttcctgc tgaac 15

<210> 949
<211> 16
<212> DNA
<213> Homo sapiens

<400> 949
ctgttcctgc tgaact 16

<210> 950
<211> 15
<212> DNA
<213> Homo sapiens

<400> 950
tcagaccgag acaag 15

<210> 951
<211> 16
<212> DNA
<213> Homo sapiens

<400> 951
tcagaccgag acaagt 16

<210> 952
<211> 19
<212> DNA
<213> Homo sapiens

<400> 952
gcctatcctg gattacttg 19

<210> 953
<211> 14
<212> DNA
<213> Homo sapiens

<400> 953
gcggaactta gcca 14

<210> 954
<211> 16
<212> DNA
<213> Homo sapiens

<400> 954
aaccgatttc agatgg 16

<210> 955
<211> 17
<212> DNA
<213> Homo sapiens

<400> 955
aaccgatttc agatggt 17

<210> 956
<211> 14

<212> DNA
<213> Homo sapiens

<400> 956
gctgcaaaca tccg 14

<210> 957
<211> 14
<212> DNA
<213> Homo sapiens

<400> 957
cttccagtcg agga 14

<210> 958
<211> 17
<212> DNA
<213> Homo sapiens

<400> 958
gcaacttagt aatgtgc 17

<210> 959
<211> 18
<212> DNA
<213> Homo sapiens

<400> 959
gcaacttagt aatgtgca 18

<210> 960
<211> 17
<212> DNA
<213> Homo sapiens

<400> 960
caatgcaact acaatgc 17

<210> 961
<211> 18
<212> DNA
<213> Homo sapiens

<400> 961
caatgcaact acaatgca 18

<210> 962
<211> 14
<212> DNA
<213> Homo sapiens

<400> 962
ctacctgcac gaac 14

<210> 963
<211> 15
<212> DNA
<213> Homo sapiens

<400> 963
ctacctgcac gaaca 15

<210> 964
<211> 16
<212> DNA
<213> Homo sapiens

<400> 964
gcaaaaatgt gctagt 16

<210> 965
<211> 17
<212> DNA
<213> Homo sapiens

<400> 965
gcaaaaatgt gctagtg 17

<210> 966
<211> 19
<212> DNA
<213> Homo sapiens

<400> 966
aacaatacaa cttactacc 19

<210> 967
<211> 20
<212> DNA
<213> Homo sapiens

<400> 967
aacaatacaa cttactacct 20

<210> 968
<211> 22
<212> DNA
<213> Homo sapiens

<400> 968
aacaatacaa cttactacct ca 22

<210> 969
<211> 15
<212> DNA
<213> Homo sapiens

<400> 969
cacaagatcg gatct 15

<210> 970
<211> 16
<212> DNA
<213> Homo sapiens

<400> 970
cacaagatcg gatcta 16

<210> 971
<211> 17
<212> DNA
<213> Homo sapiens

<400> 971
cacaagatcg gatctac 17

<210> 972
<211> 15
<212> DNA
<213> Homo sapiens

<400> 972
cacaagttcg gatct 15

<210> 973
<211> 16
<212> DNA
<213> Homo sapiens

<400> 973
cacaagttcg gatcta 16

<210> 974
<211> 18
<212> DNA
<213> Homo sapiens

<400> 974
cttcagttat cacagtac 18

<210> 975
<211> 19
<212> DNA
<213> Homo sapiens

<400> 975
cttcagttat cacagtact 19

<210> 976
<211> 20
<212> DNA
<213> Homo sapiens

<400> 976
cttcagttat cacagtactg 20

<210> 977
<211> 21
<212> DNA
<213> Homo sapiens

<400> 977
cttcagttat cacagtactg t 21

<210> 978
<211> 18

<212> DNA
<213> Homo sapiens

<400> 978
aacactgatt tcaaatgg 18

<210> 979
<211> 19
<212> DNA
<213> Homo sapiens

<400> 979
aacactgatt tcaaatggt 19

<210> 980
<211> 16
<212> DNA
<213> Homo sapiens

<400> 980
tcatagccct gtacaa 16

<210> 981
<211> 19
<212> DNA
<213> Homo sapiens

<400> 981
tcatagccct gtacaatgc 19

<210> 982
<211> 14
<212> DNA
<213> Homo sapiens

<400> 982
gctacctgca ctgt 14

<210> 983
<211> 15
<212> DNA
<213> Homo sapiens

<400> 983
gctacctgca ctgta 15

<210> 984
<211> 16
<212> DNA
<213> Homo sapiens

<400> 984
gctacctgca ctgtaa 16

<210> 985
<211> 16
<212> DNA
<213> Homo sapiens

<400> 985
tgatagccct gtacaa 16

<210> 986
<211> 17
<212> DNA
<213> Homo sapiens

<400> 986
tgatagccct gtacaat 17

<210> 987
<211> 18
<212> DNA
<213> Homo sapiens

<400> 987
tgatagccct gtacaatg 18

<210> 988
<211> 16
<212> DNA
<213> Homo sapiens

<400> 988
gaacaggtag tctgaa 16

<210> 989
<211> 17
<212> DNA
<213> Homo sapiens

<400> 989
gaacaggtag tctgaac 17

<210> 990
<211> 18
<212> DNA
<213> Homo sapiens

<400> 990
gaacaggtag tctgaaca 18

<210> 991
<211> 15
<212> DNA
<213> Homo sapiens

<400> 991
aaccaatgtg cagac 15

<210> 992
<211> 16
<212> DNA
<213> Homo sapiens

<400> 992
aaccaatgtg cagact 16

<210> 993
<211> 17
<212> DNA
<213> Homo sapiens

<400> 993
aaccaatgtg cagacta 17

<210> 994
<211> 17
<212> DNA
<213> Homo sapiens

<400> 994
acaagctttt tgctcgt 17

<210> 995
<211> 17
<212> DNA
<213> Homo sapiens

<400> 995
acaaagttct gtagtgc 17

<210> 996
<211> 18
<212> DNA
<213> Homo sapiens

<400> 996
acaaagttct gtagtgca 18

<210> 997
<211> 15
<212> DNA
<213> Homo sapiens

<400> 997
gctgagagtg tagga 15

<210> 998
<211> 16
<212> DNA
<213> Homo sapiens

<400> 998
gctgagagtg taggat 16

<210> 999
<211> 19
<212> DNA
<213> Homo sapiens

<400> 999
gctgagagtg taggatgtt 19

<210> 1000
<211> 16

<212> DNA
<213> Homo sapiens

<400> 1000
cttccagtcg gggatg 16

<210> 1001
<211> 14
<212> DNA
<213> Homo sapiens

<400> 1001
agacacgtgc actg 14

<210> 1002
<211> 15
<212> DNA
<213> Homo sapiens

<400> 1002
agacacgtgc actgt 15

<210> 1003
<211> 16
<212> DNA
<213> Homo sapiens

<400> 1003
agacacgtgc actgta 16

<210> 1004
<211> 15
<212> DNA
<213> Homo sapiens

<400> 1004
gcagaagcat ttcca 15

<210> 1005
<211> 17
<212> DNA
<213> Homo sapiens

<400> 1005
caacaaaatc actagtc 17

<210> 1006
<211> 18
<212> DNA
<213> Homo sapiens

<400> 1006
caacaaaatc actagtct 18

<210> 1007
<211> 19
<212> DNA
<213> Homo sapiens

<400> 1007
caacaaaatc actagtctt 19

<210> 1008
<211> 20
<212> DNA
<213> Homo sapiens

<400> 1008 20
caacaaaatc actagtcttc 20

<210> 1009
<211> 16
<212> DNA
<213> Homo sapiens

<400> 1009 16
cacaaattcg gatcta 16

<210> 1010
<211> 17
<212> DNA
<213> Homo sapiens

<400> 1010
cacaaattcg gatctac 17

<210> 1011
<211> 18
<212> DNA
<213> Homo sapiens

<400> 1011
cacaaattcg gatctaca 18

<210> 1012
<211> 17
<212> DNA
<213> Homo sapiens

<400> 1012
acaaattcgg ttctaca 17

<210> 1013
<211> 18
<212> DNA
<213> Homo sapiens

<400> 1013
acaaattcgg ttctacag 18

<210> 1014
<211> 16
<212> DNA
<213> Homo sapiens

<400> 1014
aacaaccagc taagac 16

<210> 1015
<211> 17
<212> DNA
<213> Homo sapiens

<400> 1015
aacaaccagc taagaca 17

<210> 1016
<211> 18
<212> DNA
<213> Homo sapiens

<400> 1016
aacaaccagc taagacac 18

<210> 1017
<211> 14
<212> DNA
<213> Homo sapiens

<400> 1017
actcaccgac aggt 14

<210> 1018
<211> 15
<212> DNA
<213> Homo sapiens

<400> 1018
actcaccgac aggtt 15

<210> 1019
<211> 16
<212> DNA
<213> Homo sapiens

<400> 1019
actcaccgac aggttg 16

<210> 1020
<211> 19
<212> DNA
<213> Homo sapiens

<400> 1020
tgtgagttct accattgcc 19

<210> 1021
<211> 18
<212> DNA
<213> Homo sapiens

<400> 1021
gaacagatag tctaaaca 18

<210> 1022
<211> 19

<212> DNA
<213> Homo sapiens

<400> 1022
gaacagatag tctaaacac 19

<210> 1023
<211> 20
<212> DNA
<213> Homo sapiens

<400> 1023
gaacagatag tctaaacact 20

<210> 1024
<211> 16
<212> DNA
<213> Homo sapiens

<400> 1024
aggcatagga tgacaa 16

<210> 1025
<211> 17
<212> DNA
<213> Homo sapiens

<400> 1025
aggcatagga tgacaaa 17

<210> 1026
<211> 18
<212> DNA
<213> Homo sapiens

<400> 1026
aggcatagga tgacaaag 18

<210> 1027
<211> 15
<212> DNA
<213> Homo sapiens

<400> 1027
tcagccgctg tcaca 15

<210> 1028
<211> 14
<212> DNA
<213> Homo sapiens

<400> 1028
aggcgaagga tgac 14

<210> 1029
<211> 15
<212> DNA
<213> Homo sapiens

<400> 1029
aggcgaagga tgaca 15

<210> 1030
<211> 16
<212> DNA
<213> Homo sapiens

<400> 1030
aggcgaagga tgacaa 16

<210> 1031
<211> 17
<212> DNA
<213> Homo sapiens

<400> 1031
aggcgaagga tgacaaa 17

<210> 1032
<211> 15
<212> DNA
<213> Homo sapiens

<400> 1032
ctgcctgtct gtgcc 15

<210> 1033
<211> 17
<212> DNA
<213> Homo sapiens

<400> 1033
acatggttag atcaagc 17

<210> 1034
<211> 18
<212> DNA
<213> Homo sapiens

<400> 1034
acatggttag atcaagca 18

<210> 1035
<211> 14
<212> DNA
<213> Homo sapiens

<400> 1035
gaaacccagc agac 14

<210> 1036
<211> 15
<212> DNA
<213> Homo sapiens

<400> 1036
gaaacccagc agaca 15

<210> 1037
<211> 16
<212> DNA
<213> Homo sapiens

<400> 1037
gaaacccagc agacaa 16

<210> 1038
<211> 17
<212> DNA
<213> Homo sapiens

<400> 1038
gaaacccagc agacaat 17

<210> 1039
<211> 14
<212> DNA
<213> Homo sapiens

<400> 1039
gagacccagt agcc 14

<210> 1040
<211> 15
<212> DNA
<213> Homo sapiens

<400> 1040
gagacccagt agcca 15

<210> 1041
<211> 16
<212> DNA
<213> Homo sapiens

<400> 1041
ggggtatttg acaaac 16

<210> 1042
<211> 17
<212> DNA
<213> Homo sapiens

<400> 1042
ggggtatttg acaaact 17

<210> 1043
<211> 18
<212> DNA
<213> Homo sapiens

<400> 1043
ggggtatttg acaaactg 18

<210> 1044
<211> 16

<212> DNA
<213> Homo sapiens

<400> 1044
taaacggaac cactag 16

<210> 1045
<211> 17
<212> DNA
<213> Homo sapiens

<400> 1045
taaacggaac cactagt 17

<210> 1046
<211> 18
<212> DNA
<213> Homo sapiens

<400> 1046
taaacggaac cactagtg 18

<210> 1047
<211> 18
<212> DNA
<213> Homo sapiens

<400> 1047
actgtacaaa ctactacc 18

<210> 1048
<211> 17
<212> DNA
<213> Homo sapiens

<400> 1048
acagcacaaa ctactac 17

<210> 1049
<211> 14
<212> DNA
<213> Homo sapiens

<400> 1049
ggtaatccct ggca 14

<210> 1050
<211> 15
<212> DNA
<213> Homo sapiens

<400> 1050
ggtaatccct ggcaa 15

<210> 1051
<211> 16
<212> DNA
<213> Homo sapiens

<400> 1051
ggtaatccct ggcaat 16

<210> 1052
<211> 15
<212> DNA
<213> Homo sapiens

<400> 1052
gcagaactta gccac 15

<210> 1053
<211> 16
<212> DNA
<213> Homo sapiens

<400> 1053
gcagaactta gccact 16

<210> 1054
<211> 21
<212> DNA
<213> Homo sapiens

<400> 1054
agctgagtgt aggatgttta c 21

<210> 1055
<211> 16
<212> DNA
<213> Homo sapiens

<400> 1055
tcacaagtta gggtct 16

<210> 1056
<211> 19
<212> DNA
<213> Homo sapiens

<400> 1056
tcacaagtta gggtctcag 19

<210> 1057
<211> 17
<212> DNA
<213> Homo sapiens

<400> 1057
atgccctttt aacattg 17

<210> 1058
<211> 20
<212> DNA
<213> Homo sapiens

<400> 1058
atgccctttt aacattgcac 20

<210> 1059
<211> 14
<212> DNA
<213> Homo sapiens

<400> 1059
cgaccatggc tgta 14

<210> 1060
<211> 15
<212> DNA
<213> Homo sapiens

<400> 1060
acagctggtt gaagg 15

<210> 1061
<211> 19
<212> DNA
<213> Homo sapiens

<400> 1061
tcacatagga ataaaaagc 19

<210> 1062
<211> 20
<212> DNA
<213> Homo sapiens

<400> 1062
tcacatagga ataaaaagcc 20

<210> 1063
<211> 17
<212> DNA
<213> Homo sapiens

<400> 1063
ctaccatagg gtaaaac 17

<210> 1064
<211> 18
<212> DNA
<213> Homo sapiens

<400> 1064
ctaccatagg gtaaaacc 18

<210> 1065
<211> 18
<212> DNA
<213> Homo sapiens

<400> 1065
tccataaagt aggaaaca 18

<210> 1066
<211> 19

<212> DNA
<213> Homo sapiens

<400> 1066
tccataaagt aggaaacac 19

<210> 1067
<211> 20
<212> DNA
<213> Homo sapiens

<400> 1067
tccataaagt aggaaacact 20

<210> 1068
<211> 21
<212> DNA
<213> Homo sapiens

<400> 1068
tccataaagt aggaaacact a 21

<210> 1069
<211> 17
<212> DNA
<213> Homo sapiens

<400> 1069
gtagtgcttt ctacttt 17

<210> 1070
<211> 18
<212> DNA
<213> Homo sapiens

<400> 1070
gtagtgcttt ctacttta 18

<210> 1071
<211> 16
<212> DNA
<213> Homo sapiens

<400> 1071
tgagctacag tgcttc 16

<210> 1072
<211> 17
<212> DNA
<213> Homo sapiens

<400> 1072
tgagctacag tgcttca 17

<210> 1073
<211> 18
<212> DNA
<213> Homo sapiens

<400> 1073
tgagctacag tgcttcat 18

<210> 1074
<211> 20
<212> DNA
<213> Homo sapiens

<400> 1074
ctagtacatc atctatactg 20

<210> 1075
<211> 21
<212> DNA
<213> Homo sapiens

<400> 1075
ctagtacatc atctatactg t 21

<210> 1076
<211> 15
<212> DNA
<213> Homo sapiens

<400> 1076
aagggattcc tggga 15

<210> 1077
<211> 16
<212> DNA
<213> Homo sapiens

<400> 1077
aagggattcc tgggaa 16

<210> 1078
<211> 17
<212> DNA
<213> Homo sapiens

<400> 1078
aagggattcc tgggaaa 17

<210> 1079
<211> 14
<212> DNA
<213> Homo sapiens

<400> 1079
cccaagttct gtca 14

<210> 1080
<211> 15
<212> DNA
<213> Homo sapiens

<400> 1080
cccaagttct gtcat 15

<210> 1081
<211> 16
<212> DNA
<213> Homo sapiens

<400> 1081
cccaagttct gtcatg 16

<210> 1082
<211> 17
<212> DNA
<213> Homo sapiens

<400> 1082
agctgctttt gggattc 17

<210> 1083
<211> 18
<212> DNA
<213> Homo sapiens

<400> 1083
agctgctttt gggattcc 18

<210> 1084
<211> 18
<212> DNA
<213> Homo sapiens

<400> 1084
tcatacagct agataacc 18

<210> 1085
<211> 15
<212> DNA
<213> Homo sapiens

<400> 1085
cacaggttaa agggt 15

<210> 1086
<211> 16
<212> DNA
<213> Homo sapiens

<400> 1086
cacaggttaa agggtc 16

<210> 1087
<211> 17
<212> DNA
<213> Homo sapiens

<400> 1087
cacaggttaa agggtct 17

<210> 1088
<211> 16

<212> DNA
<213> Homo sapiens

<400> 1088
gcattattac tcacgg 16

<210> 1089
<211> 17
<212> DNA
<213> Homo sapiens

<400> 1089
gcattattac tcacggt 17

<210> 1090
<211> 18
<212> DNA
<213> Homo sapiens

<400> 1090
gcattattac tcacggta 18

<210> 1091
<211> 14
<212> DNA
<213> Homo sapiens

<400> 1091
cgcgtaccaa aagt 14

<210> 1092
<211> 15
<212> DNA
<213> Homo sapiens

<400> 1092
cgcgtaccaa aagta 15

<210> 1093
<211> 16
<212> DNA
<213> Homo sapiens

<400> 1093
cgcgtaccaa aagtaa 16

<210> 1094
<211> 17
<212> DNA
<213> Homo sapiens

<400> 1094
cgcgtaccaa aagtaat 17

<210> 1095
<211> 18
<212> DNA
<213> Homo sapiens

<400> 1095
cgcgtaccaa aagtaata 18

<210> 1096
<211> 19
<212> DNA
<213> Homo sapiens

<400> 1096
cgcgtaccaa aagtaataa 19

<210> 1097
<211> 20
<212> DNA
<213> Homo sapiens

<400> 1097
cgcgtaccaa aagtaataat 20

<210> 1098
<211> 14
<212> DNA
<213> Homo sapiens

<400> 1098
agccaagctc agac 14

<210> 1099
<211> 15
<212> DNA
<213> Homo sapiens

<400> 1099
agccaagctc agacg 15

<210> 1100
<211> 16
<212> DNA
<213> Homo sapiens

<400> 1100
ccctctggtc aaccag 16

<210> 1101
<211> 17
<212> DNA
<213> Homo sapiens

<400> 1101
tccatcatca aaacaaa 17

<210> 1102
<211> 18
<212> DNA
<213> Homo sapiens

<400> 1102
tccatcatca aaacaaat 18

<210> 1103
<211> 19
<212> DNA
<213> Homo sapiens

<400> 1103
tccatcatca aaacaaatg 19

<210> 1104
<211> 20
<212> DNA
<213> Homo sapiens

<400> 1104
tccatcatca aaacaaatgg 20

<210> 1105
<211> 17
<212> DNA
<213> Homo sapiens

<400> 1105
acccttatca gttctcc 17

<210> 1106
<211> 16
<212> DNA
<213> Homo sapiens

<400> 1106
gaactgcctt tctctc 16

<210> 1107
<211> 17
<212> DNA
<213> Homo sapiens

<400> 1107
gaactgcctt tctctcc 17

<210> 1108
<211> 15
<212> DNA
<213> Homo sapiens

<400> 1108
aagcccaaaa ggaga 15

<210> 1109
<211> 16
<212> DNA
<213> Homo sapiens

<400> 1109
aagcccaaaa ggagaa 16

<210> 1110
<211> 17

<212> DNA
<213> Homo sapiens

<400> 1110
aagcccaaaa ggagaat 17

<210> 1111
<211> 18
<212> DNA
<213> Homo sapiens

<400> 1111
aagcccaaaa ggagaatt 18

<210> 1112
<211> 16
<212> DNA
<213> Homo sapiens

<400> 1112
accctccacc atgcaa 16

<210> 1113
<211> 17
<212> DNA
<213> Homo sapiens

<400> 1113
tccacatgga gttgctg 17

<210> 1114
<211> 16
<212> DNA
<213> Homo sapiens

<400> 1114
gccaatattt ctgtgc 16

<210> 1115
<211> 17
<212> DNA
<213> Homo sapiens

<400> 1115
gccaatattt ctgtgct 17

<210> 1116
<211> 14
<212> DNA
<213> Homo sapiens

<400> 1116
gaaagagacc ggtt 14

<210> 1117
<211> 15
<212> DNA
<213> Homo sapiens

<400> 1117
gaaagagacc ggttc 15

<210> 1118
<211> 16
<212> DNA
<213> Homo sapiens

<400> 1118
gaaagagacc ggttca 16

<210> 1119
<211> 15
<212> DNA
<213> Homo sapiens

<400> 1119
atctgcactg tcagc 15

<210> 1120
<211> 17
<212> DNA
<213> Homo sapiens

<400> 1120
atctgcactg tcagcac 17

<210> 1121
<211> 18
<212> DNA
<213> Homo sapiens

<400> 1121
atctgcactg tcagcact 18

<210> 1122
<211> 16
<212> DNA
<213> Homo sapiens

<400> 1122
accgatttca aatggt 16

<210> 1123
<211> 17
<212> DNA
<213> Homo sapiens

<400> 1123
accgatttca aatggtg 17

<210> 1124
<211> 16
<212> DNA
<213> Homo sapiens

<400> 1124
acatcgttac cagaca 16

<210> 1125
<211> 19
<212> DNA
<213> Homo sapiens

<400> 1125
acatcgttac cagacagtg 19

<210> 1126
<211> 16
<212> DNA
<213> Homo sapiens

<400> 1126
tcaccaaaac atggaa 16

<210> 1127
<211> 17
<212> DNA
<213> Homo sapiens

<400> 1127
tcaccaaaac atggaag 17

<210> 1128
<211> 17
<212> DNA
<213> Homo sapiens

<400> 1128
gcaatcagct aactaca 17

<210> 1129
<211> 20
<212> DNA
<213> Homo sapiens

<400> 1129
gcaatcagct aactacactg 20

<210> 1130
<211> 18
<212> DNA
<213> Homo sapiens

<400> 1130
gctttgacaa tactattg 18

<210> 1131
<211> 21
<212> DNA
<213> Homo sapiens

<400> 1131
gctttgacaa tactattgca c 21

<210> 1132
<211> 16

<212> DNA
<213> Homo sapiens

<400> 1132
acaggattga gggggg 16

<210> 1133
<211> 18
<212> DNA
<213> Homo sapiens

<400> 1133
atgccctttc atcattgc 18

<210> 1134
<211> 19
<212> DNA
<213> Homo sapiens

<400> 1134
atgccctttc atcattgca 19

<210> 1135
<211> 15
<212> DNA
<213> Homo sapiens

<400> 1135
gctgtaaaca tccga 15

<210> 1136
<211> 15
<212> DNA
<213> Homo sapiens

<400> 1136
tccagtcaag gatgt 15

<210> 1137
<211> 16
<212> DNA
<213> Homo sapiens

<400> 1137
tccagtcaag gatgtt 16

<210> 1138
<211> 17
<212> DNA
<213> Homo sapiens

<400> 1138
tccagtcaag gatgttt 17

<210> 1139
<211> 18
<212> DNA
<213> Homo sapiens

<400> 1139
tccagtcaag gatgttta 18

<210> 1140
<211> 19
<212> DNA
<213> Homo sapiens

<400> 1140
tccagtcaag gatgtttac 19

<210> 1141
<211> 16
<212> DNA
<213> Homo sapiens

<400> 1141
gtacccctgg agattc 16

<210> 1142
<211> 17
<212> DNA
<213> Homo sapiens

<400> 1142
gtacccctgg agattct 17

<210> 1143
<211> 18
<212> DNA
<213> Homo sapiens

<400> 1143
ctactaaaac atggaagc 18

<210> 1144
<211> 19
<212> DNA
<213> Homo sapiens

<400> 1144
ctactaaaac atggaagca 19

<210> 1145
<211> 15
<212> DNA
<213> Homo sapiens

<400> 1145
cagcaggtac cccca 15

<210> 1146
<211> 16
<212> DNA
<213> Homo sapiens

<400> 1146
acactcaaac atggaa 16

<210> 1147
<211> 17
<212> DNA
<213> Homo sapiens

<400> 1147
acactcaaac atggaag 17

<210> 1148
<211> 18
<212> DNA
<213> Homo sapiens

<400> 1148
acactcaaac atggaagc 18

<210> 1149
<211> 17
<212> DNA
<213> Homo sapiens

<400> 1149
tcaccattgc taaagtg 17

<210> 1150
<211> 16
<212> DNA
<213> Homo sapiens

<400> 1150
aaacgtggaa tttcct 16

<210> 1151
<211> 17
<212> DNA
<213> Homo sapiens

<400> 1151
aaacgtggaa tttcctc 17

<210> 1152
<211> 18
<212> DNA
<213> Homo sapiens

<400> 1152
aaacgtggaa tttcctct 18

<210> 1153
<211> 19
<212> DNA
<213> Homo sapiens

<400> 1153
aaacgtggaa tttcctcta 19

<210> 1154
<211> 20

<212> DNA
<213> Homo sapiens

<400> 1154
aaacgtggaa tttcctctat 20

<210> 1155
<211> 15
<212> DNA
<213> Homo sapiens

<400> 1155
acaccccaaa atcga 15

<210> 1156
<211> 14
<212> DNA
<213> Homo sapiens

<400> 1156
ggaaagcgcc ccca 14

<210> 1157
<211> 18
<212> DNA
<213> Homo sapiens

<400> 1157
cacttatcag gttgtatt 18

<210> 1158
<211> 19
<212> DNA
<213> Homo sapiens

<400> 1158
cacttatcag gttgtatta 19

<210> 1159
<211> 20
<212> DNA
<213> Homo sapiens

<400> 1159
cacttatcag gttgtattat 20

<210> 1160
<211> 21
<212> DNA
<213> Homo sapiens

<400> 1160
cacttatcag gttgtattat a 21

<210> 1161
<211> 15
<212> DNA
<213> Homo sapiens

<400> 1161
acgtggattt tcctc 15

<210> 1162
<211> 16
<212> DNA
<213> Homo sapiens

<400> 1162
acgtggattt tcctct 16

<210> 1163
<211> 17
<212> DNA
<213> Homo sapiens

<400> 1163
acgtggattt tcctcta 17

<210> 1164
<211> 18
<212> DNA
<213> Homo sapiens

<400> 1164
acgtggattt tcctctat 18

<210> 1165
<211> 16
<212> DNA
<213> Homo sapiens

<400> 1165
acaaaagttg cctttg 16

<210> 1166
<211> 17
<212> DNA
<213> Homo sapiens

<400> 1166
acaaaagttg cctttgt 17

<210> 1167
<211> 14
<212> DNA
<213> Homo sapiens

<400> 1167
acacaggacc tgga 14

<210> 1168
<211> 15
<212> DNA
<213> Homo sapiens

<400> 1168
acacaggacc tggag 15

<210> 1169
<211> 16
<212> DNA
<213> Homo sapiens

<400> 1169
acacaggacc tggagt 16

<210> 1170
<211> 14
<212> DNA
<213> Homo sapiens

<400> 1170
ggccttctga ctcc 14

<210> 1171
<211> 17
<212> DNA
<213> Homo sapiens

<400> 1171
tacgttccat agtctac 17

<210> 1172
<211> 15
<212> DNA
<213> Homo sapiens

<400> 1172
agccacaatc acctt 15

<210> 1173
<211> 16
<212> DNA
<213> Homo sapiens

<400> 1173
agccacaatc accttc 16

<210> 1174
<211> 17
<212> DNA
<213> Homo sapiens

<400> 1174
agccacaatc accttct 17

<210> 1175
<211> 18
<212> DNA
<213> Homo sapiens

<400> 1175
ggctataaag taactgag 18

<210> 1176
<211> 19

<212> DNA
<213> Homo sapiens

<400> 1176
ggctataaag taactgaga 19

<210> 1177
<211> 14
<212> DNA
<213> Homo sapiens

<400> 1177
acggaagggc agag 14

<210> 1178
<211> 15
<212> DNA
<213> Homo sapiens

<400> 1178
acggaagggc agaga 15

<210> 1179
<211> 14
<212> DNA
<213> Homo sapiens

<400> 1179
gacgggtgcg attt 14

<210> 1180
<211> 14
<212> DNA
<213> Homo sapiens

<400> 1180
cctcaaggag cttc 14

<210> 1181
<211> 15
<212> DNA
<213> Homo sapiens

<400> 1181
cctcaaggag cttca 15

<210> 1182
<211> 17
<212> DNA
<213> Homo sapiens

<400> 1182
acaaagttct gtgatgc 17

<210> 1183
<211> 18
<212> DNA
<213> Homo sapiens

<400> 1183
acaaagttct gtgatgca 18

<210> 1184
<211> 16
<212> DNA
<213> Homo sapiens

<400> 1184
ttctaggata ggccca 16

<210> 1185
<211> 17
<212> DNA
<213> Homo sapiens

<400> 1185
ttctaggata ggcccag 17

<210> 1186
<211> 15
<212> DNA
<213> Homo sapiens

<400> 1186
acaccaatgc cctag 15

<210> 1187
<211> 18
<212> DNA
<213> Homo sapiens

<400> 1187
tcaacaaaat cactgatg 18

<210> 1188
<211> 19
<212> DNA
<213> Homo sapiens

<400> 1188
tcaacaaaat cactgatgc 19

<210> 1189
<211> 20
<212> DNA
<213> Homo sapiens

<400> 1189
tcaacaaaat cactgatgct 20

<210> 1190
<211> 14
<212> DNA
<213> Homo sapiens

<400> 1190
tgagctcctg gagg 14

<210> 1191
<211> 15
<212> DNA
<213> Homo sapiens

<400> 1191
tgagctcctg gagga 15

<210> 1192
<211> 18
<212> DNA
<213> Homo sapiens

<400> 1192
acatttttcg ttattgct 18

<210> 1193
<211> 15
<212> DNA
<213> Homo sapiens

<400> 1193
tagctggttg aaggg 15

<210> 1194
<211> 16
<212> DNA
<213> Homo sapiens

<400> 1194
tagctggttg aagggg 16

<210> 1195
<211> 14
<212> DNA
<213> Homo sapiens

<400> 1195
gccctggact agga 14

<210> 1196
<211> 14
<212> DNA
<213> Homo sapiens

<400> 1196
ggccttctga ccct 14

<210> 1197
<211> 15
<212> DNA
<213> Homo sapiens

<400> 1197
ggccttctga cccta 15

<210> 1198
<211> 16

<212> DNA
<213> Homo sapiens

<400> 1198
ggccttctga ccctaa 16

<210> 1199
<211> 15
<212> DNA
<213> Homo sapiens

<400> 1199
ctgaggggcc tcaga 15

<210> 1200
<211> 17
<212> DNA
<213> Homo sapiens

<400> 1200
ttcaaaacat gaattgc 17

<210> 1201
<211> 18
<212> DNA
<213> Homo sapiens

<400> 1201
ttcaaaacat gaattgct 18

<210> 1202
<211> 19
<212> DNA
<213> Homo sapiens

<400> 1202
ttcaaaacat gaattgctg 19

<210> 1203
<211> 20
<212> DNA
<213> Homo sapiens

<400> 1203
ttcaaaacat gaattgctgc 20

<210> 1204
<211> 15
<212> DNA
<213> Drosophila melanogaster

<400> 1204
tgagacacac tttgc 15

<210> 1205
<211> 16
<212> DNA
<213> Drosophila melanogaster

<400> 1205
tgagacacac tttgcc 16

<210> 1206
<211> 19
<212> DNA
<213> Drosophila melanogaster

<400> 1206
actatacaac ctactacct 19

<210> 1207
<211> 20
<212> DNA
<213> Drosophila melanogaster

<400> 1207
actatacaac ctactacctc 20

<210> 1208
<211> 21
<212> DNA
<213> Drosophila melanogaster

<400> 1208
actatacaac ctactacctc a 21

<210> 1209
<211> 18
<212> DNA
<213> Drosophila melanogaster

<400> 1209
taggagagag aaaaagac 18

<210> 1210
<211> 19
<212> DNA
<213> Drosophila melanogaster

<400> 1210
taggagagag aaaaagact 19

<210> 1211
<211> 20
<212> DNA
<213> Drosophila melanogaster

<400> 1211
taggagagag aaaaagactg 20

<210> 1212
<211> 21
<212> DNA
<213> Drosophila melanogaster

<400> 1212
taggagagag aaaaagactg a 21

<210> 1213
<211> 14
<212> DNA
<213> Drosophila melanogaster

<400> 1213
tcagctatgc cgac 14

<210> 1214
<211> 15
<212> DNA
<213> Drosophila melanogaster

<400> 1214
tcagctatgc cgaca 15

<210> 1215
<211> 16
<212> DNA
<213> Drosophila melanogaster

<400> 1215
tcagctatgc cgacat 16

<210> 1216
<211> 17
<212> DNA
<213> Drosophila melanogaster

<400> 1216
tcagctatgc cgacatc 17

<210> 1217
<211> 14
<212> DNA
<213> Drosophila melanogaster

<400> 1217
gctcctcaaa gctg 14

<210> 1218
<211> 16
<212> DNA
<213> Drosophila melanogaster

<400> 1218
aaaaagaaca gccact 16

<210> 1219
<211> 17
<212> DNA
<213> Drosophila melanogaster

<400> 1219
aaaaagaaca gccactg 17

<210> 1220
<211> 18

<212> DNA
<213> Drosophila melanogaster

<400> 1220
aaaaagaaca gccactgt 18

<210> 1221
<211> 14
<212> DNA
<213> Drosophila melanogaster

<400> 1221
cggggcgaga gaat 14

<210> 1222
<211> 15
<212> DNA
<213> Drosophila melanogaster

<400> 1222
cggggcgaga gaatg 15

<210> 1223
<211> 16
<212> DNA
<213> Drosophila melanogaster

<400> 1223
gcactgattt cgaatg 16

<210> 1224
<211> 17
<212> DNA
<213> Drosophila melanogaster

<400> 1224
gcactgattt cgaatgg 17

<210> 1225
<211> 18
<212> DNA
<213> Drosophila melanogaster

<400> 1225
ctcacagtat aatcctgt 18

<210> 1226
<211> 19
<212> DNA
<213> Drosophila melanogaster

<400> 1226
ctcacagtat aatcctgtg 19

<210> 1227
<211> 20
<212> DNA
<213> Drosophila melanogaster

<400> 1227
ctcacagtat aatcctgtga 20

<210> 1228
<211> 21
<212> DNA
<213> Drosophila melanogaster

<400> 1228
ctcacagtat aatcctgtga t 21

<210> 1229
<211> 14
<212> DNA
<213> Drosophila melanogaster

<400> 1229
cgccagtaag cgga 14

<210> 1230
<211> 15
<212> DNA
<213> Drosophila melanogaster

<400> 1230
cgccagtaag cggaa 15

<210> 1231
<211> 15
<212> DNA
<213> Drosophila melanogaster

<400> 1231
gctcatcaaa gctgg 15

<210> 1232
<211> 16
<212> DNA
<213> Drosophila melanogaster

<400> 1232
gctcatcaaa gctggc 16

<210> 1233
<211> 17
<212> DNA
<213> Drosophila melanogaster

<400> 1233
gctcatcaaa gctggct 17

<210> 1234
<211> 14
<212> DNA
<213> Drosophila melanogaster

<400> 1234
gcccatcaaa gctg 14

<210> 1235
<211> 15
<212> DNA
<213> Drosophila melanogaster

<400> 1235
gcccatcaaa gctgg 15

<210> 1236
<211> 15
<212> DNA
<213> Drosophila melanogaster

<400> 1236
cagctattcc gacat 15

<210> 1237
<211> 16
<212> DNA
<213> Drosophila melanogaster

<400> 1237
cagctattcc gacatc 16

<210> 1238
<211> 17
<212> DNA
<213> Drosophila melanogaster

<400> 1238
cagctattcc gacatct 17

<210> 1239
<211> 18
<212> DNA
<213> Drosophila melanogaster

<400> 1239
cagctattcc gacatctt 18

<210> 1240
<211> 19
<212> DNA
<213> Drosophila melanogaster

<400> 1240
cagctattcc gacatcttg 19

<210> 1241
<211> 19
<212> DNA
<213> Homo sapiens

<400> 1241
ctgcactata agcacttta 19

<210> 1242
<211> 20

<212> DNA
<213> Homo sapiens

<400> 1242
cctgcactat aagcacttta 20

<210> 1243
<211> 21
<212> DNA
<213> Homo sapiens

<400> 1243
acctgcacta taagcacttt a 21

<210> 1244
<211> 22
<212> DNA
<213> Homo sapiens

<400> 1244
tacctgcact ataagcactt ta 22

<210> 1245
<211> 23
<212> DNA
<213> Homo sapiens

<400> 1245
ctacctgcac tataagcact tta 23

<210> 1246
<211> 19
<212> DNA
<213> Homo sapiens

<400> 1246
ctgcactatg agcactttg 19

<210> 1247
<211> 20
<212> DNA
<213> Homo sapiens

<400> 1247
cctgcactat gagcactttg 20

<210> 1248
<211> 21
<212> DNA
<213> Homo sapiens

<400> 1248
acctgcacta tgagcacttt g 21

<210> 1249
<211> 22
<212> DNA
<213> Homo sapiens

<400> 1249
tacctgcact atgagcactt tg 22

<210> 1250
<211> 23
<212> DNA
<213> Homo sapiens

<400> 1250
ctacctgcac tatgagcact ttg 23

<210> 1251
<211> 20
<212> DNA
<213> Homo sapiens

<400> 1251
cgctcgggtt ttcccgagcg 20

<210> 1252
<211> 30
<212> DNA
<213> Homo sapiens

<400> 1252
gaactataca acctactacc tttttttttt 30

<210> 1253
<211> 31
<212> DNA
<213> Homo sapiens

<400> 1253
gaactataca acctactacc tttttttttt t 31

<210> 1254
<211> 28
<212> DNA
<213> Homo sapiens

<400> 1254
gaaccacaca acctactatt tttttttt 28

<210> 1255
<211> 29
<212> DNA
<213> Homo sapiens

<400> 1255
gaaccacaca acctactact ttttttttt 29

<210> 1256
<211> 29
<212> DNA
<213> Homo sapiens

<400> 1256
gaaccataca acctactact ttttttttt 29

<210> 1257
<211> 30
<212> DNA
<213> Homo sapiens

<400> 1257
gaaccataca acctactacc tttttttttt 30

<210> 1258
<211> 31
<212> DNA
<213> Homo sapiens

<400> 1258
gaaccataca acctactacc tttttttttt t 31

<210> 1259
<211> 30
<212> DNA
<213> Homo sapiens

<400> 1259
gactatgcaa cctactacct tttttttttt 30

<210> 1260
<211> 50
<212> DNA
<213> Homo sapiens

<400> 1260
cgctcgggtt ttcccgagcg gaactataca acctactacc tttttttttt 50

<210> 1261
<211> 51
<212> DNA
<213> Homo sapiens

<400> 1261
cgctcgggtt ttcccgagcg gaactataca acctactacc tttttttttt t 51

<210> 1262
<211> 48
<212> DNA
<213> Homo sapiens

<400> 1262.
cgctcgggtt ttcccgagcg gaaccacaca acctactatt tttttttt 48

<210> 1263
<211> 49
<212> DNA
<213> Homo sapiens

<400> 1263
cgctcgggtt ttcccgagcg gaaccacaca acctactact ttttttttt 49

<210> 1264
<211> 49

<212> DNA
<213> Homo sapiens

<400> 1264
cgctcgggtt ttcccgagcg gaaccataca acctactact ttttttttt 49

<210> 1265
<211> 50
<212> DNA
<213> Homo sapiens

<400> 1265
cgctcgggtt ttcccgagcg gaaccataca acctactacc tttttttttt 50

<210> 1266
<211> 51
<212> DNA
<213> Homo sapiens

<400> 1266
cgctcgggtt ttcccgagcg gaaccataca acctactacc tttttttttt t 51

<210> 1267
<211> 50
<212> DNA
<213> Homo sapiens

<400> 1267
cgctcgggtt ttcccgagcg gactatgcaa cctactacct tttttttttt 50

<210> 1268
<211> 22
<212> RNA
<213> Homo sapiens

<400> 1268
ugagguagua gguuguauag uu 22

<210> 1269
<211> 22
<212> RNA
<213> Homo sapiens

<400> 1269
ugagguagua gguugugugg uu 22

<210> 1270
<211> 22
<212> RNA
<213> Homo sapiens

<400> 1270
ugagguagua gguuguaugg uu 22

<210> 1271
<211> 21
<212> RNA
<213> Homo sapiens

<400> 1271
agagguagua gguugcauag u 21

<210> 1272
<211> 21
<212> RNA
<213> Homo sapiens

<400> 1272
ugagguagga gguuguauag u 21

<210> 1273
<211> 22
<212> RNA
<213> Homo sapiens

<400> 1273
ugagguagua gauuguauag uu 22

<210> 1274
<211> 21
<212> RNA
<213> Homo sapiens

<400> 1274
ugagguagua guuuguacag u 21

<210> 1275
<211> 21
<212> RNA
<213> Homo sapiens

<400> 1275
ugagguagua guuugugcug u 21

<210> 1276
<211> 21
<212> RNA
<213> Homo sapiens

<400> 1276
uggaauguaa agaaguaugu a 21

<210> 1277
<211> 22
<212> RNA
<213> Homo sapiens

<400> 1277
aacccguaga uccgaacuug ug 22

<210> 1278
<211> 22
<212> RNA
<213> Homo sapiens

<400> 1278
uacaguacug ugauaacuga ag 22

<210> 1279
<211> 23
<212> RNA
<213> Homo sapiens

<400> 1279
agcagcauug uacagggcua uga 23

<210> 1280
<211> 20
<212> RNA
<213> Homo sapiens

<400> 1280
ucaaaugcuc agacuccugu 20

<210> 1281
<211> 24
<212> RNA
<213> Homo sapiens

<400> 1281
aaaagugcuu acagugcagg uagc 24

<210> 1282
<211> 21
<212> RNA
<213> Homo sapiens

<400> 1282
uaaagugcug acagugcaga u 21

<210> 1283
<211> 23
<212> RNA
<213> Homo sapiens

<400> 1283
agcagcauug uacagggcua uca 23

<210> 1284
<211> 21
<212> RNA
<213> Homo sapiens

<400> 1284
auaaggauuu uuaggggcau u 21

<210> 1285
<211> 23
<212> RNA
<213> Homo sapiens

<400> 1285
uacccuguag auccgaauuu gug 23

<210> 1286
<211> 22

<212> RNA
<213> Homo sapiens

<400> 1286
uacccuguag aaccgaauuu gu 22

<210> 1287
<211> 23
<212> RNA
<213> Homo sapiens

<400> 1287
uggaguguga caaugguguu ugu 23

<210> 1288
<211> 22
<212> RNA
<213> Homo sapiens

<400> 1288
uuaaggcacg cggugaaugc ca 22

<210> 1289
<211> 23
<212> RNA
<213> Homo sapiens

<400> 1289
ucccugagac ccuuuaaccu gug 23

<210> 1290
<211> 22
<212> RNA
<213> Homo sapiens

<400> 1290
ucccugagac ccuaacuugu ga 22

<210> 1291
<211> 21
<212> RNA
<213> Homo sapiens

<400> 1291
ucguaccgug aguaauaaug c 21

<210> 1292
<211> 21
<212> RNA
<213> Homo sapiens

<400> 1292
cauuauuacu uuugguacgc g 21

<210> 1293
<211> 22
<212> RNA
<213> Homo sapiens

<400> 1293
ucggauccgu cugagcuugg cu 22

<210> 1294
<211> 22
<212> RNA
<213> Homo sapiens

<400> 1294
ucacagugaa ccggucucuu uu 22

<210> 1295
<211> 22
<212> RNA
<213> Homo sapiens

<400> 1295
ucacagugaa ccggucucuu uc 22

<210> 1296
<211> 21
<212> RNA
<213> Homo sapiens

<400> 1296
cuuuuugcgg ucugggcuug c 21

<210> 1297
<211> 22
<212> RNA
<213> Homo sapiens

<400> 1297
cagugcaaug uuaaaagggc au 22

<210> 1298
<211> 22
<212> RNA
<213> Homo sapiens

<400> 1298
cagugcaaug augaaagggc au 22

<210> 1299
<211> 22
<212> RNA
<213> Homo sapiens

<400> 1299
uaacagucua cagccauggu cg 22

<210> 1300
<211> 22
<212> RNA
<213> Homo sapiens

<400> 1300
uugguccccu ucaaccagcu gu 22

<210> 1301
<211> 21
<212> RNA
<213> Homo sapiens

<400> 1301
uugguccccu ucaaccagcu a 21

<210> 1302
<211> 21
<212> RNA
<213> Homo sapiens

<400> 1302
ugugacuggu ugaccagagg g 21

<210> 1303
<211> 23
<212> RNA
<213> Homo sapiens

<400> 1303
uauggcuuuu uauuccuaug uga 23

<210> 1304
<211> 22
<212> RNA
<213> Homo sapiens

<400> 1304
uauggcuuuu cauuccuaug ug 22

<210> 1305
<211> 23
<212> RNA
<213> Homo sapiens

<400> 1305
acuccauuug uuuugaugau gga 23

<210> 1306
<211> 22
<212> RNA
<213> Homo sapiens

<400> 1306
uauugcuuaa gaauacgcgu ag 22

<210> 1307
<211> 17
<212> RNA
<213> Homo sapiens

<400> 1307
agcugguguu gugaauc 17

<210> 1308
<211> 18

<212> RNA
<213> Homo sapiens

<400> 1308
ucuacagugc acgugucu 18

<210> 1309
<211> 21
<212> RNA
<213> Homo sapiens

<400> 1309
agugguuuua cccuauggua g 21

<210> 1310
<211> 22
<212> RNA
<213> Homo sapiens

<400> 1310
uaacacuguc ugguaaagau gg 22

<210> 1311
<211> 23
<212> RNA
<213> Homo sapiens

<400> 1311
uguaguguuu ccuacuuuau gga 23

<210> 1312
<211> 20
<212> RNA
<213> Homo sapiens

<400> 1312
cauaaaguag aaagcacuac 20

<210> 1313
<211> 22
<212> RNA
<213> Homo sapiens

<400> 1313
ugagaugaag cacuguagcu ca 22

<210> 1314
<211> 22
<212> RNA
<213> Homo sapiens

<400> 1314
uacaguauag augauguacu ag 22

<210> 1315
<211> 24
<212> RNA
<213> Homo sapiens

<400> 1315
guccaguuuu cccaggaauc ccuu 24

<210> 1316
<211> 22
<212> RNA
<213> Homo sapiens

<400> 1316
ugagaacuga auuccauggg uu 22

<210> 1317
<211> 22
<212> RNA
<213> Homo sapiens

<400> 1317
ugagaacuga auuccauagg cu 22

<210> 1318
<211> 20
<212> RNA
<213> Homo sapiens

<400> 1318
guguguggaa augcuucugc 20

<210> 1319
<211> 22
<212> RNA
<213> Homo sapiens

<400> 1319
ucagugcacu acagaacuuu gu 22

<210> 1320
<211> 22
<212> RNA
<213> Homo sapiens

<400> 1320
ucagugcauc acagaacuuu gu 22

<210> 1321
<211> 22
<212> RNA
<213> Homo sapiens

<400> 1321
ucuggcuccg ugucuucacu cc 22

<210> 1322
<211> 22
<212> RNA
<213> Homo sapiens

<400> 1322
ucucccaacc cuuguaccag ug 22

<210> 1323
<211> 22
<212> RNA
<213> Homo sapiens

<400> 1323
acuagacuga agcuccuuga gg 22

<210> 1324
<211> 22
<212> RNA
<213> Homo sapiens

<400> 1324
ucagugcaug acagaacuug gg 22

<210> 1325
<211> 20
<212> RNA
<213> Homo sapiens

<400> 1325
uugcauaguc acaaaaguga 20

<210> 1326
<211> 22
<212> RNA
<213> Homo sapiens

<400> 1326
uagguuaucc guguugccuu cg 22

<210> 1327
<211> 22
<212> RNA
<213> Homo sapiens

<400> 1327
aaucauacac gguugaccua uu 22

<210> 1328
<211> 22
<212> RNA
<213> Homo sapiens

<400> 1328
uuaaugcuaa ucgugauagg gg 22

<210> 1329
<211> 22
<212> RNA
<213> Homo sapiens

<400> 1329
uagcagcaca uaaugguuug ug 22

<210> 1330
<211> 22

<212> RNA
<213> Homo sapiens

<400> 1330
uagcagcaca ucaugguuua ca 22

<210> 1331
<211> 22
<212> RNA
<213> Homo sapiens

<400> 1331
uagcagcacg uaaauauugg cg 22

<210> 1332
<211> 20
<212> RNA
<213> Homo sapiens

<400> 1332
acugcaguga aggcacuugu 20

<210> 1333
<211> 24
<212> RNA
<213> Homo sapiens

<400> 1333
caaagugcuu acagugcagg uagu 24

<210> 1334
<211> 23
<212> RNA
<213> Homo sapiens

<400> 1334
aacauucaac gcugucggug agu 23

<210> 1335
<211> 22
<212> RNA
<213> Homo sapiens

<400> 1335
aacauucauu gcugucggug gg 22

<210> 1336
<211> 22
<212> RNA
<213> Homo sapiens

<400> 1336
aacauucaac cugucgguga gu 22

<210> 1337
<211> 24
<212> RNA
<213> Homo sapiens

<400> 1337
aacauucauu guugucggug gguu 24

<210> 1338
<211> 22
<212> RNA
<213> Homo sapiens

<400> 1338
uuuggcaaug guagaacuca ca 22

<210> 1339
<211> 21
<212> RNA
<213> Homo sapiens

<400> 1339
ugguucuaga cuugccaacu a 21

<210> 1340
<211> 23
<212> RNA
<213> Homo sapiens

<400> 1340
uauggcacug guagaauuca cug 23

<210> 1341
<211> 22
<212> RNA
<213> Homo sapiens

<400> 1341
uggacggaga acugauaagg gu 22

<210> 1342
<211> 18
<212> RNA
<213> Homo sapiens

<400> 1342
uggagagaaa ggcaguuc 18

<210> 1343
<211> 23
<212> RNA
<213> Homo sapiens

<400> 1343
caaagaauuc uccuuuuggg cuu 23

<210> 1344
<211> 21
<212> RNA
<213> Homo sapiens

<400> 1344
ucgugucuug uguugcagcc g 21

<210> 1345
<211> 22
<212> RNA
<213> Homo sapiens

<400> 1345
caucccuugc augguggagg gu 22

<210> 1346
<211> 23
<212> RNA
<213> Homo sapiens

<400> 1346
gugccuacug agcugauauc agu 23

<210> 1347
<211> 22
<212> RNA
<213> Homo sapiens

<400> 1347
uaaggugcau cuagugcaga ua 22

<210> 1348
<211> 22
<212> RNA
<213> Homo sapiens

<400> 1348
uaaggugcau cuagugcagu ua 22

<210> 1349
<211> 22
<212> RNA
<213> Homo sapiens

<400> 1349
ugauauguuu gauauauuag gu 22

<210> 1350
<211> 22
<212> RNA
<213> Homo sapiens

<400> 1350
caacggaauc ccaaaagcag cu 22

<210> 1351
<211> 22
<212> RNA
<213> Homo sapiens

<400> 1351
gcugcgcuug gauuucgucc cc 22

<210> 1352
<211> 21

<212> RNA
<213> Homo sapiens

<400> 1352
cugaccuaug aauugacagc c 21

<210> 1353
<211> 21
<212> RNA
<213> Homo sapiens

<400> 1353
aacuggccua caaaguccca g 21

<210> 1354
<211> 24
<212> RNA
<213> Homo sapiens

<400> 1354
aacuggcccu caaagucccg cuuu 24

<210> 1355
<211> 22
<212> RNA
<213> Homo sapiens

<400> 1355
uguaacagca acuccaugug ga 22

<210> 1356
<211> 21
<212> RNA
<213> Homo sapiens

<400> 1356
uagcagcaca gaaauauugg c 21

<210> 1357
<211> 21
<212> RNA
<213> Homo sapiens

<400> 1357
uagguaguuu cauguuguug g 21

<210> 1358
<211> 21
<212> RNA
<213> Homo sapiens

<400> 1358
uagguaguuu ccuguuguug g 21

<210> 1359
<211> 22
<212> RNA
<213> Homo sapiens

<400> 1359
uucaccaccu ucuccaccca gc 22

<210> 1360
<211> 19
<212> RNA
<213> Homo sapiens

<400> 1360
gguccagagg ggagauagg 19

<210> 1361
<211> 22
<212> RNA
<213> Homo sapiens

<400> 1361
uacaguaguc ugcacauugg uu 22

<210> 1362
<211> 23
<212> RNA
<213> Homo sapiens

<400> 1362
cccaguguuc agacuaccug uuc 23

<210> 1363
<211> 23
<212> RNA
<213> Homo sapiens

<400> 1363
cccaguguuu agacuaucug uuc 23

<210> 1364
<211> 23
<212> RNA
<213> Homo sapiens

<400> 1364
ugugcaaauc uaugcaaaac uga 23

<210> 1365
<211> 23
<212> RNA
<213> Homo sapiens

<400> 1365
ugugcaaauc caugcaaaac uga 23

<210> 1366
<211> 22
<212> RNA
<213> Homo sapiens

<400> 1366
caucuuaccg gacagugcug ga 22

<210> 1367
<211> 22
<212> RNA
<213> Homo sapiens

<400> 1367
uaacacugucugguaacgau gu 22

<210> 1368
<211> 23
<212> RNA
<213> Homo sapiens

<400> 1368
uaauacugcc ugguaaugau gac 23

<210> 1369
<211> 22
<212> RNA
<213> Homo sapiens

<400> 1369
uaauacugcc ggguaaugau gg 22

<210> 1370
<211> 22
<212> RNA
<213> Homo sapiens

<400> 1370
agagguauag ggcaugggaa aa 22

<210> 1371
<211> 22
<212> RNA
<213> Homo sapiens

<400> 1371
uuuccuaugc auauacuucu uu 22

<210> 1372
<211> 22
<212> RNA
<213> Homo sapiens

<400> 1372
gugaaauguu uaggaccacu ag 22

<210> 1373
<211> 22
<212> RNA
<213> Homo sapiens

<400> 1373
uucccuuugu cauccuaugc cu 22

<210> 1374
<211> 22

<212> RNA
<213> Homo sapiens

<400> 1374
uccuucauuc caccggaguc ug 22

<210> 1375
<211> 22
<212> RNA
<213> Homo sapiens

<400> 1375
uggaauguaa ggaagugugu gg 22

<210> 1376
<211> 22
<212> RNA
<213> Homo sapiens

<400> 1376
auaagacgag caaaaagcuu gu 22

<210> 1377
<211> 23
<212> RNA
<213> Homo sapiens

<400> 1377
uaaagugcuu auagugcagg uag 23

<210> 1378
<211> 23
<212> RNA
<213> Homo sapiens

<400> 1378
caaagugcuc auagugcagg uag 23

<210> 1379
<211> 22
<212> RNA
<213> Homo sapiens

<400> 1379
uagcuuauca gacugauguu ga 22

<210> 1380
<211> 22
<212> RNA
<213> Homo sapiens

<400> 1380
cugugcgugu gacagcggcu ga 22

<210> 1381
<211> 22
<212> RNA
<213> Homo sapiens

<400> 1381
uucccuuugu cauccuucgc cu 22

<210> 1382
<211> 21
<212> RNA
<213> Homo sapiens

<400> 1382
uaacagucuc cagucacggc c 21

<210> 1383
<211> 22
<212> RNA
<213> Homo sapiens

<400> 1383
accaucgacc guugauugua cc 22

<210> 1384
<211> 21
<212> RNA
<213> Homo sapiens

<400> 1384
acagcaggca cagacaggca g 21

<210> 1385
<211> 21
<212> RNA
<213> Homo sapiens

<400> 1385
augaccuaug aauugacaga c 21

<210> 1386
<211> 21
<212> RNA
<213> Homo sapiens

<400> 1386
uaaucucagc uggcaacugu g 21

<210> 1387
<211> 24
<212> RNA
<213> Homo sapiens

<400> 1387
uacugcauca ggaacugauu ggau 24

<210> 1388
<211> 21
<212> RNA
<213> Homo sapiens

<400> 1388
uugugcuuga ucuaaccaug u 21

<210> 1389
<211> 21
<212> RNA
<213> Homo sapiens

<400> 1389
ugauugucca aacgcaauuc u 21

<210> 1390
<211> 22
<212> RNA
<213> Homo sapiens

<400> 1390
aagcugccag uugaagaacu gu 22

<210> 1391
<211> 21
<212> RNA
<213> Homo sapiens

<400> 1391
ccacaccgua ucugacacuu u 21

<210> 1392
<211> 23
<212> RNA
<213> Homo sapiens

<400> 1392
agcuacauug ucugcugggu uuc 23

<210> 1393
<211> 24
<212> RNA
<213> Homo sapiens

<400> 1393
agcuacaucu ggcuacuggg ucuc 24

<210> 1394
<211> 21
<212> RNA
<213> Homo sapiens

<400> 1394
ugucaguuug ucaaauaccc c 21

<210> 1395
<211> 23
<212> RNA
<213> Homo sapiens

<400> 1395
caagucacua gugguuccgu uua 23

<210> 1396
<211> 21

<212> RNA
<213> Homo sapiens

<400> 1396
aucacauugc cagggauuuc c 21

<210> 1397
<211> 21
<212> RNA
<213> Homo sapiens

<400> 1397
aucacauugc cagggauuac c 21

<210> 1398
<211> .22
<212> RNA
<213> Homo sapiens

<400> 1398
uggcucaguu cagcaggaac ag 22

<210> 1399
<211> 22
<212> RNA
<213> Homo sapiens

<400> 1399
cauugcacuu gucucggucu ga 22

<210> 1400
<211> 21
<212> RNA
<213> Homo sapiens

<400> 1400
uucaaguaau ccaggauagg c 21

<210> 1401
<211> 22
<212> RNA
<213> Homo sapiens

<400> 1401
uucaaguaau ucaggauagg uu 22

<210> 1402
<211> 21
<212> RNA
<213> Homo sapiens

<400> 1402
uucacagugg cuaaguuccg c 21

<210> 1403
<211> 21
<212> RNA
<213> Homo sapiens

<400> 1403
uucacagugg cuaaguucug c 21

<210> 1404
<211> 22
<212> RNA
<213> Homo sapiens

<400> 1404
aaggagcuca cagucuauug ag 22

<210> 1405
<211> 21
<212> RNA
<213> Homo sapiens

<400> 1405
agggcccccc cucaauccug u 21

<210> 1406
<211> 22
<212> RNA
<213> Homo sapiens

<400> 1406
uaugugggau gguaaaccgc uu 22

<210> 1407
<211> 22
<212> RNA
<213> Homo sapiens

<400> 1407
ugguuuaccg ucccacauac au 22

<210> 1408
<211> 21
<212> RNA
<213> Homo sapiens

<400> 1408
uagcaccauc ugaaaucggu u 21

<210> 1409
<211> 23
<212> RNA
<213> Homo sapiens

<400> 1409
uagcaccauu ugaaaucagu guu 23

<210> 1410
<211> 20
<212> RNA
<213> Homo sapiens

<400> 1410
uagcaccauu ugaaaucggu 20

<210> 1411
<211> 23
<212> RNA
<213> Homo sapiens

<400> 1411
cagugcaaua guauugucaa agc 23

<210> 1412
<211> 22
<212> RNA
<213> Homo sapiens

<400> 1412
uaaacgugga uguacuugcu uu 22

<210> 1413
<211> 23
<212> RNA
<213> Homo sapiens

<400> 1413
acuuuaacau ggaagugcuu ucu 23

<210> 1414
<211> 22
<212> RNA
<213> Homo sapiens

<400> 1414
uuuaacaugg ggguaccugc ug 22

<210> 1415
<211> 23
<212> RNA
<213> Homo sapiens

<400> 1415
uaagugcuuc cauguuuugg uga 23

<210> 1416
<211> 23
<212> RNA
<213> Homo sapiens

<400> 1416
uaagugcuuc cauguuuuag uag 23

<210> 1417
<211> 23
<212> RNA
<213> Homo sapiens

<400> 1417
uaagugcuuc cauguuucag ugg 23

<210> 1418
<211> 23

<212> RNA
<213> Homo sapiens

<400> 1418
uaagugcuuc cauguuugag ugu 23

<210> 1419
<211> 22
<212> RNA
<213> Homo sapiens

<400> 1419
cuuucagucg gauguuugca gc 22

<210> 1420
<211> 22
<212> RNA
<213> Homo sapiens

<400> 1420
uguaaacauc cucgacugga ag 22

<210> 1421
<211> 22
<212> RNA
<213> Homo sapiens

<400> 1421
uguaaacauc cuacacucag cu 22

<210> 1422
<211> 23
<212> RNA
<213> Homo sapiens

<400> 1422
uguaaacauc cuacacucuc agc 23

<210> 1423
<211> 22
<212> RNA
<213> Homo sapiens

<400> 1423
uguaaacauc cccgacugga ag 22

<210> 1424
<211> 22
<212> RNA
<213> Homo sapiens

<400> 1424
cuuucagucg gauguuuaca gc 22

<210> 1425
<211> 20
<212> RNA
<213> Homo sapiens

<400> 1425
uguaaacauc cuugacugga 20

<210> 1426
<211> 21
<212> RNA
<213> Homo sapiens

<400> 1426
ggcaagaugc uggcauagcu g 21

<210> 1427
<211> 21
<212> RNA
<213> Homo sapiens

<400> 1427
uauugcacau uacuaaguug c 21

<210> 1428
<211> 23
<212> RNA
<213> Homo sapiens

<400> 1428
aaaagcuggg uugagagggc gaa 23

<210> 1429
<211> 22
<212> RNA
<213> Homo sapiens

<400> 1429
gcacauuaca cggucgaccu cu 22

<210> 1430
<211> 22
<212> RNA
<213> Homo sapiens

<400> 1430
ccacugcccc aggugcugcu gg 22

<210> 1431
<211> 23
<212> RNA
<213> Homo sapiens

<400> 1431
cgcauccccu agggcauugg ugu 23

<210> 1432
<211> 23
<212> RNA
<213> Homo sapiens

<400> 1432
ccuaguaggu guccaguaag ugu 23

<210> 1433
<211> 20
<212> RNA
<213> Homo sapiens

<400> 1433
ccucugggcc cuuccuccag 20

<210> 1434
<211> 22
<212> RNA
<213> Homo sapiens

<400> 1434
cuggcccucu cugcccuucc gu 22

<210> 1435
<211> 22
<212> RNA
<213> Homo sapiens

<400> 1435
aacacaccug guuaaccucu uu 22

<210> 1436
<211> 19
<212> RNA
<213> Homo sapiens

<400> 1436
gugcauugua guugcauug 19

<210> 1437
<211> 23
<212> RNA
<213> Homo sapiens

<400> 1437
gcaaagcaca cggccugcag aga 23

<210> 1438
<211> 21
<212> RNA
<213> Homo sapiens

<400> 1438
gccccugggc cuauccuaga a 21

<210> 1439
<211> 23
<212> RNA
<213> Homo sapiens

<400> 1439
ucaagagcaa uaacgaaaaa ugu 23

<210> 1440
<211> 23

<212> RNA
<213> Homo sapiens

<400> 1440
uccagcuccu auaugaugcc uuu 23

<210> 1441
<211> 23
<212> RNA
<213> Homo sapiens

<400> 1441
uccagcauca gugauuuugu uga 23

<210> 1442
<211> 21
<212> RNA
<213> Homo sapiens

<400> 1442
ucccuguccu ccaggagcuc a 21

<210> 1443
<211> 23
<212> RNA
<213> Homo sapiens

<400> 1443
uccgucucag uuacuuuaua gcc 23

<210> 1444
<211> 24
<212> RNA
<213> Homo sapiens

<400> 1444
ucucacacag aaaucgcacc cguc 24

<210> 1445
<211> 21
<212> RNA
<213> Homo sapiens

<400> 1445
ugcugacucc uaguccaggg c 21

<210> 1446
<211> 23
<212> RNA
<213> Homo sapiens

<400> 1446
ugucugcccg caugccugcc ucu 23

<210> 1447
<211> 23
<212> RNA
<213> Homo sapiens

<400> 1447
uggcaguguc uuagcugguu guu 23

<210> 1448
<211> 23
<212> RNA
<213> Homo sapiens

<400> 1448
uaggcagugu cauuagcuga uug 23

<210> 1449
<211> 23
<212> RNA
<213> Homo sapiens

<400> 1449
aggcagugua guuagcugau ugc 23

<210> 1450
<211> 22
<212> RNA
<213> Homo sapiens

<400> 1450
uuaucag-aau cuccaggggu ac 22

<210> 1451
<211> 23
<212> RNA
<213> Homo sapiens

<400> 1451
aauccuugga accuaggugu gag 23

<210> 1452
<211> 22
<212> RNA
<213> Homo sapiens

<400> 1452
auugcacggu auccaucugu aa 22

<210> 1453
<211> 22
<212> RNA
<213> Homo sapiens

<400> 1453
uaaugccccu aaaaauccuu au 22

<210> 1454
<211> 22
<212> RNA
<213> Homo sapiens

<400> 1454
aauugcacuu uagcaauggu ga 22

<210> 1455
<211> 22
<212> RNA
<213> Homo sapiens

<400> 1455
acauagagga aauuccacgu uu 22

<210> 1456
<211> 21
<212> RNA
<213> Homo sapiens

<400> 1456
aauaauacau gguugaucuu u 21

<210> 1457
<211> 22
<212> RNA
<213> Homo sapiens

<400> 1457
agaucgaccg uguuauauuc gc 22

<210> 1458
<211> 21
<212> RNA
<213> Homo sapiens

<400> 1458
gccugcuggg guggaaccug g 21

<210> 1459
<211> 21
<212> RNA
<213> Homo sapiens

<400> 1459
gugccgccau cuuuugagug u 21

<210> 1460
<211> 23
<212> RNA
<213> Homo sapiens

<400> 1460
aaagugcugc gacauuugag cgu 23

<210> 1461
<211> 23
<212> RNA
<213> Homo sapiens

<400> 1461
gaagugcuuc gauuuugggg ugu 23

<210> 1462
<211> 22

<212> RNA
<213> Homo sapiens

<400> 1462
acucaaaaug ggggcgcuuu cc 22

<210> 1463
<211> 22
<212> RNA
<213> Homo sapiens

<400> 1463
uuauaauaca accugauaag ug 22

<210> 1464
<211> 22
<212> RNA
<213> Homo sapiens

<400> 1464
uuuguucguu cggcucgcgu ga 22

<210> 1465
<211> 21
<212> RNA
<213> Homo sapiens

<400> 1465
aucauagagg aaaauccacg u 21

<210> 1466
<211> 22
<212> RNA
<213> Homo sapiens

<400> 1466
aucauagagg aaaauccaug uu 22

<210> 1467
<211> 22
<212> RNA
<213> Homo sapiens

<400> 1467
aucacacaaa ggcaacuuuu gu 22

<210> 1468
<211> 22
<212> RNA
<213> Homo sapiens

<400> 1468
cuccugacuc cagguccugu gu 22

<210> 1469
<211> 19
<212> RNA
<213> Homo sapiens

<400> 1469
ugguagacua uggaacgua 19

<210> 1470
<211> 22
<212> RNA
<213> Homo sapiens

<400> 1470
uauguaauau gguccacauc uu 22

<210> 1471
<211> 22
<212> RNA
<213> Homo sapiens

<400> 1471
ugguugacca uagaacaugc gc 22

<210> 1472
<211> 22
<212> RNA
<213> Homo sapiens

<400> 1472
uauacaaggg caagcucucu gu 22

<210> 1473
<211> 22
<212> RNA
<213> Homo sapiens

<400> 1473
gaaguuguuc gugguggauu cg 22

<210> 1474
<211> 22
<212> RNA
<213> Homo sapiens

<400> 1474
agaucagaag gugauugugg cu 22

<210> 1475
<211> 20
<212> RNA
<213> Homo sapiens

<400> 1475
auuccuagaa auuguucaua 20

<210> 1476
<211> 23
<212> RNA
<213> Homo sapiens

<400> 1476
cgaauguugc ucggugaacc ccu 23

<210> 1477
<211> 22
<212> RNA
<213> Homo sapiens

<400> 1477
agguuacccg agcaacuuug ca 22

<210> 1478
<211> 22
<212> RNA
<213> Homo sapiens

<400> 1478
aauauaacac agauggccug uu 22

<210> 1479
<211> 23
<212> RNA
<213> Homo sapiens

<400> 1479
acuucaccug guccacuagc cgu 23

<210> 1480
<211> 22
<212> RNA
<213> Homo sapiens

<400> 1480
cuggacuuag ggucagaagg cc 22

<210> 1481
<211> 22
<212> RNA
<213> Homo sapiens

<400> 1481
cuggacuugg agucagaagg cc 22

<210> 1482
<211> 22
<212> RNA
<213> Homo sapiens

<400> 1482
agcucggucu gaggccccuc ag 22

<210> 1483
<211> 22
<212> RNA
<213> Homo sapiens

<400> 1483
cagcagcaau ucauguuuug aa 22

<210> 1484
<211> 21

<212> RNA
<213> Homo sapiens

<400> 1484
aucgggaaug ucguguccgc c 21

<210> 1485
<211> 22
<212> RNA
<213> Homo sapiens

<400> 1485
uaauacuguc ugguaaaacc gu 22

<210> 1486
<211> 21
<212> RNA
<213> Homo sapiens

<400> 1486
ugucuugcag gccgucaugc a 21

<210> 1487
<211> 23
<212> RNA
<213> Homo sapiens

<400> 1487
ucuuggagua ggucauuggg ugg 23

<210> 1488
<211> 21
<212> RNA
<213> Homo sapiens

<400> 1488
cuggauggcu ccuccauguc u 21

<210> 1489
<211> 22
<212> RNA
<213> Homo sapiens

<400> 1489
aucaugaugg gcuccucggu gu 22

<210> 1490
<211> 22
<212> RNA
<213> Homo sapiens

<400> 1490
uugcauaugu aggauguccc au 22

<210> 1491
<211> 22
<212> RNA
<213> Homo sapiens

<400> 1491
uggcagugua uuguuagcug gu 22

<210> 1492
<211> 22
<212> RNA
<213> Homo sapiens

<400> 1492
uuuuugcgau guguuccuaa ua 22

<210> 1493
<211> 23
<212> RNA
<213> Homo sapiens

<400> 1493
aaaccguuac cauuacugag uuu 23

<210> 1494
<211> 22
<212> RNA
<213> Homo sapiens

<400> 1494
uguuugcaga ggaaacugag ac 22

<210> 1495
<211> 21
<212> RNA
<213> Homo sapiens

<400> 1495
ucagucucau cugcaaagaa g 21

<210> 1496
<211> 22
<212> RNA
<213> Homo sapiens

<400> 1496
gagguugucc guggugaguu cg 22

<210> 1497
<211> 20
<212> RNA
<213> Homo sapiens

<400> 1497
gucauacacg gcucuccucu 20

<210> 1498
<211> 22
<212> RNA
<213> Homo sapiens

<400> 1498
agaggcuggc cgugaugaau uc 22

<210> 1499
<211> 21
<212> RNA
<213> Homo sapiens

<400> 1499
cccagauaau ggcacucuca a 21

<210> 1500
<211> 23
<212> RNA
<213> Homo sapiens

<400> 1500
agugacauca cauauacggc age 23

<210> 1501
<211> 22
<212> RNA
<213> Homo sapiens

<400> 1501
caaccuggag gacuccaugc ug 22

<210> 1502
<211> 23
<212> RNA
<213> Homo sapiens

<400> 1502
aguggggaac ccuuccauga gga 23

<210> 1503
<211> 23
<212> RNA
<213> Homo sapiens

<400> 1503
aggaccugcg ggacaagauu cuu 23

<210> 1504
<211> 22
<212> RNA
<213> Homo sapiens

<400> 1504
uuguacaugg uaggcuuuca uu 22

<210> 1505
<211> 24
<212> RNA
<213> Homo sapiens

<400> 1505
ugaaacauac acgggaaacc ucuu 24

<210> 1506
<211> 23

<212> RNA
<213> Homo sapiens

<400> 1506
aaacaaacau ggugcacuuc uuu 23

<210> 1507
<211> 17
<212> RNA
<213> Homo sapiens

<400> 1507
auuacauggc caaucuc 17

<210> 1508
<211> 21
<212> RNA
<213> Homo sapiens

<400> 1508
cagcagcaca cugugguuug u 21

<210> 1509
<211> 23
<212> RNA
<213> Homo sapiens

<400> 1509
uuucaagcca gggggcguuu uuc 23

<210> 1510
<211> 23
<212> RNA
<213> Homo sapiens

<400> 1510
uuaagacuug cagugauguu uaa 23

<210> 1511
<211> 22
<212> RNA
<213> Homo sapiens

<400> 1511
augcaccugg gcaaggauuc ug 22

<210> 1512
<211> 22
<212> RNA
<213> Homo sapiens

<400> 1512
aauccuuugu cccuggguga ga 22

<210> 1513
<211> 21
<212> RNA
<213> Homo sapiens

<400> 1513
auccuugcua ucugggugcu a 21

<210> 1514
<211> 23
<212> RNA
<213> Homo sapiens

<400> 1514
uagcagcggg aacaguucug cag 23

<210> 1515
<211> 21
<212> RNA
<213> Homo sapiens

<400> 1515
agacccuggu cugcacucua u 21

<210> 1516
<211> 22
<212> RNA
<213> Homo sapiens

<400> 1516
gucaacacuu gcugguuucc uc 22

<210> 1517
<211> 21
<212> RNA
<213> Homo sapiens

<400> 1517
uaaggcaccc uucugaguag a 21

<210> 1518
<211> 21
<212> RNA
<213> Homo sapiens

<400> 1518
uuuugcaccu uuuggaguga a 21

<210> 1519
<211> 23
<212> RNA
<213> Homo sapiens

<400> 1519
ugauuguagc cuuuuggagu aga 23

<210> 1520
<211> 23
<212> RNA
<213> Homo sapiens

<400> 1520
ugauugguac gucugugggu aga 23

<210> 1521
<211> 23
<212> RNA
<213> Homo sapiens

<400> 1521
uacucaggag aguggcaauc aca 23

<210> 1522
<211> 21
<212> RNA
<213> Homo sapiens

<400> 1522
gugucuuuug cucugcaguc a 21

<210> 1523
<211> 22
<212> RNA
<213> Homo sapiens

<400> 1523
aagugcuguc auagcugagg uc 22

<210> 1524
<211> 23
<212> RNA
<213> Homo sapiens

<400> 1524
cacucagccu ugagggcacu uuc 23

<210> 1525
<211> 22
<212> RNA
<213> Homo sapiens

<400> 1525
uucacaggga ggugucauuu au 22

<210> 1526
<211> 20
<212> RNA
<213> Homo sapiens

<400> 1526
auugacacuu cugugaguag 20

<210> 1527
<211> 21
<212> RNA
<213> Homo sapiens

<400> 1527
gagugccuuc uuuuggagcg u 21

<210> 1528
<211> 24

<212> RNA
<213> Homo sapiens

<400> 1528
uucuccaaaa gaaagcacuu ucug 24

<210> 1529
<211> 18
<212> RNA
<213> Homo sapiens

<400> 1529
ugcuuccuuu cagagggu 18

<210> 1530
<211> 22
<212> RNA
<213> Homo sapiens

<400> 1530
aucuggaggu aagaagcacu uu 22

<210> 1531
<211> 22
<212> RNA
<213> Homo sapiens

<400> 1531
ccucuagaug gaagcacugu cu 22

<210> 1532
<211> 23
<212> RNA
<213> Homo sapiens

<400> 1532
aucgugcauc ccuuuagagu guu 23

<210> 1533
<211> 22
<212> RNA
<213> Homo sapiens

<400> 1533
ucgugcaucc cuuuagagug uu 22

<210> 1534
<211> 22
<212> RNA
<213> Homo sapiens

<400> 1534
aucgugcauc cuuuuagagu gu 22

<210> 1535
<211> 20
<212> RNA
<213> Homo sapiens

<400> 1535
ucugcaaagg gaagcccuuu 20

<210> 1536
<211> 23
<212> RNA
<213> Homo sapiens

<400> 1536
ucucuggagg gaagcacuuu cug 23

<210> 1537
<211> 23
<212> RNA
<213> Homo sapiens

<400> 1537
cucuagaggg aagcacuuuc ucu 23

<210> 1538
<211> 21
<212> RNA
<213> Homo sapiens

<400> 1538
aaagcgcuuc ccuuugcugg a 21

<210> 1539
<211> 22
<212> RNA
<213> Homo sapiens

<400> 1539
caaagcgcuc cccuuuagag gu 22

<210> 1540
<211> 22
<212> RNA
<213> Homo sapiens

<400> 1540
caaagcgcuu cucuuuagag ug 22

<210> 1541
<211> 21
<212> RNA
<213> Homo sapiens

<400> 1541
caaagcgcuu cccuuuggag c 21

<210> 1542
<211> 22
<212> RNA
<213> Homo sapiens

<400> 1542
aaagcgcuuc ccuucagagu gu 22

<210> 1543
<211> 21
<212> RNA
<213> Homo sapiens

<400> 1543
aaagcgcuuc ucuuuagagg a 21

<210> 1544
<211> 22
<212> RNA
<213> Homo sapiens

<400> 1544
uucuccaaaa gggagcacuu uc 22

<210> 1545
<211> 25
<212> RNA
<213> Homo sapiens

<400> 1545
aaagugcauc cuuuuagagu guuac 25

<210> 1546
<211> 23
<212> RNA
<213> Homo sapiens

<400> 1546
aaagugcauc cuuuuagagg uuu 23

<210> 1547
<211> 22
<212> RNA
<213> Homo sapiens

<400> 1547
aaagugcauc uuuuuagagg au 22

<210> 1548
<211> 23
<212> RNA
<213> Homo sapiens

<400> 1548
caaagugccu cccuuuagag ugu 23

<210> 1549
<211> 22
<212> RNA
<213> Homo sapiens

<400> 1549
aaagugccuc cuuuuagagu gu 22

<210> 1550
<211> 21

<212> RNA
<213> Homo sapiens

<400> 1550
cuccagaggg aaguacuuuc u 21

<210> 1551
<211> 23
<212> RNA
<213> Homo sapiens

<400> 1551
ucuacaaagg gaagcccuuu cug 23

<210> 1552
<211> 22
<212> RNA
<213> Homo sapiens

<400> 1552
aaagugcuuc ccuuuggacu gu 22

<210> 1553
<211> 21
<212> RNA
<213> Homo sapiens

<400> 1553
aaagugcuuc cuuuuagagg g 21

<210> 1554
<211> 23
<212> RNA
<213> Homo sapiens

<400> 1554
aaagugcuuc cuuuuagagg guu 23

<210> 1555
<211> 23
<212> RNA
<213> Homo sapiens

<400> 1555
aaagugcuuc ucuuuggugg guu 23

<210> 1556
<211> 21
<212> RNA
<213> Homo sapiens

<400> 1556
aaagugcuuc cuuuuugagg g 21

<210> 1557
<211> 22
<212> RNA
<213> Homo sapiens

<400> 1557
aagugcuucc uuuuagaggg uu 22

<210> 1558
<211> 24
<212> RNA
<213> Homo sapiens

<400> 1558
acaaagugcu ucccuuuaga gugu 24

<210> 1559
<211> 22
<212> RNA
<213> Homo sapiens

<400> 1559
acaaagugcu ucccuuuaga gu 22

<210> 1560
<211> 22
<212> RNA
<213> Homo sapiens

<400> 1560
aacgcacuuc ccuuuagagu gu 22

<210> 1561
<211> 23
<212> RNA
<213> Homo sapiens

<400> 1561
aaaaugguuc ccuuuagagu guu 23

<210> 1562
<211> 21
<212> RNA
<213> Homo sapiens

<400> 1562
aacgcgcuuc ccuauagagg g 21

<210> 1563
<211> 21
<212> RNA
<213> Homo sapiens

<400> 1563
gaaggcgcuu cccuuuggag u 21

<210> 1564
<211> 22
<212> RNA
<213> Homo sapiens

<400> 1564
cuacaaaggg aagcacuuuc uc 22

<210> 1565
<211> 21
<212> RNA
<213> Homo sapiens

<400> 1565
cuccagaggg augcacuuuc u 21

<210> 1566
<211> 21
<212> RNA
<213> Homo sapiens

<400> 1566
gaaggcgcuu cccuuuagag c 21

<210> 1567
<211> 21
<212> RNA
<213> Homo sapiens

<400> 1567
aaagugcuuc cuuuuagagg c 21

<210> 1568
<211> 21
<212> RNA
<213> Homo sapiens

<400> 1568
cucuagaggg aagcacuuuc u 21

<210> 1569
<211> 24
<212> RNA
<213> Homo sapiens

<400> 1569
cucuugaggg aagcacuuuc uguu 24

<210> 1570
<211> 24
<212> RNA
<213> Homo sapiens

<400> 1570
cucuagaggg aagcgcuuuc uguu 24

<210> 1571
<211> 21
<212> RNA
<213> Homo sapiens

<400> 1571
cugcaaaggg aagcccuuuc u 21

<210> 1572
<211> 22

<212> RNA
<213> Homo sapiens

<400> 1572
uggaagacua gugauuuugu ug 22

<210> 1573
<211> 23
<212> RNA
<213> Homo sapiens

<400> 1573
ucuuugguua ucuagcugua uga 23

<210> 1574
<211> 21
<212> RNA
<213> Homo sapiens

<400> 1574
uaaagcuaga uaaccgaaag u 21

<210> 1575
<211> 21
<212> RNA
<213> Homo sapiens

<400> 1575
uauugcacuu gucccggccu g 21

<210> 1576
<211> 22
<212> RNA
<213> Homo sapiens

<400> 1576
aaagugcugu ucgugcaggu ag 22

<210> 1577
<211> 22
<212> RNA
<213> Homo sapiens

<400> 1577
uucaacgggu auuuauugag ca 22

<210> 1578
<211> 22
<212> RNA
<213> Homo sapiens

<400> 1578
uuuggcacua gcacauuuuu gc 22

<211> 22
<212> RNA
<213> Homo sapiens

<400> 1579
ugagguagua aguuguauug uu 22

<210> 1580
<211> 22
<212> RNA
<213> Homo sapiens

<400> 1580
aacccguaga uccgaucuug ug 22

<210> 1581
<211> 22
<212> RNA
<213> Homo sapiens

<400> 1581
cacccguaga accgaccuug cg 22